# EUROPEAN PATENT SPECIFICATION

(11) **EP 2 170 403 B1**
(45) Date of publication and mention of the grant of the patent: **16.04.2014**
(21) Application number: 08763631.2
(22) Date of filing: 26.06.2008
(51) Int. Cl.: C12N 15/113, A61K 31/713

(54) **Compositions and methods for inhibiting expression of pro-apoptotic genes**
Zusammensetzungen und Verfahren zur Unterdrückung der Expression von pro-apoptotischen Genen
Compositions et procédés d'inhibition de l'expression de gènes pro-apoptotiques

(30) Priority: 27.06.2007 US 937318 P; 10.08.2007 US 964325 P
(43) Date of publication of application: 07.04.2010
(73) Proprietor: Quark Pharmaceuticals, Inc., Fremont, California 94555 (US)
(72) Inventor: KALINSKI, Hagar, 75700 Rishon-le-zion (IL); METT, Igor, 76248 Rehovot (IL); FEINSTEIN, Elena, 76214 Rehovot (IL); ERLICH, Shai, Dr, Belmont, CA 94002 (US)
(74) Representative: Almond-Martin, Carol
(86) International application number: PCT/IL2008/000874
(87) International publication number: WO 2009/001359

(56) References cited:
- WO-A2-00/24885
- WO-A2-2004/045543
- US-A1- 2005 153 337
- US-A1- 2006 069 056
- HOCHEGGER KATHRIN ET AL: "p21 and mTERT are novel markers for determining different ischemic time periods in renal ischemia-reperfusion injury.", AMERICAN JOURNAL OF PHYSIOLOGY. RENAL PHYSIOLOGY FEB 2007 LNKD- PUBMED:16968891, vol. 292, no. 2, February 2007 (2007-02), pages F762-F768, XP002632272, ISSN: 1931-857X
- DRAGUN DUSKA ET AL: "Inhibition of intercellular adhesion molecule-1 with antisense deoxynucleotides prolongs renal isograft survival in the rat", KIDNEY INTERNATIONAL, NATURE PUBLISHING GROUP, LONDON, GB, vol. 54, no. 6, 1 December 1998 (1998-12-01), pages 2113-2122, XP008135344, ISSN: 0085-2538, DOI: DOI:10.1046/J.1523-1755.1998.00189.X
- KELLY K J ET AL: "P53 mediates the apoptotic response to GTP depletion after renal ischemia-reperfusion: Protective role of a p53 inhibitor", JOURNAL OF THE AMERICAN SOCIETY OF NEPHROLOGY 20030101 US LNKD- DOI:10.1097/01.ASN.0000040596.23073.01, vol. 14, no. 1, 1 January 2003 (2003-01-01), pages 128-138, XP002632231, ISSN: 1046-6673
- HEALY D A ET AL: "Heat shock-induced protection of renal proximal tubular epithelial cells from cold storage and rewarming injury", JOURNAL OF THE AMERICAN SOCIETY OF NEPHROLOGY 200603 US LNKD- DOI:10.1681/ASN.2005090980, vol. 17, no. 3, March 2006 (2006-03), pages 805-812, XP002632234, ISSN: 1046-6673
- PERICO NORBERTO ET AL: "Delayed graft function in kidney transplantation", LANCET (NORTH AMERICAN EDITION), vol. 364, no. 9447, 13 November 2004 (2004-11-13), pages 1814-1827, XP002632232, ISSN: 0099-5355

## Description

This application claims the benefit of US provisional application No. 60/937318 filed June 27, 2007 and US provisional application No. 60/964325 filed August 10, 2007.

Throughout this application various patent and scientific publications are cited. The disclosures for these publications in their entireties are hereby mentioned to more fully describe the state of the art to which this invention pertains.

### FIELD OF THE INVENTION

The present description relates to compounds, pharmaceutical compositions comprising same and methods of use thereof for the inhibition of certain pro-apoptotic genes, useful in the treatment of diseases and disorders in which gene expression is adverse. In particular embodiments, the invention provides siRNA oligonucleotides, compositions comprising same for use in the treatment of various diseases.

### BACKGROUND OF THE INVENTION

### RNA interference

RNA interference (RNAi) is a phenomenon involving double-stranded (ds) RNA-dependent gene-specific posttranscriptional gene silencing. Originally, attempts to study this phenomenon and to manipulate mammalian cells experimentally were hindered by a non-specific antiviral defense mechanism activated in response to long dsRNA molecules (see Gil et al. Apoptosis 2000, 5:107-114). Later it was discovered that short, synthetic RNA duplexes of 21 nucleotides could mediate gene specific RNAi in mammalian cells, precluding stimulation of the generic antiviral defense mechanisms (see Elbashir et al. Nature 2001, 411:494-498; Caplen et al. PNAS USA 2001, 98:9742-9747). As a result, small interfering RNAs (siRNAs) have become powerful tools in attempting to understand gene function.

RNA interference (RNAi) in mammals is mediated by small interfering RNAs (siRNAs) (Fire et al, Nature 1998, 391:806) or microRNAs (miRNAs) (Ambros, Nature 2004, 431(7006):350-355; Bartel, Cell 2004, 116(2): 281-97). The corresponding process in plants is commonly referred to as specific post-transcriptional gene silencing (PTGS) or RNA silencing and is also referred to as quelling in fungi.

An siRNA is a double-stranded RNA or modified RNA molecule which down-regulates or silences (prevents) the expression of a gene/ mRNA of its endogenous (cellular) counterpart. The mechanism of RNA interference is detailed infra.

Several studies have revealed that siRNA therapeutics is effective in vivo in both mammals and in humans. Bitko et al., have shown that specific siRNA molecules directed against the respiratory syncytial virus (RSV) nucleocapsid N gene are effective in treating mice when administered intranasally (Nat. Med. 2005, 11(1):50-55). Recent reviews discussing siRNA therapeutics are available (Barik, et al., J. Mol. Med 2005, 83:764-773; Dallas and Vlassov, Med. Sci. Monitor 2006, 12(4):RA67-74; Chakraborty Current Drug Targets 2007,8(3);469-82).

Mucke (IDrugs 2007 10(1):37-41) presents a review of current therapeutics, including siRNA to various targets, for the treatment of ocular diseases, for example age related macular degeneration (AMD) and glaucoma.

### Pro- apoptotic genes

Pro-apoptotic genes are genes that encode proteins that play a role in apoptotic cell death. A non-limiting list of pro-apoptotic genes are: tumor protein p53 (P53 or TP53 which terms are used herein interchangeably); HtrA serine peptidase 2 (HTRA2); Kelch-like ECH associated protein 1 (KEAP1); Src homology 2 domain containing transforming protein 1 (SHC1-SHC, p66); zinc finger HIT type (ZNHIT1); lectin galactose-binding soluble 3 (LGALS3); and sestrin2 (HI95, SESN2).

Inhibition of one or more of the above genes is useful in the treatment and/or prevention of at least one of the following diseases or disorders and of other diseases disclosed herein: hearing loss, in particular chemical-induced ototoxicity, acute renal failure (ARF), chronic obstructive pulmonary disease (COPD), ischemia reperfusion injury following lung transplantation, lung cancer, acute respiratory disease syndrome (ARDS), spinal cord injury, pressure sores, osteoarthritis, diabetic retinopathy, oral mucositis, dry eye syndrome, ocular ischemic conditions, injury associated with organ transplant and Delayed Graft Function (DGF) in a recipient of kidney transplant. The function of HI95 has been disclosed in Budanov et al., 2002, 21(39):6017-31.

### Acute renal failure

Acute renal failure (ARF) is a clinical syndrome characterized by rapid deterioration of renal function that occurs within days. The principal feature of ARF is an abrupt decline in glomerular filtration rate (GFR), resulting in the retention of nitrogenous wastes (urea, creatinine). Worldwide, severe ARF occurs in about 170-200 per million population annually. To date, there is no specific treatment for established ARF. Several drugs have been found to ameliorate toxic and ischemic experimental ARF, as manifested by lower serum creatinine levels, reduced histological damage and faster recovery of renal function in different animal models. These include anti-oxidants, calcium channel blockers, diuretics, vasoactive substances, growth factors, anti-inflammatory agents and more. However, the drugs tested in clinical trials showed no benefit, and their use in clinical ARF has not been approved.

In the majority of hospitalized ARF patients, ARF is caused by acute tubular necrosis (ATN), which results from ischemic and/or nephrotoxic insults. Renal hypoperfusion is caused by hypovolemic, cardiogenic and septic shock, by administration of vasoconstrictive drugs, renovascular injury or kidney transplant. Nephrotoxins include exogenous toxins such as contrast media, aminoglycosides and cisplatin and cisplatin-like compounds as well as endogenous toxin such as myoglobin. Any chemical, biological or other agent which causes ARF or other kidney disease or disorder may be considered a nephrotoxin. Recent studies, however, support the theory that apoptosis in renal tissues is prominent in most human cases of ARF. The principal site of apoptotic cell death is the distal nephron. During the initial phase of ischemic injury, loss of integrity of the actin cytoskeleton leads to flattening of the epithelium, with loss of the brush border, loss of focal cell contacts, and subsequent disengagement of the cell from the underlying substratum. It has been suggested that apoptotic tubule cell death may be more predictive of functional changes than necrotic cell death (Komarov et al., Science 1999, 10;285(5434):1733-7); Supavekin et al., Kidney Int. 2003, 63(5):1714-24).

US 2006/0069056 discloses the use of siRNA targeting p53 for the treatement of ischemic acute renal failure.

In conclusion, currently there are no satisfactory modes of therapy for the prevention and/or treatment of acute renal failure, and there is a need therefore to develop novel compounds for this purpose.

An effective therapy to treat the above mentioned diseases and disorders would be of great therapeutic value.

### SUMMARY OF THE INVENTION

The present invention is directed to a double-stranded siRNA compound which reduces or inhibits expression of the tumor protein p53 (P53 or TP53) gene, for use in preventing or reducing Delayed Graft Function in a recipient of a kidney transplant, said siRNA compound having the structure:
5' (N)ₓ - Z 3' (antisense strand)
3' Z'-(N')_{y} 5' (sense strand)
wherein each of N and N' is a ribonucleotide which may be modified or unmodified in its sugar residue;
wherein each of (N)ₓ and (N')_{y} is an oligonucleotide in which each consecutive N or N' is joined to the next N or N' by a covalent bond;
wherein each of x and y is an integer between 18 and 40;
wherein in each of (N)ₓ and (N')_{y} the ribonucleotides alternate between modified ribonucleotides and unmodified ribonucleotides, each modified ribonucleotide being a 2'-O-methyl sugar modified ribonucleotide;
wherein each of Z and Z' may be present or absent, but if present is 1-5 deoxyribonucleotides covalently attached at the 3' terminus of the oligonucleotide in which it is present;
wherein the sequence of (N)ₓ comprises the sequence 5' UGAAGGGUGAAAUAUUCUC 3' (SEQ ID NO:2) and the sequence of (N')_{y} comprises the sequence 5' GAGAAUAUUUCACCCUUCA 3' (SEQ ID NO:1); and wherein a therapeutically effective dose of said siRNA compound is to be administered intravenously to the recipient of the kidney transplant between 15 minutes and 4 hours following revascularization of the transplanted kidney, thereby preventing or reducing Delayed Graft Function in the recipient.

The present description discloses inhibitors of a pro-apoptotic gene selected from the group consisting of TP53; HTRA2; KEAP1; SHC1-SHC, ZNHIT1, LGALS3, and HI95. In particular the present description discloses novel double stranded oligonucleotides that inhibit or reduce expression of a pro-apoptotic gene selected from the group consisting of TP53; HTRA2; KEAP1; SHC1-SHC, ZNHIT1, LGALS3, and HI95, and pharmaceutical compositions comprising one or more such oligonucleotides or a vector capable of expressing the oligoribonucleotide. The present description, further relates to methods for treating or preventing the incidence or severity of various diseases or conditions in which gene expression is associated with the etiology or progression of the disease or condition.

In one aspect the present invention provides a compound having the structure:
5' (N)ₓ-Z 3' (antisense strand)
3' Z'-(N')_{y} 5' (sense strand)
wherein each ofN and N' is a nucleotide which may be modified or unmodified in its sugar residue; wherein each of (N)ₓ and (N')_{y} is an oligonucleotide in which each consecutive N or N' is joined to the next N or N' by a covalent bond;
wherein each of x and y is an integer between 18 and 40;
wherein each of Z and Z' may be present or absent, but if present is 1-5 nucleotides covalently attached at the 3' terminus of the strand in which it is present; and
wherein the sequence of (N)ₓ comprises an antisense sequence relative to the mRNA transcribed from the mammalian gene TP53,, wherein the sequence of (N)ₓ comprises the sequence 5' UGAAGGGUGAAAUAUUCUC 3' (SEQ ID NO:2) and the sequence of (N')_{y} comprises the sequence 5' GAGAAUAUUUCACCCUUCA 3'(SEQ ID NO:1), for use in preventing or reducing Delayed Graft Function in a recipient of a kidney transplant, wherein a therapeutically effective dose of said siRNA compound is to be administered intravenously to the recipient of the kidney transplant between 15 minutes and 4 hours following revascularization of the transplanted kidney, thereby preventing or reducing Delayed Graft Function in the recipient.

In some embodiments the compound comprises a phosphodiester bond. In various embodiments the compound comprises ribonucleotides wherein x = y and wherein x is an integer selected from the group consisting of 19, 20 and 21. In preferred embodiments x = y =19.

In some embodiments the compound is blunt ended, for example wherein Z and Z' are both absent. In an alternative embodiment, the compound comprises at least one 3' overhang, wherein at least one of Z or Z' is present. Z and Z' can be independently comprise one or more covalently linked modified or non-modified nucleotides, as described infra, for example inverted dT or dA; dT, LNA, mirror nucleotide and the like. In some embodiments each of Z and Z' are independently selected from dT and dTdT.

The compound comprises a modification at the 2' position of the sugar residue, the modification being a ribonucleotide comprising a methoxy moiety at the 2' position (2'-O-methyl; 2'-O-Me; 2'-O-CH₃) of the sugar residue.

The compound comprises modified alternating ribonucleotides in the antisense and the sense strands. In some preferred embodiments the middle ribonucleotide of the antisense strand is not modified; e.g. ribonucleotide in position 10 in a 19-mer strand.

In additional embodiments the compound comprises modified ribonucleotides in alternating positions wherein the ribonucleotides at the 5' and 3' termini of the antisense strand are modified in their sugar residues, and the ribonucleotides at the 5' and 3' termini of the sense strand are unmodified in their sugar residues. In some embodiments, neither the antisense nor the sense strands are phosphorylated at the 3' and 5' termini. In other embodiments either or both the antisense and the sense strands are phosphorylated at the 3' termini.

The compound comprises the antisense sequence present in Tables A #1. In other embodiments the present disclosure provides a mammalian expression vector comprising the antisense sequence present in Tables A #1.

In certain preferred embodiments the present invention provides a compound having the structure
5' (N)x 3' antisense strand
3' (N')y5' sense strand
wherein x and y =19; and (N)x and (N)y are fully complementary;
wherein alternating ribonucleotides in the antisense and the sense strands are modified to result in a 2'-O-methyl modification in the sugar residue of the ribonucleotides;
wherein the ribonucleotides at the 5' and 3' termini of the antisense strand are modified;
wherein the ribonucleotides at the 5' and 3' termini of the sense strand are unmodified;
wherein the antisense and the sense strands are phosphorylated or non-phosphorylated at the 3' and 5' termini; and
wherein each of N and N' is selected from the group of oligomers set forth in Table A #1; for use in preventing or reducing Delayed Graft Function as detailed above.

In a second aspect the present invention provides a pharmaceutical composition comprising one or more compounds for use of the present invention, in an amount effective to inhibit human gene expression wherein the gene is TP53; and a pharmaceutically acceptable carrier.

More specifically, the present description discloses methods and compositions useful in treating a subject suffering from or at risk of acute renal failure (ARF), hearing loss including chemical-induced oxotoxicity, glaucoma, diabetic retinopathy, ischemic optic neuropathy (ION), dry eye syndrome, acute respiratory distress syndrome (ARDS) and other acute lung and respiratory injuries, injury (e.g. ischemia-reperfusion injury) in organ transplant including lung, kidney, bone marrow, heart, pancreas, cornea or liver transplantation, nephrotoxicity, nephritis, neurotoxicity, spinal cord injury, osteoarthritis (OA), oral mucositis, pressure sores, and chronic obstructive pulmonary disease (COPD).

The disclosed methods comprise administering to a subject in need thereof one or more inhibitory compounds which down-regulate expression of the pro-apoptotic gene TP53; in a therapeutically effective dose so as to thereby treat the subject.

The inhibitor is siRNA.

The present description further discloses the use of compounds which down-regulate the expression of a proapoptotic gene, particularly to small interfering RNAs (siRNAs), in the treatment of various diseases, conditions or disorders associated with TP53 gene expression including acute renal failure (ARF), hearing loss including chemical-induced oxotoxicity, glaucoma, diabetic retinopathy, ischemic optic neuropathy, dry eye syndrome, acute respiratory distress syndrome (ARDS) and other acute lung and respiratory injuries, injury (e.g. ischemia-reperfusion injury) in organ transplant including lung, kidney, bone marrow, heart, pancreas, cornea or liver transplantation, nephrotoxicity, nephritis, neurotoxicity, spinal cord injury, osteoarthritis (OA), oral mucositis, pressure sores, and chronic obstructive pulmonary disease (COPD).

The invention more specifically provides, for use in preventing or reducing the symptoms of Delayed Graft Function in a recipient of a kidney transplant, for administration to the recipient of the kidney transplant, a composition comprising a therapeutically effective dose of a compound having the structure:
5' (N)ₓ - Z3' (antisense strand)
3' Z'-(N')_{y} 5' (sense strand)
wherein each of N and N' is a nucleotide selected from an unmodified ribonucleotide, and a modified ribonucleotide;
wherein each of (N)x and (N')y is an oligomer in which each consecutive ribonucleotide is joined to the next ribonucleotide by a covalent bond and each of x and y is an integer between 18 and 40; wherein in each of (N)x and (N')y the nucleotides alternate between modified ribonucleotides and unmodified ribonucleotides each modified ribonucleotide being modified so as to have a 2'-O-methyl on its sugar;
wherein each of Z and Z' may be present or absent, but if present is 1-5 deoxyribonucleotides covalently attached at the 3' terminus of the oligomer to which it is attached;
and wherein the antisense strand of the compound comprises the sequence 5' UGAAGGGUGAAAUAUUCUC 3' and the sense strand of the compound comprises the sequence 5' GAGAAUAUUUCACCCUUCA 3', thereby preventing or reducing the symptoms of Delayed Graft Function in the recipient.

Another aspect of the disclosure is a method of preventing or reducing the symptoms of Delayed Graft Function in a recipient of a kidney transplant, comprising administering to one or both of the donor and the recipient of the kidney transplant an siRNA, which reduces or inhibits expression of the TP53 gene, thereby preventing or reducing the symptoms of Delayed Graft Function in the recipient.

Lists of 19- and 21-mer sense and corresponding antisense sequences useful in preparation of siRNA compounds are set forth in Tables A-N, for illustrative purposes.

A list of siRNA to TP53 is provided in tables A-B, infra, for illustrative purposes.

A list of siRNA to HTRA2 is provided in tables C-D, infra, for illustrative purposes.

A list of siRNA to KEAP 1 is provided in tables E-F, infra, for illustrative purposes.

A list of siRNA to SHC1-SHC is provided in tables G-H, infra for illustrative purposes.

A list of siRNA to ZNHIT1 is provided in tables I-J, infra, for illustrative purposes.

A list of siRNA to LGAL3 is provided in tables K-L, infra, for illustrative purposes.

A list of siRNA to HI95 is provided in tables M-N, infra, for illustrative purposes. Additional siRNA compounds are set forth in Table P.

Known compounds, compositions and methods are explicitly excluded from the present invention. These and further features of the present invention will be better understood in conjunction with the detailed description, examples and claims that follow.

### DETAILED DESCRIPTION OF THE INVENTION

The present description relates generally to compounds which down-regulate expression of TP53; HTRA2; KEAP1; SHC1-SHC, ZNHIT1, LGALS3, and HI95 genes, particularly to novel small interfering RNAs (siRNAs), and to the use of these siRNAs in the treatment of various diseases and medical conditions. Particular diseases and conditions to be treated are acute renal failure (ARF), Delayed Graft Function (DGF) in a recipient of kidney transplant, hearing loss including chemical-induced oxotoxicity, glaucoma, diabetic retinopathy, ischemic optic neuropathy, dry eye syndrome, acute respiratory distress syndrome (ARDS) and other acute lung and respiratory injuries, injury (e.g. ischemia-reperfusion injury) in organ transplant including lung, kidney, bone marrow, heart, pancreas, cornea or liver transplantation, nephrotoxicity, nephritis, neurotoxicity, spinal cord injury, osteoarthritis (OA), oral mucositis, pressure sores, and chronic obstructive pulmonary disease (COPD).

Lists of siRNA are provided in Tables A-N infra. The separate lists of 19-mer and 21-mer siRNAs are prioritized based on their score according to a proprietary algorithm as the best sequences for targeting the human gene expression. Methods, molecules and compositions, which inhibit the pro-apoptotic genes are discussed herein at length, and any of said molecules and/or compositions may be beneficially employed in the treatment of a subject suffering from or at risk of developing any of said conditions. Tables A, C, E, G, I, K, M set forth 19-mer oligomers. Tables B, D, F, H, J, L, N set forth 21-mer oligomers. The oligomers are useful in the synthesis of siRNA compounds and pharmaceutical compositions comprising same.

### Definitions

For convenience certain terms employed in the specification, examples and claims are described herein.

An "inhibitor" is a compound which is capable of inhibiting or reducing the expression or activity of a gene or the product of such gene to an extent sufficient to achieve a desired biological or physiological effect. The term "inhibitor" as used herein refers to one or more of an oligonucleotide inhibitor, including siRNA, shRNA, aptamers, antisense molecules, miRNA and ribozymes, as well as antibodies. Table 1 below provides a list of the proapoptotic genes of the present description, the gene identification (gi) numbers, as well as Genbank identifiers, known isoforms, and indications.

**Table 1:**

| Gene | Full name and Human Gene ID | Indications or diseases to be treated * |
|---|---|---|
| p53 (TP53) | tumor protein p53 | organ transplant (e.g. lung, kidney), glaucoma, hearing loss, acute kidney injury, acute lung injury, ischemic optic neuropathy (ION) |
| | gi8400737, NM_000546.2 | |
| HTRA2 | Htra serine peptidase 2 | acute renal failure, hearing loss, ARDS, glaucoma, spinal cord injury, COPD, osteoarthritis, diabetic retinopathy, toxicity, organ transplant, acute ischemia-reperfusion lung injury |
| | | |
| | var 1 gi:73747817, NM_013247 | |
| | var 2 gi:73747818, NM_145074 | |
| KEAP 1 | Kelch-like ECH-associated protein 1 | acute renal failure, hearing loss, ARDS, glaucoma, spinal cord injury, COPD, osteoarthritis, diabetic retinopathy, pressure sores, nephrotoxicity, neurotoxicity, organ transplant |
| | var 1 gi:45269144 NM_203500 | |
| | var 2 gi:45269143 NM_012289 | |
| SHC1 | Src homology 2 domain containing) transforming prot. 1 | acute renal failure, hearing loss, ARDS, glaucoma, spinal cord injury, COPD, osteoarthritis , diabetic retinopathy, pressure sores, toxicity, organ transplant, acute ischemia-reperfusion lung injury |
| | var 1 gi:52693920 NM_183001 | |
| | var 2 gi:34147725 NM_003029 | |
| ZNHIT1 | Zn finger HIT type 1 | acute renal failure, hearing loss, ARDS, glaucoma, spinal cord injury, COPD, osteoarthritis, diabetic retinopathy, pressure sores, toxicity, organ transplant |
| | | |
| | gi:37594439\|; NM_006349 | |
| LGALS3 | lectin galactoside-binding soluble 3 | acute renal failure, hearing loss, ARDS, glaucoma, spinal cord injury, COPD, osteoarthritis , diabetic retinopathy, pressure sores, organ transplant |
| | var 1 gi:115430222 NM_002306 | |
| | var 2 gi:115430224 NR_003225 | |
| HI95 | Sestrin2 | acute renal failure, hearing loss, ARDS, glaucoma, spinal cord injury, COPD, osteoarthritis , diabetic retinopathy, pressure sores, organ transplant |
| | gi:32454742 NM_031459 | |

| | | |
|---|---|---|
| *Toxicity includes chemically induced (in a non-limiting example cisplatin and cisplatin analogs and aminoglycoside antibiotic ototoxicity, neurotoxicity and nephrotoxicity. Organ transplant includes *inter alia* lung, heart, kidney, liver and bone marrow. ION: ischemic ocular neuropathy; COPD: chronic obstructive pulmonary disorder; ARDS: acute respiratory distress syndrome; | | |

As used herein, the term "polypeptide" refers to, in addition to a polypeptide, an oligopeptide, peptide and a full protein. The present invention provides compounds that inhibit one or more isoforms of a gene, in the event that more than one isoforms exits.

### RNA interference and siRNA

RNA interference (RNAi) is based on the ability of dsRNA species to enter a cytoplasmic protein complex, where it is then targeted to the complementary cellular RNA and specifically degrade it. The RNA interference response features an endonuclease complex containing an siRNA, commonly referred to as an RNA-induced silencing complex (RISC), which mediates cleavage of single-stranded RNA having a sequence complementary to the antisense strand of the siRNA duplex. Cleavage of the target RNA may take place in the middle of the region complementary to the antisense strand of the siRNA duplex (Elbashir et al., Genes Dev., 2001, 15(2):188-200). In more detail, longer dsRNAs are digested into short (17-29 bp) dsRNA fragments (also referred to as short inhibitory RNAs, "siRNAs") by type III RNAses (DICER, DROSHA, etc.; Bernstein et al., Nature, 2001, 409(6818):363-6; Lee et al., Nature, 2003, 425(6956):415-9). The RISC protein complex recognizes these fragments and complementary mRNA. The whole process is culminated by endonuclease cleavage of target mRNA (McManus & Sharp, Nature Rev Genet, 2002, 3(10):737-47; Paddison & Hannon, Curr Opin Mol Ther. 2003, 5(3):217-24). (For additional information on these terms and proposed mechanisms, see for example Bernstein et al., RNA 2001, 7(11):1509-21; Nishikura, Cell 2001, 107(4):415-8 and PCT publication WO 01/36646).

The selection and synthesis of siRNA corresponding to known genes has been widely reported; see for example Ui-Tei et al., J Biomed Biotechnol. 2006; 2006: 65052; Chalk et al., Biochem. Biophys. Res. Comm. 2004, 319(1): 264-74; Sioud & Leirdal, Met. Mol Biol.; 2004, 252:457-69; Levenkova et al., Bioinform. 2004, 20(3):430-2; Ui-Tei et al., Nuc. Acid Res. 2004, 32(3):936-48. For examples of the use of, and production of, modified siRNA see Braasch et al., Biochem., 2003, 42(26):7967-75; Chiu et al., RNA, 2003, 9(9):1034-48; PCT publications WO 2004/015107 (Atugen); WO 02/44321 (Tuschl et al), and US Patent Nos. 5,898,031 and 6,107,094. See also Dykxhoom et al Gene Therapy (2006),13,541-552.

Several groups have described the development of DNA-based vectors capable of generating siRNA within cells. The method generally involves transcription of short hairpin RNAs that are efficiently processed to form siRNAs within cells (Paddison et al. PNAS USA 2002, 99:1443-1448; Paddison et al. Genes & Dev 2002, 16:948-958; Sui et al. PNAS USA 2002, 8:5515-5520; and Brummelkamp et al. Science 2002, 296:550-553). These reports describe methods to generate siRNAs capable of specifically targeting numerous endogenously and exogenously expressed genes.

As used herein, the term "nucleic acid" refers to polynucleotides such as deoxyribonucleic acid (DNA), and, where appropriate, ribonucleic acid (RNA). The terms should also be understood to include, as equivalents, analogs of either RNA or DNA made from nucleotide analogs, and, as applicable to the embodiment being described, double-stranded polynucleotides and single-stranded polynucleotides such as sense or antisense.

"Oligonucleotide" refers to a sequence having from about 2 to about 50 linked nucleotides or linked modified nucleotides, or a combination of modified and unmodified nucleotide. Oligonucleotide includes the terms oligomer, antisense strand and sense strand.

"Nucleotide" is meant to encompass deoxyribonucleotides and ribonucleotides, which may be natural or synthetic, and or modified or unmodified. Modifications include changes to the sugar moiety, the base moiety and or the linkages between ribonucleotides in the oligoribonucleotide.

All analogues of, or modifications to, a nucleotide / oligonucleotide may be employed with the present invention, provided that said analogue or modification does not substantially affect the function of the nucleotide / oligonucleotide. The nucleotides can be selected from naturally occurring or synthetic modified bases. Naturally occurring bases include adenine, guanine, cytosine, thymine and uracil. Modified bases of nucleotides include inosine, xanthine, hypoxanthine, 2-aminoadenine, 6-methyl, 2-propyl and other alkyl adenines, 5-halo uracil, 5-halo cytosine, 6-aza cytosine and 6-aza thymine, pseudo uracil, 4- thiouracil, 8-halo adenine, 8-aminoadenine, 8-thiol adenine, 8-thiolalkyl adenines, 8-hydroxyl adenine and other 8-substituted adenines, 8-halo guanines, 8-amino guanine, 8-thiol guanine, 8-thioalkyl guanines, 8- hydroxyl guanine and other substituted guanines, other aza and deaza adenines, other aza and deaza guanines, 5-trifluoromethyl uracil and 5- trifluoro cytosine.

In addition, analogues of polynucleotides can be prepared wherein the structure of one or more nucleotide is fundamentally altered and better suited as therapeutic or experimental reagents. An example of a nucleotide analogue is a peptide nucleic acid (PNA) wherein the deoxyribose (or ribose) phosphate backbone in DNA (or RNA) is replaced with a polyamide backbone which is similar to that found in peptides. PNA analogues have been shown to be resistant to enzymatic degradation and to have extended lives *in vivo* and *in vitro.* Other modifications that can be made to oligonucleotides include polymer backbones, cyclic backbones, acyclic backbones, thiophosphate-D-ribose backbones, triester backbones, thioate backbones, 2'-5' bridged backbone, artificial nucleic acids, morpholino nucleic acids, locked nucleic acid (LNA), glycol nucleic acid (GNA), threose nucleic acid (TNA), arabinoside, and mirror nucleoside (for example, beta-L-deoxynucleoside instead of beta-D-deoxynucleoside). Examples of siRNA compounds comprising LNA nucleotides are disclosed in Elmen et al., (NAR 2005. 33(1):439-447).

The compounds of the present invention can be synthesized using one or more inverted nucleotides, for example inverted thymidine or inverted adenine (for example see Takei, et al., 2002. JBC 277(26):23800-06.

Certain structures include an siRNA compound having one or a plurality of 2'-5' internucleotide linkages (bridges).

A "mirror" nucleotide is a nucleotide with reversed chirality to the naturally occurring or commonly employed nucleotide, i.e., a mirror image (L-nucleotide) of the naturally occurring (D-nucleotide). The nucleotide can be a ribonucleotide or a deoxyribonucleotide and my further comprise at least one sugar, base and or backbone modification. US Patent No. 6,602,858 discloses nucleic acid catalysts comprising at least one L-nucleotide substitution.

The present description discloses methods and compositions for inhibiting expression of a pro-apoptotic gene in vivo. In general, the method includes administering oligoribonucleotides, in particular small interfering RNAs (i.e., siRNAs) or a nucleic acid material that can produce siRNA in a cell, that targets an mRNA transcribed from a pro-apoptotic gene in an amount sufficient to down-regulate expression of a target gene by an RNA interference mechanism. In particular, the disclosed method can be used to inhibit expression of pro-apoptotic gene for treatment of a disease.

In accordance with the present disclosure, the siRNA compounds or other inhibitors of the pro-apoptotic genes are used as drugs to treat various pathologies.

The present invention provides double-stranded oligoribonucleotides (eg. siRNAs), which down-regulate the expression of the pro-apoptotic genes TP53 for use as detailed above.

There are at least four variant TP53 polypeptides (Bourdon et al. Genes Dev. 2005; 19: 2122-2137). All variants are included in the definition of TP53 polypeptides and in the definition of the TP53 genes encoding them.

An siRNA for use of the invention is a duplex oligoribonucleotide in which the sense strand is derived from the mRNA sequence of the pro-apoptotic genes, and the antisense strand is complementary to the sense strand. In general, some deviation from the target mRNA sequence is tolerated without compromising the siRNA activity (see e.g. Czauderna et al., NAR 2003, 31(11):2705-2716). An siRNA for use of the invention inhibits gene expression on a post-transcriptional level with or without destroying the mRNA. Without being bound by theory, siRNA may target the mRNA for specific cleavage and degradation and/ or may inhibit translation from the targeted message.

The oligoribonucleotide according to the present invention comprises modified siRNA. The siRNA comprises an RNA duplex comprising a first strand and a second strand, whereby the first strand comprises a ribonucleotide sequence at least partially complementary to about 18 to about 40 consecutive nucleotides of a target nucleic acid, and the second strand comprises ribonucleotide sequence at least partially complementary to the first strand and wherein said first strand and/or said second strand comprises a plurality of groups of modified ribonucleotides having a modification at the 2'-position of the sugar moiety whereby within each strand each group of modified ribonucleotides is flanked on one or both sides by a group of flanking ribonucleotides whereby each ribonucleotide forming the group of flanking ribonucleotides is an unmodified ribonucleotide.

The group of modified ribonucleotides and the group of flanking ribonucleotides comprise a one oligoribonucleotide.

The first and second strands comprise a pattern of modified nucleotides. In various embodiments the pattern of modified nucleotides of said first strand is identical relative to the pattern of modified nucleotides of the second strand.

In other embodiments the pattern of modified nucleotides of said first strand is shifted by one or more nucleotides relative to the pattern of modified nucleotides of the second strand.

In some preferred embodiments the middle ribonucleotide in the first strand (antisense) is an unmodified nucleotide. For example, in a 19-oligomer antisense strand, ribonucleotide number 10 is unmodified; in a 21-oligomer antisense strand, ribonucleotide number 11 is unmodified; and in a 23-oligomer antisense strand, ribonucleotide number 12 is unmodified. The modifications or pattern of modification, if any, of the siRNA must be planned to allow for this.

The modifications on the 2' moiety of the sugar residue are methoxy modifications.

In some embodiments the siRNA is blunt ended, on one or both ends. More specifically, the siRNA may be blunt ended on the end defined by the 5'- terminus of the first strand and the 3'-terminus of the second strand, or the end defined by the 3'-terminus of the first strand and the 5'-terminus of the second strand, or both ends.

In other embodiments at least one of the two strands may have an overhang of at least one nucleotide at the 5'-terminus; the overhang may consist of at least one deoxyribonucleotide. At least one of the strands may also optionally have an overhang of at least one nucleotide at the 3'-terminus. The overhang may consist of from about 1 to about 4 nucleotides.

The length of RNA duplex is from about 18 to about 40 ribonucleotides, preferably 19 to 23 ribonucleotides. Further, the length of each strand (oligomer) may independently have a length selected from 19, 20 or 21 ribonucleotides.

Additionally, in certain preferred embodiments the complementarity between said first strand and the target nucleic acid can be perfect. In some embodiments, the strands are substantially complementary, i.e. having one, two or up to three mismatches between said first strand and the target nucleic acid.

The first strand and the second strand each comprise groups of modified ribonucleotides and groups of flanking ribonucleotides, whereby each group of modified ribonucleotides consists of a single ribonucleotide and whereby each group of flanking ribonucleotides consists of a single ribonucleotide, wherein each group of modified ribonucleotides of the first strand is aligned with a group of flanking ribonucleotides on the second strand, and wherein the 5' most terminal ribonucleotide is selected from a group of modified ribonucleotides, and the 3' most terminal ribonucleotide of the second strand is a selected from the group of flanking ribonucleotide.

In yet other embodiments the ribonucleotide forming the group of flanking ribonucleotides on the first strand is an unmodified ribonucleotide arranged in a 3' direction relative to the ribonucleotide forming the group of modified ribonucleotides, and the ribonucleotide forming the group of modified ribonucleotides on the second strand is a modified ribonucleotide which is arranged in 5' direction relative to the ribonucleotide forming the group of flanking ribonucleotides. In some embodiments the first strand of the siRNA comprises five to about twenty, eight to twelve, preferably ten or twelve groups of modified ribonucleotides, and the second strand comprises seven to eleven, preferably nine or eleven groups of modified ribonucleotides. The first strand and the second strand may be linked by a loop structure, which may be comprised of a non-nucleic acid polymer such as, inter alia, polyethylene glycol. Alternatively, the loop structure may be comprised of a nucleic acid, including modified and non-modified ribonucleotides and modified and non-modified deoxyribonucleotides.

Further, the 5'-terminus of the first strand of the siRNA may be linked to the 3'-terminus of the second strand, or the 3'-terminus of the first strand may be linked to the 5'-terminus of the second strand, said linkage being via a nucleic acid linker typically having a length between 3-100 nucleotides, preferably about 3 to about 10 nucleotides.

In various embodiments, the present invention provides a compound having structure A:
5' (N)ₓ - Z 3' (antisense strand)
3' Z'-(N')_{y} 5' (sense strand)
wherein each N and N' is a ribonucleotide selected from the group consisting of a modified ribonucleotide or an unmodified ribonucleotide and each of (N)ₓ and (N')_{y} is an oligomer in which each consecutive N or N' is joined to the next N or N' by a covalent bond;
wherein each of x and y is an integer between 18 and 40;
wherein each of Z and Z' may be present or absent, but if present is 1-5 nucleotides covalently attached at the 3' terminus of the strand in which it is present;
and wherein the sequence of (N)ₓ comprises an antisense sequence relative to the mRNA transcribed from the mammalian gene TP53, namely 5' UGAAGGGUGAAAUAUUCUC 3' for use in preventing or reducing Delayed Graft Function as detailed above.

Z and Z' are preferably independently selected from the group consisting of dT (deoxythymine) and dTdT.

The antisense and sense sequences are the sequences presented in Tables A#1-N.

The abbreviations in the tables A-N are as follows: CHMP chimpanzee, MO mouse, CHL chinchilla, MK monkey, RB rabbit, GP guinea pig.

The siRNA compounds comprise the following oligomer pairs (both sense and antisense oligomers are shown in 5'-3' orientation):
TP53: (#1 in Table A) (Sequence disclosed in WO 2006/035434; 15)
   Sense: GAGAAUAUUUCACCCUUCA
   Antisense: UGAAGGGUGAAAUAUUCUC

For comparative purposes, other siRNAs compounds are disclosed in the description as follows:
TP53 (#44 in Table A) (Sequence disclosed in WO 2006/035434; F3)
   Sense: CCGAGUGGAAGGAAAUUUG
   Antisense: CAAAUUUCCUUCCACUCGG
TP53 (#198 in Table A) (Sequence disclosed in WO 2006/035434; G1)
   Sense: GACAGAAACACUUUUCGAC
   Antisense: GUCGAAAAGUGUUUCUGUC
HTRA2_11 (#2 in Table C and present in Table P)
   Sense: GAAUCACAGAAACACUUUU
   Antisense: AAAAGUGUUUCUGUGAUUC
HTRA2_(#3 in Table C)
   Sense: GGCCUGGUGAUGUGAUUUU
   Antisense: AAAAUCACAUCACCAGGCC
HTRA2_13 (#5 in Table C)
   Sense: CCGUGGUCUAUAUCGAGAU
   Antisense: AUCUCGAUAUAGACCACGG
HTRA2_15 (#13 in Table C)
   Sense: CCUAGCAACAUAUUAUAGU
   Antisense: ACUAUAAUAUGUUGCUAGG
HTRA2_16 (#20 in Table C and present in Table P)
   Sense: GCCGUGGUCUAUAUCGAGA
   Antisense: UCUCGAUAUAGACCACGGC
HTRA2_17 (#55 in Table C and present in Table P)
   Sense: GUGCUGCUCUUUGUGGUGU
   Antisense: ACACCACAAAGAGCAGCAC
HTRA22_18 (#179 in Table C)
   Sense: CAGCUAUUGAUUUUGGAAA
   Antisense: UUUCCAAAAUCAAUAGCUG
HTRA2_21 (#180 in Table C)
   Sense: GCUAUUGAUUUUGGAAACU
   Antisense: AGUUUCCAAAAUCAAUAGC
HTRA2_22 (#181 in Table C)
   Sense: AGCUAUUGAUUUUGGAAAC
   Antisense: GUUUCCAAAAUCAAUAGCU
KEAP1_2 (#5 in Table E)
   Sense: GCCUCAUUGAAUUCGCCUA
   Antisense: UAGGCGAAUUCAAUGAGGC
KEAP1_11 (#20 in Table E and present in Table P)
   Sense: CACCAUGUGAUUUAUUCUU
   Antisense: AAGAAUAAAUCACAUGGUG
KEAP1_12 (#21 in Table E and present in Table P)
   Sense: ACUGCAAAUAACCCAUCUU
   Antisense: AAGAUGGGUUAUUUGCAGU
KEAP1_13 (#28 in Table E and present in Table P)
   Sense: CACUGCAAAUAACCCAUCU
   Antisense: AGAUGGGUUAUUUGCAGUG
KEAP1_14 (#37 in Table E and present in Table P)
   Sense: GCAGCUGUCACCAUGUGAU
   Antisense: AUCACAUGGUGACAGCUGC
KEAP1_17 (#56 in Table E and present in Table P)
   Sense: UGCAUCAACUGGGUCAAGU
   Antisense: ACUUGACCCAGUUGAUGCA
SHC1_1 (#1 in Table G)
   Sense: ACCUGAAAUUUGCUGGAAU
   Antisense: AUUCCAGCAAAUUUCAGGU
SHC1_2 (#3 in Table G)
   Sense: CAGAGAGCUUUUUGAUGAU
   Antisense: AUCAUCAAAAAGCUCUCUG
SHC1_3 (#8 in Table G)
   Sense: CACAUGCAAUCUAUCUCAU
   Antisense: AUGAGAUAGAUUGCAUGUG
SHC1_6 (#28 in Table G)
   Sense: CGGGAGCUUUGUCAAUAAG
   Antisense: CUUAUUGACAAAGCUCCCG
SHC1_8 (#140 in Table G)
   Sense: GGGUUCUUAUAAUGGAAAA
   Antisense: UUUUCCAUUAUAAGAACCC
SHC1_11 (#141 in Table G)
   Sense: CCCAAGCCCAAGUACAAUC
   Antisense: GAUUGUACUUGGGCUUGGG
SHC1_14 (#142 in Table G)
   Sense: AGGAAGGGCAGCUGAUGAU
   Antisense: AUCAUCAGCUGCCCUUCCU
ZNHIT1_1 (#1 in Table I)
   Sense: CCGAGGUGAUCAUUUUAAA
   Antisense: UUUAAAAUGAUCACCUCGG
ZNHIT1_5 (#5 in Table I)
   Sense: GUGACCACAUCUUUAAAAU
   Antisense: AUUUUAAAGAUGUGGUCAC
ZNHIT1_10 (#34 in Table I)
   Sense: CUGGAAAGAAAAAGAAGAA
   Antisense: UUCUUCUUUUUCUUUCCAG
ZNHIT1_11 (#50 in Table I)
   Sense: ACACUGGAAAGAAAAAGAA
   Antisense: UUCUUUUUCUUUCCAGUGU
LGALS3_3: (#4 in Table K)
   Sense: GGGAAUUUCUGGUGACAUA
   Antisense: UAUGUCACCAGAAAUUCCC
LGALS3_5: (#159 in Table K)
   Sense: GCAGACGGCUUCUCACUUA
   Antisense: UAAGUGAGAAGCCGUCUGC
LGALS3_17 (#160 in Table K and present in Table P)
   Sense: AGCGGAAAAUGGCAGACAA
   Antisense: UUGUCUGCCAUUUUCCGCU
LGALS3_18 (#15 in Table K)
   Sense: GGGUUAAAAAACUCAAUGA
   Antisense: UCAUUGAGUUUUUUAACCC

It will be readily understood by those skilled in the art that the compounds for use of the present invention consist of a plurality of modified and/or unmodified ribonucleotides, which are linked through covalent linkages. Each such covalent linkage may be a phosphodiester linkage, a phosphorothioate linkage, or a combination of both, along the length of the ribonucleotide sequence of the individual strand. Other possible backbone modifications are described inter alia in U.S. Patent Nos. 5,587,361; 6,242,589; 6,277,967; 6,326,358; 5,399,676; 5,489,677; 5,596,086; 6,693,187 and 7,067,641.

In particular embodiments, x and y are independently an integer between about 18 to about 40, preferably from about 19 to about 23. In a particular embodiment, x is equal to y (i.e. x = y) and in preferred embodiments x = y = 19, x = y = 20 or x = y = 21. In a particularly preferred embodiment x=y= 19.

In one embodiment of the compound for use of the invention, Z and Z' are both absent; in another embodiment one or both of Z or Z' is present.

The modified ribonucleotides are modified in their sugar residue, by the addition of a moiety at the 2' position which is methoxy (2'-O-Me).

Alternating ribonucleotides are modified in both the antisense and the sense strands of the compound. In particular the exemplified siRNA has been modified such that a 2'-O-methyl (Me) group was present on the first, third, fifth, seventh, ninth, eleventh, thirteenth, fifteenth, seventeenth and nineteenth nucleotide of the antisense strand, whereby the very same modification, i. e. a 2'-O-Me group, was present at the second, fourth, sixth, eighth, tenth, twelfth, fourteenth, sixteenth and eighteenth nucleotide of the sense strand. Additionally, it is to be noted that these particular siRNA compounds are also blunt ended.

The compounds of the invention comprise alternating modified and unmodified ribonucleotides in both the antisense and the sense strands of the compound. In certain embodiments in the 19-mer oligomers and 23-mer oligomers the ribonucleotides at the 5' and 3' termini of the antisense strand are modified in their sugar residues, and the ribonucleotides at the 5' and 3' termini of the sense strand are unmodified in their sugar residues. For 21-mer oligomers the ribonucleotides at the 5' and 3' termini of the sense strand are modified in their sugar residues, and the ribonucleotides at the 5' and 3' termini of the antisense strand are unmodified in their sugar residues. As mentioned above, it is preferred that the middle nucleotide of the antisense strand is unmodified.

According to one preferred embodiment of the invention, the antisense and the sense strands of the siRNA are phosphorylated only at the 3'-terminus and not at the 5'-terminus. According to another preferred embodiment of the invention, the antisense and the sense strands are non-phosphorylated. According to yet another preferred embodiment of the invention, the 5' ribonucleotide in the sense strand is modified, for example to abolish any possibility of *in vivo* 5'-phosphorylation.

The description further discloses a vector capable of expressing any of the aforementioned oligoribonucleotides in unmodified form in a cell after which appropriate modification may be made. In preferred embodiment the cell is a mammalian cell, preferably a human cell.

Substantially complementary refers to complementarity of greater than about 84%, to another sequence. For example in a duplex region consisting of 19 base pairs one mismatch results in 94.7% complementarity, two mismatches results in about 89.5% complementarity and 3 mismatches results in about 84.2% complementarity, rendering the duplex region substantially complementary. Accordingly substantially identical refers to identity of greater than about 84%, to another sequence. More particularly, the invention provides an oligoribonucleotide for the subject use wherein one strand comprises consecutive nucleotides having, from 5' to 3', the sequence set forth in Tables A #1.

Tables A-N provide illustrative 19- and 21-mer oligomers useful in the preparation of siRNA compounds for targeting the expression of a gene selected from TP53; HTRA2; KEAP1; SHC1-SHC, ZNHIT1, LGALS3, and HI95 respectively. The presently most preferred compound of the invention is a blunt-ended 19-mer siRNA, i.e. x=y=19 and Z and Z' are both absent. The siRNA is either phosphorylated at 3' termini of both sense and anti-sense strands, or non-phosphorylated at all; or having the 5' most ribonucleotide on the sense strand specifically modified to abolish any possibility of *in vivo* 5'-phosphorylation. The alternating ribonucleotides are modified at the 2' position of the sugar residue in both the antisense and the sense strands, wherein the moiety at the 2' position is methoxy (2'-O-methyl) and wherein the ribonucleotides at the 5' and 3' termini of the antisense strand are modified in their sugar residues, and the ribonucleotides at the 5' and 3' termini of the sense strand are unmodified in their sugar residues.

The compounds for use of the present invention can be synthesized by any of the methods that are well known in the art for synthesis of ribonucleic (or deoxyribonucleic) oligonucleotides. Such synthesis is, among others, described in Beaucage and Iyer Tetrahedron 1992; 48: 2223-2311, Beaucage and Iyer, Tetrahedron 1993; 49: 6123-6194 and Caruthers et. al., Methods Enzymol. 1987; 154: 287-313; the synthesis of thioates is, among others, described in Eckstein, Annu. Rev. Biochem. 1985; 54: 367-402, the synthesis of RNA molecules is described in Sproat ,in Humana Press 2005, Herdewijn ed.; Kap. 2: 17-31 and respective downstream processes are, among others, described in Pingoud et al., in IRL Press 1989 edited by Oliver; Kap. 7: 183-208 and Sproat (ibid). Other synthetic procedures are known in the art e.g. the procedures as described in Usman et al., 1987, J. Am. Chem. Soc., 109, 7845; Scaringe et al., 1990, NAR, 18, 5433; Wincott et al., 1995, NAR, 23, 2677-2684; and Wincott et al., 1997, Methods Mol. Bio., 74, 59, and these procedures may make use of common nucleic acid protecting and coupling groups, such as dimethoxytrityl at the 5'-end, and phosphoramidites at the 3'-end. The modified (e.g. 2'-O-methylated) nucleotides and unmodified nucleotides are incorporated as desired.

The oligonucleotides for use of the present invention can be synthesized separately and joined together post-synthetically, for example, by ligation (Moore et al., 1992, Science 256, 9923;

International PCT publication No. WO 93/23569; Shabarova et al., 1991, NAR 19, 4247; Bellon et al., 1997, Nucleosides & Nucleotides, 16, 951; Bellon et al., 1997, Bioconj. Chem. 8, 204), or by hybridization following synthesis and/or deprotection.

It is noted that a commercially available machine (available, inter alia, from Applied Biosystems) can be used; the oligonucleotides are prepared according to the sequences disclosed herein. Overlapping pairs of chemically synthesized fragments can be ligated using methods well known in the art (e.g., see US Patent No. 6,121,426). The strands are synthesized separately and then are annealed to each other in the tube. Then, the double-stranded siRNAs are separated from the single-stranded oligonucleotides that were not annealed (e.g. because of the excess of one of them) by HPLC. In relation to the siRNAs or siRNA fragments of the present invention, two or more such sequences can be synthesized and linked together for use in the present invention.

The compounds of the invention can also be synthesized via a tandem synthesis methodology, as described in US patent application publication No. 2004/0019001 wherein both siRNA strands are synthesized as a single contiguous oligonucleotide fragment or strand separated by a cleavable linker which is subsequently cleaved to provide separate siRNA fragments or strands that hybridize and permit purification of the siRNA duplex. The linker can be a polynucleotide linker or a non-nucleotide linker.

### Pharmaceutical Compositions

While it may be possible for the compounds for use of the present invention to be administered as the raw chemical, it is preferable to present them as a pharmaceutical composition. Accordingly the present invention provides a pharmaceutical composition comprising one or more of the compounds of the invention; and a pharmaceutically acceptable carrier. This composition may comprise a mixture of two or more different siRNA compounds.

The invention further provides a pharmaceutical composition comprising at least one compound for use of the invention covalently or non-covalently bound to one or more compounds of the invention in an amount effective to inhibit the mammalian pro-apoptotic genes; and a pharmaceutically acceptable carrier. The compound may be processed intracellularly by endogenous cellular complexes to produce one or more oligoribonucleotides of the invention.

The invention further provides a pharmaceutical composition comprising a pharmaceutically acceptable carrier and one or more of the compounds of the invention in an amount effective to down-regulate expression in a cell of a mammalian pro-apoptotic gene of the present invention, the compound comprising a sequence complementary to the sequence of 5' UGAAGGGUGAAAUAUUCUC 3'.

### Methods of Treatment

In preferred embodiments the subject to be treated is a warm-blooded animal and, in particular, mammals including human, afflicted with or suffering from a disease or disorder associated with TP53 expression. The term "treatment" as used herein refers to administration of a therapeutic substance to a subject in need thereof in an amount effective to ameliorate symptoms associated with a disease, to lessen the severity or cure the disease, or to prevent the disease from occurring.

In cases where treatment is for the purpose of prevention, then the present invention relates to the subject siRNA for use in a method for delaying the onset of or averting the development of the disease or disorder.

The term "organ transplant" is meant to encompass transplant of kidney. Although a xenotransplant can be contemplated in certain situations, an allotransplant is usually preferable.

The present invention relates to a compound according to the present invention for use in a method of treating an organ recipient wherein said compound is to be administered to the organ recipient, in a therapeutically effective amount.

The description further discloses a method for preserving an organ comprising contacting the organ with an effective amount of compound of the present invention. Also disclosed is a method for reducing or preventing injury (in particular reperfusion injury) of an organ during surgery and/or following removal of the organ from a subject comprising placing the organ in an organ preserving solution wherein the solution comprises a compound according to the present invention.

Additionally, the description discloses a method of down-regulating the expression of the mammalian pro-apoptotic gene TP53 by at least 50% as compared to a control comprising contacting a mRNA transcript of TP53; with one or more of the compounds as disclosed.

The compound for use of the present invention down-regulates the mammalian gene TP53 whereby the down-regulation is selected from the group comprising down-regulation of gene function, down-regulation of polypeptide and down-regulation of mRNA expression.

The down-regulation is selected from the group comprising down-regulation of function (which may be examined by an enzymatic assay or a binding assay with a known interactor of the native gene / polypeptide, inter alia), down-regulation of protein (which may be examined by Western blotting, ELISA or immuno-precipitation, inter alia) and down-regulation of mRNA expression (which may be examined by Northern blotting, quantitative RT-PCR, in-situ hybridization or microarray hybridization, inter alia).

In additional embodiments the description discloses a method of treating a patient suffering from a disease accompanied by an elevated level of a mammalian pro-apoptotic gene disclosed herein, the method comprising administering to the patient a compound or composition as disclosed in a therapeutically effective dose thereby treating the patient.

The present description discloses the use of compounds which down-regulate the expression of a mammalian pro-apoptotic gene, particularly to novel small interfering RNAs (siRNAs), in the treatment of the following diseases or conditions in which inhibition of the expression of the mammalian TP53 gene is beneficial: acute renal failure (ARF), hearing loss including chemical-induced oxotoxicity, glaucoma, diabetic retinopathy, ischemic optic neuropathy, dry eye syndrome, acute respiratory distress syndrome (ARDS) and other acute lung and respiratory injuries, injury (e.g. ischemia-reperfusion injury) in organ transplant including lung, kidney, bone marrow, heart, pancreas, cornea or liver transplantation, nephrotoxicity, nephritis, neurotoxicity, spinal cord injury, osteoarthritis (OA), oral mucositis, pressure sores, and chronic obstructive pulmonary disease (COPD).

Other indications include chemical-induced nephrotoxicity and chemical-induced neurotoxicity, for example toxicity induced by cisplatin and cisplatin-like compounds (platinum - based compounds), by aminoglycosides, by loop diuretics, and by hydroquinone and their analogs.

Methods, molecules and compositions which inhibit a mammalian proapoptotic gene or polypeptide of the present invention are discussed herein at length, and any of said molecules and/or compositions may be beneficially employed in the treatment of a patient suffering from any of said conditions. It is to be explicitly understood that the present invention covers novel compounds and compositions as well as novel methods of treatment of known compounds (such as the compounds disclosed in co-assigned PCT Publication No. WO 2006/035434.

"Treatment" refers to both therapeutic treatment and prophylactic or preventative measures, wherein the object is to prevent or slow down (lessen) pro-apoptotic-related disorder as listed above. Those in need of treatment include those already experiencing the disease or condition, those prone to having the disease or condition, and those in which the disease or condition is to be prevented. The compounds of the invention may be administered before, during or subsequent to the onset of the disease or condition.

The disclosed method includes administering a therapeutically effective amount of one or more compounds which down-regulate expression of TP53 gene, particularly the novel siRNAs of the present invention.

The compounds of the invention are used for treating acute renal failure, in particular acute renal failure due to ischemia in post surgical patients.

The patients at high-risk of developing acute renal failure can be identified using various scoring methods such as the Cleveland Clinic algorithm or that developed by US Academic Hospitals (QMMI) and by Veterans' Administration (CICSS). Preferred uses of the compounds of the invention are for the prevention of ischemic acute renal failure in kidney transplant patients.

The siRNA compounds of the invention are used for treating acute kidney injury in patients undergoing kidney transplantation. In one embodiment, the acute kidney injury is a result of kidney ischemia-reperfusion injury during kidney transplantation.

The siRNA compounds of the invention for treating acute kidney injury in patients undergoing kidney transplantation are siRNA compounds directed to the TP53 gene, more preferably the siRNA compound is the 15 compound (#1 in Table A).

The siRNA compounds of the invention for treating acute kidney injury in patients undergoing kidney transplantation are administered intravenously, more preferably as a single slow intravenous push.

In one preferred embodiment, the siRNA compounds of the invention are used to treat patients undergoing deceased donor kidney transplant for prophylaxis of Delayed Graft Function. Delayed Graft Function is defined as the need for dialysis within the first seven days after kidney transplantation and is associated with poorer graft function and survival. The occurrence of Delayed Graft Function is significantly higher when the graft is obtained from a donor who died from brain or cardiac death, or when the graft has been kept in cold storage conditions for more than 24h prior to transplantation (cold storage prior to transplantation).

In order to achieve prophylaxis of Delayed Graft Function following kidney transplantation, the siRNA compounds of the invention are administered to the recipient following the transplantation, 15 minutes to 4 hours following revascularization of the graft in the recipient. Preferred doses of the siRNA compounds of the invention are between 0.1- 50 mg/kg, more preferably between 0.5-10 mg/kg. Additionally, the siRNA compounds of the invention may be administered to a living donor preferably 24 hours or less, most preferably one hour or less prior to harvest of the organ.

For kidney transplantation, the recipient or both the donor and the recipient may be treated with a compound or composition of the present invention. Accordingly, the present invention relates to a compound of the invention for use in a method of treating a kidney recipient wherein the compound is to be administered to the recipient in a therapeutically effective amount.

The description further discloses a method for preserving a kidney transplant or graft comprising contacting the transplant or graft with an effective amount of compound of the present invention. Also provided is a method for reducing or preventing injury (in particular reperfusion injury) of a kidney transplant or graft during surgery and/or following removal of the organ from a subject comprising placing the kidney transplant or graft in an organ preserving solution wherein the solution comprises a compound according to the present invention.

In other aspects the disclosed compounds and methods are useful for treating or preventing the incidence or severity of various diseases and conditions in a patient, in particular

conditions which are result from ischemic/reperfusion injury or oxidative stress, ischemic optic neuropathy, dry eye syndrome, acute respiratory distress syndrome (ARDS) for example due to coronavirus infection or endotoxins, severe acute respiratory syndrome (SARS), and other acute lung injuries, ischemia reperfusion injury associated with organ transplantation such as kidney or lung transplantation, glaucoma, spinal cord injury, pressure sores, oral mucositis, osteoarthritis, chronic obstructive pulmonary disease (COPD) and chemical-induced toxicity. The methods comprising administering to the patient a composition comprising one or more inhibitors (such as siRNA compounds), which inhibit TP53 in a therapeutically effective dose, thereby treating the patient.

The TP53 siRNA compounds of the present invention are particularly useful in ameliorating or treating the adverse effects of organ transplant, including ameliorating, treating or preventing perfusion injury.

The present invention relates to a compound of the present invention for use in a method of treating an organ recipient wherein the compound is to be administered to the organ recipient in a therapeutically effective amount.

The description further discloses a method for preserving an organ comprising contacting the organ with an effective amount of compound of the present invention. Also disclosed is a method for reducing or preventing injury (in particular reperfusion injury) of an organ during surgery and/or following removal of the organ from a subject comprising placing the organ in an organ preserving solution wherein the solution comprises a compound according to the present invention.

The present invention also provides for a process of preparing a pharmaceutical composition, which comprises:
providing one or more double stranded compound of the invention ; and
admixing said compound with a pharmaceutically acceptable carrier.

In a preferred embodiment, the compound used in the preparation of a pharmaceutical composition is admixed with a carrier in a pharmaceutically effective dose. In a particular embodiment the compound of the present invention is conjugated to a steroid or to a lipid or to another suitable molecule e.g. to cholesterol.

### Delivery

The siRNA molecules for use of the present invention may be delivered to the target tissue by direct application of the naked molecules prepared with a carrier or a diluent.

The term "naked siRNA" refers to siRNA molecules that are substantially free from any delivery vehicle that acts to assist, promote or facilitate entry into the cell, including viral sequences, viral particles, liposome formulations, lipofectin or precipitating agents and the like. For example, siRNA in PBS is "naked siRNA".

However, in some embodiments the siRNA molecules for use of the invention are delivered in liposome formulations and lipofectin formulations and the like and can be prepared by methods well known to those skilled in the art. Such methods are described, for example, in U.S. Pat. Nos. 5,593,972, 5,589,466, and 5,580,859.

Delivery systems aimed specifically at the enhanced and improved delivery of siRNA into mammalian cells have been developed (see, for example, Shen et al FEBS Let. 539: 111-114 (2003), Xia et al., Nat. Biotech. 20: 1006-1010 (2002), Reich et al., Mol. Vision 9: 210-216 (2003), Sorensen et al., J. Mol. Biol. 327: 761-766 (2003), Lewis et al., Nat. Gen. 32: 107-108 (2002) and Simeoni et al., NAR 31, 11: 2717-2724 (2003)). siRNA has recently been successfully used for inhibition of gene expression in primates; (for details see for example, Tolentino et al., Retina 24(1):132-138).

Respiratory formulations for siRNA are described in US patent application publication No. 2004/0063654. Cholesterol-conjugated siRNAs (and other steroid and lipid conjugated siRNAs) can been used for delivery (see for example Soutschek et al Nature 2004. 432: 173-177. ; and Lorenz et al. Bioorg. Med. Chem.. Lett. 14:4975-4977 (2004).

Pharmaceutically acceptable carriers, solvents, diluents, excipients, adjuvants and vehicles as well as implant carriers generally refer to inert, non-toxic solid or liquid fillers, diluents or encapsulating material not reacting with the active ingredients of the invention and they include liposomes and microspheres. Examples of delivery systems useful in the present invention include U.S. Patent Nos. 5,225,182; 5,169,383; 5,167,616; 4,959,217; 4,925,678; 4,487,603; 4,486,194; 4,447,233; 4,447,224; 4,439,196; and 4,475,196. Many other such implants, delivery systems, and modules are well known to those skilled in the art. In one specific embodiment of this invention topical and transdermal formulations may be selected. The siRNAs or pharmaceutical compositions of the present invention are administered and dosed in accordance with good medical practice, taking into account the clinical condition of the individual patient, the disease to be treated, the site and method of administration, scheduling of administration, patient age, sex, body weight and other factors known to medical practitioners. The compounds and compositions of the present invention may be administered alone or in combination with other pharmaceuticals. For example in transplant patients, it may be beneficial to co-administration of one or more compounds of the invention with an immunosuppressant, including cyclosporine, tacrolimus, azathioprine, prednisone and the like.

By "co-administration" is meant administration before, concurrently with, or after administration of an siRNA inhibitor as described above.

A "therapeutically effective dose" for purposes herein is thus determined by such considerations as are known in the art. The dose must be effective to achieve improvement including but not limited to improved survival rate or more rapid recovery, or improvement or elimination of symptoms and other indicators as are selected as appropriate measures by those skilled in the art.

In general, the active dose of compound for humans is in the range of from 1 ng/kg to about 20-100 mg/kg body weight per day, preferably about 0.01 mg to about 2-10 mg/kg body weight per day, in a regimen of one dose per day or twice or three or more times per day for a period of 1-4 weeks or longer.

It should be noted that the compound can be administered as the compound or as pharmaceutically acceptable salt and can be administered alone or as an active ingredient in combination with pharmaceutically acceptable carriers, solvents, diluents, excipients, adjuvants and vehicles. The compounds are to be administered intravenously. Liquid forms may be prepared for intravenous injection. The liquid compositions include aqueous solutions, with and without organic co-solvents, aqueous or oil suspensions, emulsions with edible oils, as well as similar pharmaceutical vehicles. The administration comprises intravenous administration.

The present description further discloses a pharmaceutical composition comprising two or more siRNA molecules for the treatment of any of the diseases and conditions mentioned herein, whereby said two molecules may be physically mixed together in the pharmaceutical composition in amounts which generate equal or otherwise beneficial activity, or may be covalently or non-covalently bound, or joined together by a nucleic acid linker of a length ranging from 2-100, preferably 2-50 or 2-30 nucleotides. In one aspect, the siRNA molecules are comprised of a double-stranded nucleic acid structure as described herein, wherein the two siRNA sequences are selected from Tables A-N.

The siRNA molecules are covalently or non-covalently bound or joined by a linker to form a tandem siRNA molecule. Such tandem siRNA molecules comprising two siRNA sequences are typically of 38-150 nucleotides in length, more preferably 38 or 40-60 nucleotides in length, and longer accordingly if more than two siRNA sequences are included in the tandem molecule. A longer tandem molecule comprised of two or more longer sequences which encode siRNA produced via internal cellular processing, e.g., long dsRNAs, is also disclosed, as is a tandem molecule encoding two or more shRNAs.

Particularly preferred tandem molecules are tandem molecules comprising one or two of the preferred siRNAs disclosed herein (the siRNAs, supra).

siRNA compounds for use according to the invention may be the main active component in a pharmaceutical composition, or may be one active component of a pharmaceutical composition containing two or more siRNAs (or molecules which encode or endogenously produce two or more siRNAs, be it a mixture of molecules or one or more tandem molecules which encode two or more siRNAs), said pharmaceutical composition further being comprised of one or more additional siRNA molecule which targets one or more additional gene. Simultaneous inhibition of said additional gene(s) will likely have an additive or synergistic effect for treatment of the diseases disclosed herein.

Additionally, the siRNA compounds disclosed herein or any nucleic acid molecule comprising or encoding such siRNA can be linked or bound (covalently or non-covalently) to antibodies (including aptamer molecules) against cell surface internalizable molecules expressed on the target cells, in order to achieve enhanced targeting for treatment of the diseases disclosed herein. For example, anti-Fas antibody (preferably a neutralizing antibody) may be combined (covalently or non-covalently) with siRNA. In another example, an aptamer which can act like a ligand/antibody may be combined (covalently or non-covalently) with any siRNA to a pro-apoptotic gene disclosed herein.

The compounds for use of the present invention can be delivered directly. When delivered directly the sequences are generally rendered nuclease resistant.

It is also envisaged that a long oligonucleotide (typically 25-500 nucleotides in length) comprising one or more stem and loop structures, where stem regions comprise the sequences of the oligonucleotides of the invention, may be delivered in a carrier, preferably a pharmaceutically acceptable carrier, and may be processed intracellularly by endogenous cellular complexes (e.g. by DROSHA and DICER as described above) to produce one or more smaller double stranded oligonucleotides (siRNAs) which are oligonucleotides of the invention. This oligonucleotide can be termed a tandem shRNA construct. It is envisaged that this long oligonucleotide is a single stranded oligonucleotide comprising one or more stem and loop structures, wherein each stem region comprises a sense and corresponding antisense siRNA sequence of the pro-apoptotic genes of the invention. In particular, it is envisaged that this oligonucleotide comprises sense and antisense siRNA sequences as depicted in Tables A #1.

The present invention is illustrated in detail below with reference to Examples, but is not to be construed as being limited thereto.

Citation of any document herein is not intended as an admission that such document is pertinent prior art, or considered material to the patentability of any claim of the present application. Any statement as to content or a date of any document is based on the information available to applicant at the time of filing and does not constitute an admission as to the correctness of such a statement.

### EXAMPLES

### General methods in molecular biology

Standard molecular biology techniques known in the art and not specifically described were generally followed as in Sambrook et al., Molecular Cloning: A Laboratory Manual, Cold Spring Harbor Laboratory Press, New York (1989), and as in Ausubel et al., Current Protocols in Molecular Biology, John Wiley and Sons, Baltimore, Maryland (1989) and as in Perbal, A Practical Guide to Molecular Cloning, John Wiley & Sons, New York (1988), and as in Watson et al., Recombinant DNA, Scientific American Books, New York and in Birren et al (eds) Genome Analysis: A Laboratory Manual Series, Vols. 1-4 Cold Spring Harbor Laboratory Press, New York (1998) and methodology as set forth in US patent Nos. 4,666,828; 4,683,202; 4,801,531; 5,192,659 and 5,272,057. Polymerase chain reaction (PCR) was carried out generally as in PCR Protocols: A Guide To Methods And Applications, Academic Press, San Diego, CA (1990). In situ PCR in combination with Flow Cytometry can be used for detection of cells containing specific DNA and mRNA sequences (Testoni et al., 1996, Blood 87:3822.) Methods of performing RT-PCR are also well known in the art.

### EXAMPLE 1: In vitro testing of the siRNA compounds for pro-apoptotic genes

General: About 1.5-2x10⁵ tested cells (HeLa cells or 293T cells for siRNA targeted the human gene and NRK52 cells or NMUMG cells for siRNA targeted the rat/mouse gene) were seeded per well in 6 wells plate (70-80% confluent).

At 24h subsequently, cells were transfected with siRNA compounds using Lipofectamine® 2000 reagent (Invitrogene) at final concentration of 500pM, 5nM, 20nM or 40nM. The cells were incubated at 37°C in a CO₂ incubator for 72h.

As positive control for cells transfection PTEN-Cy3 labeled siRNA compounds were used. As negative control for siRNA activity GFP siRNA compounds were used.

At 72h after transfection cells were harvested and RNA was extracted from cells. Transfection efficiency was tested by fluorescent microscopy.

Results: The percent of inhibition of gene expression using specific preferred siRNAs was determined using qPCR analysis of target gene in cells expressing the endogenous gene. The data demonstrate the percent of knockdown of the expression of the target gene in cells. In general, the siRNAs having specific sequences that were selected for in vitro testing were specific for both human and the rat/rabbit genes. Similar results of reduced expression of specific genes are obtained with other siRNAs, the sequences of which are listed in Tables A-N.

**Table 2: Percent of knockdown of the expression of selected genes in cells using 19-mer siRNA molecules.**

| **SiRNA tested** | **sense seq 5'>3'** | **anti-sense seq 5'>3'** | **% of control**** |
|---|---|---|---|
| HTRA2_11 | GAAUCACAGAAACACUUUU | AAAAGUGUUUCUGUGAUUC | 18, 18 |
| HTRA2_13 | CCGUGGUCUAUAUCGAGAU | AUCUCGAUAUAGACCACGG | 11, 15 |
| HTRA2_16 | GCCGUGGUCUAUAUCGAGA | UCUCGAUAUAGACCACGGC | 27 |
| HTRA2_18 | CAGCUAUUGAUUUUGGAA | UUUCCAAAAUCAAUAGCUG | 17, 11, 15 |
| HTRA2_21 | GCUAUUGAUUUUGGAAACU | AGUUUCCAAAAUCAAUAGC | 15, 14, 9 |
| HTRA2_22 | AGCUAUUGAUUUUGGAAAC | GUUUCCAAAAUCAAUAGCU | 19, 10, 8 |
| KEAP1_2 | GCCUCAUUGAAUUCGCCUA | UAGGCGAAUUCAAUGAGGC | 18, 32 |
| KEAP1_8 | GGGCAAAAAUACAGUCCAA | UUGGACUGUAUUUUUGCCC | 18, 7 (5nM) |
| KEAP1_9 | GGAGUAUCAUUGUUUUUGC | ACAAAAACAAUGAUACUCC | 8, 7 |
| KEAP1_10 | GGCAAAAAUACAGUCCAAU | AUUGGACUGUAUUUUUGCC | 15, 6 (5nM) |
| KEAP1_11 | CACCAUGUGAUUUAUUCUU | AAGAAUAAAUCACAUGGUG | 24 (5nM) |
| KEAP1_12 | ACUGCAAAUAACCCAUCUU | AAGAUGGGUUAUUUGCAGU | 38, 12 (5nM) |
| KEAP1_13 | CACUGCAAAUAACCCAUCU | AGAUGGGUUAUUUGCAGUG | 37 |
| KEAP1_14 | GCAGCUGUCACCAUGUGAU | AUCACAUGGUGACAGCUGC | 24 |
| SHC1_1 | ACCUGAAAUUUGCUGGAAU | AUUCCAGCAAAUUUCAGGU | 16, 12 (5nM) |
| SHC1_2 | CAGAGAGCUUUUUGAUGAU | AUCAUCAAAAAGCUCUCUG | 5, 13 |
| SHC1_3 | CACAUGCAAUCUAUCUCAU | AUGAGAUAGAUUGCAUGUG | 21, 12 |
| ZNHIT1_1 | CCGAGGUGAUCAUUUUAAA | UUUAAAAUGAUCACCUCGG | 8, 14 |
| ZNHIT1_2 | GUGACCACAUCUUUAAAAU | AUUUUAAAGAUGUGGUCAC | 22 |
| ZNHIT1_10 | CUGGAAAGAAAAAGAAGAA | UUCUUCUUUUUCUUUCCAG | 18 |
| ZNHIT1_11 | ACACUGGAAAGAAAAAGAA | UUCUUUUUCUUUCCAGUGU | 20 |
| LGALS3_12 | GUGCCUUAUAACCUGCCUU | AAGGCAGGUUAUAAGGCAC | 32, 51 |
| LGALS3_13 | GGAAGAAAGACAGUCGGUU | AACCGACUGUCUUUCUUCC | 18, 29, 27 |
| LGALS3_14 | GCAGUACAAUCAUCGGGUU | AACCCGAUGAUUGUACUGC | 26, 51 |
| LGALS3_15 | GAGAGUCAUUGUUUGCAAU | AUUGCAAACAAUGACUCUC | 24, 29, 13 |
| LGALS3_18 | GGGUUAAAAAACUCAAUGA | UCAUUGAGUUUUUUAACCC | 14, 30 |

| | | | |
|---|---|---|---|
| ** % of control in separate tests using 20nM concentration of siRNA molecules (unless indicated otherwise). All sequences are presented in a 5'-3' orientation. | | | |

### EXAMPLE 2: Model systems of acute renal failure (ARF) (for illustrative purposes)

ARF is a clinical syndrome characterized by rapid deterioration of renal function that occurs within days. Without being bound by theory the acute kidney injury may be the result of renal ischemia-reperfusion injury such as renal ischemia-reperfusion injury in patients undergoing major surgery such as major cardiac surgery. The principal feature of ARF is an abrupt decline in glomerular filtration rate (GFR), resulting in the retention of nitrogenous wastes (urea, creatinine). Recent studies support the hypothesis that apoptosis in renal tissues is prominent in most human cases of ARF. The principal site of apoptotic cell death is the distal nephron. During the initial phase of ischemic injury, loss of integrity of the actin cytoskeleton leads to flattening of the epithelium, with loss of the brush border, loss of focal cell contacts, and subsequent disengagement of the cell from the underlying substratum.

Testing the active siRNA for each pro-apoptotic gene separately for treating ARF is done using an animal model for ischemia-reperfusion-induced ARF.

Ischemia-reperfusion induced ARF: Ischemia-reperfusion injury is induced in rats following 45 minutes bilateral kidney arterial clamp and subsequent release of the clamp to allow 24 hours of reperfusion. Twelve mg/kg of siRNA of the invention (i.e. siRNA to a specific pro-apoptotic gene) are injected into the jugular vein 30 minutes prior to and 4 hours following the clamp. ARF progression is monitored by measurement of serum creatinine levels before (baseline) and 24 hrs post surgery. At the end of the experiment, the rats are perfused via an indwelling femoral line with warm PBS followed by 4% paraformaldehyde. The left kidneys are removed and stored in 4% paraformaldehyde for subsequent histological analysis. Acute renal failure is frequently defined as an acute increase of the serum creatinine level from baseline. An increase of at least 0.5 mg per dL or 44.2 µmol per L of serum creatinine is considered as an indication for acute renal failure. Serum creatinine is measured at time zero before the surgery and at 24 hours post ARF surgery. The results show that the siRNA compounds of the invention prevent onset of acute renal failure in this model.

### EXAMPLE 3: Model systems for transplantation-associated acute kidney injury

Warm ischemia - A left nephrectomy was performed, followed by auto transplantation that resulted in a warm kidney graft preservation period of 45 minutes. Following auto transplantation, a right nephrectomy was performed on the same animal. An anti-TP53 gene siRNA, QM5 siRNA, (sense strand: 5' GAAGAAAAUUUCCGCAAAA 3'; antisense strand: 5'UUUUGCGGAAAUUUUCUUC 3') was administered intravenously via the femoral vein either before harvesting of the kidney graft (mimicking donor treatment) ("pre"), or after the kidney autotransplantation (mimicking recipient treatment), or both before harvest and after transplantation (combined donor and recipient treatment) ("pre-post").

Cold ischemia - A left nephrectomy was performed on a donor animal, followed by a cold preservation (on ice) of the harvested kidney for a period of 5 hours. At the end of this period, the recipient rat underwent a bilateral nephrectomy, followed by transplantation of the cold-preserved kidney graft. The total warm ischemia time (including surgical procedure) was 30 minutes. QM5 was administered intravenously via the femoral vein, either to the donor animal prior to the kidney harvest ("pre"), or to the recipient animal 15 minutes ("post 15 min") or 4 hours (post 4 hrs) post-transplantation.

To assess the efficacy of QM5 siRNA in improvement of post-transplantation renal function, serum creatinine levels were measured on days 1, 2, and 7 post-transplantation in both warm and cold ischemia models.

In the warm ischemia experiments, serum creatinine levels in post-transplantation animals were lower in all QM5-treated groups compared to the control group, at all time points analyzed (Table 3 below); however, only at 24 hours post-transplantation were serum creatinine levels in all treatment groups statistically significantly lower than the control group. Group 3 ("post") and Group 4 ("pre-post") showed the lowest serum creatinine levels (1.11±0.68 mg/dL and 0.84±0.67 mg/dL, respectively) relative to the control group (2.36±0.99 mg/dL). No significant differences in the serum creatinine levels were found between siRNA treatment groups (adjusted p-value> 0.90). These data demonstrated the ability of p53 siRNA to protect rat kidney from transplantation-related acute kidney injury associated with warm ischemia and reperfusion.

**Table 3A: Mean serum creatinine values in warm ischemia model**

| | | 24 hours | | | 48 hours | | | 168 hours | | | Adjusted P-value (Tukey) | | |
|---|---|---|---|---|---|---|---|---|---|---|---|---|---|
| | Group | N | Mean | Std | N | Mean | Std | N | Mean | Std | Pre | Pre-Post | Post |
| | Control | 10 | 2.36 | 0.99 | 5 | 1.35 | 0.99 | 3 | 0.60 | 0.24 | 0.3575 | 0.1071 | 0.1890 |
| | Pre Injection | 6 | 1.45 | 1.03 | 5 | 0.94 | 0.75 | 3 | 0.44 | 0.05 | | 0.9134 | 0.9654 |
| | Pre-Post Injection | 7 | 0.84 | 0.67 | 4 | 0.61 | 0.17 | 3 | 0.28 | 0.05 | | | 0.9976 |
| | Post Injection | 6 | 1.11 | 0.68 | 5 | 0.77 | 0.21 | 3 | 0.38 | 0.23 | | | |

An important time point is 24 hours when the acute kidney damage develops. Table 3 shows the adjusted p-values according to Tukey method for time point 24 hours.

**Table 3B:_Tukey adjusted P-value for 24 hour time point**

| 24 hours | Pre Injection | Pre-Post Injection | Post Injection |
|---|---|---|---|
| Control | 0.0653 | 0.0034 | 0.0198 |
| PreInjection | | 0.1703 | 0.4415 |
| Pre-Post Injection | | | 0.4415 |

In the cold ischemia experiments, serum creatinine levels were statistically significantly reduced in all siRNA-treated groups, at all experimental time points compared to PBS-treated control (Table 4). No significant differences were found between siRNA treatment groups themselves. Serum creatinine levels in the control animals remained elevated one week post-transplantation (mean creatinine 1.25±0.71 mg/dL), indicating the occurrence of kidney injury. QM5-treated animals demonstrated almost two-fold lower (nearly basal) serum creatinine levels one week post-transplantation (0.63±0.18; 0.64±0.17; 0.63±0.31 for "pre", "post" and "pre-post" groups, respectively), indicating the ability of QM5 to protect the kidney from DGF (delayed graft function) associated with cold and warm ischemia and subsequent reperfusion.

**Table 4: Mean serum creatinine levels in cold ischemia model**

| | Time | 24 hours | | 48 hours | | 168 hours | |
|---|---|---|---|---|---|---|---|
| Group | N | Mean | Std | Mean | | Mean | Std |
| Control | 6 | 1.88 | 0.76 | 1.45 | 1.27 | 1.25 | 0.71 |
| Pre_30m | 6 | 1.12 | 0.32 | 0.97 | 0.39 | 0.63 | 0.18 |
| Pos_15m | 5 | 0.92 | 0.27 | 0.70 | 0.29 | 0.64 | 0.17 |
| Post_4h | 6 | 1.15 | 0.22 | 0.70 | 0.13 | 0.63 | 0.31 |

In conclusion, TP53-targeted siRNA protects rats from transplantation-associated acute kidney injury and from cold ischemia-associated delayed graft function. The maximum siRNA efficacy in preventing warm ischemia associated kidney dysfunction in transplantation model in rats was achieved when siRNA was administered (at a dose of 12 mg/kg) either to the recipient within hours post-transplantation, or both to donor within minutes pre harvest, and to recipient within hours post-transplantation. The difference between these two treatment regiments was not statistically significant. The maximum efficacy of QM5 in preventing cold ischemia-associated delayed graft function was achieved when siRNA was administered to the recipient as a single bolus intravenous injection (12 mg/kg) within minutes or hours post-transplantation. There was no statistically significant difference between these two treatment regiments.

For further elaboration on model systems which are used to test the compounds of the present invention, see International patent publication Nos. WO 06/023544A2, WO 2006/035434 and WO 2007/084684A2, co-assigned or assigned to the assignee of the present invention.

### EXAMPLE 4: Generation of sequences for active siRNA compounds to the pro-auoptotic genes and production of the siRNAs

Using proprietary algorithms and the known sequence of each pro-apoptotic gene, the sequences of potential siRNAs were generated. Tables A and B show TP53 19-mer and 21-mer siRNAs, respectively, as 5'-3' sequences, which are prioritized based on their score in the proprietary algorithm as the best sequences for targeting the human gene expression. A list of preferred siRNA to HTRA2 is provided in tables C and D, infra. A list of preferred siRNA to KEAP1 is provided in tables E and F, infra. A list of preferred siRNA to SHC1-SHC is provided in tables G and H, infra. A list of preferred siRNA to ZNHIT1 is provided in tables I and J, infra. A list of preferred siRNA to LGALS3 is provided in tables K and L infra. A list of preferred siRNA to HI95 is provided in tables M and N, infra.

The siRNAs used in the experiments described herein are all 19-mers, having alternating ribonucleotides modified in both the antisense and the sense strands of the compound. The modification is such that a 2'-O-methyl (Me) group is present on the first, third, fifth, seventh, ninth, eleventh, thirteenth, fifteenth, seventeenth and nineteenth nucleotide of the antisense strand, whereby the very same modification, i. e. a 2'-O-Me group, is present at the second, fourth, sixth, eighth, tenth, twelfth, fourteenth, sixteenth and eighteenth nucleotide of the sense strand. These particular siRNA compounds are also blunt ended and are non-phosphorylated at the termini; however, comparative experiments have shown that siRNA compounds phosphorylated at one or both of the 3'-termini have similar activity *in vivo* compared to the non-phosphorylated compounds.

It will be appreciated by a person skilled in the art that the above specific embodiments are illustrative and that the present invention is not limited by what has been particularly shown and described hereinabove.

**Table A: 19-mer P53 - tumor protein p53**

| Number | Sense siRNA | AntiSense siRNA | Other Sp | Human-8400737 ORF:252-1433 |
|---|---|---|---|---|
| 1 | GAGAAUAUUUCACCCUUCA | UGAAGGGUGAAAUAUUCUC | MK, Dog | [1221-1240] ORF |
| 2 | CCAUUUUUAUAUCGAUCUC | GAGAUCGAUAUAAAAAUGG | | [2564-2602] 3'UTR |
| 3 | CCCUUUUUAUAUCCCAUUU | AAAUGGGAUAUAAAAAGGG | | [2571-2589] 3'UTR |
| 4 | GCCCUCAACAAGAUGUUUU | AAAACAUCUUGUUGAGGGC | | [636-654] ORF |
| 5 | CCCAUUUUUAUAUCGAUCU | AGAUCGAUAUAAAAAUGGG | | [2583-2601] 3'UTR |
| 6 | CAGCUUAGAUUUUAAGGUU | AACCUUAAAAUCUAAGCUG | | [1646-1664] 3'UTR |
| 7 | CCAGCUUAGAUUUUAAGGU | ACCUUAAAAUCUAAGCUGG | | [1645-1663] 3'UTR |
| 8 | GGAAACAUUUUCAGACCUA | UAGGUCUGAAAAUGUUUCC | MK | [299-317] ORF |
| 9 | GGAUGACAGAAACACUUUU | AAAAGUGUUUCUGUCAUCC | MK,Pig | [869-887] ORF |
| 10 | GCCACCUGAAGUCCAAAAA | UUUUUGGACUUCAGGUGGC | | [1351-1369] ORF |
| 11 | GGGUCAACAUCUUUUACAU | AUGUAAAAGAUGUUGACCC | | [2507-2525] 3'UTR |
| 12 | CCCUCAACAAGAUGUUUUG | CAAAACAUCUUGUUGAGGG | | [637-655] ORF |
| 13 | ACAUACCAGCUUAGAUUUU | AAAAUCUAAGCUGGUAUGU | | [1640-1658] 3'UTR |
| 14 | UCCCAUUUUUAUAUCGAUC | GAUCGAUAUAAAAAUGGGA | | [2582-2600] 3'UTR |
| 15 | UCCCUUUUUAUAUCCCAUU | AAUGGGAUAUAAAAAGGGA | | [2570-2588] 3'UTR |
| 16 | GUGAGGGUUAAUGAAAUAA | UUAUUUCAUUAACCCUCAC | | [1865-1883] 3'UTR |
| 17 | CCAUAUCUGUGAAAUGCUG | CAGCAUUUCACAGAUAUGG | | [1817-1835] 3'UTR |
| 18 | CCCAUCCUCACCAUCAUCA | UGAUGAUGGUGAGGAUGGG | Pig,GP,RB | [999-1017] ORF |
| 19 | CCGGACGAUAUUGAACAAU | AUUGUUCAAUAUCGUCCGG | | [390-408] ORF |
| 20 | CCCGGACGAUAUUGAACAA | UUGUUCAAUAUCGUCCGGG | | [389-407] ORF |
| 21 | CUCUUGUAUAUGAUGAUCU | AGAUCAUCAUAUACAAGAG | | [2131-2149] 3'UTR |
| 22 | CCCUCAUGUUGAAUUUUCU | AGAAAAUUCAACAUGAGGG | | [1785-1803] 3'UTR |
| 23 | CCACCUGAAGUCCAAAAAG | CUUUUUGGACUUCAGGUGG | | [1352-1370] ORF |
| 24 | CCACCAAGACUUGUUUUAU | AUAAAACAAGUCUUGGUGG | | [2154-2172] 3'UTR |
| 25 | GCAUUGUGAGGGUUAAUGA | UCAUUAACCCUCACAAUGC | | [1860-1878] 3'UTR |
| 26 | CUAACUUCAAGGCCCAUAU | AUAUGGGCCUUGAAGUUAG | | [1804-1822] 3'UTR |
| 27 | AGUUUACAAUCAGCCACAU | AUGUGGCUGAUUGUAAACU | | [1719-1737] 3'UTR |
| 28 | AGGGUUAGUUUACAAUCAG | CUGAUUGUAAACUAACCCU | | [1713-1731] 3'UTR |
| 29 | GACUUCCAUUUGCUUUGUC | GACAAAGCAAAUGGAAGUC | | [1554-1572] 3'UTR |
| 30 | GAAACAUUUUCAGACCUAU | AUAGGUCUGAAAAUGUUUC | MK | [300-318] ORF |
| 31 | GAAACACUUUUCGACAUAG | CUAUGUCGAAAAGUGUUUC | MK | [877-895] ORF |
| 32 | UGCCCUCAACAAGAUGUUU | AAACAUCUUGUUGAGGGCA | | [635-653] ORF |
| 33 | CGGACGAUAUUGAACAAUG | CAUUGUUCAAUAUCGUCCG | | [391-409] ORF |
| 34 | CUCCCUUUUUAUAUCCCAU | AUGGGAUAUAAAAAGGGAG | | [2569-2587] 3'UTR |
| 35 | GGUCAACAUCUUUUACAUU | AAUGUAAAAGAUGUUGACC | | [2508-2526] 3'UTR |
| 36 | UCUUGUAUAUGAUGAUCUG | CAGAUCAUCAUAUACAAGA | | [2132-2150] 3'UTR |
| 37 | GCUGGUGGGUUGGUAGUUU | AAACUACCAACCCACCAGC | | [1975-1993] 3'UTR |
| 38 | UUGUGAGGGUUAAUGAAAU | AUUUCAUUAACCCUCACAA | | [1863-1881] 3'UTR |
| 39 | GGGAGAUGUAAGAAAUGUU | AACAUUUCUUACAUCUCCC | | [1684-1702] 3'UTR |
| 40 | CUGACAUUCUCCACUUCUU | AAGAAGUGGAGAAUGUCAG | | [1430-1448] ORF+3'UTR |
| 41 | CCAAAAAGGGUCAGUCUAC | GUAGACUGACCCUUUUUGG | | [1363-1381] ORF |
| 42 | GUAAUCUACUGGGACGGAA | UUCCGUCCCAGUAGAUUAC | MK | [1036-1054] ORF |
| 43 | CCAUCCACUACAACUACAU | AUGUAGUUGUAGUGGAUGG | MK,Dog,GP | [943-961] ORF |
| 44 | CCGAGUGGAAGGAAAUUUG | CAAAUUUCCUUCCACUCGG | MK,Dog | [836-854] ORF |
| 45 | CUGUCAUCUUCUGUCCCUU | AAGGGACAGAAGAUGACAG | | [528-546] ORF |
| 46 | CCCUCCUUCUCCCUUUUUA | UAAAAAGGGAGAAGGAGGG | | [2561-2579] 3'UTR |
| 47 | GGAAGGGUCAACAUCUUUU | AAAAGAUGUUGACCCUUCC | | [2503-2521] 3'UTR |
| 48 | CAAGACUUGUUUUAUGCUC | GAGCAUAAAACAAGUCUUG | | [2158-2176] 3'UTR |
| 49 | UCCACCAAGACUUGUUUUA | UAAAACAAGUCUUGGUGGA | | [2153-2171] 3'UTR |
| 50 | GGAGUUGUCAAGUCUUGCU | AGCAAGACUUGACAACUCC | | [2022-2040] 3'UTR |
| 51 | CCCAUAUCUGUGAAAUGCU | AGCAUUUCACAGAUAUGGG | | [1816-1834] 3'UTR |
| 52 | GGGUUAGUUUACAAUCAGC | GCUGAUUGUAAACUAACCC | | [1714-1732] 3'UTR |
| 53 | ACAUUCUCCACUUCUUGUU | AACAAGAAGUGGAGAAUGU | | [1433-1451] 3'UTR |
| 54 | CCAUCCUCACCAUCAUCAC | GUGAUGAUGGUGAGGAUGG | Pig,GP,RB | [1000-1018] ORF |
| 55 | AAACACUUUUCGACAUAGU | ACUAUGUCGAAAAGUGUUU | MK | [878-896] ORF |
| 56 | CCUGUCAUCUUCUGUCCCU | AGGGACAGAAGAUGACAGG | | [527-545] ORF |
| 57 | ACGAUAUUGAACAAUGGUU | AACCAUUGUUCAAUAUCGU | | [394-412] ORF |
| 58 | GGACGAUAUUGAACAAUGG | CCAUUGUUCAAUAUCGUCC | | [392-410] ORF |
| 59 | ACAUCUUUUACAUUCUGCA | UGCAGAAUGUAAAAGAUGU | | [2513-2531] 3'UTR |
| 60 | CCAAGACUUGUUUUAUGCU | AGCAUAAAACAAGUCUUGG | | [2157-2175] 3'UTR |
| 61 | CACCAAGACUUGUUUUAUG | CAUAAAACAAGUCUUGGUG | | [2155-2173] 3'UTR |
| 62 | CGACCUUAGUACCUAAAAG | CUUUUAGGUACUAAGGUCG | | [2075-2093] 3'UTR |
| 63 | GGUGGGUUGGUAGUUUCUA | UAGAAACUACCAACCCACC | | [1978-1996] 3'UTR |
| 64 | UGCAUUGUGAGGGUUAAUG | CAUUAACCCUCACAAUGCA | | [1859-1877] 3'UTR |
| 65 | GUGCAUUGUGAGGGUUAAU | AUUAACCCUCACAAUGCAC | | [1858-1876] 3'UTR |
| 66 | GCAUUUGCACCUACCUCAC | GUGAGGUAGGUGCAAAUGC | | [1836-1854] 3'UTR |
| 67 | GGCCCAUAUCUGUGAAAUG | CAUUUCACAGAUAUGGGCC | | [1814-1832] 3'UTR |
| 68 | AGGCCCAUAUCUGUGAAAU | AUUUCACAGAUAUGGGCCU | | [1813-1831] 3'UTR |
| 69 | CUCAUGUUGAAUUUUCUCU | AGAGAAAAUUCAACAUGAG | | [1787-1805] 3'UTR |
| 70 | UCCCUCAUGUUGAAUUUUC | GAAAAUUCAACAUGAGGGA | | [1784-1802] 3'UTR |
| 71 | CUGUCCCUCAUGUUGAAUU | AAUUCAACAUGAGGGACAG | | [1781-1799] 3'UTR |
| 72 | AGCUGUCCCUCAUGUUGAA | UUCAACAUGAGGGACAGCU | | [1779 7977] 3'UTR |
| 73 | AGACCUAUGGAAACUACUU | AAGUAGUUUCCAUAGGUCU | MK | [311-329] ORF |
| 74 | GUGAGCGCUUCGAGAUGUU | AACAUCUCGAAGCGCUCAC | MK,Rat | [1255-1273] ORF |
| 75 | UGGAUGACAGAAACACUUU | AAAGUGUUUCUGUCAUCCA | Pig | [868-886] ORF |
| 76 | GGAAGGAAAUUUGCGUGUG | CACACGCAAAUUUCCUUCC | MK | [842-860] ORF |
| 77 | GCUAAAAGUUUUGAGCUUC | GAAGCUCAAAACUUUUAGC | | [92-110] 5'UTR |
| 78 | GACUGGCGCUAAAAGUUUU | AAAACUUUUAGCGCCAGUC | GP | [85103] 5'UTR |
| 79 | AGGGUCAACAUCUUUUACA | UGUAAAAGAUGUUGACCCU | | [2506-2524] 3'UTR |
| 80 | GCCUUGAAACCACCUUUUA | UAAAAGGUGGUUUCAAGGC | | [1894-1912] 3'UTR |
| 81 | AGUGCAUUGUGAGGGUUAA | UUAACCCUCACAAUGCACU | | [1857-1875] 3'UTR |
| 82 | AGAGUGCAUUGUGAGGGUU | AACCCUCACAAUGCACUCU | | [1855-1873] 3'TR |
| 83 | UGUCCCUCAUGUUGAAUUU | AAAUUCAACAUGAGGGACA | | [1782-1800] 3'UTR |
| 84 | GACCUAUGGAAACUACUUC | GAAGUAGUUUCCAUAGGUC | MK | [312-330] ORF |
| 85 | GGAUGUUUGGGAGAUGUAA | UUACAUCUCCCAAACAUCC | | [1676-1694] 3'UTR |
| 86 | GGGAUGUUUGGGAGAUGUA | UACAUCUCCCAAACAUCCC | | [1675-1693] 3'UTR |
| 87 | AGGAAACAUUUUCAGACCU | AGGUCUGAAAAUGUUUCCU | MK | [298-316] ORF |
| 88 | UGGAUGGAGAAUAUUUCAC | GUGAAAUAUUCUCCAUCCA | MK | [1219-1237] ORF |
| 89 | AGUAUUUGGAUGACAGAAA | UUUCUGUCAUCCAAAUACU | RB | [862-880] ORF |
| 90 | GGAGUAUUUGGAUGACAGA | UCUGUCAUCCAAAUACUCC | RB | [860-878] ORF |
| 91 | CCUGAAAACAACGUUCUGU | ACAGAACGUUGUUUUCAGG | GP | [330-348] ORF |
| 92 | CUGGUGGGUUGGUAGUUUC | GAAACUACCAACCCACCAG | | [1976-1994] 3'UTR |
| 93 | ACUUCCUGAAAACAACGUU | AACGUUGUUUUCAGGAAGU | GP | [326-344] ORF |
| 94 | CUCAAGACUGGCGCUAAAA | UUUUAGCGCCAGUCUUGAG | | [80-98] 5'UTR |
| 95 | AUCUGUGAAAUGCUGGCAU | AUGCCAGCAUUUCACAGAU | | [1821-1839] 3'UTR |
| 96 | UCUAACUUCAAGGCCCAUA | UAUGGGCCUUGAAGUUAGA | | [1803-1821] 3'UTR |
| 97 | GUCCCUCAUGUUGAAUUUU | AAAAUUCAACAUGAGGGAC | | [1783-1801] 3'UTR |
| 98 | AGAAAUGUUCUUGCAGUUA | UAACUGCAAGAACAUUUCU | | [1694-1712] 3'UTR |
| 99 | CCAUUUUGGGUUUUGGGUC | GACCCAAAACCCAAAAUGG | | [1492-1510] 3'UTR |
| 100 | GACUCAGACUGACAUUCUC | GAGAAUGUCAGUCUGAGUC | | [1422-1440] ORF+3'UTR |
| 101 | CCUGACUCAGACUGACAUU | AAUGUCAGUCUGAGUCAGG | | [1419-1437] ORF+3'UTR |
| 102 | UUGGAUGACAGAAACACUU | AAGUGUUUCUGUCAUCCAA | Pig | [867-885] ORF |
| 103 | GUGGAGUAUUUGGAUGACA | UGUCAUCCAAAUACUCCAC | | [858-876] ORF |
| 104 | UGGCGCUAAAAGUUUUGAG | CUCAAAACUUUUAGCGCCA | GP | [88-106] 5'UTR |
| 105 | AGACUGGCGCUAAAAGUUU | AAACUUUUAGCGCCAGUCU | GP | [84-102] 5'UTR |
| 106 | GUCGACCUUAGUACCUAAA | UUUAGGUACUAAGGUCGAC | | [2073-2091] 3'UTR |
| 107 | CUUGAAACCACCUUUUAUU | AAUAAAAGGUGGUUUCAAG | | [1896-1914] 3'UTR |
| 108 | UGCAGUUAAGGGUUAGUUU | AAACUAACCCUUAACUGCA | | [1705-1723] 3'UTR |
| 109 | UUCAGACCUAUGGAAACUA | UAGUUUCCAUAGGUCUGAA | MK | [308-326] ORF |
| 110 | CCUGCACUGGUGUUUUGUU | AACAAAACACCAGUGCAGG | | [1597-1615] 3'UTR |
| 111 | CAUUUUCAGACCUAUGGAA | UUCCAUAGGUCUGAAAAUG | MK | [304-322] ORF |
| 112 | GACUGACAUUCUCCACUUC | GAAGUGGAGAAUGUCAGUC | | [1428-1446] ORF+3'UTR |
| 113 | UGACUCAGACUGACAUUCU | AGAAUGUCAGUCUGAGUCA | | [1421-1439] ORF+3'UTR |
| 114 | CACUGGAUGGAGAAUAUUU | AAAUAUUCUCCAUCCAGUG | MK | [1216-1234] ORF |
| 115 | AAAGAAGAAACCACUGGAU | AUCCAGUGGUUUCUUCUUU | MK,GP | [1205-1223] ORF |
| 116 | UCAUCACACUGGAAGACUC | GAGUCUUCCAGUGUGAUGA | RB,MO | [1012-1030] ORF |
| 117 | UGGAGUAUUUGGAUGACAG | CUGUCAUCCAAAUACUCCA | | [859-877] ORF |
| 118 | GCGUGUGGAGUAUUUGGAU | AUCCAAAUACUCCACACGC | | [854-872] ORF |
| 119 | CUGGCGCUAAAAGUUUUGA | UCAAAACUUUUAGCGCCAG | GP | [87-705] 5'UTR |
| 120 | CCUUCUCCCUUUUUAUAUC | GAUAUAAAAAGGGAGAAGG | | [2565-2583] 3'UTR |
| 121 | AAGCAAUGGAUGAUUUGAU | AUCAAAUCAUCCAUUGCUU | | [364-382] ORF |
| 122 | AGGGAGUUGUCAAGUCUUG | CAAGACUUGACAACUCCCU | | [2020-2038] 3'UTR |
| 123 | CCUUGAAACCACCUUUUAU | AUAAAAGGUGGUUUCAAGG | | [1895-1913] 3'UTR |
| 124 | GCUGUCCCUCAUGUUGAAU | AUUCAACAUGAGGGACAGC | | [1780-1798] 3'UTR |
| 125 | AAAUGUUCUUGCAGUUAAG | CUUAACUGCAAGAACAUUU | | [1696-1714] 3'UTR |
| 126 | AAGAAAUGUUCUUGCAGUU | AACUGCAAGAACAUUUCUU | | [1693-1711] 3'UTR |
| 127 | GUUUGGGAGAUGUAAGAAA | UUUCUUACAUCUCCCAAAC | | [1680-1698] 3'UTR |
| 128 | CUGCACUGGUGUUUUGUUG | CAACAAAACACCAGUGCAG | | [1598-1616] 3'UTR |
| 129 | CAGACUGACAUUCUCCACU | AGUGGAGAAUGUCAGUCUG | | [1426-1444] ORF+3'UTR |
| 130 | UCAGGAAACAUUUUCAGAC | GUCUGAAAAUGUUUCCUGA | MK | [296-314] ORF |
| 131 | GCUGAAUGAGGCCUUGGAA | UUCCAAGGCCUCAUUCAGC | MK,Rat | [1280-1298] ORF |
| 132 | CAAAGAAGAAACCACUGGA | UCCAGUGGUUUCUUCUUUG | MK,GP | [1204-1222] ORF |
| 133 | GCCAAAGAAGAAACCACUG | CAGUGGUUUCUUCUUUGGC | MK,GP | [1202-1220] ORF |
| 134 | 1AGCCAAAGAAGAAACCACU | AGUGGUUUCUUCUUUGGCU | MK,GP | [1201-1219] ORF |
| 135 | GCUCUGACUGUACCACCAU | AUGGUGGUACAGUCAGAGC | MK,HM,Pig,RB | [928-946] ORF |
| 136 | GAGUAUUUGGAUGACAGAA | UUCUGUCAUCCAAAUACUC | RB | [861-879] ORF |
| 137 | GUGUGGAGUAUUUGGAUGA | UCAUCCAAAUACUCCACAC | | [856-874] ORF |
| 138 | UGGAAGGAAAUUUGCGUGU | ACACGCAAAUUUCCUUCCA | MK | [841-859] ORF |
| 139 | CCCUCCUCAGCAUCUUAUC | GAUAAGAUGCUGAGGAGGG | | [818-836] ORF |
| 140 | ACAGCACAUGACGGAGGUU | AACCUCCGUCAUGUGCUGU | | [749-767] ORF |
| 141 | UCGACCUUAGUACCUAAAA | UUUUAGGUACUAAGGUCGA | | [2074-2092] 3'UTR |
| 142 | GAGUUGUCAAGUCUUGCUG | CAGCAAGACUUGACAACUC | | [2023-2041] 3'UTR |
| 143 | CAGAGUGCAUUGUGAGGGU | ACCCUCACAAUGCACUCUG | | [1854-1872] 3'UTR |
| 144 | AAGGCCCAUAUCUGUGAAA | UUUCACAGAUAUGGGCCUU | | [1812-1830] 3'UTR |
| 145 | UUGCAGUUAAGGGUUAGUU | AACUAACCCUUAACUGCAA | | [1704-1722] 3'UTR |
| 146 | UUCUUGCAGUUAAGGGUUA | UAACCCUUAACUGCAAGAA | | [1701-1719] 3'UTR |
| 147 | AGGGAUGUUUGGGAGAUGU | ACAUCUCCCAAACAUCCCU | | [1674-1692] 3'UTR |
| 148 | GAGGGAUGUUUGGGAGAUG | CAUCUCCCAAACAUCCCUC | | [1673-1691] 3'UTR |
| 149 | CAGGAAACAUUUUCAGACC | GGUCUGAAAAUGUUUCCUG | MK | [297-315] ORF |
| 150 | GCCAUAAAAAACUCAUGUU | AACAUGAGUUUUUUAUGGC | | [1387-1405] ORF |
| 151 | CGUGUGGAGUAUUUGGAUG | CAUCCAAAUACUCCACACG | | [855-873] ORF |
| 152 | UGGAUGAUUUGAUGCUGUC | GACAGCAUCAAAUCAUCCA | MK | [370-388] ORF |
| 153 | CAAUAAAACUUUGCUGCCA | UGGCAGCAAAGUUUUAUUG | | [2611-2629] 3'TR |
| 154 | CAAUGGAUGAUUUGAUGCU | AGCAUCAAAUCAUCCAUUG | | [367-385] ORF |
| 155 | GAGGGAGUUGUCAAGUCUU | AAGACUUGACAACUCCCUC | | [2019-2037] 3'UTR |
| 156 | UGGUUAGGUAGAGGGAGUU | AACUCCCUCUACCUAACCA | | [2009-2027] 3'UTR |
| 157 | AUGUACAUCUGGCCUUGAA | UUCAAGGCCAGAUGUACAU | | [1883-1901] 3'UTR |
| 158 | UGGGUUUUGGGUCUUUGAA | UUCAAAGACCCAAAACCCA | | [1498-1516] 3'UTR |
| 159 | CCCGCCAUAAAAAACUCAU | AUGAGUUUUUUAUGGCGGG | | [1384-1402] ORF |
| 160 | GAUGUUCCGAGAGCUGAAU | AUUCAGCUCUCGGAACAUC | MK,Pig,Rat | [1268-1286] ORF |
| 161 | AGAUGUUCCGAGAGCUGAA | UUCAGCUCUCGGAACAUCU | MK,Pig,Rat,RB | [1267-1285] ORF |
| 162 | ACAUGUGUAACAGUUCCUG | CAGGAACUGUUACACAUGU | MK,Dog | [958-976] ORF |
| 163 | ACCAUCCACUACAACUACA | UGUAGUUGUAGUGGAUGGU | MK,Dog,GP | [942-960] ORF |
| 164 | GAGCUUCUCAAAAGUCUAG | CUAGACUUUUGAGAAGCUC | | [104-122] 5'UTR |
| 165 | UCGACAUAGUGUGGUGGUG | CACCACCACACUAUGUCGA | MK | [887-905] ORF |
| 166 | GAAGGAAAUUUGCGUGUGG | CCACACGCAAAUUUCCUUC | MK | [843-861] ORF |
| 167 | AUAAUGUACAUCUGGCCUU | AAGGCCAGAUGUACAUUAU | | [1880-1898] 3'UTR |
| 168 | GGGUUUUGGGUCUUUGAAC | GUUCAAAGACCCAAAACCC | | [1499-1517] 3'UTR |
| 169 | GAAGUCCAAAAAGGGUCAG | CUGACCCUUUUUGGACUUC | | [1358-1376] ORF |
| 170 | AGCUUCUCAAAAGUCUAGA | UCUAGACUUUUGAGAAGCU | | [105-123] 5'UTR |
| 171 | GGAAAUUUGCGUGUGGAGU | ACUCCACACGCAAAUUUCC | MK | [846-864] ORF |
| 172 | CUGGUUAGGUAGAGGGAGU | ACUCCCUCUACCUAACCAG | | [2008-2026] 3'UTR |
| 173 | AGCCACCUGAAGUCCAAAA | UUUUGGACUUCAGGUGGCU | | [1350-1368] ORF |
| 174 | CAGAAACACUUUUCGACAU | AUGUCGAAAAGUGUUUCUG | MK | [875-893] ORF |
| 175 | UGCGUGUGGAGUAUUUGGA | UCCAAAUACUCCACACGCA | | [853-871] ORF |
| 176 | GGUAGAGGGAGUUGUCAAG | CUUGACAACUCCCUCUACC | | [2015-2033] 3'UTR |
| 177 | GCCCACUUCACCGUACUAA | UUAGUACGGUGAAGUGGGC | | [1753-1771] 3'UTR |
| 178 | UGGGACGGAAGAGCUUUGA | UCAAAGCUGUUCCGUCCCA | MK,HM,Pig,RB | [1045-1063] ORF |
| 179 | GCUUCUUGCAUUCUGGGAC | GUCCCAGAAUGCAAGAAGC | | [586-604] ORF |
| 180 | AUGCUGGCAUUUGCACCUA | UAGGUGCAAAUGCCAGCAU | | [1830-1848] 3'UTR |
| 181 | GGAAACUACUUCCUGAAAA | UUUUCAGGAAGUAGUUUCC | MK,GP | [319-337] ORF |
| 182 | GGAGAAUAUUUCACCCUUC | GAAGGGUGAAAUAUUCUCC | MK,Dog | [1224-1242] ORF |
| 183 | AGAAACACUUUUCGACAUA | UAUGUCGAAAAGUGUUUCU | MK | [876-894] ORF |
| 184 | CAAGAUGUUUUGCCAACUG | CAGUUGGCAAAACAUCUUG | | [644-662] ORF |
| 185 | CUGCCCUCAACAAGAUGUU | AACAUCUUGUUGAGGGCAG | | [634-652] ORF |
| 186 | CGCUAAAAGUUUUGAGCUU | AAGCUCAAAACUUUUAGCG | | [91-109] 5'UTR |
| 187 | UCAGCCACAUUCUAGGUAG | CUACCUAGAAUGUGGCUGA | | [1728-1746] 3'UTR |
| 188 | CCAGGACUUCCAUUUGCUU | AAGCAAAUGGAAGUCCUGG | | [1550-1568] 3'UTR |
| 189 | CCAUAAAAAACUCAUGUUC | GAACAUGAGUUUUUUAUGG | | [1388-1406] ORF |
| 190 | UCCUCACCAUCAUCACACU | AGUGUGAUGAUGGUGAGGA | Pig,RB | [1003-1021] ORF |
| 191 | CCAUCUACAAGCAGUCACA | UGUGACUGCUUGUAGAUGG | MK | [733-751] ORF |
| 192 | CUCUUGGUCGACCUUAGUA | UACUAAGGUCGACCAAGAG | | [2067-2085] 3'UTR |
| 193 | GCCACAUUCUAGGUAGGUA | UACCUACCUAGAAUGUGGC | | [1731-1749] 3'UTR |
| 194 | GGUUUUUACUGUGAGGGAU | AUCCCUCACAGUAAAAACC | | [1661-1679] 3'UTR |
| 195 | CCCAGGACUUCCAUUUGCU | AGCAAAUGGAAGUCCUGGG | | [1549-1567] 3'UTR |
| 196 | CGCCAUAAAAAACUCAUGU | ACAUGAGUUUUUUAUGGCG | | [1386-1404] ORF |
| 197 | CCGCCAUAAAAAACUCAUG | CAUGAGUUUUUUAUGGCGG | | [1385-1403] ORF |
| 198 | GACAGAAACACUUUUCGAC | GUCGAAAAGUGUUUCUGUC | MK,Dog,Pig | [873-891] ORF |
| 199 | CUGGAGGAUUUCAUCUCUU | AAGAGAUGAAAUCCUCCAG | | [2117-2135] 3'UTR |
| 200 | CCCUGUCUGACAACCUCUU | AAGAGGUUGUCAGACAGGG | | [2053-2071] 3'UTR |
| 201 | GUUGGUAGUUUCUACAGUU | AACUGUAGAAACUACCAAC | | [1983-2001] 3'UTR |
| 202 | GGUUGGUAGUUUCUACAGU | ACUGUAGAAACUACCAACC | | [1982-2000] 3'UTR |
| 203 | GGGUUGGUAGUUUCUACAG | CUGUAGAAACUACCAACCC | | [1981-1999] 3'UTR |
| 204 | CCACAUUCUAGGUAGGUAG | CUACCUACCUAGAAUGUGG | | [1732-1750] 3'UTR |
| 205 | GUAGGACAUACCAGCUUAG | CUAAGCUGGUAUGUCCUAC | | [1635-1653] 3'UTR |
| 206 | CCCUGCCAUUUUGGGUUUU | AAAACCCAAAAUGGCAGGG | | [1487-1505] 3'UTR |
| 207 | UCCCGCCAUAAAAAACUCA | UGAGUUUUUUAUGGCGGGA | | [1383-1401] ORF |
| 208 | CUCCCGCCAUAAAAAACUC | GAGUUUUUUAUGGCGGGAG | | [1382-1400] ORF |
| 209 | GGCCUUGGAACUCAAGGAU | AUCCUUGAGUUCCAAGGCC | MK | [1289-1307] ORF |
| 210 | CUCUGAGUCAGGAAACAUU | AAUGUUUCCUGACUCAGAG | MK | [289-307] ORF |
| 211 | UGGAGAAUAUUUCACCCUU | AAGGGUGAAAUAUUCUCCA | MK,Dog,Rat | [1223-1241] ORF |
| 212 | CCUGCCCUCAACAAGAUGU | ACAUCUUGUUGAGGGCAGG | | [633-651] ORF |
| 213 | GAACAAUGGUUCACUGAAG | CUUCAGUGAACCAUUGUUC | | [402-420] ORF |
| 214 | GGCAUUUGCACCUACCUCA | UGAGGUAGGUGCAAAUGCC | | [1835-1853] 3'UTR |
| 215 | UGCUGGCAUUUGCACCUAC | GUAGGUGCAAAUGCCAGCA | | [1831-1849] 3'UTR |
| 216 | CCUGCCAUUUUGGGUUUUG | CAAAACCCAAAAUGGCAGG | | [1488-1506] 3'UTR |
| 217 | GUCUACCUCCCGCCAUAAA | UUUAUGGCGGGAGGUAGAC | RB | [1376-1394] ORF |
| 218 | GGAAGAGAAUCUCCGCAAG | CUUGCGGAGAUUCUCUUCC | | [1106-1124] ORF |
| 219 | ACAACUACAUGUGUAACAG | CUGUUACACAUGUAGUUGU | MK,Dog | [952-970] ORF |
| 220 | GCCAUCUACAAGCAGUCAC | GUGACUGCUUGUAGAUGGC | MK | [732-750] ORF |
| 221 | UCCUUCUCCCUUUUUAUAU | AUAUAAAAAGGGAGAAGGA | | [2564-2582] 3'UTR |
| 222 | CCUCCUUCUCCCUUUUUAU | AUAAAAAGGGAGAAGGAGG | | [2562-2580] 3'UTR |
| 223 | GCCAAACCCUGUCUGACAA | UUGUCAGACAGGGUUUGGC | | [2047-2065] 3'UTR |
| 224 | CAGGACUUCCAUUUGCUUU | AAAGCAAAUGGAAGUCCUG | | [1551-1569] 3'UTR |
| 225 | UGAAGUCCAAAAAGGGUCA | UGACCCUUUUUGGACUUCA | | [1357-1375] ORF |
| 226 | UGAGUCAGGAAACAUUUUC | GAAAAUGUUUCCUGACUCA | MK | [292-310] ORF |
| 227 | GAGAGCUGAAUGAGGCCUU | AAGGCCUCAUUCAGCUCUC | MK,Rat | [1276-1294] ORF |
| 228 | AGAGAAUCUCCGCAAGAAA | UUUCUUGCGGAGAUUCUCU | | [1109-1127] ORF |
| 229 | CACCAUCAUCACACUGGAA | UUCCAGUGUGAUGAUGGUG | Pig,RB | [1007-1025] ORF |
| 230 | CUGCUCAGAUAGCGAUGGU | ACCAUCGCUAUCUGAGCAG | | [794-812] ORF |
| 231 | CUCCUUCUCCCUUUUUAUA | UAUAAAAAGGGAGAAGGAG | | [2563-2581] 3'UTR |
| 232 | UGAUCUGGAUCCACCAAGA | UCUUGGUGGAUCCAGAUCA | | [2144-2162] 3'UTR |
| 233 | CCCACUUCACCGUACUAAC | GUUAGUACGGUGAAGUGGG | | [1754-1772] 3'UTR |
| 234 | GCCUGACUCAGACUGACAU | AUGUCAGUCUGAGUCAGGC | | [1418-1436] ORF+3'UTR |
| 235 | CUGAGUCAGGAAACAUUUU | AAAAUGUUUCCUGACUCAG | MK | [291-309] ORF |
| 236 | AGGAAGAGAAUCUCCGCAA | UUGCGGAGAUUCUCUUCCU | | [1105-1123] ORF |
| 237 | GUGUAACAGUUCCUGCAUG | CAUGCAGGAACUGUUACAC | MK,Dog | [962-980] ORF |
| 238 | AACUACAUGUGUAACAGUU | AACUGUUACACAUGUAGUU | MK,Dog | [954-972] ORF |
| 239 | CAACUACAUGUGUAACAGU | ACUGUUACACAUGUAGUUG | MK,Dog | [953-971] ORF |
| 240 | ACAAGAUGUUUUGCCAACU | AGUUGGCAAAACAUCUUGU | | [643-661] ORF |
| 241 | CGAUAUUGAACAAUGGUUC | GAACCAUUGUUCAAUAUCG | | [395-413] ORF |
| 242 | GUACCUAAAAGGAAAUCUC | GAGAUUUCCUUUUAGGUAC | | [2083-2101] 3'UTR |
| 243 | GUCCCAAGCAAUGGAUGAU | AUCAUCCAUUGCUUGGGAC | | [359-377] ORF |
| 244 | CCACCUUUUAUUACAUGGG | CCCAUGUAAUAAAAGGUGG | | [1903-1921] 3'UTR |
| 245 | GGCCUUGAAACCACCUUUU | AAAAGGUGGUUUCAAGGCC | | [1893-1911] 3'UTR |
| 246 | UCUGUGAAAUGCUGGCAUU | AAUGCCAGCAUUUCACAGA | | [1822-1840] 3'UTR |
| 247 | AUGGAAACUACUUCCUGAA | UUCAGGAAGUAGUUUCCAU | MK,GP | [317-335] ORF |
| 248 | CACCGUACUAACCAGGGAA | UUCCCUGGUUAGUACGGUG | | [1761-1779] 3'UTR |
| 249 | AGGACAUACCAGCUUAGAU | AUCUAAGCUGGUAUGUCCU | | [1637-1655] 3'UTR |
| 250 | UGGCCUGCACUGGUGUUUU | AAAACACCAGUGCAGGCCA | | [1594-1612] 3'UTR |
| 251 | GCUUGCAAUAGGUGUGCGU | ACGCACACCUAUUGCAAGC | | [1522-1540] 3'UTR |
| 252 | CCUCCCGCCAUAAAAAACU | AGUUUUUUAUGGCGGGAGG | | [1381-1399] ORF |
| 253 | CUACCUCCCGCCAUAAAAA | UUUUUAUGGCGGGAGGUAG | MK,RB | [1378-1396] ORF |
| 254 | GAGUCAGGAAACAUUUUCA | UGAAAAUGUUUCCUGACUC | MK | [293-311] ORF |
| 255 | AGAGCUGAAUGAGGCCUUG | CAAGGCCUCAUUCAGCUCU | MK,Rat | [1277-1295] ORF |
| 256 | GGAUGGAGAAUAUUUCACC | GGUGAAAUAUUCUCCAUCC | MK | [1220-1238] ORF |
| 257 | CCACUGGAUGGAGAAUAUU | AAUAUUCUCCAUCCAGUGG | MK | [1215-1233] ORF |
| 258 | AAGAGAAUCUCCGCAAGAA | UUCUUGCGGAGAUUCUCUU | | [1108-1126] ORF |
| 259 | UCCGAGUGGAAGGAAAUUU | AAAUUUCCUUCCACUCGGA | MK,Dog | [835-853] ORF |
| 260 | CAUGAGCGCUGCUCAGAUA | UAUCUGAGCAGCGCUCAUG | | [786-804] ORF |
| 261 | ACAAGCAGUCACAGCACAU | AUGUGCUGUGACUGCUUGU | MK | [739-757] ORF |
| 262 | CUAAAAGUUUUGAGCUUCU | AGAAGCUCAAAACUUUUAG | | [93-111] 5'UTR |
| 263 | CAACAAGAUGUUUUGCCAA | UUGGCAAAACAUCUUGUUG | | [641-659] ORF |
| 264 | UCAACAAGAUGUUUUGCCA | UGGCAAAACAUCUUGUUGA | MK | [640-658] ORF |
| 265 | GCCAAGUCUGUGACUUGCA | UGCAAGUCACAGACUUGGC | MK | [606-624] ORF |
| 266 | GUUUCCGUCUGGGCUUCUU | AAGAAGCCCAGACGGAAAC | | [574-592] ORF |
| 267 | ACACCCUGGAGGAUUUCAU | AUGAAAUCCUCCAGGGUGU | | [2112-2130] 3'UTR |
| 268 | CUGUGAAAUGCUGGCAUUU | AAAUGCCAGCAUUUCACAG | | [1823-1841] 3'UTR |
| 269 | AGGACUUCCAUUUGCUUUG | CAAAGCAAAUGGAAGUCCU | | [1552-1570] 3'UTR |
| 270 | UCAGAAGCACCCAGGACUU | AAGUCCUGGGUGCUUCUGA | | [1540-1558] 3'UTR |
| 271 | AGAAUAUUUCACCCUUCAG | CUGAAGGGUGAAAUAUUCU | MK,Dog | [1226-1244] ORF |
| 272 | AACCACUGGAUGGAGAAUA | UAUUCUCCAUCCAGUGGUU | MK | [1213-1231] ORF |
| 273 | CCAGUGGUAAUCUACUGGG | CCCAGUAGAUUACCACUGG | MK | [1030-1048] ORF |
| 274 | CUCCAGUGGUAAUCUACUG | CAGUAGAUUACCACUGGAG | MK | [1028-1046] ORF |
| 275 | CUGGAAGACUCCAGUGGUA | UACCACUGGAGUCUUCCAG | MK | [1020-1038] ORF |
| 276 | CCAUCAUCACACUGGAAGA | UCUUCCAGUGUGAUGAUGG | Pig,RB,MO | [1009-1027] ORF |
| 277 | UUAUCCGAGUGGAAGGAAA | UUUCCUUCCACUCGGAUAA | MK,HM | [832-850] ORF |
| 278 | GCAUCUUAUCCGAGUGGAA | UUCCACUCGGAUAAGAUGC | HM | [827-845] ORF |
| 279 | CAAGCAGUCACAGCACAUG | CAUGUGCUGUGACUGCUUG | MK | [740-758] ORF |
| 280 | GUCUGUGACUUGCACGUAC | GUACGUGCAAGUCACAGAC | MK | [611-629] ORF |
| 281 | CCAAGUCUGUGACUUGCAC | GUGCAAGUCACAGACUUGG | MK | [607-625] ORF |
| 282 | GCGCUAAAAGUUUUGAGCU | AGCUCAAAACUUUUAGCGC | GP | [90-108] 5'UTR |
| 283 | UCUGUCCCUUCCCAGAAAA | UUUUCUGGGAAGGGACAGA | MK | [537-555] ORF |
| 284 | UGAAGACCCAGGUCCAGAU | AUCUGGACCUGGGUCUUCA | MK | [416-434] ORF |
| 285 | ACAUUCUGCAAGCACAUCU | AGAUGUGCUUGCAGAAUGU | | [2522-2540] 3'UTR |
| 286 | UCCCAAGCAAUGGAUGAUU | AAUCAUCCAUUGCUUGGGA | | [360-378] ORF |
| 287 | UGGGUUGGUAGUUUCUACA | UGUAGAAACUACCAACCCA | | [1980-1998] 3'UTR |
| 288 | GUGGGUUGGUAGUUUCUAC | GUAGAAACUACCAACCCAC | | [1979-1997] 3'UTR |
| 289 | UCCAAAAAGGGUCAGUCUA | UAGACUGACCCUUUUUGGA | | [1362-1380] ORF |
| 290 | GCUUCGAGAUGUUCCGAGA | UCUCGGAACAUCUCGAAGC | MK,Pig,Rat | [1261-1279] ORF |
| 291 | AGAAACCACUGGAUGGAGA | UCUCCAUCCAGUGGUUUCU | MK | [1210-1228] ORF |
| 292 | GAAGAGAAUCUCCGCAAGA | UCUUGCGGAGAUUCUCUUC | | [1107-1125] ORF |
| 293 | GAACAGCUUUGAGGUGCGU | ACGCACCUCAAAGCUGUUC | Pig | [1052-1070] ORF |
| 294 | CAGUGGUAAUCUACUGGGA | UCCCAGUAGAUUACCACUG | MK | [1031-1049] ORF |
| 295 | GAAGACUCCAGUGGUAAUC | GAUUACCACUGGAGUCUUC | MK | [1023-1041] ORF |
| 296 | CACUUUUCGACAUAGUGUG | CACACUAUGUCGAAAAGUG | MK | [881-899] ORF |
| 297 | UGGCCAUCUACAAGCAGUC | GACUGCUUGUAGAUGGCCA | MK | [730-748] ORF |
| 298 | GGCGCUAAAAGUUUUGAGC | GCUCAAAACUUUUAGCGCC | GP | [89-107] 5'UTR |
| 299 | AGACCCAGGUCCAGAUGAA | UUCAUCUGGACCUGGGUCU | MK | [419-437] ORF |
| 300 | GGAUCCACCAAGACUUGUU | AACAAGUCUUGGUGGAUCC | | [2150-2168] 3'UTR |
| 301 | GGAGGAUUUCAUCUCUUGU | ACAAGAGAUGAAAUCCUCC | | [2119-2137] 3'UTR |
| 302 | UGGAAACUACUUCCUGAAA | UUUCAGGAAGUAGUUUCCA | MK,GP | [318-336] ORF |
| 303 | CCUAUGGAAACUACUUCCU | AGGAAGUAGUUUCCAUAGG | MK | [314-332] ORF |
| 304 | CCACUGAACAAGUUGGCCU | AGGCCAACUUGUUCAGUGG | | [1581-1599] 3'UTR |
| 305 | UGAACCCUUGCUUGCAAUA | UAUUGCAAGCAAGGGUUCA | | [1513-1531] 3'UTR |
| 306 | GGUCUUUGAACCCUUGCUU | AAGCAAGGGUUCAAAGACC | | [1507-1525] 3'UTR |
| 307 | CAGCUUUGAGGUGCGUGUU | AACACGCACCUCAAAGCUG | Pig | [1055-1073] ORF |
| 308 | ACCAUCAUCACACUGGAAG | CUUCCAGUGUGAUGAUGGU | Pig,RB,MO | [1008-1026] ORF |
| 309 | UCACCAUCAUCACACUGGA | UCCAGUGUGAUGAUGGUGA | Pig,RB | [1006-1024] ORF |
| 310 | CACUACAACUACAUGUGUA | UACACAUGUAGUUGUAGUG | MK,Dog | [948-966] ORF |
| 311 | AUAGUGUGGUGGUGCCCUA | UAGGGCACCACCACACUAU | MK | [892-910] ORF |
| 312 | ACAGCCAAGUCUGUGACUU | AAGUCACAGACUUGGCUGU | MK | [603-621] ORF |
| 313 | UUCUGUCCCUUCCCAGAAA | UUUCUGGGAAGGGACAGAA | | [536-554] ORF |
| 314 | UCUGGAUCCACCAAGACUU | AAGUCUUGGUGGAUCCAGA | | [2147-2165] 3'UTR |
| 315 | CCCUGGAGGAUUUCAUCUC | GAGAUGAAAUCCUCCAGGG | | [2115-2133] 3'U'TR |
| 316 | CUUAGUACCUAAAAGGAAA | UUUCCUUUUAGGUACUAAG | | [2079-2097] 3'UTR |
| 317 | GGUCGACCUUAGUACCUAA | UUAGGUACUAAGGUCGACC | | [2072-2090] 3'UTR |
| 318 | CCAAACCCUGUCUGACAAC | GUUGUCAGACAGGGUUUGG | | [2048-2066] 3'UTR |
| 319 | CUGAAAACAACGUUCUGUC | GACAGAACGUUGUUUUCAG | GP | [331-349] ORF |
| 320 | GGAGUAGGACAUACCAGCU | AGCUGGUAUGUCCUACUCC | | [1632-1650] 3'UTR |
| 321 | UGCAAUAGGUGUGCGUCAG | CUGACGCACACCUAUUGCA | | [1525-1543] 3'UTR |
| 322 | UGCUCAAGACUGGCGCUAA | UUAGCGCCAGUCUUGAGCA | | [78-96] 5'UTR |
| 323 | GCCAUUUUGGGUUUUGGGU | ACCCAAAACCCAAAAUGGC | | [1491-1509] 3'UTR |
| 324 | CCCUCUGAGUCAGGAAACA | UGUUUCCUGACUCAGAGGG | MK | [287-305] ORF |
| 325 | CCAAAGAAGAAACCACUGG | CCAGUGGUUUCUUCUUUGG | MK,GP | [1203-1221] ORF |
| 326 | AGAAAACCUACCAGGGCAG | CUGCCCUGGUAGGUUUUCU | | [550-568] ORF |
| 327 | UGAACAAUGGUUCACUGAA | UUCAGUGAACCAUUGUUCA | | [401-419] ORF |
| 328 | GAGCUGGAAGGGUCAACAU | AUGUUGACCCUUCCAGCUC | | [2498-2516] 3'UTR |
| 329 | GAUCCACCAAGACUUGUUU | AAACAAGUCUUGGUGGAUC | | [2151-2169] 3'UTR |
| 330 | ACCUCUUGGUCGACCUUAG | CUAAGGUCGACCAAGAGGU | | [2065-2083] 3'UTR |
| 331 | ACCUCACAGAGUGCAUUGU | ACAAUGCACUCUGUGAGGU | | [1848-1866] 3'UTR |
| 332 | CUACCUCACAGAGUGCAUU | AAUGCACUCUGUGAGGUAG | | [1846-1864] 3'UTR |
| 333 | GUACUAACCAGGGAAGCUG | CAGCUUCCCUGGUUAGUAC | | [1765-1783] 3'UTR |
| 334 | GGGUCUUUGAACCCUUGCU | AGCAAGGGUUCAAAGACCC | | [1506-1524] 3'UTR |
| 335 | CUCAUGUUCAAGACAGAAG | CUUCUGUCUUGAACAUGAG | | [1398-1416] ORF |
| 336 | AGCUGAAUGAGGCCUUGGA | UCCAAGGCCUCAUUCAGCU | MK,Rat | [1279-1297] ORF |
| 337 | ACUACAACUACAUGUGUAA | UUACACAUGUAGUUGUAGU | MK,Dog | [949-967] ORF |
| 338 | UGGUCGACCUUAGUACCUA | UAGGUACUAAGGUCGACCA | | [2071-2089] 3'UTR |
| 339 | UGGCCUUGAAACCACCUUU | AAAGGUGGUUUCAAGGCCA | | [1892-1910] 3'UTR |
| 340 | CUGGCCUUGAAACCACCUU | AAGGUGGUUUCAAGGCCAG | | [1891-1909] 3'UTR |
| 341 | GCCUGCACUGGUGUUUUGU | ACAAAACACCAGUGCAGGC | | [1596-1614] 3'UTR |
| 342 | UGAACAAGUUGGCCUGCAC | GUGCAGGCCAACUUGUUCA | | [1585-1603] 3'UTR |
| 343 | ACCCUUGCUUGCAAUAGGU | ACCUAUUGCAAGCAAGGGU | | [1516-1534] 3'UTR |
| 344 | CCAGCCAAAGAAGAAACCA | UGGUUUCUUCUUUGGCUGG | MK | [1199-1217] ORF |
| 345 | CCCAGCCAAAGAAGAAACC | GGUUUCUUCUUUGGCUGGG | MK | [1198-1216] ORF |
| 346 | AGCUUUGAGGUGCGUGUUU | AAACACGCACCUCAAAGCU | Pig | [1056-1074] ORF |
| 347 | UCACACUGGAAGACUCCAG | CUGGAGUCUUCCAGUGUGA | MK,RB,MO | [1015-1033] ORF |
| 348 | GCUUCUCAAAAGUCUAGAG | CUCUAGACUUUUGAGAAGC | | [106-124] 5'UTR |
| 349 | CUCUGACUGUACCACCAUC | GAUGGUGGUACAGUCAGAG | MK,HM,Pig,RB | [929-947] ORF |
| 350 | AGCAUCUUAUCCGAGUGGA | UCCACUCGGAUAAGAUGCU | HM | [826-844] ORF |
| 351 | UCAGCAUCUUAUCCGAGUG | CACUCGGAUAAGAUGCUGA | HM | [824-842] ORF |
| 352 | AAAGUUUUGAGCUUCUCAA | UUGAGAAGCUCAAAACUUU | | [96-114] 5'UTR |
| 353 | AGCAGUCACAGCACAUGAC | GUCAUGUGCUGUGACUGCU | MK | [742-760] ORF |
| 354 | GGCCAUCUACAAGCAGUCA | UGACUGCUUGUAGAUGGCC | MK | [731-749] ORF |
| 355 | GCUGUGGGUUGAUUCCACA | UGUGGAAUCAACCCACAGC | | [683-701] ORF |
| 356 | AGCUGUGGGUUGAUUCCAC | GUGGAAUCAACCCACAGCU | | [682-700] ORF |
| 357 | UCCCUUCCCAGAAAACCUA | UAGGUUUUCUGGGAAGGGA | MK | [541-559] ORF |
| 358 | CCUACCUCACAGAGUGCAU | AUGCACUCUGUGAGGUAGG | | [1845-1863] 3'UTR |
| 359 | ACCGUACUAACCAGGGAAG | CUUCCCUGGUUAGUACGGU | | [1762-1780] 3'UTR |
| 360 | UCACCGUACUAACCAGGGA | UCCCUGGUUAGUACGGUGA | | [1760-1778] 3'UTR |
| 361 | CUGAACAAGUUGGCCUGCA | UGCAGGCCAACUUGUUCAG | | [1584-1602] 3'UTR |
| 362 | GCCUUGGAACUCAAGGAUG | CAUCCUUGAGUUCCAAGGC | MK | [1290-1308] ORF |
| 363 | CAGCCAAAGAAGAAACCAC | GUGGUUUCUUCUUUGGCUG | MK,GP | [1200-1218] ORF |
| 364 | ACAGCUUUGAGGUGCGUGU | ACACGCACCUCAAAGCUGU | Pig | [1054-1072] ORF |
| 365 | UCCACUACAACUACAUGUG | CACAUGUAGUUGUAGUGGA | MK,Dog,GP | [946-964] ORF |
| 366 | UGAGCUUCUCAAAAGUCUA | UAGACUUUUGAGAAGCUCA | | [103-121] 5'UTR |
| 367 | CUCAGCAUCUUAUCCGAGU | ACUCGGAUAAGAUGCUGAG | HM | [823-841] ORF |
| 368 | CAAGCACAUCUGCAUUUUC | GAAAAUGCAGAUGUGCUUG | | [2530-2548] 3'UTR |
| 369 | UGCAAGCACAUCUGCAUUU | AAAUGCAGAUGUGCUUGCA | | [2528-2546] 3'UTR |
| 370 | CCUUAGUACCUAAAAGGAA | UUCCUUUUAGGUACUAAGG | | [2078-2096] 3'UTR |
| 371 | UAAGAAAUGUUCUUGCAGU | ACUGCAAGAACAUUUCUUA | | [1692-1710] 3'UTR |
| 372 | CCCUUGCUUGCAAUAGGUG | CACCUAUUGCAAGCAAGGG | | [1517-1535] 3'UTR |
| 373 | GUUUUGAGCUUCUCAAAAG | CUUUUGAGAAGCUCAAAAC | | [99-117] 5'UTR |
| 374 | UCCUCAGCAUCUUAUCCGA | UCGGAUAAGAUGCUGAGGA | HM | [821-839] ORF |
| 375 | CUCACAGAGUGCAUUGUGA | UCACAAUGCACUCUGUGAG | | [1850-1868] 3'UTR |
| 376 | UGUAAGAAAUGUUCUUGCA | UGCAAGAACAUUUCUUACA | | [1690-1708] 3'UTR |
| 377 | GGCUCCACUGAACAAGUUG | CAACUUGUUCAGUGGAGCC | | [1577-1595] 3'UTR |
| 378 | UCGAGAUGUUCCGAGAGCU | AGCUCUCGGAACAUCUCGA | MK,Pig,Rat,RB | [1264-1282] ORF |
| 379 | CAACCUCUUGGUCGACCUU | AAGGUCGACCAAGAGGUUG | | [2063-2081] 3'UTR |
| 360 | UUGAACCCUUGCUUGCAAU | AUUGCAAGCAAGGGUUCAA | | [1512-1530] 3'UTR |
| 381 | UCCAGAUGAAGCUCCCAGA | UCUGGGAGCUUCAUCUGGA | | [428-446] ORF |
| 382 | ACAACCUCUUGGUCGACCU | AGGUCGACCAAGAGGUUGU | | [2062-2080] 3'UTR |
| 383 | GGCUUCUUGCAUUCUGGGA | UCCCAGAAUGCAAGAAGCC | | [585-603] ORF |
| 384 | UCUGCAAGCACAUCUGCAU | AUGCAGAUGUGCUUGCAGA | | [2526-2544] 3'UTR |
| 385 | CUGACAACCUCUUGGUCGA | UCGACCAAGAGGUUGUCAG | | [2059-2077] 3'UTR |
| 386 | CUGCAAGCACAUCUGCAUU | AAUGCAGAUGUGCUUGCAG | | [2527-2545] 3'UTR |
| 387 | CUGGGCUUCUUGCAUUCUG | CAGAAUGCAAGAAGCCCAG | | [582-600] ORF |
| 388 | CAGAAAACCUACCAGGGCA | UGCCCUGGUAGGUUUUCUG | | [549-567] ORF |
| 389 | GUCUGGGCUUCUUGCAUUC | GAAUGCAAGAAGCCCAGAC | | [580-598] ORF |
| 390 | CCUCUGAGUCAGGAAACAU | AUGUUUCCUGACUCAGAGG | MK | [288-306] ORF |
| 391 | AGGCCUUGGAACUCAAGGA | UCCUUGAGUUCCAAGGCCU | MK | [1288-1306] ORF |

**Table B 21-mer P53 - tumor protein p53**

| Number | Sense siRNA | AntiSense siRNA | Other Sp | Human 8400737 ORF:252-1433 |
|---|---|---|---|---|
| 1 | CCAGCUUAGAUUUUAAGGUUU | AAACCUUAAAAUCUAAGCUGG | | [1645-1665] 3'UTR |
| 2 | CCCAUUUUUAUAUCGAUCUCU | AGAGAUCGAUAUAAAAAUGGG | | [2583-2603] 3'UTR |
| 3 | GGACAUACCAGCUUAGAUUUU | AAAAUCUAAGCUGGUAUGUCC | | [1638-1658] 3'UTR |
| 4 | UCCCAUUUUUAUAUCGAUCUC | GAGAUCGAUAUAAAAAUGGGA | | [2582-2602] 3'UTR |
| 5 | CUCCCUUUUUAUAUCCCAUUU | AAAUGGGAUAUAAAAAGGGAG | | [2569-2589] 3'UTR |
| 6 | GCAUUGUGAGGGUUAAUGAAA | UUUCAUUAACCCUCACAAUGC | | [1860-1880] 3'UTR |
| 7 | CCAUUUUGGGUUUUGGGUCUU | AAGACCCAAAACCCAAAAUGG | | [1492-1512] 3'UTR |
| 8 | CCCUGUCAUCUUCUGUCCCUU | AAGGGACAGAAGAUGACAGGG | | [526-546] ORF |
| 9 | GGAUUUCAUCUCUUGUAUAUG | CAUAUACAAGAGAUGAAAUCC | | [2122-2142] 3'UTR |
| 10 | CCUCAUGUUGAAUUUUCUCUA | UAGAGAAAAUUCAACAUGAGG | | [1786-1806] 3'UTR |
| 11 | CAGUUAAGGGUUAGUUUACAA | UUGUAAACUAACCCUUAACUG | | [1707-1727] 3'UTR |
| 12 | GACAUACCAGCUUAGAUUUUA | UAAAAUCUAAGCUGGUAUGUC | | [1639-1659] 3'UTR |
| 13 | CCCUUCCCAGAAAACCUACCA | UGGUAGGUUUUCUGGGAAGGG | MK | [542-562] ORF |
| 14 | CUUGUAUAUGAUGAUCUGGAU | AUCCAGAUCAUCAUAUACAAG | | [2133-2153] 3'UTR |
| 15 | CUACUUCCUGAAAACAACGUU | AACGUUGUUUUCAGGAAGUAG | GP | [324-344] ORF |
| 16 | GGCCCAUAUCUGUGAAAUGCU | AGCAUUUCACAGAUAUGGGCC | | [1814-1834] 3'UTR |
| 17 | CUCUAACUUCAAGGCCCAUAU | AUAUGGGCCUUGAAGUUAGAG | | [1802-1822] 3'UTR |
| 18 | GCUGUCCCUCAUGUUGAAUUU | AAAUUCAACAUGAGGGACAGC | | [1780-1800] 3'UTR |
| 19 | UAGUUUACAAUCAGCCACAUU | AAUGUGGCUGAUUGUAAACUA | | [1718-1738] 3'UTR |
| 20 | GGAAACAUUUUCAGACCUAUG | CAUAGGUCUGAAAAUGUUUCC | MK | [299-319] ORF |
| 21 | UGCCCUCAACAAGAUGUUUUG | CAAAACAUCUUGUUGAGGGCA | | [635-655] ORF |
| 22 | GGUCAACAUCUUUUACAUUCU | AGAAUGUAAAAGAUGUUGACC | | [2508-2528] 3'UTR |
| 23 | AGGGUCAACAUCUUUUACAUU | AAUGUAAAAGAUGUUGACCCU | | [2506-2526] 3'UTR |
| 24 | GAGUGCAUUGUGAGGGUUAAU | AUUAACCCUCACAAUGCACUC | | [1856-1876] 3'UTR |
| 25 | GUUUACAAUCAGCCACAUUCU | AGAAUGUGGCUGAUUGUAAAC | | [1720-1740] 3'UTR |
| 26 | GGGUUAGUUUACAAUCAGCCA | UGGCUGAUUGUAAACUAACCC | | [1714-1734] 3'UTR |
| 27 | GGAGUAUUUGGAUGACAGAAA | UUUCUGUCAUCCAAAUACUCC | RB | [860-880] ORF |
| 28 | GUGAGGGUUAAUGAAAUAAUG | CAUUAUUUCAUUAACCCUCAC | | [1865-1885] 3'UTR |
| 29 | GUGCAUUGUGAGGGUUAAUGA | UCAUUAACCCUCACAAUGCAC | | [1858-1878] 3'UTR |
| 30 | UUAGUUUACAAUCAGCCACAU | AUGUGGCUGAUUGUAAACUAA | | [1717-1737] 3'UTR |
| 31 | AGAAAUGUUCUUGCAGUUAAG | CUUAACUGCAAGAACAUUUCU | | [1694-1714] 3'UTR |
| 32 | ACAUUUUCAGACCUAUGGAAA | UUUCCAUAGGUCUGAAAAUGU | MK | [303-323] ORF |
| 33 | UCAGACUGACAUUCUCCACUU | AAGUGGAGAAUGUCAGUCUGA | | [1425-1445] ORF+3'UTR |
| 34 | AGGAAACAUUUUCAGJ1CCUAU | AUAGGUCUGAAAAUGUUUCCU | MK | [298-318] ORF |
| 35 | CCCGGACGAUAUUGAACAAUG | CAUUGUUCAAUAUCGUCCGGG | | [389-409] ORF |
| 36 | CCACCAAGACUUGUUUUAUGC | GCAUAAAACAAGUCUUGGUGG | | [2154-2174] 3'UTR |
| 37 | UCUCUUGUAUAUGAUGAUCUG | CAGAUCAUCAUAUACAAGAGA | | [2130-2150] 3'UTR |
| 38 | CCUUGAAACCACCUUUUAUUA | UAAUAAAAGGUGGUUUCAAGG | | [1895-1915] 3'UTR |
| 39 | UGCAUUGUGAGGGUUAAUGAA | UUCAUUAACCCUCACAAUGCA | | [1859-1879] 3'UTR |
| 40 | GUCCCUCAUGUUGAAUUUUCU | AGAAAAUUCAACAUGAGGGAC | | [1783-1803] 3'UTR |
| 41 | CUGUCCCUCAUGUUGAAUUUU | AAAAUUCAACAUGAGGGACAG | | [1781-1801] 3'UTR |
| 42 | GCAGUUAAGGGUUAGUUUACA | UGUAAACUAACCCUUAACUGC | | [1706-1726] 3'UTR |
| 43 | CCUGUCAUCUUCUGUCCCUUC | GAAGGGACAGAAGAUGACAGG | | [527-547] ORF |
| 44 | GGACGAUAUUGAACAAUGGUU | AACCAUUGUUCAAUAUCGUCC | | [392-412] ORF |
| 45 | CGGACGAUAUUGAACAAUGGU | ACCAUUGUUCAAUAUCGUCCG | | [391-411] ORF |
| 46 | UCUCCCUUUUUAUAUCCCAUU | AAUGGGAUAUAAAAAGGGAGA | | [2568-2588] 3'UTR |
| 47 | CAACAUCUUUUACAUUCUGCA | UGCAGAAUGUAAAAGAUGUUG | | [2511-2531] 3'UTR |
| 48 | GUCAACAUCUUUUACAUUCUG | CAGAAUGUAAAAGAUGUUGAC | | [2509-2529] 3'UTR |
| 49 | GCCUUGAAACCACCUUUUAUU | AAUAAAAGGUGGUUUCAAGGC | | [1894-1914] 3'UTR |
| 50 | GCCCAUAUCUGUGAAAUGCUG | CAGCAUUUCACAGAUAUGGGC | | [1815-1835] 3'UTR |
| 51 | GAAACUACUUCCUGAAAACAA | UUGUUUUCAGGAAGUAGUUUC | GP | [320-340] ORF |
| 52 | UAAGGGUUAGUUUACAAUCAG | CUGAUUGUAAACUAACCCUUA | | [1711-1731] 3'UTR |
| 53 | CCUGACUCAGACUGACAUUCU | AGAAUGUCAGUCUGAGUCAGG | | [1419-1439] ORF+3'UTR |
| 54 | CGAGAUGUUCCGAGAGCUGAA | UUCAGCUCUCGGAACAUCUCG | MK,Pig,Rat,RB | [1265-1285] ORF |
| 55 | GGUAAUCUACUGGGACGGAAC | GUUCCGUCCCAGUAGAUUACC | MK | [1035-1055] ORF |
| 56 | GGAAGGAAAUUUGCGUGUGGA | UCCACACGCAAAUUUCCUUCC | MK | [842-862] ORF |
| 57 | CCGGACGAUAUUGAACAAUGG | CCAUUGUUCAAUAUCGUCCGG | | [390-410] ORF |
| 58 | GGGUCAACAUCUUUUACAUUC | GAAUGUAAAAGAUGUUGACCC | | [2507-2527] 3'UTR |
| 59 | GGAAGGGUCAACAUCUUUUAC | GUAAAAGAUGUUGACCCUUCC | | [2503-2523] 3'UTR |
| 60 | UGGAAGGGUCAACAUCUUUUA | UAAAAGAUGUUGACCCUUCCA | | [2502-2522] 3'UTR |
| 61 | CACCAAGACUUGUUUUAUGCU | AGCAUAAAACAAGUCUUGGUG | | [2155-2175] 3'UTR |
| 62 | GCUGGUGGGUUGGUAGUUUCU | AGAAACUACCAACCCACCAGC | | [1975-1995] 3'UTR |
| 63 | CAGAGUGCAUUGUGAGGGUUA | UAACCCUCACAAUGCACUCUG | | [1854-1874] 3'UTR |
| 64 | GAAUUUUCUCUAACUUCAAGG | CCUUGAAGUUAGAGAAAAUUC | | [1795-1815] 3'UTR |
| 65 | CCCUCAUGUUGAAUUUUCUCU | AGAGAAAAUUCAACAUGAGGG | | [1785-1805] 3'UTR |
| 66 | UCCCUCAUGUUGAAUUUUCUC | GAGAAAAUUCAACAUGAGGGA | | [1784-1804] 3'UTR |
| 67 | UCAGACCUAUGGAAACUACUU | AAGUAGUUUCCAUAGGUCUGA | MK | [309-329] ORF |
| 68 | GCGUGUGGAGUAUUUGGAUGA | UCAUCCAAAUACUCCACACGC | | [854-874] ORF |
| 69 | GGAAAUUUGCGUGUGGAGUAU | AUACUCCACACGCAAAUUUCC | MK | [846-866] ORF |
| 70 | AAGGGUCAACAUCUUUUACAU | AUGUAAAAGAUGUUGACCCUU | | [2505-2525] 3'UTR |
| 71 | GGGAGUUGUCAAGUCUUGCUG | CAGCAAGACUUGACAACUCCC | | [2021-2041] 3'UTR |
| 72 | AGAGUGCAUUGUGAGGGUUAA | UUAACCCUCACAAUGCACUCU | | [1855-1875] 3'UTR |
| 73 | ACAGAGUGCAUUGUGAGGGUU | AACCCUCACAAUGCACUCUGU | | [1853-1873] 3'UTR |
| 74 | CAAGGCCCAUAUCUGUGAAAU | AUUUCACAGAUAUGGGCCUUG | | [1811-1831] 3'UTR |
| 75 | GAGGGAUGUUUGGGAGAUGUA | UACAUCUCCCAAACAUCCCUC | | [1673-1693] 3'UTR |
| 76 | CAUUUUGGGUUUUGGGUCUUU | AAAGACCCAAAACCCAAAAUG | | [1493-1513] 3'UTR |
| 77 | UGACAUUCUCCACUUCUUGUU | AACAAGAAGUGGAGAAUGUCA | | [1431-1451] ORF+3'UTR |
| 78 | CACUGGAUGGAGAAUAUUUCA | UGAAAUAUUCUCCAUCCAGUG | MK | [1216-1236] ORF |
| 79 | CACCAUCCACUACAACUACAU | AUGUAGUUGUAGUGGAUGGUG | MK,Dog,GP | [941-961] ORF |
| 80 | GAGUAUUUGGAUGACAGAAAC | GUUUCUGUCAUCCAAAUACUC | RB | [861-881] ORF |
| 81 | GAAAUUUGCGUGUGGAGUAUU | AAUACUCCACACGCAAAUUUC | MK | [847-867] ORF |
| 82 | AGACUGGCGCUAAAAGUUUUG | CAAAACUUUUAGCGCCAGUCU | GP | [84-104] 5'UTR |
| 83 | UUCUCCCUUUUUAUAUCCCAU | AUGGGAUAUAAAAAGGGAGAA | | [2567-2587] 3'UTR |
| 84 | UCAACAUCUUUUACAUUCUGC | GCAGAAUGUAAAAGAUGUUGA | | [2510-2530] 3'UTR |
| 85 | UCAAGACUGGCGCUAAAAGUU | AACUUUUAGCGCCAGUCUUGA | | [81-101] 5'UTR |
| 86 | UCAAGGCCCAUAUCUGUGAAA | UUUCACAGAUAUGGGCCUUGA | | [1810-1830] 3'UTR |
| 87 | GACUGACAUUCUCCACUUCUU | AAGAAGUGGAGAAUGUCAGUC | | [1428-1448] ORF+3'UTR |
| 88 | CAGACUGACAUUCUCCACUUC | GAAGUGGAGAAUGUCAGUCUG | | [1426-1446] ORF+3'UTR |
| 89 | GCGCACAGAGGAAGAGAAUCU | AGAUUCUCUUCCUCUGUGCGC | | [1097-1117] ORF |
| 90 | UGGUAAUCUACUGGGACGGAA | UUCCGUCCCAGUAGAUUACCA | MK | [1034-1054] ORF |
| 91 | GUGGUAAUCUACUGGGACGGA | UCCGUCCCAGUAGAUUACCAC | MK | [1033-1053] ORF |
| 92 | GUGGAGUAUUUGGAUGACAGA | UCUGUCAUCCAAAUACUCCAC | | [858-878] ORF |
| 93 | CGAGUGGAAGGAAAUUUGCGU | ACGCAAAUUUCCUUCCACUCG | MK | [837-857] ORF |
| 94 | ACAGCACAUGACGGAGGUUGU | ACAACCUCCGUCAUGUGCUGU | | [749-769] ORF |
| 95 | CAAGACUGGCGCUAAAAGUUU | AAACUUUUAGCGCCAGUCUUG | | [82-102] 5'UTR |
| 96 | CAAGCAAUGGAUGAUUUGAUG | CAUCAAAUCAUCCAUUGCUUG | | [363-383] ORF |
| 97 | UGGCUGGUGGGUUGGUAGUUU | AAACUACCAACCCACCAGCCA | | [1973-1993] 3'UTR |
| 98 | AAGCUGUCCCUCAUGUUGAAU | AUUCAACAUGAGGGACAGCUU | | [1778-1798] 3'UTR |
| 99 | CUUGCAGUUAAGGGUUAGUUU | AAACUAACCCUUAACUGCAAG | | [1703-1723] 3'UTR |
| 100 | UUGGGAGAUGUAAGAAAUGUU | AACAUUUCUUACAUCUCCCAA | | [1682-1702] 3'UTR |
| 101 | UGUUUGGGAGAUGUAAGAAAU | AUUUCUUACAUCUCCCAAACA | | [1679-1699] 3'UTR |
| 102 | GGAUGUUUGGGAGAUGUAAGA | UCUUACAUCUCCCAAACAUCC | | [1676-1696] 3'UTR |
| 103 | CCUGCACUGGUGUUUUGUUGU | ACAACAAAACACCAGUGCAGG | | [1597-1617] 3'UTR |
| 104 | AAACAUUUUCAGACCUAUGGA | UCCAUAGGUCUGAAAAUGUUU | MK | [301-321] ORF |
| 105 | GACAUUCUCCACUUCUUGUUC | GAACAAGAAGUGGAGAAUGUC | | [1432-1452] ORF+3'UTR |
| 106 | AGAUGUUCCGAGAGCUGAAUG | CAUUCAGCUCUCGGAACAUCU | MK,Pig,Rat | [1267-1287] ORF |
| 107 | AGUAUUUGGAUGACAGAAACA | UGUUUCUGUCAUCCAAAUACU | RB | [862-882] ORF |
| 108 | GAAGGAAAUUUGCGUGUGGAG | CUCCACACGCAAAUUUCCUUC | MK | [843-863] ORF |
| 109 | CAAUGGAUGAUUUGAUGCUGU | ACAGCAUCAAAUCAUCCAUUG | | [367-387] ORF |
| 110 | GGAGUUGUCAAGUCUUGCUGG | CCAGCAAGACUUGACAACUCC | | [2022-2042] 3'UTR |
| 111 | GUAGAGGGAGUUGUCAAGUCU | AGACUUGACAACUCCCUCUAC | | [2016-2036] 3'UTR |
| 112 | GCUGGUUAGGUAGAGGGAGUU | AACUCCCUCUACCUAACCAGC | | [2007-2027] 3'UTR |
| 113 | GAAAUAAUGUACAUCUGGCCU | AGGCCAGAUGUACAUUAUUUC | | [1677-1897] 3'UTR |
| 114 | UGAAAUAAUGUACAUCUGGCC | GGCCAGAUGUACAUUAUUUCA | | [1876-1896] 3'UTR |
| 115 | GAUGUUUGGGAGAUGUAAGAA | UUCUUACAUCUCCCAAACAUC | | [1677-1697] 3'UTR |
| 116 | GGGAUGUUUGGGAGAUGUAAG | CUUACAUCUCCCAAACAUCCC | | [1675-1695] 3'UTR |
| 117 | UGAGGGAUGUUUGGGAGAUGU | ACAUCUCCCAAACAUCCCUCA | | [1672-1692] 3'UTR |
| 118 | GUGAGGGAUGUUUGGGAGAUG | CAUCUCCCAAACAUCCCUCAC | | [1671-1691] 3'UTR |
| 119 | ACUGACAUUCUCCACUUCUUG | CAAGAAGUGGAGAAUGUCAGU | | [1429-1449] ORF+3'UTR |
| 120 | GAGAUGUUCCGAGAGCUGAAU | AUUCAGCUCUCGGAACAUCUC | MK,Pig,Rat | [1266-1286] ORF |
| 121 | CAAAGAAGAAACCACUGGAUG | CAUCCAGUGGUUUCUUCUUUG | MK,GP | [1204-1224] ORF |
| 122 | UGGAGUAUUUGGAUGACAGAA | UUCUGUCAUCCAAAUACUCCA | | [859-879] ORF |
| 123 | GUGUGGAGUAUUUGGAUGACA | UGUCAUCCAAAUACUCCACAC | | [856-876] ORF |
| 124 | CGUGUGGAGUAUUUGGAUGAC | GUCAUCCAAAUACUCCACACG | | [855-875] ORF |
| 125 | GUGGAAGGAAAUUUGCGUGUG | CACACGCAAAUUUCCUUCCAC | MK | [840-860] ORF |
| 126 | CUGGAAGGGUCAACAUCUUUU | AAAAGAUGUUGACCCUUCCAG | | [2501-2521] 3'UTR |
| 127 | UCUUGCAGUUAAGGGUUAGUU | AACUAACCCUUAACUGCAAGA | | [1702-1722] 3'UTR |
| 128 | UGGGAGAUGUAAGAAAUGUUC | GAACAUUUCUUACAUCUCCCA | | [1683-1703] 3'UTR |
| 129 | AGGGAUGUUUGGGAGAUGUAA | UUACAUCUCCCAAACAUCCCU | | [1674-1694] 3'UTR |
| 130 | GCACUGGUGUUUUGUUGUGGG | CCCACAACAAAACACCAGUGC | | [1600-1620] 3'UTR |
| 131 | CCACCAUCCACUACAACUACA | UGUAGUUGUAGUGGAUGGUGG | MK,Dog,GP | [940-960] ORF |
| 132 | AGGAAAUUUGCGUGUGGAGUA | UACUCCACACGCAAAUUUCCU | MK | [845-865] ORF |
| 133 | CCGCCAUAAAAAACUCAUGUU | AACAUGAGUUUUUUAUGGCGG | | [1385-1405] ORF |
| 134 | UGAAGUCCAAAAAGGGUCAGU | ACUGACCCUUUUUGGACUUCA | | [1357-1377] ORF |
| 135 | UCAAAAGUCUAGAGCCACCGU | ACGGUGGCUCUAGACUUUUGA | | [111-131] 5'UTR |
| 136 | ACAGAAACACUUUUCGACAUA | UAUGUCGAAAAGUGUUUCUGU | MK | [874-894] ORF |
| 137 | GGUUAGGUAGAGGGAGUUGUC | GACAACUCCCUCUACCUAACC | | [2010-2030] 3'UTR |
| 138 | AAUGUACAUCUGGCCUUGAAA | UUUCAAGGCCAGAUGUACAUU | | [1882-1902] 3'UTR |
| 139 | GAAGUCCAAAAAGGGUCAGUC | GACUGACCCUUUUUGGACUUC | | [1358-1378] ORF |
| 140 | ACCACCAUCCACUACAACUAC | GUAGUUGUAGUGGAUGGUGGU | MK,Dog,GP | [939-959] ORF |
| 141 | GCUUCUUGCAUUCUGGGACAG | CUGUCCCAGAAUGCAAGAAGC | | [586-606] ORF |
| 142 | GGUUGGUAGUUUCUACAGUUG | CAACUGUAGAAACUACCAACC | | [1982-2002] 3'UTR |
| 143 | GGGUUGGUAGUUUCUACAGUU | AACUGUAGAAACUACCAACCC | | [1981-2001] 3'UTR |
| 144 | GGAGUAGGACAUACCAGCUUA | UAAGCUGGUAUGUCCUACUCC | | [1632-1652] 3'UTR |
| 145 | AGCACCCAGGACUUCCAUUUG | CAAAUGGAAGUCCUGGGUGCU | | [1545-1565] 3'UTR |
| 146 | CCCUGCCCUCAACAAGAUGUU | AACAUCUUGUUGAGGGCAGGG | | [632-652] ORF |
| 147 | CUGGUUAGGUAGAGGGAGUUG | CAACUCCCUCUACCUAACCAG | | [2008-2028] 3'UTR |
| 148 | GCAUUUGCACCUACCUCACAG | CUGUGAGGUAGGUGCAAAUGC | | [1836-1856] 3'UTR |
| 149 | GCCAUAAAAAACUCAUGUUCA | UGAACAUGAGUUUUUUAUGGC | | [1387-1407] ORF |
| 150 | CGCCAUAAAAAACUCAUGUUC | GAACAUGAGUUUUUUAUGGCG | | [1386-1406] ORF |
| 151 | AGCCACCUGAAGUCCAAAAAG | CUUUUUGGACUUCAGGUGGCU | | [1350-1370] ORF |
| 152 | CCCAUCCUCACCAUCAUCACA | UGUGAUGAUGGUGAGGAUGGG | Pig,RB | [999-1019] ORF |
| 153 | CCAUCCUCACCAUCAUCACAC | GUGUGAUGAUGGUGAGGAUGG | Pig,RB | [1000-1020] ORF |
| 154 | GCGCUAAAAGUUUUGAGCUUC | GAAGCUCAAAACUUUUAGCGC | | [90-110] 5'UTR |
| 155 | ACCCUGUCUGACAACCUCUUG | CAAGAGGUUGUCAGACAGGGU | | [2052-2072] 3'UTR |
| 156 | UGACAGAAACACUUUUCGACA | UGUCGAAAAGUGUUUCUGUCA | MK,Pig | [872-892] ORF |
| 157 | GGCGCUAAAAGUUUUGAGCUU | AAGCUCAAAACUUUUAGCGCC | | [89-109] 5'UTR |
| 158 | GGAGGAUUUCAUCUCUUGUAU | AUACAAGAGAUGAAAUCCUCC | | [2119-2139] 3'UTR |
| 159 | GGGAGAUGUAAGAAAUGUUCU | AGAACAUUUCUUACAUCUCCC | | [1684-1704] 3'UTR |
| 160 | GGAGAAUAUUUCACCCUUCAG | CUGAAGGGUGAAAUAUUCUCC | MK,Dog | [1224-1244] ORF |
| 161 | GAAGAGAAUCUCCGCAAGAAA | UUUCUUGCGGAGAUUCUCUUC | | [1107-1127] ORF |
| 162 | UCAUGGCGACUGUCCAGCUUU | AAAGCUGGACAGUCGCCAUGA | GP | [6-26] 5'UTR |
| 163 | CCCUCCUUCUCCCUUUUUAUA | UAUAAAAAGGGAGAAGGAGGG | | [2561-2581] 3'UTR |
| 164 | CCCUGGAGGAUUUCAUCUCUU | AAGAGAUGAAAUCCUCCAGGG | | [2115-2135] 3'UTR |
| 165 | CUAUGGAAACUACUUCCUGAA | UUCAGGAAGUAGUUUCCAUAG | MK | [315-335] ORF |
| 166 | GUUUUUACUGUGAGGGAUGUU | AACAUCCCUCACAGUAAAAAC | | [1662-1682] 3'UTR |
| 167 | CUCCCGCCAUAAAAAACUCAU | AUGAGUUUUUUAUGGCGGGAG | | [1382-1402] ORF |
| 168 | AGAAUAUUUCACCCUUCAGAU | AUCUGAAGGGUGAAAUAUUCU | MK,Dog | [1226-1246] ORF |
| 169 | AGAAACCACUGGAUGGAGAAU | AUUCUCCAUCCAGUGGUUUCU | MK | [1210-1230] ORF |
| 170 | AGAGGAAGAGAAUCUCCGCAA | UUGCGGAGAUUCUCUUCCUCU | | [1103-1123] ORF |
| 171 | GACAGAAACACUUUUCGACAU | AUGUCGAAAAGUGUUUCUGUC | MK | [873-893] ORF |
| 172 | CUACAAGCAGUCACAGCACAU | AUGUGCUGUGACUGCUUGUAG | MK | [737-757] ORF |
| 173 | CCUGCCCUCAACAAGAUGUUU | AAACAUCUUGUUGAGGGCAGG | | [633-653] ORF |
| 174 | CCUCCUUCUCCCUUUUUAUAU | AUAUAAAAAGGGAGAAGGAGG | | [2562-2582] 3'UTR |
| 175 | GUAUAUGAUGAUCUGGAUCCA | UGGAUCCAGAUCAUCAUAUAC | | [2136-2156] 3'UTR |
| 176 | AGCCACAUUCUAGGUAGGUAG | CUACCUACCUAGAAUGUGGCU | | [1730-1750] 3'UTR |
| 177 | GUAAGAAAUGUUCUUGCAGUU | AACUGCAAGAACAUUUCUUAC | | [1691-1711] 3'UTR |
| 178 | AGGUUUUUACUGUGAGGGAUG | CAUCCCUCACAGUAAAAACCU | | [1660-1680] 3'UTR |
| 179 | ACCAGCUUAGAUUUUAAGGUU | AACCUUAAAAUCUAAGCUGGU | | [1644-1664] 3'UTR |
| 180 | AGGACAUACCAGCUUAGAUUU | AAAUCUAAGCUGGUAUGUCCU | | [1637-1657] 3'UTR |
| 181 | GUAGGACAUACCAGCUUAGAU | AUCUAAGCUGGUAUGUCCUAC | | [1635-1655] 3'UTR |
| 182 | CAGGACUUCCAUUUGCUUUGU | ACAAAGCAAAUGGAAGUCCUG | | [1551-1571] 3'UTR |
| 183 | CCUCCCGCCAUAAAAAACUCA | UGAGUUUUUUAUGGCGGGAGG | | [1381-1401] ORF |
| 184 | CCAAAAAGGGUCAGUCUACCU | AGGUAGACUGACCCUUUUUGG | | [1363-1383] ORF |
| 185 | CCACUACAACUACAUGUGUAA | UUACACAUGUAGUUGUAGUGG | MK,Dog | [947-967] ORF |
| 186 | CAGAAACACUUUUCGACAUAG | CUAUGUCGAAAAGUGUUUCUG | MK | [875-895] ORF |
| 187 | UCAGCCACAUUCUAGGUAGGU | ACCUACCUAGAAUGUGGCUGA | | [1728-1748] 3'UTR |
| 188 | CCAGGACUUCCAUUUGCUUUG | CAAAGCAAAUGGAAGUCCUGG | | [1550-1570] 3'UTR |
| 189 | CCCAGGACUUCCAUUUGCUUU | AAAGCAAAUGGAAGUCCUGGG | | [1549-1569] 3'UTR |
| 190 | GUGCUCAAGACUGGCGCUAAA | UUUAGCGCCAGUCUUGAGCAC | | [77-97] 5'UTR |
| 191 | GUCUACCUCCCGCCAUAAAAA | UUUUUAUGGCGGGAGGUAGAC | RB | [1376-1396] ORF |
| 192 | CCUGAAGUCCAAAAAGGGUCA | UGACCCUUUUUGGACUUCAGG | | [1355-1375] ORF |
| 193 | GGAAGAGAAUCUCCGCAAGAA | UUCUUGCGGAGAUUCUCUUCC | | [1106-1126] ORF |
| 194 | CACCAUCAUCACACUGGAAGA | UCUUCCAGUGUGAUGAUGGUG | Pig,RB | [1007-1027] ORF |
| 195 | CUCACCAUCAUCACACUGGAA | UUCCAGUGUGAUGAUGGUGAG | Pig,RB | [1005-1025] ORF |
| 196 | CGCUAAAAGUUUUGAGCUUCU | AGAAGCUCAAAACUUUUAGCG | | [91-111] 5'UTR |
| 197 | GAGGAUUUCAUCUCUUGUAUA | UAUACAAGAGAUGAAAUCCUC | | [2120-2140] 3'UTR |
| 198 | GUCCCAAGCAAUGGAUGAUUU | AAAUCAUCCAUUGCUUGGGAC | | [359-379] ORF |
| 199 | UGCUGGCAUUUGCACCUACCU | AGGUAGGUGCAAAUGCCAGCA | | [1831-1851] 3'UTR |
| 200 | GGAAACUACUUCCUGAAAACA | UGUUUUCAGGAAGUAGUUUCC | GP | [319-339] ORF |
| 201 | CAGCCACAUUCUAGGUAGGUA | UACCUACCUAGAAUGUGGCUG | | [1729-1749] 3'UTR |
| 202 | GGACUUCCAUUUGCUUUGUCC | GGACAAAGCAAAUGGAAGUCC | | [1553-1573] 3'UTR |
| 203 | GCAAUAGGUGUGCGUCAGAAG | CUUCUGACGCACACCUAUUGC | | [1526-1546] 3'UTR |
| 204 | GGCCUGACUCAGACUGACAUU | AAUGUCAGUCUGAGUCAGGCC | | [1417-1437] ORF+3'UTR |
| 205 | CCCGCCAUAAAAAACUCAUGU | ACAUGAGUUUUUUAUGGCGGG | | [1384-1404] ORF |
| 206 | UGAGUCAGGAAACAUUUUCAG | CUGAAAAUGUUUCCUGACUCA | MK | [292-312] ORF |
| 207 | GAUGGAGAAUAUUUCACCCUU | AAGGGUGAAAUAUUCUCCAUC | MK,Dog,Rat | [1221-1241] ORF |
| 208 | AAACCACUGGAUGGAGAAUAU | AUAUUCUCCAUCCAGUGGUUU | MK | [1212-1232] ORF |
| 209 | CCAGCCAAAGAAGAAACCACU | AGUGGUUUCUUCUUUGGCUGG | MK | [1199-1219] ORF |
| 210 | AGAAACACUUUUCGACAUAGU | ACUAUGUCGAAAAGUGUUUCU | MK | [876-896] ORF |
| 211 | CAACAAGAUGUUUUGCCAACU | AGUUGGCAAAACAUCUUGUUG | | [641-661] ORF |
| 212 | CUCAACAAGAUGUUUUGCCAA | UUGGCAAAACAUCUUGUUGAG | | [639-659] ORF |
| 213 | GUGGGUUGGUAGUUUCUACAG | CUGUAGAAACUACCAACCCAC | | [1979-1999] 3'UTR |
| 214 | ACAAUCAGCCACAUUCUAGGU | ACCUAGAAUGUGGCUGAUUGU | | [1724-1744] 3'UTR |
| 215 | GGCCUGCACUGGUGUUUUGUU | AACAAAACACCAGUGCAGGCC | | [1595-1615] 3'UTR |
| 216 | UGCUCAAGACUGGCGCUAAAA | UUUUAGCGCCAGUCUUGAGCA | | [78-98] 5'UTR |
| 217 | CAGGAAACAUUUUCAGACCUA | UAGGUCUGAAAAUGUUUCCUG | MK | [297-317] ORF |
| 218 | CAGUCUACCUCCCGCCAUAAA | UUUAUGGCGGGAGGUAGACUG | RB | [1374-1394] ORF |
| 219 | CCACCUGAAGUCCAAAAAGGG | CCCUUUUUGGACUUCAGGUGG | | [1352-1372] ORF |
| 220 | GAGUCAGGAAACAUUUUCAGA | UCUGAAAAUGUUUCCUGACUC | MK | [293-313] ORF |
| 221 | CUGAGUCAGGAAACAUUUUCA | UGAAAAUGUUUCCUGACUCAG | MK | [291-311] ORF |
| 222 | GGAUGGAGAAUAUUUCACCCU | AGGGUGAAAUAUUCUCCAUCC | MK | [1220-1240] ORF |
| 223 | CUCUGAGUCAGGAAACAUUUU | AAAAUGUUUCCUGACUCAGAG | MK | [289-309] ORF |
| 224 | CCAAAGAAGAAACCACUGGAU | AUCCAGUGGUUUCUUCUUUGG | MK,GP | [1203-1223] ORF |
| 225 | GAGGAAGAGAAUCUCCGCAAG | CUUGCGGAGAUUCUCUUCCUC | | [1104-1124] ORF |
| 226 | GGAAGACUCCAGUGGUAAUCU | AGAUUACCACUGGAGUCUUCC | MK | [1022-1042] ORF |
| 227 | CCAUCAUCACACUGGAAGACU | AGUCUUCCAGUGUGAUGAUGG | RB,MO | [1009-1029] ORF |
| 228 | GGAUGACAGAAACACUUUUCG | CGAAAAGUGUUUCUGUCAUCC | MK,Pig | [869-889] ORF |
| 229 | ACAAGAUGUUUUGCCAACUGG | CCAGUUGGCAAAACAUCUUGU | | [643-663] ORF |
| 230 | CCUCAACAAGAUGUUUUGCCA | UGGCAAAACAUCUUGUUGAGG | MK | [638-658] ORF |
| 231 | CUGCCCUCAACAAGAUGUUUU | AAAACAUCUUGUUGAGGGCAG | | [634-654] ORF |
| 232 | GAACAAUGGUUCACUGAAGAC | GUCUUCAGUGAACCAUUGUUC | | [402-422] ORF |
| 233 | CUGGAGGAUUUCAUCUCUUGU | ACAAGAGAUGAAAUCCUCCAG | | [2117-2137] 3'UTR |
| 234 | CCUGGAGGAUUUCAUCUCUUG | CAAGAGAUGAAAUCCUCCAGG | | [2116-2136] 3'UTR |
| 235 | CCACACCCUGGAGGAUUUCAU | AUGAAAUCCUCCAGGGUGUGG | | [2110-2130] 3'UTR |
| 236 | CGUCCCAAGCAAUGGAUGAUU | AAUCAUCCAUUGCUUGGGACG | | [358-378] ORF |
| 237 | CAGCCAAACCCUGUCUGACAA | UUGUCAGACAGGGUUUGGCUG | | [2045-2065] 3'UTR |
| 238 | CCCACUUCACCGUACUAACCA | UGGUUAGUACGGUGAAGUGGG | | [1754-1774] 3'UTR |
| 239 | AGGACUUCCAUUUGCUUUGUC | GACAAAGCAAAUGGAAGUCCU | | [1552-1572] 3'UTR |
| 240 | UCAGUCUACCUCCCGCCAUAA | UUAUGGCGGGAGGUAGACUGA | | [1373-1393] ORF |
| 241 | UGGAGAAUAUUUCACCCUUCA | UGAAGGGUGAAAUAUUCUCCA | MK,Dog | [1223-1243] ORF |
| 242 | AACCACUGGAUGGAGAAUAUU | AAUAUUCUCCAUCCAGUGGUU | MK | [1213-1233] ORF |
| 243 | CCAGUGGUAAUCUACUGGGAC | GUCCCAGUAGAUUACCACUGG | MK | [1030-1050] ORF |
| 244 | GAAACACUUUUCGACAUAGUG | CACUAUGUCGAAAAGUGUUUC | MK | [877-897] ORF |
| 245 | UGGAUGACAGAAACACUUUUC | GAAAAGUGUUUCUGUCAUCCA | Pig | [868-888] ORF |
| 246 | CUUAUCCGAGUGGAAGGAAAU | AUUUCCUUCCACUCGGAUAAG | MK,HM | [831-851] ORF |
| 247 | GCCCUCAACAAGAUGUUUUGC | GCAAAACAUCUUGUUGAGGGC | | [636-656] ORF |
| 248 | CAGCCAAGUCUGUGACUUGCA | UGCAAGUCACAGACUUGGCUG | MK | [604-624] ORF |
| 249 | UGGAGGAUUUCAUCUCUUGUA | UACAAGAGAUGAAAUCCUCCA | | [2118-2138] 3'UTR |
| 250 | UGGCAUUUGCACCUACCUCAC | GUGAGGUAGGUGCAAAUGCCA | | [1834-1854] 3'UTR |
| 251 | AGGGAAGCUGUCCCUCAUGUU | AACAUGAGGGACAGCUUCCCU | | [1774-1794] 3'UTR |
| 252 | UGGAAACUACUUCCUGAAAAC | GUUUUCAGGAAGUAGUUUCCA | MK,GP | [318-338] ORF |
| 253 | ACCUAUGGAAACUACUUCCUG | CAGGAAGUAGUUUCCAUAGGU | MK | [313-333] ORF |
| 254 | UGGCCUGCACUGGUGUUUUGU | ACAAAACACCAGUGCAGGCCA | | [1594-1614] 3'UTR |
| 255 | AGAAGCACCCAGGACUUCCAU | AUGGAAGUCCUGGGUGCUUCU | | [1542-1562] 3'UTR |
| 256 | GAAGACUCCAGUGGUAAUCUA | UAGAUUACCACUGGAGUCUUC | MK | [1023-1043] ORF |
| 257 | CAUGUGUAACAGUUCCUGCAU | AUGCAGGAACUGUUACACAUG | MK,Dog | [959-979] ORF |
| 258 | GGCCAUCUACAAGCAGUCACA | UGUGACUGCUUGUAGAUGGCC | MK | [731-751] ORF |
| 259 | GAAAACCUACCAGGGCAGCUA | UAGCUGCCCUGGUAGGUUUUC | | [551-571] ORF |
| 260 | CUUCUGUCCCUUCCCAGAAAA | UUUUCUGGGAAGGGACAGAAG | | [535-555] ORF |
| 261 | UCAUCUUCUGUCCCUUCCCAG | CUGGGAAGGGACAGAAGAUGA | | [531-551] ORF |
| 262 | CCAAACCCUGUCUGACAACCU | AGGUUGUCAGACAGGGUUUGG | | [2048-2068] 3'UTR |
| 263 | CCGUCCCAAGCAAUGGAUGAU | AUCAUCCAUUGCUUGGGACGG | | [357-377] ORF |
| 264 | GGUGGGUUGGUAGUUUCUACA | UGUAGAAACUACCAACCCACC | | [1978-1998] 3'UTR |
| 265 | GGCAUUUGCACCUACCUCACA | UGUGAGGUAGGUGCAAAUGCC | | [1835-1855] 3'UTR |
| 266 | AUCUGUGAAAUGCUGGCAUUU | AAAUGCCAGCAUUUCACAGAU | | [1821-1841] 3'UTR |
| 267 | CCCAUAUCUGUGAAAUGCUGG | CCAGCAUUUCACAGAUAUGGG | | [1816-1836] 3'UTR |
| 268 | ACCCAGGACUUCCAUUUGCUU | AAGCAAAUGGAAGUCCUGGGU | | [1548-1568] 3'UTR |
| 269 | GAAACCACUGGAUGGAGAAUA | UAUUCUCCAUCCAGUGGUUUC | MK | [1211-1231] ORF |
| 270 | AGGAAGAGAAUCUCCGCAAGA | UCUUGCGGAGAUUCUCUUCCU | | [1105-1125] ORF |
| 271 | CAACUACAUGUGUAACAGUUC | GAACUGUUACACAUGUAGUUG | MK,Dog | [953-973] ORF |
| 272 | ACAUAGUGUGGUGGUGCCCUA | UAGGGCACCACCACACUAUGU | MK | [890-910] ORF |
| 273 | CUGCUCAGAUAGCGAUGGUCU | AGACCAUCGCUAUCUGAGCAG | | [794-814] ORF |
| 274 | UCACAGCACAUGACGGAGGUU | AACCUCCGUCAUGUGCUGUGA | | [747-767] ORF |
| 275 | GCUAAAAGUUUUGAGCUUCUC | GAGAAGCUCAAAACUUUUAGC | | [92-112] 5'UTR |
| 276 | GAAGACCCAGGUCCAGAUGAA | UUCAUCUGGACCUGGGUCUUC | MK | [417-437] ORF |
| 277 | GCUGGAAGGGUCAACAUCUUU | AAAGAUGUUGACCCUUCCAGC | | [2500-2520] 3'UTR |
| 278 | GGAUCCACCAAGACUUGUUUU | AAAACAAGUCUUGGUGGAUCC | | [2150-2170] 3'UTR |
| 279 | CCAAGCAAUGGAUGAUUUGAU | AUCAAAUCAUCCAUUGCUUGG | | [362-382] ORF |
| 280 | GGCCUUGAAACCACCUUUUAU | AUAAAAGGUGGUUUCAAGGCC | | [1893-1913] 3'UTR |
| 281 | CCUACCUCACAGAGUGCAUUG | CAAUGCACUCUGUGAGGUAGG | | [1845-1865] 3'UTR |
| 282 | UGUGAAAUGCUGGCAUUUGCA | UGCAAAUGCCAGCAUUUCACA | | [1824-1844] 3'UTR |
| 283 | GCCACAUUCUAGGUAGGUAGG | CCUACCUACCUAGAAUGUGGC | | [1731-1751] 3'UTR |
| 284 | GAGUAGGACAUACCAGCUUAG | CUAAGCUGGUAUGUCCUACUC | | [1633-1653] 3'UTR |
| 285 | GGGAGUAGGACAUACCAGCUU | AAGCUGGUAUGUCCUACUCCC | | [1631-1651] 3'UTR |
| 286 | UCCCGCCAUAAAAAACUCAUG | CAUGAGUUUUUUAUGGCGGGA | | [1383-1403] ORF |
| 287 | GAGCUGAAUGAGGCCUUGGAA | UUCCAAGGCCUCAUUCAGCUC | MK,Rat | [1276-1298] ORF |
| 288 | CCACUGGAUGGAGAAUAUUUC | GAAAUAUUCUCCAUCCAGUGG | MK | [1215-1235] ORF |
| 289 | AAGAAACCACUGGAUGGAGAA | UUCUCCAUCCAGUGGUUUCUU | MK | [1209-1229] ORF |
| 290 | GCCAAAGAAGAAACCACUGGA | UCCAGUGGUUUCUUCUUUGGC | MK,GP | [1202-1222] ORF |
| 291 | CGCACAGAGGAAGAGAAUCUC | GAGAUUCUCUUCCUCUGUGCG | | [1098-1118] ORF |
| 292 | CUACAACUACAUGUGUAACAG | CUGUUACACAUGUAGUUGUAG | MK,Dog | [950-970] ORF |
| 293 | CUGUACCACCAUCCACUACAA | UUGUAGUGGAUGGUGGUACAG | MK,Pig | [935-955] ORF |
| 294 | UGACUGUACCACCAUCCACUA | UAGUGGAUGGUGGUACAGUCA | MK,HM,Pig,RB | [932-952] ORF |
| 295 | CAUAGUGUGGUGGUGCCCUAU | AUAGGGCACCACCACACUAUG | MK | [891-911] ORF |
| 296 | CAGCAUCUUAUCCGAGUGGAA | UUCCACUCGGAUAAGAUGCUG | HM | [825-845] ORF |
| 297 | CUAAAAGUUUUGAGCUUCUCA | UGAGAAGCUCAAAACUUUUAG | | [93-113] 5'UTR |
| 298 | GUCUGUGACUUGCACGUACUC | GAGUACGUGCAAGUCACAGAC | MK | [611-631] ORF |
| 299 | CAAGUCUGUGACUUGCACGUA | UACGUGCAAGUCACAGACUUG | MK | [608-628] ORF |
| 300 | CGUCUGGGCUUCUUGCAUUCU | AGAAUGCAAGAAGCCCAGACG | | [579-599] ORF |
| 301 | CCAGAUGAAGCUCCCAGAAUG | CAUUCUGGGAGCUUCAUCUGG | | [429-449] ORF |
| 302 | UGAACAAUGGUUCACUGAAGA | UCUUCAGUGAACCAUUGUUCA | | [401-421] ORF |
| 303 | CAAGCACAUCUGCAUUUUCAC | GUGAAAAUGCAGAUGUGCUUG | | [2530-2550] 3'UTR |
| 304 | UGGAGCUGGAAGGGUCAACAU | AUGUUGACCCUUCCAGCUCCA | | [2496-2516] 3'UTR |
| 305 | GACAACCUCUUGGUCGACCUU | AAGGUCGACCAAGAGGUUGUC | | [2061-2081] 3'UTR |
| 306 | UGGCCUUGAAACCACCUUUUA | UAAAAGGUGGUUUCAAGGCCA | | [1892-1912] 3'UTR |
| 307 | CACCUACCUCACAGAGUGCAU | AUGCACUCUGUGAGGUAGGUG | | [1843-1863] 3'UTR |
| 308 | GACCUAUGGAAACUACUUCCU | AGGAAGUAGUUUCCAUAGGUC | MK | [312-332] ORF |
| 309 | UGCAAUAGGUGUGCGUCAGAA | UUCUGACGCACACCUAUUGCA | | [1525-1545] 3'UTR |
| 310 | GCCAUUUUGGGUUUUGGGUCU | AGACCCAAAACCCAAAAUGGC | | [1491-1511] 3'UTR |
| 311 | AGACUGACAUUCUCCACUUCU | AGAAGUGGAGAAUGUCAGUCU | | [1427-1447] ORF+3'UTR |
| 312 | AAACUCAUGUUCAAGACAGAA | UUCUGUCUUGAACAUGAGUUU | | [1395-1415] ORF |
| 313 | AAAACUCAUGUUCAAGACAGA | UCUGUCUUGAACAUGAGUUUU | | [1394-1414] ORF |
| 314 | UCAGGAAACAUUUUCAGACCU | AGGUCUGAAAAUGUUUCCUGA | MK | [296-316] ORF |
| 315 | ACAACUACAUGUGUAACAGUU | AACUGUUACACAUGUAGUUGU | MK,Dog | [952-972] ORF |
| 316 | UGGCUCUGACUGUACCACCAU | AUGGUGGUACAGUCAGAGCCA | MK,HM,RB | [926-946] ORF |
| 317 | UGAGGUUGGCUCUGACUGUAC | GUACAGUCAGAGCCAACCUCA | MK,HM | [920-940] ORF |
| 318 | AGUUUUGAGCUUCUCAAAAGU | ACUUUUGAGAAGCUCAAAACU | | [98-118] 5'UTR |
| 319 | GCCAUCUACAAGCAGUCACAG | CUGUGACUGCUUGUAGAUGGC | MK | [732-752] ORF |
| 320 | CGAUAUUGAACAAUGGUUCAC | GUGAACCAUUGUUCAAUAUCG | | [395415] ORF |
| 321 | GCAAGCACAUCUGCAUUUUCA | UGAAAAUGCAGAUGUGCUUGC | | [2529-2549] 3'UTR |
| 322 | GCAAUGGAUGAUUUGAUGCUG | CAGCAUCAAAUCAUCCAUUGC | | [366-366] ORF |
| 323 | UCCCAAGCAAUGGAUGAUUUG | CAAAUCAUCCAUUGCUUGGGA | | [360-380] ORF |
| 324 | CCUUAGUACCUAAAAGGAAAU | AUUUCCUUUUAGGUACUAAGG | | [2078-2098] 3'UTR |
| 325 | GACCUUAGUACCUAAAAGGAA | UUCCUUUUAGGUACUAAGGUC | | [2076-2096] 3'UTR |
| 326 | CGACCUUAGUACCUAAAAGGA | UCCUUUUAGGUACUAAGGUCG | | [2075-2095] 3'UTR |
| 327 | GGUCGACCUUAGUACCUAAAA | UUUUAGGUACUAAGGUCGACC | | [2072-2092] 3'UTR |
| 328 | UUGGUCGACCUUAGUACCUAA | UUAGGUACUAAGGUCGACCAA | | [2070-2090] 3'UTR |
| 329 | AGCCAAACCCUGUCUGACAAC | GUUGUCAGACAGGGUUUGGCU | | [2046-2066] 3'UTR |
| 330 | UCUGGCCUUGAAACCACCUUU | AAAGGUGGUUUCAAGGCCAGA | | [1890-1910] 3'UTR |
| 331 | GAAGCUGUCCCUCAUGUUGAA | UUCAACAUGAGGGACAGCUUC | | [1777-1797] 3'UTR |
| 332 | CCACUUCACCGUACUAACCAG | CUGGUUAGUACGGUGAAGUGG | | [1755-1775] 3'UTR |
| 333 | CACCCAGGACUUCCAUUUGCU | AGCAAAUGGAAGUCCUGGGUG | | [1547-1567] 3'UTR |
| 334 | CCCUGCCAUUUUGGGUUUUGG | CCAAAACCCAAAAUGGCAGGG | | [1487-1507] 3'UTR |
| 335 | UCAAGACAGAAGGGCCUGACU | AGUCAGGCCCUUCUGUCUUGA | | [1405-1425] ORF |
| 336 | ACCACUGGAUGGAGAAUAUUU | AAAUAUUCUCCAUCCAGUGGU | MK | [1214-1234] ORF |
| 337 | AGAAGAAACCACUGGAUGGAG | CUCCAUCCAGUGGUUUCUUCU | MK | [1207-1227] ORF |
| 338 | UGUGCUCAAGACUGGCGCUAA | UUAGCGCCAGUCUUGAGCACA | | [76-96] 5'UTR |
| 339 | GCACAGAGGAAGAGAAUCUCC | GGAGAUUCUCUUCCUCUGUGC | | [1099-1119] ORF |
| 340 | UCCAGUGGUAAUCUACUGGGA | UCCCAGUAGAUUACCACUGGA | MK | [1029-1049] ORF |
| 341 | CUGGAAGACUCCAGUGGUAAU | AUUACCACUGGAGUCUUCCAG | MK | [1020-1040] ORF |
| 342 | CCUCACCAUCAUCACACUGGA | UCCAGUGUGAUGAUGGUGAGG | Pig,RB | [1004-1024] ORF |
| 343 | CCUCAGCAUCUUAUCCGAGUG | CACUCGGAUAAGAUGCUGAGG | HM | [822-842] ORF |
| 344 | ACCAUGAGCGCUGCUCAGAUA | UAUCUGAGCAGCGCUCAUGGU | | [764-804] ORF |
| 345 | ACAAGCAGUCACAGCACAUGA | UCAUGUGCUGUGACUGCUUGU | MK | [739-759] ORF |
| 346 | GGACAGCCAAGUCUGUGACUU | AAGUCACAGACUUGGCUGUCC | | [601-621] ORF |
| 347 | UGGCGCUAAAAGUUUUGAGCU | AGCUCAAAACUUUUAGCGCCA | GP | [88-108] 5'UTR |
| 348 | ACGAUAUUGAACAAUGGUUCA | UGAACCAUUGUUCAAUAUCGU | | [394-414] ORF |
| 349 | GACGAUAUUGAACAAUGGUUC | GAACCAUUGUUCAAUAUCGUC | | [393-413] ORF |
| 350 | AUCCACCAAGACUUGUUUUAU | AUAAAACAAGUCUUGGUGGAU | | [2152-2172] 3'UTR |
| 351 | UCCUGAAAACAACGUUCUGUC | GACAGAACGUUGUUUUCAGGA | GP | [329-349] ORF |
| 352 | CUGGCCUUGAAACCACCUUUU | AAAAGGUGGUUUCAAGGCCAG | | [1891-1911] 3'UTR |
| 353 | UGGGUCUUUGAACCCUUGCUU | AAGCAAGGGUUCAAAGACCCA | | [1505-1525] 3'UTR |
| 354 | AGCUUUGAGGUGCGUGUUUGU | ACAAACACGCACCUCAAAGCU | Pig | [1056-1076] ORF |
| 355 | ACUGGAAGACUCCAGUGGUAA | UUACCACUGGAGUCUUCCAGU | MK | [1019-1039] ORF |
| 356 | CAGCUGUGGGUUGAUUCCACA | UGUGGAAUCAACCCACAGCUG | | [681-701] ORF |
| 357 | GGCUUCUUGCAUUCUGGGACA | UGUCCCAGAAUGCAAGAAGCC | | [585-605] ORF |
| 358 | UGGAUCCACCAAGACUUGUUU | AAACAAGUCUUGGUGGAUCCA | | [2149-2169] 3'UTR |
| 359 | UGGUCGACCUUAGUACCUAAA | UUUAGGUACUAAGGUCGACCA | | [2071-2091] 3'UTR |
| 360 | ACCUCUUGGUCGACCUUAGUA | UACUAAGGUCGACCAAGAGGU | | [2065-2085] 3'UTR |
| 361 | GGUAGUUUCUACAGUUGGGCA | UGCCCAACUGUAGAAACUACC | | [1986-2006] 3'UTR |
| 362 | GGAAGCUGUCCCUCAUGUUGA | UCAACAUGAGGGACAGCUUCC | | [1776-1796] 3'UTR |
| 363 | GCCUGCACUGGUGUUUUGUUG | CAACAAAACACCAGUGCAGGC | | [1596-1616] 3'UTR |
| 364 | CUGAACAAGUUGGCCUGCACU | AGUGCAGGCCAACUUGUUCAG | | [1584-1604] 3'UTR |
| 365 | GAACCCUUGCUUGCAAUAGGU | ACCUAUUGCAAGCAAGGGUUC | | [1514-1534] 3'UTR |
| 366 | GGGUCUUUGAACCCUUGCUUG | CAAGCAAGGGUUCAAAGACCC | | [1506-1526] 3'UTR |
| 367 | GUUUUGGGUCUUUGAACCCUU | AAGGGUUCAAAGACCCAAAAC | | [1501-1521] 3'UTR |
| 368 | ACACUGGAAGACUCCAGUGGU | ACCACUGGAGUCUUCCAGUGU | MK | [1017-1037] ORF |
| 369 | UCCACUACAACUACAUGUGUA | UACACAUGUAGUUGUAGUGGA | MK,Dog | [946-966] ORF |
| 370 | AGGUUGGCUCUGACUGUACCA | UGGUACAGUCAGAGCCAACCU | MK,HM,RB | [922-942] ORF |
| 371 | UCAGCAUCUUAUCCGAGUGGA | UCCACUCGGAUAAGAUGCUGA | HM | [824-844] ORF |
| 372 | CCUCCUCAGCAUCUUAUCCGA | UCGGAUAAGAUGCUGAGGAGG | HM | [819-839] ORF |
| 373 | CUGCAAGCACAUCUGCAUUUU | AAAAUGCAGAUGUGCUUGCAG | | [2527-2547] 3'UTR |
| 374 | ACCCUGGAGGAUUUCAUCUCU | AGAGAUGAAAUCCUCCAGGGU | | [2114-2134] 3'UTR |
| 375 | CCCAAGCAAUGGAUGAUUUGA | UCAAAUCAUCCAUUGCUUGGG | | [361-381] ORF |
| 376 | CUACAGUUGGGCAGCUGGUUA | UAACCAGCUGCCCAACUGUAG | | [1994-2014] 3'UTR |
| 377 | CCUCACAGAGUGCAUUGUGAG | CUCACAAUGCACUCUGUGAGG | | [1849-1869] 3'UTR |
| 378 | UCACCGUACUAACCAGGGAAG | CUUCCCUGGUUAGUACGGUGA | | [1760-1760] 3'UTR |
| 379 | ACUGAACAAGUUGGCCUGCAC | GUGCAGGCCAACUUGUUCAGU | | [1583-1603] 3'UTR |
| 380 | CAGCCACCUGAAGUCCAAAAA | UUUUUGGACUUCAGGUGGCUG | | [1349-1369] ORF |
| 381 | CAGCCAAAGAAGAAACCACUG | CAGUGGUUUCUUCUUUGGCUG | MK,GP | [1200-1220] ORF |
| 382 | ACAGCUUUGAGGUGCGUGUUU | AAACACGCACCUCAAAGCUGU | Pig | [1054-1074] ORF |
| 383 | CACUGGAAGACUCCAGUGGUA | UACCACUGGAGUCUUCCAGUG | MK | [1018-1038] ORF |
| 384 | UCACACUGGAAGACUCCAGUG | CACUGGAGUCUUCCAGUGUGA | MK,RB,MO | [1015-1035] ORF |
| 385 | CACUACAACUACAUGUGUAAC | GUUACACAUGUAGUUGUAGUG | MK,Dog | [948-968] ORF |
| 386 | CUCUGACUGUACCACCAUCCA | UGGAUGGUGGUACAGUCAGAG | MK,HM,Pig,RB | [929-949] ORF |
| 387 | AGUCUGUGACUUGCACGUACU | AGUACGUGCAAGUCACAGACU | MK | [610-630] ORF |
| 388 | CCAAGUCUGUGACUUGCACGU | ACGUGCAAGUCACAGACUUGG | MK | [607-627] ORF |
| 389 | GACAGCCAAGUCUGUGACUUG | CAAGUCACAGACUUGGCUGUC | | [602-622] ORF |
| 390 | GUCCAGAUGAAGCUCCCAGAA | UUCUGGGAGCUUCAUCUGGAC | | [427-447] ORF |
| 391 | UGGUUCACUGAAGACCCAGGU | ACCUGGGUCUUCAGUGAACCA | | [408-428] ORF |
| 392 | AGCUGGAAGGGUCAACAUCUU | AAGAUGUUGACCCUUCCAGCU | | [2499-2519] 3'UTR |
| 393 | ACCUUAGUACCUAAAAGGAAA | UUUCCUUUUAGGUACUAAGGU | | [2077-2097] 3'UTR |
| 394 | CCUGUCUGACAACCUCUUGGU | ACCAAGAGGUUGUCAGACAGG | | [2054-2074] 3'UTR |
| 395 | CACAGAGUGCAUUGUGAGGGU | ACCCUCACAAUGCACUCUGUG | | [1852-1872] 3'UTR |
| 396 | CUUUGAACCCUUGCUUGCAAU | AUUGCAAGCAAGGGUUCAAAG | | [1510-1530] 3'UTR |
| 397 | CCAGCCACCUGAAGUCCAAAA | UUUUGGACUUCAGGUGGCUGG | | [1348-1368] ORF |
| 398 | CUACAUGUGUAACAGUUCCUG | CAGGAACUGUUACACAUGUAG | MK,Dog | [956-976] ORF |
| 399 | CCAUCCACUACAACUACAUGU | ACAUGUAGUUGUAGUGGAUGG | MK,Dog,GP | [943-963] ORF |
| 400 | GAGCUUCUCAAAAGUCUAGAG | CUCUAGACUUUUGAGAAGCUC | | [104-124] 5'UTR |
| 401 | UGAGCUUCUCAAAAGUCUAGA | UCUAGACUUUUGAGAAGCUCA | | [103-123] 5'UTR |
| 402 | CCCUUGCUUGCAAUAGGUGUG | CACACCUAUUGCAAGCAAGGG | | [1517-1537] 3'UTR |
| 403 | GGGUUUUGGGUCUUUGAACCC | GGGUUCAAAGACCCAAAACCC | | [1499-1519] 3'UTR |
| 404 | ACCAUCCACUACAACUACAUG | CAUGUAGUUGUAGUGGAUGGU | MK,Dog,GP | [942-962] ORF |
| 405 | UGACAACCUCUUGGUCGACCU | AGGUCGACCAAGAGGUUGUCA | | [2060-2080] 3'UTR |
| 406 | UCCAGCCACCUGAAGUCCAAA | UUUGGACUUCAGGUGGCUGGA | | [1347-1367] ORF |
| 407 | GAGGCCUUGGAACUCAAGGAU | AUCCUUGAGUUCCAAGGCCUC | MK | [1287-1307] ORF |
| 408 | UCCAGAUGAAGCUCCCAGAAU | AUUCUGGGAGCUUCAUCUGGA | | [428-448] ORF |
| 409 | UGCAAGCACAUCUGCAUUUUC | GAAAAUGCAGAUGUGCUUGCA | | [2528-2548] 3'UTR |
| 410 | GAAUGAGGCCUUGGAACUCAA | UUGAGUUCCAAGGCCUCAUUC | MK | [1283-1303] ORF |
| 411 | GAAACCACCUUUUAUUACAUG | CAUGUAAUAAAAGGUGGUUUC | | [1899-1919] 3'UTR |
| 412 | CUACUGGGACGGAACAGCUUU | AAAGCUGUUCCGUCCCAGUAG | MK | [1041-1061] ORF |
| 413 | GGAGAUGUAAGAAAUGUUCUU | AAGAACAUUUCUUACAUCUCC | | [1685-1705] 3'UTR |
| 414 | CUGGAUCCACCAAGACUUGUU | AACAAGUCUUGGUGGAUCCAG | | [2148-2168] 3'UTR |
| 415 | CCUCUGAGUCAGGAAACAUUU | AAAUGUUUCCUGACUCAGAGG | MK | [288-308] ORF |
| 416 | CCAUGGCCAUCUACAAGCAGU | ACUGCUUGUAGAUGGCCAUGG | MK | [727-747] ORF |
| 417 | CGUACUAACCAGGGAAGCUGU | ACAGCUUCCCUGGUUAGUACG | | [1764-1784] 3'UTR |
| 418 | CCCUCUGAGUCAGGAAACAUU | AAUGUUUCCUGACUCAGAGGG | MK | [287-307] ORF |
| 419 | UCCGUCUGGGCUUCUUGCAUU | AAUGCAAGAAGCCCAGACGGA | | [577-597] ORF |
| 420 | GAGAUGUAAGAAAUGUUCUUG | CAAGAACAUUUCUUACAUCUC | | [1686-1706] 3'UTR |
| 421 | GAUGUAAGAAAUGUUCUUGCA | UGCAAGAACAUUUCUUACAUC | | [1688-1708] 3'UTR |

**Table C: 19-mer HTRA2 - HtrA serine peptidase 2**

| No. | Sense siRNA | AntiSense siRNA | Other Sp | Human-73747817 ORF:603-1979 | Human-73747818 ORF:603-1688 |
|---|---|---|---|---|---|
| 1 | CCGAGACAGAGGGUUAAAU | AUUUAACCCUCUGUCUCGG | | [2051-2069] 3'UTR | [1760-1778] 3'UTR |
| 2 | GAAUCACAGAAACACUUUU | AAAAGUGUUUCUGUGAUUC | | [2124-2142] 3'UTR | [1833-1851] 3'UTR |
| 3 | GGCCUGGUGAUGUGAUUUU | AAAAUCACAUCACCAGGCC | | [1825-1843] ORF | [1534-1552] ORF |
| 4 | GUGAUGUGAUUUUGGCCAU | AUGGCCAAAAUCACAUCAC | | [1831-1849] ORF | [1540-1558] ORF |
| 5 | CCGUGGUCUAUAUCGAGAU | AUCUCGAUAUAGACCACGG | | [1081-1099] ORF | [1081-1099] ORF |
| 6 | UGACAUGGGUUUCUUGGUA | UACCAAGAAACCCAUGUCA | | [2508-2526] 3'UTR | [2217-2235] 3'UTR |
| 7 | AGGGUGAAAACUUCUGCUU | AAGCAGAAGUUUUCACCCU | | [2445-2463] 3'UTR | [2154-2172] 3'UTR |
| 8 | UGAAGAAUCACAGAAACAC | GUGUUUCUGUGAUUCUUCA | | [2120-2138] 3'UTR | [1829-1847] 3'UTR |
| 9 | UGAAGCUGUUCGAACCCAA | UUGGGUUCGAACAGCUUCA | MO | [1886-1904] ORF | [1595-1613] ORF |
| 10 | GACAUGGGUUUCUUGGUAA | UUACCAAGAAACCCAUGUC | | [2509-2527] 3'UTR | [2218-2236] 3'UTR |
| 11 | CGGUUGCUGACAUGGGUUU | AAACCCAUGUCAGCAACCG | | [2501-2519] 3'UTR | [2210-2228] 3'UTR |
| 12 | GGUGAAAACUUCUGCUUGA | UCAAGCAGAAGUUUUCACC | | [2447-2465] 3'UTR | [2156-2174] 3'UTR |
| 13 | CCUAGCAACAUAUUAUAGU | ACUAUAAUAUGUUGCUAGG | | [2162-2180] 3'UTR | [1871-1889] 3'UTR |
| 14 | ACCGAGACAGAGGGUUAAA | UUUAACCCUCUGUCUCGGU | | [2050-2068] 3'UTR | [1759-1777] 3'UTR |
| 15 | GCCUGGUGAUGUGAUUUUG | CAAAAUCACAUCACCAGGC | | [1826-1844] ORF | [1535-1553] ORF |
| 16 | GGUAAGCUCCUGAGGUAAU | AUUACCUCAGGAGCUUACC | Rat | [2523-2541] 3'UTR | [2232-2250] 3'UTR |
| 17 | GGGUGAAAACUUCUGCUUG | CAAGCAGAAGUUUUCACCC | | [2446-2464] 3'UTR | [2155-2173] 3'UTR |
| 18 | CUGCUCUGAUUUCCUCCUU | AAGGAGGAAAUCAGAGCAG | | [2005-2023] 3'UTR | [1714-1732] 3'UTR |
| 19 | GGUGAUGUGAUUUUGGCCA | UGGCCAAAAUCACAUCACC | | [1830-1848] ORF | [1539-1557] ORF |
| 20 | GCCGUGGUCUAUAUCGAGA | UCUCGAUAUAGACCACGGC | | [1080-1098] ORF | [1080-1098] ORF |
| 21 | GUGAAAACUUCUGCUUGAC | GUCAAGCAGAAGUUUUCAC | | [2448-2466] 3'UTR | [2157-2175] 3'UTR |
| 22 | AGAUGUUUAUGAAGCUGUU | AACAGCUUCAUAAACAUCU | MO | [1877-1895] ORF | [1586-1604] ORF |
| 23 | GGAGCAGAUGGUACAAAAU | AUUUUGUACCAUCUGCUCC | | [1853-1871] ORF | [1562-1560] ORF |
| 24 | UGAAGGUCACAGCUGGAAU | AUUCCAGCUGUGACCUUCA | | [1570-1588] ORF | [1375-1393] ORF |
| 25 | CGUGGUCUAUAUCGAGAUC | GAUCUCGAUAUAGACCACG | | [1082-1100] ORF | [1082-1100] ORF |
| 26 | GUAAGCUCCUGAGGUAAUG | CAUUACCUCAGGAGCUUAC | Rat | [2524-2542] 3'UTR | [2233-2251] 3'UTR |
| 27 | UGGGUUUCUUGGUAAGCUC | GAGCUUACCAAGAAACCCA | | [2513-2531] 3'UTR | [2222-2240] 3'UTR |
| 28 | ACAUGGGUUUCUUGGUAAG | CUUACCAAGAAACCCAUGU | | [2510-2520] 3'UTR | [2219-2237] 3'UTR |
| 29 | CCAUCUUUUGUGGGCAGUU | AACUGCCCACAAAAGAUGG | | [2311-2329] 3'UTR | [2020-2038] 3'UTR |
| 30 | CUGAUUUCCUCCUUGCCUU | AAGGCAAGGAGGAAAUCAG | | [2010-2028] 3'UTR | [171-1737] 3'UTR |
| 31 | CACUGACCUUAUAUGUGAC | GUCACAUAUAAGGUCAGUG | | [1942-1960] ORF | [1651-1669] ORF |
| 32 | GCAGAUGGUACAAAAUGCU | AGCAUUUUGUACCAUCUGC | | [1856-1874] ORF | [1565-1583] ORF |
| 33 | CGGCCUGGUGAUGUGAUUU | AAAUCACAUCACCAGGCCG | | [1824-1842] ORF | [1533-1551] ORF |
| 34 | UGAAAACUUCUGCUUGACA | UGUCAAGCAGAAGUUUUCA | | [2449-2467] 3'UTR | [2158-2176] 3'UTR |
| 35 | GAGGGUGAAAACUUCUGCU | AGCAGAAGUUUUCACCCUC | | [2444-2462] 3'UTR | [2153-2171] 3'UTR |
| 36 | GGAGGGUGAAAACUUCUGC | GCAGAAGUUUUCACCCUCC | | [2443-2461] 3'UTR | [2152-2170] 3'UTR |
| 37 | ACCAUCCUGACCUCCUAUU | AAUAGGAGGUCAGGAUGGU | | [2274-2292] 3'UTR | [1983-2001] 3'UTR |
| 38 | GAAGGAGGGUGAAAACUUC | GAAGUUUUCACCCUCCUUC | | [2440-2458] 3'UTR | [2149-2167] 3'UTR |
| 39 | UGAAGGAGGGUGAAAACUU | AAGUUUUCACCCUCCUUCA | | [2439-2457] 3'UTR | [2146-2166] 3'UTR |
| 40 | CAAAAGGCUAGAGGUAAAG | CUUUACCUCUAGCCUUUUG | | [2218-2236] 3'UTR | [1927-1945] 3'UTR |
| 41 | UGCUCUGAUUUCCUCCUUG | CAAGGAGGAAAUCAGAGCA | Rat | [2006-2024] 3'UTR | [1715-1733] 3'UTR |
| 42 | UGAUGUGAUUUUGGCCAUU | AAUGGCCAAAAUCACAUCA | | [1832-1850] ORF | [1541-1559] ORF |
| 43 | UCCUUUGCCAUCCCUUCUG | CAGAAGGGAUGGCAAAGGA | Rat,MO | [1590-1608] ORF | [1395-1413] ORF |
| 44 | CUGCCGUGGUCUAUAUCGA | CGAUAUAGACCACGGCAG | | [1078-1096] ORF | [1078-1096] ORF |
| 45 | UGAGUGCGGUUGCUGACAU | AUGUCAGCAACCGCACUCA | | [2495-2513] 3'UTR | [2204-2222] 3'UTR |
| 46 | CAUCUUUUGUGGGCAGUUA | UAACUGCCCACAAAAGAUG | | [2312-2330] 3'UTR | [2021-2039] 3'UTR |
| 47 | GCUGCUGCCAUCUUUUGUG | CACAAAAGAUGGCAGCAGC | | [2304-2322] 3'UTR | [2013-2031] 3'UTR |
| 48 | UGAUUUCCUCCUUGCCUUU | AAAGGCAAGGAGGAAAUCA | | [2011-2029] 3'UTR | [1720-1738] 3'UTR |
| 49 | CCUGCUCUGAUUUCCUCCU | AGGAGGAAAUCAGAGCAGG | | [2004+2022] 3'UTR | [1713-1731] 3'UTR |
| 50 | ACAUCGCAACGCUGAGGAU | AUCCUCAGCGUUGCGAUGU | | [1285-1303] ORF | [1285-1303] ORF |
| 51 | UGCUCUUUGUGGUGUGGUG | CACCACACCACAAAGAGCA | | [2340-2358] 3'UTR | [2049-2067] 3'UTR |
| 52 | UGCUGCUCUUUGUGGUGUG | CACACCACAAAGAGCAGCA | | [2337-2355] 3'UTR | [2046-2064] 3'UTR |
| 53 | GUCAGGUGCUGCUCUUUGU | ACAAAGAGCAGCACCUGAC | | [2331-2349] 3'UTR | [2040-2058] 3'UTR |
| 54 | CGAGAGUUUCUGCAUCGUG | CACGAUGCAGAAACUCUCG | | [1617-1635] ORF | [1422-1440] ORF |
| 55 | GUGCUGCUCUUUGUGGUGU | ACACCACAAAGAGCAGCAC | | [2336-2354] 3'UTR | [2045-2063] 3'UTR |
| 56 | GGCAGUGCUGUUGUUGUUG | CAACAACAACAGCACUGCC | | [947-965] ORF | [947-965] ORF |
| 57 | CUCCUGCUCUGAUUUCCUC | GAGGAAAUCAGAGCAGGAG | | [2002-2020] 3'UTR | [1711-1729] 3'UTR |
| 58 | CAAAAUGCUGAAGAUGUUU | AAACAUCUUCAGCAUUUUG | Rat,MO | [1866-1884] ORF | [1575-1593] ORF |
| 59 | GAUGGUACAAAAUGCUGAA | UUCAGCAUUUUGUACCAUC | | [1859-1877] ORF | [1568-1586] ORF |
| 60 | AGAUGGUACAAAAUGCUGA | UCAGCAUUUUGUACCAUCU | | [1858-1876] ORF | [1567-1585] ORF |
| 61 | AGACCACAAUUCCCGGCAU | AUGCCGGGAAUUGUGGUCU | | [423-441] 5'UTR | [423-441] 5'UTR |
| 62 | CCCGGAGUCAGUACAACUU | AAGUUGUACUGACUCCGGG | | [1030-1048] ORF | [1030-1048] ORF |
| 63 | AGAAUGAAUAGAUCACCAA | UUGGUGAUCUAUUCAUUCU | | [1973-1991] ORF+3'UTR | [1682-1700] ORF+3'UTR |
| 64 | GGACGAGAAACACUGACCU | AGGUCAGUGUUUCUCGUCC | | [1932-1950] ORF | [1641-1659] ORF |
| 65 | GCCUCAGAGAACUCUGGAA | UUCCAGAGUUCUCUGAGGC | | [685-903] ORF | [885-903] ORF |
| 66 | CUGCUGACGUCAGGAACUU | AAGUUCCUGACGUCAGCAG | | [714-732] ORF | [714-732] ORF |
| 67 | UGAACACCAUGAAGGUCAC | GUGACCUUCAUGGUGUUCA | | [1561-1579] ORF | [1366-1384] ORF |
| 68 | CUCUGAAGAAUCACAGAAA | UUUCUGUGAUUCUUCAGAG | | [2117-2135] 3'UTR | [1826-1844] 3'UTR |
| 69 | CAGUGCUGUUGUUGUUGUG | CACAACAACAACAGCACUG | | [949-967] ORF | [949-967] ORF |
| 70 | CGCUGAGGAUUCAGACUAA | UUAGUCUGAAUCCUCAGCG | | [1294-1312] ORF | [1294-1312] ORF |
| 71 | ACGCUGAGGAUUCAGACUAI | UAGUCUGAAUCCUCAGCGU | | [1293-1311] ORF | [1293-1311] ORF |
| 72 | UUGCUGACAUGGGUUUCUU | AAGAAACCCAUGUCAGCAA | | [2504-2522] 3'UTR | [2213-2231] 3'UTR |
| 73 | GCUCUGAAGAAUCACAGAA | UUCUGUGAUUCUUCAGAGC | | [2116-2134] 3'UTR | [1825-1843] 3'UTR |
| 74 | CCAAGAGUAUGAGGCUCCU | AGGAGCCUCAUACUCUUGG | | [1988-2006] 3'UTR | [1697-1715] 3'UTR |
| 75 | AGGUCACAGAAUGAAUAGA | UCUAUUCAUUCUGUGACCU | | [1966-1984] ORF+3'UTR | [1675-1693] ORF+3'UTR |
| 76 | UGGUACAAAAUGCUGAAGA | UCUUCAGCAUUUUGUACCA | | [1861-1879] ORF | [1570-1588] ORF |
| 77 | UGAUGAUGCUGACCCUGAG | CUCAGGGUCAGCAUCAUCA | | [1693-1711] ORF | [1498-1516] ORF |
| 78 | GUGCACUUCUGAAGGACUU | AAGUCCUUCAGAAGUGCAC | | [487-505] 5'UTR | [487-505] 5'UTR |
| 79 | GGGAAAAGAAGAAUUCCUC | GAGGAAUUCUUCUUUUCCC | | [1636-1654] ORF | [1441-1459] ORF |
| 80 | UCCCAUGCUUGGCUACAGA | UCUGUAGCCAAGCAUGGGA | | [2403-2421] 3'UTR | [2112-2130] 3'UTR |
| 81 | CCGGAGUCAGUACAACUUC | GAAGUUGUACUGACUCCGG | | [1031-1049] ORF | [1031-1049] ORF |
| 82 | AGGGUUAAAUGAACCAGUG | CACUGGUUCAUUUAACCCU | | [2060-2078] 3'UTR | [1769-1787] 3'UTR |
| 83 | GAGGGUUAAAUGAACCAGU | ACUGGUUCAUUUAACCCUC | | [2059-2077] 3'UTR | [1768-1786] 3'UTR |
| 84 | CACCGAGACAGAGGGUUAAI | UUAACCCUCUGUCUCGGUG | | [2049-2067] 3'UTR | [1758-1776] 3'UTR |
| 85 | CCUCAGAGAACUCUGGAAC | GUUCCAGAGUUCUCUGAGG | | [886-904] ORF | [886-904] ORF |
| 86 | UGAUUGGAGUGAACACCAU | AUGGUGUUCACUCCAAUCA | Rat,MO | [1552-1570] ORF | [1357-1375] ORF |
| 87 | UUGGUAAGCUCCUGAGGUA | UACCUCAGGAGCUUACCAA | | [2521-2539] 3'UTR | [2230-2248] 3'UTR |
| 88 | CCAUAGUGCAUGGUCUGAU | AUCAGACCAUGCACUAUGG | | [2477-2495] 3'UTR | [2186-2204] 3'UTR |
| 89 | GGGCUCUGAAGAAUCACAG | CUGUGAUUCUUCAGAGCCC | | [2114-2132] 3'UTR | [1823-1841] 3'UTR |
| 90 | CUCCUGGGCUCUGAAGAAU | AUUCUUCAGAGCCCAGGAG | | [2109-2127] 3'UTR | [1818-1836] 3'UTR |
| 91 | AGAGGGUUAAAUGAACCAG | CUGGUUCAUUUAACCCUCU | | [2058-2076] 3'TR | [1767-1785] 3'UTR |
| 92 | GAAGAUGUUUAUGAAGCUG | CAGCUUCAUAAACAUCUUC | MO | [1875-1893] ORF | [1584-1602] ORF |
| 93 | GAAAAGAAGAAUUCCUCCU | AGGAGGAAUUCUUCUUUUC | | [1638-1656] ORF | [1443-1461] ORF |
| 94 | UGAUCGUCUUCGAGAGUUU | AAACUCUCGAAGACGAUCA | | [1607-1625] ORF | [1412-1430] ORF |
| 95 | CUGCUUGACAGUUCCACAU | AUGUGGAACUGUCAAGCAG | | [2458-2476] 3'UTR | [2167-2185] 3'UTR |
| 96 | UGAGCUGCUGCCAUCUUUU | AAAAGAUGGCAGCAGCUCA | | [2301-2319] 3'UTR | [2010-2028] 3'UTR |
| 97 | CCACCAAAAGGCUAGAGGU | ACCUCUAGCCUUUUGGUGG | | [2214-2232] 3'UTR | [1923-1941] 3'UTR |
| 98 | AGAUCACCAAGAGUAUGAG | CUCAUACUCUUGGUGAUCU | | [1982-2000] 3'UTR | [1691-1709] 3'UTR |
| 99 | CUGAUCGUCUUCGAGAGUU | AACUCUCGAAGACGAUCAG | | [1606-1624] ORF | [1411-1429] ORF |
| 100 | CCCUUCUGAUCGUCUUCGA | UCGAAGACGAUCAGAAGGG | | [1601-1619] ORF | [1406-1424] ORF |
| 101 | UUGGAGUGAACACCAUGAA | UUCAUGGUGUUCACUCCAA | Rat,MO | [1555-1573] ORF | [1360-1378] ORF |
| 102 | GUCCGUGUGAGACUGCUAA | UUAGCAGUCUCACACGGAC | | [1218-1236] ORF | [1218-1236] ORF |
| 103 | ACAACUUCAUCGCAGAUGU | ACAUCUGCGAUGAAGUUGU | | [1042-1060] ORF | [1042-1060] ORF |
| 104 | CAGUACAACUUCAUCGCAG | CUGCGAUGAAGUUGUACUG | | [1038-1056] ORF | [1038-1056] ORF |
| 105 | CUCUGAAGGAGGGUGAAAA | UUUUCACCCUCCUUCAGAG | | [2436-2454] 3'UTR | [2145-2163] 3'UTR |
| 106 | CCAUGCUUGGCUACAGAUA | UAUCUGUAGCCAAGCAUGG | | [2405-2423] 3'UTR | [2114-2132] 3'UTR |
| 107 | AGCAAAGAUUCCCAUGCUU | AAGCAUGGGAAUCUUUGCU | | [2394-2412] 3'UTR | [2103-2121] 3'UTR |
| 108 | UGACCUCCUAUUAAAGAAA | UUUCUUUAAUAGGAGGUCA | | [2281-2299] 3'UTR | [1990-2008] 3'UTR |
| 109 | UCUGAAGAAUCACAGAAAC | GUUUCUGUGAUUCUUCAGA | | [2118-2136] 3'UTR | [1827-1845] 3'UTR |
| 110 | UCCUUGCCUUUCUGGCUGA | UCAGCCAGAAAGGCAAGGA | | [2019-2037] 3'UTR | [1728-1746] 3'UTR |
| 111 | CAGAAUGAAUAGAUCACCA | UGGUGAUCUAUUCAUUCUG | | [1972-1990] ORF+3'UTR | [1681-1699] ORF+3'UTR |
| 112 | GCUGACGUCAGGAACUUCU | AGMGUUCCUGACGUCAGC | | [716-734] ORF | [716-734] ORF |
| 113 | GCUGAAGAUGUUUAUGAAG | CUUCAUAAACAUCUUCAGC | MO | [1872-1890] ORF | [1581-1599] ORF |
| 114 | GGAAGCUCCAUAACUGCUG | CAGCAGUUAUGGAGCUUCC | | [215-233] 5'UTR | [215-233] 5'UTR |
| 115 | UGGUCUAUAUCGAGAUCCU | AGGAUCUCGAUAUAGACCA | Rat,MO | [1084-1102] ORF | [1084-1102] ORF |
| 116 | GAAAACUUCUGCUUGACAG | CUGUCAAGCAGAAGUUUUC | | [2450-2468] 3'UTR | [2159-2177] 3'UTR |
| 117 | CCCAUGCUUGGCUACAGAU | AUCUGUAGCCAAGCAUGGG | | [2404-2422] 3'UTR | [2113-2131] 3'UTR |
| 118 | GGCUCUGAAGAAUCACAGA | UCUGUGAUUCUUCAGAGCC | | [2115-2133] 3'UTR | [1824-1842] 3'UTR |
| 119 | AGAGAACUCUGGAACCCGU | ACGGGUUCCAGAGUUCUCU | | [890-908] ORF | [890-908] ORF |
| 120 | GCACUUCUGAAGGACUUCA | UGAAGUCCUUCAGAAGUGC | | [489-507] 5'UTR | [489-507] 5'UTR |
| 121 | CCUCUGAAGGAGGGUGAAA | UUUCACCCUCCUUCAGAGG | | [2435-2453] 3'UTR | [2144-2162] 3'UTR |
| 122 | GAAAAUGAGCUGCUGCCAU | AUGGCAGCAGCUCAUUUUC | | [2296-2314] 3'UTR | [2005-2023] 3'UTR |
| 123 | CCUCCUAUUAAAGAAAAUG | CAUUUUCUUUAAUAGGAGG | | [2284-2302] 3'UTR | [1993-2011] 3'UTR |
| 124 | UGGGCUCUGAAGAAUCACA | UGUGAUUCUUCAGAGCCCA | | [2113-2131] 3'UTR | [1822-1840] 3'UTR |
| 125 | CAGAGGGUUAAAUGAACCAI | UGGUUCAUUUAACCCUCUG | | [2057-2075] 3'UTR | [1766-1784] 3'UTR |
| 126 | AGAAUUCCUCCUCCGGAAU | AUUCCGGAGGAGGAAUUCU | | [1645-1663] ORF | [1450-1468] ORF |
| 127 | AAGAAUUCCUCCUCCGGAA | UUCCGGAGGAGGAAUUCUU | | [1644-1662] ORF | [1449-1467] ORF |
| 128 | CUGGAAUCUCCUUUGCCAU | AUGGCAAAGGAGAUUCCAG | Rat,MO | [1582-1600] ORF | [1387-1405] ORF |
| 129 | CCACAUCCAUAGUGCAUGG | CCAUGCACUAUGGAUGUGG | | [2471-2489] 3'UTR | [2180-2198] 3'UTR |
| 130 | GCAAAGAUUCCCAUGCUUG | CAAGCAUGGGAAUCUUUGC | | [2395-2413] 3'UTR | [2104-2122] 3'UTR |
| 131 | GAGCAAAGAUUCCCAUGCU | AGCAUGGGAAUCUUUGCUC | | [2393-2411] 3'UTR | [2102-2120] 3'UTR |
| 132 | GCCAUCUUUUGUGGGCAGU | ACUGCCCACAAAAGAUGGC | | [2310-2328] 3'UTR | [2019-2037] 3'UTR |
| 133 | CUGACCUCCUAUUAAAGAA | UUCUUUAAUAGGAGGUCAG | | [2280-2298] 3'UTR | [1989-2007] 3'UTR |
| 134 | CACAGAAUGAAUAGAUCAC | GUGAUCUAUUCAUUCUGUG | | [1970-1988] ORF+3'UTR | [1679-1697] ORF+3'UTR |
| 135 | CCCUGAGGUCACAGAAUGA | UCAUUCUGUGACCUCAGGG | | [1961-1979] ORF | [1670-1688] ORF |
| 136 | UGCCAUCCCUUCUGAUCGU | ACGAUCAGAAGGGAUGGCA | | [1595-1613] ORF | [1400-1418] ORF |
| 137 | GGCUACAGAUACUGACAGC | GCUGUCAGUAUCUGUAGCC | | [2413-2431] 3'UTR | [2122-2140] 3'UTR |
| 138 | UGGCUACAGAUACUGACAG | CUGUCAGUAUCUGUAGCCA | | [2412-2430] 3'UTR | [2121-2139] 3'UTR |
| 139 | GGAGCAAAGAUUCCCAUGC | GCAUGGGAAUCUUUGCUCC | | [2392-2410] 3'UTR | [2101-2119] 3'UTR |
| 140 | AGAUACUGGAGCUGACCAU | AUGGUCAGCUCCAGUAUCU | | [2260-2278] 3'UTR | [1969-1987] 3'UTR |
| 141 | AGCUCCAUAACUGCUGCUU | AAGCAGCAGUUAUGGAGCU | | [218-236] 5'UTR | [218-236] 5'UTR |
| 142 | GCUGAGGAUUCAGACUAAG | CUUAGUCUGAAUCCUCAGC | | [1295-1313] ORF | [1295-1313] ORF |
| 143 | CAGUUCCACAUCCAUAGUG | CACUAUGGAUGUGGAACUG | | [2466-2484] 3'UTR | [2175-2193] 3'UTR |
| 144 | UGACAGUUCCACAUCCAUA | UAUGGAUGUGGAACUGUCA | | [2463-2481] 3'UTR | [2172-2190] 3'UTR |
| 145 | UGGGAGCAAAGAUUCCCAU | AUGGGAAUCUUUGCUCCCA | | [2390-2408] 3'UTR | [2099-2117] 3'UTR |
| 146 | UGCCAUCUUUUGUGGGCAG | CUGCCCACAAAAGAUGGCA | | [2309-2327] 3'UTR | [2018-2036] 3'UTR |
| 147 | AUGAGCUGCUGCCAUCUUU | AAAGAUGGCAGCAGCUCAU | | [2300-2318] 3'UTR | [2009-2027] 3'UTR |
| 148 | GCUAGAGGUAAAGCUGUAU | AUACAGCUUUACCUCUAGC | | [2224-2242] 3'UTR | [1933-1951] 3'UTR |
| 149 | CACCAAAAGGCUAGAGGUA | UACCUCUAGCCUUUUGGUG | | [2215-2233] 3'UTR | [1924-1942] 3'UTR |
| 150 | CACUUCUGAAGGACUUCAG | CUGAAGUCCUUCAGAAGUG | | [490-508] 5'UTR | [490-508] 5'UTR |
| 151 | CAUCCCUUCUGAUCGUCUU | AAGACGAUCAGAAGGGAUG | | [1598-1616] ORF | [1403-1421] ORF |
| 152 | CCAUCCCUUCUGAUCGUCU | AGACGAUCAGAAGGGAUGG | | [1597-1615] ORF | [1402-1420] ORF |
| 153 | GGAAUCUCCUUUGCCAUCC | GGAUGGCAAAGGAGAUUCC | Rat,MO | [1584-1602] ORF | [1389-1407] ORF |
| 154 | GUGAACACCAUGAAGGUCA | UGACCUUCAUGGUGUUCAC | | [1560-1578] ORF | [1365-1383] ORF |
| 155 | UGGGCUCAUUGUCACCAAC | GUUGGUGACAAUGAGCCCA | | [1172-1190] ORF | [1172-1190] ORF |
| 156 | AGAUUCCCAUGCUUGGCUA | UAGCCAAGCAUGGGAAUCUI | | [2399-2417] 3'UTR | [2108-2126] 3'UTR |
| 157 | CUGCCAUCUUUUGUGGGCA | UGCCCACAAAAGAUGGCAG | | [2308-2326] 3'UTR | [2017-2035] 3'UTR |
| 158 | ACCAAAAGGCUAGAGGUAA | UUACCUCUAGCCUUUUGGU | | [2216-2234] 3'UTR | [1925-1943] 3'UTR |
| 159 | UGCCUUUCUGGCUGAGGUU | AACCUCAGCCAGAAAGGCA | | [2023-2041] 3'UTR | [1732-1750] 3'UTR |
| 160 | GCUCUGAUUUCCUCCUUGC | GCAAGGAGGAAAUCAGAGC | Rat | [2007-2025] 3'UTR | [1716-1734] 3'UTR |
| 161 | AUGGGCUCAUUGUCACCAA | UUGGUGACAAUGAGCCCAU | | [1171-1189] ORF | [1171-1189] ORF |
| 162 | UCAUCGCAGAUGUGGUGGA | UCCACCACAUCUGCGAUGA | MO | [1048-1066] ORF | [7048-1066] ORF |
| 163 | GACAGUUCCAGAUCCAUAG | CUAUGGAUGUGGAACUGUC | | [2464-2482] 3'UTR | [2173-2191] 3'UTR |
| 164 | GCUACAGAUACUGACAGCU | AGCUGUCAGUAUCUGUAGC | | [2414-2432] 3'UTR | [2123-2141] 3'UTR |
| 165 | CAGUUAGUCAGGUGCUGCU | AGCAGCACCUGACUAACUG | | [2325-2343] 3'UTR | [2034-2052] 3'UTR |
| 166 | GAGGCUCCUGCUCUGAUUU | AAAUCAGAGCAGGAGCCUC | | [1998-2016] 3'UTR | [1707-1725] 3'UTR |
| 167 | ACAGCUGGAAUCUCCUUUG | CAAAGGAGAUUCCAGCUGU | Rat,MO | [1578-1596] ORF | [1383-1401] ORF |
| 168 | CACAGCUGGAAUCUCCUUU | AAAGGAGAUUCCAGCUGUG | | [1577-1595] ORF | [1382-1400] ORF |
| 169 | GGGAGCAAAGAUUCCCAUG | CAUGGGAAUCUUUGCUCCC | | [2391-2409] 3'UTR | [2100-2118] 3'UTR |
| 170 | GACCAUCCUGACCUCCUAU | AUAGGAGGUCAGGAUGGUC | | [2273-2291] 3'UTR | [1982-2000] 3'UTR |
| 171 | UCGAGAGUUUCUGCAUCGU | ACGAUGCAGAAACUCUCGA | | [1616-1634] ORF | [1421-1439] ORF |
| 172 | GGAGUGAACACCAUGAAGG | CCUUCAUGGUGUUCACUCC | Rat,MO | [1557-1575] ORF | [1362-1380] ORF |
| 173 | CCUGAGGUCACAGAAUGAA | UUCAUUCUGUGACCUCAGG | | [1962-1980] ORF+3'UTR | [1671-1689] ORF+3'UTR |
| 174 | UCACAGCUGGAAUCUCCUU | AAGGAGAUUCCAGCUGUGA | | [1576-1594] ORF | [1381-1399] ORF |
| 175 | GCUGGAAUCUCCUUUGCCA | UGGCAAAGGAGAUUCCAGC | Rat,MO | [1581-1599] ORF | [1386-1404] ORF |
| 176 | GCAACGCUGAGGAUUCAGA | UCUGAAUCCUCAGCGUUGC | | [1290-1308] ORF | [1290-1308] ORF |
| 177 | CUGAGGUCACAGAAUGAAU | AUUCAUUCUGUGACCUCAG | | [1963-1981] ORF+3'UTR | [1672-1690] ORF+3'UTR |
| 178 | CACAUCCAUAGUGCAUGGU | ACCAUGCACUAUGGAUGUG | | [2472-2490] 3'UTR | [2181-2199] 3'UTR |
| 179 | CAGCUAUUGAUUUUGGAAA | UUUCCAAAAUCAAUAGCUG | | | |
| 180 | GCUAUUGAUUUUGGAAACU | AGUUUCCAAAAUCAAUAGC | | | |
| 181 | AGCUAUUGAUUUUGGAAAC | **GUUUCCAAAAUCAAUAGCU** | | | |

**Table D: 21-mer HTRA2 - HtrA serine peptidase 2**

| No. | Sense siRNA | AntiSense siRNA | Other Sp | Human 73747817 ORF:603-1979 | Human 73747818 ORF:603-1688 |
|---|---|---|---|---|---|
| 1 | CGAGAAACACUGACCUUAUAU | AUAUAAGGUCAGUGUUUCUCG | | [1935-1955] ORF | [1644-1664] ORF |
| 2 | GAAGAAUCACAGAAACACUUU | AAAGUGUUUCUGUGAUUCUUC | | [2121-2141] 3'UTR | [1830-1850] 3'UTR |
| 3 | UGAAUAGAUCACCAAGAGUAU | AUACUCUUGGUGAUCUAUUCA | | 1997-1997]ORF+3'UTR | [1686-1706]ORF+3'UTR |
| 4 | GACGAGAAACACUGACCUUAU | AUAAGGUCAGUGUUUCUCGUC | | [1933-1953] ORF | [1642-1662] ORF |
| 5 | CGAGACAGAGGGUUAAAUGAA | UUCAUUUAACCCUCUGUCUCG | | [2052-2072] 3'UTR | [1761-1781] 3'UTR |
| 6 | ACGAGAAACACUGACCUUAUA | UAUAAGGUCAGUGUUUCUCGU | | [1934-1954] ORF | [1643-1663] ORF |
| 7 | GGGUGAAAACUUCUGCUUGAC | GUCAAGCAGAAGUUUUCACCC | | [2446-2466] 3'UTR | [2155-2175] 3'UTR |
| 8 | GGAGGGUGAAAACUUCUGCUU | AAGCAGAAGUUUUCACCCUCC | | [2443-2463] 3'UTR | [2152-2172] 3'UTR |
| 9 | AGAAACACUGACCUUAUAUGU | ACAUAUAAGGUCAGUGUUUCU | | [1937-1957] ORF | [1646-1666] ORF |
| 10 | UGAAGAAUCAGAGAAACACUU | AAGUGUUUCUGUGAUUCUUCA | | [2120-2140] 3'UTR | [1829-1849] 3'UTR |
| 11 | ACCGAGACAGAGGGUUAAAUG | CAUUUAACCCUCUGUCUCGGU | | [2050-2070] 3'UTR | [1759-1779] 3'UTR |
| 12 | UGGUGAUGUGAUUUUGGCCAU | AUGGCCAAAAUCACAUCACCA | | [1829-1849] ORF | [1538-1558] ORF |
| 13 | GGUGAAAACUUCUGCUUGACA | UGUCAAGCAGAAGUUUUCACC | | [2447-2467] 3'UTR | [2156-2176] 3'UTR |
| 14 | CCGAGACAGAGGGUUAAAUGA | UCAUUUAACCCUCUGUCUCGG | | [2051-2071] 3'UTR | [1760-1780] 3'UTR |
| 15 | CGGCCUGGUGAUGUGAUUUUG | CAAAAUCACAUCACCAGGCCG | | [1824-1844] ORF | [1533-1553] ORF |
| 16 | GCCGUGGUCUAUAUCGAGAUC | GAUCUCGAUAUAGACCACGGC | | [1080-1100] ORF | [1080-1100] ORF |
| 17 | UGACAUGGGUUUCUUGGUAAG | CUUACCAAGAAACCCAUGUCA | | [2508-2528] 3'UTR | [2217-2237] 3'UTR |
| 18 | GCGGCCUGGUGAUGUGAUUUU | AAAAUCACAUCACCAGGCCGC | | [1823-1843] ORF | [1532-1552] ORF |
| 19 | GCAGAUGUGGUGGAGAAGACA | UGUCUUCUCCACCACAUCUGC | MO | [1053-1073] ORF | [1053-1073] ORF |
| 20 | CCAUCUUUUGUGGGCAGUUAG | CUAACUGCCCACAAAAGAUGG | | [2311-2331] 3'UTR | [2020-2040] 3'UTR |
| 21 | CCAUCCUGACCUCCUAUUAAA | UUUAAUAGGAGGUCAGGAUGG | | [2275-2295] 3'UTR | [1984-2004] 3'UTR |
| 22 | AUGAAGCUGUUCGAACCCAAU | AUUGGGUUCGAACAGCUUCAU | MO | [1885-1905] ORF | [1594-1614] ORF |
| 23 | GGCCUGGUGAUGUGAUUUUGG | CCAAAAUCACAUCACCAGGCC | | [1825-1845] ORF | [1534-1554] ORF |
| 24 | UGCGGCCUGGUGAUGUGAUUU | AAAUCACAUCACCAGGCCGCA | | [1822-1842] ORF | [1531-1551] ORF |
| 25 | GGGAGGUGAUUGGAGUGAACA | UGUUCACUCCAAUCACCUCCC | Rat,MO | [1546-1566] ORF | [1351-1371] ORF |
| 26 | UGCCGUGGUCUAUAUCGAGAU | AUCUCGAUAUAGACCACGGCA | | [1079-1099] ORF | [1079-1099] ORF |
| 27 | CCUAUUAAAGAAAAUGAGCUG | CAGCUCAUUUUCUUUAAUAGG | | [2287-2307] 3'UTR | [1996-2016] 3'UTR |
| 28 | UCCUUUGCCAUCCCUUCUGAU | AUCAGAAGGGAUGGCAAAGGA | Rat,MO | [1590-1610] ORF | [1395-1415] ORF |
| 29 | GAGGGUGAAAACUUCUGCUUG | CAAGCAGAAGUUUUCACCCUC | | [2444-2464] 3'UTR | [2153-2173] 3'UTR |
| 30 | UCCUGCUCUGAUUUCCUCCUU | AAGGAGGAAAUCAGAGCAGGA | | [2003-2023] 3'UTR | [1712-1732] 3'UTR |
| 31 | GGAGGUGAUUGGAGUGAACAC | GUGUUCACUCCAAUCACCUCC | Rat,MO | [1547-1567] ORF | [1352-1372] ORF |
| 32 | GUCUAUAUCGAGAUCCUGGAC | GUCCAGGAUCUCGAUAUAGAC | | [1086-1106] ORF | [1086-1106] ORF |
| 33 | CCUUUCUGGCUGAGGUUCUGA | UCAGAACCUCAGCCAGAAAGG | | [2025-2045] 3'UTR | [1734-1754] 3'UTR |
| 34 | UCUGAUUUCCUCCUUGCCUUU | AAAGGCAAGGAGGAAAUCAGA | | [2009-2029] 3'UTR | [1718-1738] 3'UTR |
| 35 | CUCUGAUUUCCUCCUUGCCUU | AAGGCAAGGAGGAAAUCAGAG | | [2008-2028] 3'UTR | [1717-1737] 3'UTR |
| 36 | CCUGCUCUGAUUUCCUCCUUG | CAAGGAGGAAAUCAGAGCAGG | | [200+2024] 3'UTR | [1713-1733] 3'UTR |
| 37 | CAGAUGGUACAAAAUGCUGAA | UUCAGCAUUUUGUACCAUCUG | | [1857-1877] ORF | [1566-1586] ORF |
| 38 | GGUGAUGUGAUUUUGGCCAUU | AAUGGCCAAAAUCACAUCACC | | [1830-1850] ORF | [1539-1559] ORF |
| 39 | CGUCUUCGAGAGUUUCUGCAU | AUGCAGAAACUCUCGAAGACG | | [1611-1631] ORF | [1416-1436] ORF |
| 40 | CAUGGUCUGAUGAGUGCGGUU | AACCGCACUCAUCAGACCAUG | | [2485-2505] 3'UTR | [2194-2214] 3'UTR |
| 41 | AUGUUUAUGAAGCUGUUCGAA | UUCGAACAGCUUCAUAAACAU | MO | [1879-1899] ORF | [1588-1608] ORF |
| 42 | GAUGUUUAUGAAGCUGUUCGA | UCGAACAGCUUCAUAAACAUC | MO | [1878-1898] ORF | [1587-1607] ORF |
| 43 | ACCAUCCUGACCUCCUAUUAA | UUAAUAGGAGGUCAGGAUGGU | | [227+22M] 3'UTR | [1983-2003] 3'UTR |
| 44 | GCACCGAGACAGAGGGUUAAA | UUUAACCCUCUGUCUCGGUGC | | [2048-2068] 3'UTR | [1757-1777] 3'UTR |
| 45 | CUGACAUGGGUUUCUUGGUAA | UUACCAAGAAACCCAUGUCAG | | [2507-2527] 3'UTR | [2216-2236] 3'UTR |
| 46 | AGAUGGUACAAAAUGCUGAAG | CUUCAGCAUUUUGUACCAUCU | | [1858-1878] ORF | [1567-1587] ORF |
| 47 | GCAGAUGGUACAAAAUGCUGA | UCAGCAUUUUGUACCAUCUGC | | [1856-1876] ORF | [1565-1585] ORF |
| 48 | GGGAGCAGAUGGUACAAAAUG | CAUUUUGUACCAUCUGCUCCC | | [1852-1872] ORF | [1561-1581] ORF |
| 49 | GGGACGAGAAACACUGACCUU | AAGGUCAGUGUUUCUCGUCCC | | [1931-1951] ORF | [1640-1660] ORF |
| 50 | GUGAUGUGAUUUUGGCCAUUG | CAAUGGCCAAAAUCACAUCAC | | [1831-1851] ORF | [1540-1560] ORF |
| 51 | GGCAGUGCUGUUGUUGUUGUG | CACAACAACAACAGCACUGCC | | [947-967] ORF | [947-967] ORF |
| 52 | GAGGUCACAGAAUGAAUAGAU | AUCUAUUCAUUCUGUGACCUC | | [1965-1985] ORF+3'UTR | [1674-1194] ORF+3'UTR |
| 53 | UGAUGUGAUUUUGGCCAUUGG | CCAAUGGCCAAAAUCACAUCA | | [1832-1852] ORF | [1541-1561] ORF |
| 54 | UGAAGGUCACAGCUGGAAUCU | AGAUUCCAGCUGUGACCUUCA | | [1570-1590] ORF | [1375-1395] ORF |
| 55 | GGUUGCUGACAUGGGUUUCUU | AAGAAACCCAUGUCAGCAACC | | [2502-2522] 3'UTR | [2211-2231] 3'UTR |
| 56 | CAGUACAACUUCAUCGCAGAU | AUCUGCGAUGAAGUUGUACUG | | [1038-1058] ORF | [1038-1058] ORF |
| 57 | CCGGAGUCAGUACAACUUCAU | AUGAAGUUGUACUGACUCCGG | | [1031-1051] ORF | [1031-1051] ORF |
| 58 | ACAGAAUGAAUAGAUCACCAA | UUGGUGAUCUAUUCAUUCUGU | | [1971-1991] ORF+3'UTR | [1680-1700] ORF+3'UTR |
| 59 | GAGACCACAAUUCCCGGCAUU | AAUGCCGGGAAUUGUGGUCUC | | [422-442] 5'UTR | [422-442] 5'UTR |
| 60 | GCUGACAUGGGUUUCUUGGUA | UACCAAGAAACCCAUGUCAGC | | [2506-2526] 3'UTR | [2215-2235] 3'UTR |
| 61 | UCAGUACAACUUCAUCGCAGA | UCUGCGAUGAAGUUGUACUGA | | [1037-1057] ORF | [1037-1057] ORF |
| 62 | GAAGAUGUUUAUGAAGCUGUU | AACAGCUUCAUAAACAUCUUC | MO | [1875-1895] ORF | [1584-1604] ORF |
| 63 | GAAUCUCCUUUGCCAUCCCUU | AAGGGAUGGCAAAGGAGAUUC | RaT,MO | [1585-1605] ORF | [1390-1410] ORF |
| 64 | GGCUCUGAAGAAUCACAGAAA | UUUCUGUGAUUCUUCAGAGCC | | [2115-2135] 3'UTR | [1824-1844] 3'UTR |
| 65 | GGACGAGAAACACUGACCUUA | UAAGGUCAGUGUUUCUCGUCC | | [1932-1952] ORF | [1641-1661] ORF |
| 66 | GUGAUGAUGCUGACCCUGAGU | ACUCAGGGUCAGCAUCAUCAC | | [1692-1712] ORF | [1497-1517] ORF |
| 67 | GCUUGGCUACAGAUACUGACA | UGUCAGUAUCUGUAGCCAAGC | | [2409-1429] 3'UTR | [2118-2138] 3'UTR |
| 68 | GUACAAAAUGCUGAAGAUGUU | AACAUCUUCAGCAUUUUGUAC | | [1863-1883] ORF | [1572-1592] ORF |
| 69 | GGAAAAGAAGAAUUCCUCCUC | GAGGAGGAAUUCUUCUUUUCC | | [1637-1657] ORF | [1442-1462] ORF |
| 70 | CUCCUUUGCCAUCCCUUCUGA | UCAGAAGGGAUGGCAAAGGAG | Rat,MO | [1589-1609] ORF | [1394-1414] ORF |
| 71 | AGACCACAAUUCCCGGCAUUC | GAAUGCCGGGAAUUGUGGUCU | | [423-443] 5'UTR | [423-443] 5'UTR |
| 72 | GCUUGACAGUUCCACAUCCAU | AUGGAUGUGGAACUGUCAAGC | | [2460-2480] 3'UTR | [2169-2189] 3'UTR |
| 73 | UGACUCCUGGGCUCUGAAGAA | UUCUUCAGAGCCCAGGAGUCA | | [2106-2126] 3'UTR | [1815-1835] 3'UTR |
| 74 | GCAGUGCUGUUGUUGUUGUGG | CCACAACAACAACAGCACUGC | | [948-968] ORF | [948-968] ORF |
| 75 | AGGUCACAGAAUGAAUAGAUC | GAUCUAUUCAUUCUGUGACCU | | [1966-1986] ORF+3'UTR | [1675-1695] ORF+3'UTR |
| 76 | GCUGAAGAUGUUUAUGAAGCU | AGCUUCAUAAACAUCUUCAGC | MO | [1872-1892] ORF | [1581-1601] ORF |
| 77 | CGGUUGCUGACAUGGGUUUCU | AGAAACCCAUGUCAGCAACCG | | [2501-2521] 3'UTR | [2210-2230] 3'UTR |
| 78 | UGCUUGACAGUUCCACAUCCA | UGGAUGUGGAACUGUCAAGCA | | [2459-2479] 3'UTR | [2168-2188] 3'UTR |
| 79 | GGGCAGUGCUGUUGUUGUUGU | ACAACAACAACAGCACUGCCC | | [946-966] ORF | [946-966] ORF |
| 80 | GAAUGAAUAGAUCACCAAGAG | CUCUUGGUGAUCUAUUCAUUC | | [1974-1994] ORF+3'UTR | [1683-1703] ORF+3'UTR |
| 81 | CAGAAUGAAUAGAUCACCAAG | CUUGGUGAUCUAUUCAUUCUG | | [1972-1992] ORF+3'UTR | [1681-1701] ORF+3'UTR |
| 82 | AUGGUACAAAAUGCUGAAGAU | AUCUUCAGCAUUUUGUACCAU | | [1860-1880] ORF | [1569-1589] ORF |
| 83 | GAAGAAUUCCUCCUCCGGAAU | AUUCCGGAGGAGGAAUUCUUC | | [1643-1663] ORF | [1448-1468] ORF |
| 84 | GAUUGGAGUGAACACCAUGAA | UUCAUGGUGUUCACUCCAAUC | Rat,MO | [1553-1573] ORF | [1358-1378] ORF |
| 85 | CCAUAACUGCUGCUUCAGGAG | CUCCUGAAGCAGCAGUUAUGG | | [222-242] 5'UTR | [222-242] 5'UTR |
| 86 | GGAGCAAAGAUUCCCAUGCUU | AAGCAUGGGAAUCUUUGCUCC | | [2392-2412] 3'UTR | [2101-2121] 3'UTR |
| 87 | CUGAAGAAUCACAGAAACACU | AGUGUUUCUGUGAUUCUUCAG | | [2119-2139] 3'UTR | [1828-1848] 3'UTR |
| 88 | CUCUGAAGAAUCACAGAAACA | UGUUUCUGUGAUUCUUCAGAG | | [2117-2137] 3'UTR | [1826-1846] 3'UTR |
| 89 | UGAUUUCCUCCUUGCCUUUCU | AGAAAGGCAAGGAGGAAAUCA | | [2011-2031] 3'UTR | [1720-1740] 3'UTR |
| 90 | UGGUACAAAAUGCUGAAGAUG | CAUCUUCAGCAUUUUGUACCA | | [1861-1881] ORF | [1570-1590] ORF |
| 91 | GUGAUUGGAGUGAACACCAUG | CAUGGUGUUCACUCCAAUCAC | Rat,MO | [1551-1571] ORF | [1356-1376] ORF |
| 92 | CUCUUACCGGUGCGAGUCAAA | UUUGACUCGCACCGGUAAGAG | | [169-189] 5'UTR | [169-189] 5'UTR |
| 93 | AGAGUCCGUGUGAGACUGCUA | UAGCAGUCUCACACGGACUCU | | [1215-1235] ORF | [1215-1235] ORF |
| 94 | GUUUCUUGGUAAGCUCCUGA | UCAGGAGCUUACCAAGAAACC | | [2515-2535] 3'UTR | [2224-2244] 3'UTR |
| 95 | CCACCAAAAGGCUAGAGGUAA | UUACCUCUAGCCUUUUGGUGG | | [2214-2234] 3'UTR | [1923-1943] 3'UTR |
| 96 | GCUCUGAAGAAUCACAGAAAC | GUUUCUGUGAUUCUUCAGAGC | | [2116-2136] 3'UTR | [1825-1845] 3'UTR |
| 97 | CGGAGUCAGUACAACUUCAUC | GAUGAAGUUGUACUGACUCCG | | [1032-1052] ORF | [1032-1052] ORF |
| 98 | AGAGGGUUAAAUGAACCAGUG | CACUGGUUCAUUUAACCCUCU | | [2058-2078] 3'UTR | [1767-1787] 3'UTR |
| 99 | UGAAGAUGUUUAUGAAGCUGU | ACAGCUUCAUAAACAUCUUCA | MO | [1874-1894] ORF | [1583-1603] ORF |
| 100 | GCGUGCACUUCUGAAGGACUU | AAGUCCUUCAGAAGUGCACGC | | [485-505] 5'UTR | [485-505] 5'UTR |
| 101 | AGACAUCGCAACGCUGAGGAU | AUCCUCAGCGUUGCGAUGUCU | | [1283-1303] ORF | [1283-1303] ORF |
| 102 | CUUGGUAAGCUCCUGAGGUAA | UUACCUCAGGAGCUUACCAAG | | [2520-2540] 3'UTR | [2229-2249] 3'UTR |
| 103 | GGGUUUCUUGGUAAGCUCCUG | CAGGAGCUUACCAAGAAACCC | | [2514-2534] 3'UTR | [2223-2243] 3'UTR |
| 104 | GUACAACUUCAUCGCAGAUGU | ACAUCUGCGAUGAAGUUGUAC | | [1040-1060] ORF | [1040-1060] ORF |
| 105 | GGGCUCUGAAGAAUCACAGAA | UUCUGUGAUUCUUCAGAGCCC | | [2114-2134] 3'UTR | [1823-1843] 3'UTR |
| 106 | CCCGGAGUCAGUACAACUUCA | UGAAGUUGUACUGACUCCGGG | | [1030-1050] ORF | [1030-1050] ORF |
| 107 | CAGAGAACUCUGGAACCCGUU | AACGGGUUCCAGAGUUCUCUG | | [889-909] ORF | [889-909] ORF |
| 108 | CUGAAGAUGUUUAUGAAGCUG | CAGCUUCAUAAACAUCUUCAG | MO | [1873-1893] ORF | [1582-1602] ORF |
| 109 | CCAUGAAGGUCACAGCUGGAA | UUCCAGCUGUGACCUUCAUGG | | [1567-1587] ORF | [1372-1392] ORF |
| 110 | AACGCUGAGGAUUCAGACUAA | UUAGUCUGAAUCCUCAGCGUU | | [1292-1312] ORF | [1292-1312] ORF |
| 111 | CGAUGGGCUCAUUGUCACCAA | UUGGUGACAAUGAGCCCAUCG | | [1169-1189] ORF | [1169-1189] ORF |
| 112 | GUGGUCUAUAUCGAGAUCCUG | CAGGAUCUCGAUAUAGACCAC | | [1083-1103] ORF | [1083-1103] ORF |
| 113 | GAAAACUUCUGCUUGACAGUU | AACUGUCAAGCAGAAGUUUUC | | [2450-2470] 3'UTR | [2159-2179] 3'UTR |
| 114 | UCCCAUGCUUGGCUACAGAUA | UAUCUGUAGCCAAGCAUGGGA | | [2403-2423] 3'UTR | [2112-2132] 3'UTR |
| 115 | AGGCUAGAGGUAAAGCUGUAU | AUACAGCUUUACCUCUAGCCU | | [2222-2242] 3'UTR | [1931-1951] 3'UTR |
| 116 | CACCGAGACAGAGGGUUAAAU | AUUUAACCCUCUGUCUCGGUG | | [2049-2069] 3'UTR | [1758-1778] 3'UTR |
| 117 | CAAGAGUAUGAGGCUCCUGCU | AGCAGGAGCCUCAUACUCUUG | | [1989-2009] 3'UTR | [1698-1718] 3'UTR |
| 118 | AGAAGAAUUCCUCCUCCGGAA | UUCCGGAGGAGGAAUUCUUCU | | [1642-1662] ORF | [1447-1467] ORF |
| 119 | GGGAAAAGAAGAAUUCCUCCU | AGGAGGAAUUCUUCUUUUCCC | | [1636-1656] ORF | [1441-1461] ORF |
| 120 | ACGCUGAGGAUUCAGACUAAG | CUUAGUCUGAAUCCUCAGCGU | | [1293-1313] ORF | [1293-1313] ORF |
| 121 | CAACGCUGAGGAUUCAGACUA | UAGUCUGAAUCCUCAGCGUUG | | [1291-1311] ORF | [1291-1311] ORF |
| 122 | AGCACCUGCCGUGGUCUAUAU | AUAUAGACCACGGCAGGUGCU | | [1073-1093] ORF | [1073-1093] ORF |
| 123 | CCAUAGUGCAUGGUCUGAUGA | UCAUCAGACCAUGCACUAUGG | | [2477-2497] 3'UTR | [2166-2206] 3'UTR |
| 124 | UGAAAACUUCUGCUUGACAGU | ACUGUCAAGCAGAAGUUUUCA | | [2449-2469] 3'UTR | [2158-2178] 3'UTR |
| 125 | GCCUCUGAAGGAGGGUGAAAA | UUUUCACCCUCCUUCAGAGGC | | [2434-2454] 3'UTR | [2143-2163] 3'UTR |
| 126 | CCUGACCUCCUAUUAAAGAAA | UUUCUUUAAUAGGAGGUCAGG | | [2279-2299] 3'UTR | [1988-2008] 3'UTR |
| 127 | CUGACCAUCCUGACCUCCUAU | AUAGGAGGUCAGGAUGGUCAG | | [2271-2291] 3'UTR | [1980-2000] 3'UTR |
| 128 | CUUGCCUUUCUGGCUGAGGUU | AACCUCAGCCAGAAAGGCAAG | | [2021-2041] 3'UTR | [1730-1750] 3'UTR |
| 129 | ACCAAGAGUAUGAGGCUCCUG | CAGGAGCCUCAUACUCUUGGU | | [1987-2007] 3'UTR | [1696-1716] 3'UTR |
| 130 | CAUGAAGGUCACAGCUGGAAU | AUUCCAGCUGUGACCUUCAUG | | [1568-1588] ORF | [1373-1383] ORF |
| 131 | ACCAUGAAGGUCACAGCUGGA | UCCAGCUGUGACCUUCAUGGU | | [1566-1586] ORF | [1371-1391] ORF |
| 132 | CGUGGUCUAUAUCGAGAUCCU | AGGAUCUCGAUAUAGACCACG | | [1082-1102] ORF | [1082-1102] ORF |
| 133 | CAGUUCCACAUCCAUAGUGCA | UGCACUAUGGAUGUGGAACUG | | [2466-2486] 3'UTR | [2175-2195] 3'UTR |
| 134 | CCUCUGAAGGAGGGUGAAAAC | GUUUUCACCCUCCUUCAGAGG | | [2435-2455] 3'UTR | [2144-2164] 3'UTR |
| 135 | GACAGAGGGUUAAAUGAACCA | UGGUUCAUUUAACCCUCUGUC | | [2055-2075] 3'UTR | [1764-1784] 3'UTR |
| 136 | AUGAGGCUCCUGCUCUGAUUU | AAAUCAGAGCAGGAGCCUCAU | | [1996-2016] 3'UTR | [1705-1725] 3'UTR |
| 137 | CACCAAGAGUAUGAGGCUCCU | AGGAGCCUCAUACUCUUGGUG | | [1986-2008] 3'UTR | [1695-1715] 3'UTR |
| 138 | UGCUGACGUCAGGAACUUCUG | CAGAAGUUCCUGACGUCAGCA | | [715-735] ORF | [715-735] ORF |
| 139 | CCCUUCUGAUCGUCUUCGAGA | UCUCGAAGACGAUCAGAAGGG | | [1601-1621] ORF | [1406-1426] ORF |
| 140 | CCAUCCCUUCUGAUCGUCUUC | GAAGACGAUCAGAAGGGAUGG | | [1597-1617] ORF | [1402-1422] ORF |
| 141 | GGUGAUUGGAGUGAACACGAU | AUGGUGUUCACUCCAAUCACC | Rat,MO | [1550-1570] ORF | [1355-1375] ORF |
| 142 | GGAAGCUCCAUAACUGCUGCU | AGCAGCAGUUAUGGAGCUUCC. | | [215-235] 5'UTR | [215-235] 5'UTR |
| 143 | UACUGACAGCUGGCCUCUGAA | UUCAGAGGCCAGCUGUCAGUA | | [2422-2442] 3'UTR | [2131-2151] 3'UTR |
| 144 | AGAUACUGACAGCUGGCCUCU | AGAGGCCAGCUGUCAGUAUCU | | [2419-2439] 3'UTR | [2128-2148] 3'UTR |
| 145 | GGGAGCAAAGAUUCCCAUGCU | AGCAUGGGAAUCUUUGCUCCC | | [2391-2411] 3'UTR | [2100-2120] 3'UTR |
| 146 | GUUAGUCAGGUGCUGCUCUUU | AAAGAGCAGCACCUGACUAAC | | [2327-2347] 3'UTR | [2036-2056] 3'UTR |
| 147 | GAAAAUGAGCUGCUGCCAUCU | AGAUGGCAGCAGCUCAUUUUC | | [2296-2316] 3'UTR | [2005-2025] 3'UTR |
| 148 | CCUCCUAUUAAAGAAAAUGAG | CUCAUUUUCUUUAAUAGGAGG | | [2284-2304] 3'UTR | [1993-2013] 3'UTR |
| 149 | CUGACCUCCUAUUAAAGAAAA | UUUUCUUUAAUAGGAGGUCAG | | [2280-2300] 3'UTR | [1989-2009] 3'UTR |
| 150 | GACCAUCCUGACCUCCUAUUA | UAAUAGGAGGUCAGGAUGGUC | | [2273-2293] 3'UTR | [1982-2002] 3'UTR |
| 151 | CACAGAAUGAAUAGAUCACCA | UGGUGAUCUAUUCAUUCUGUG | | [1970-1990] ORF+3'UTR | [1679-1699] ORF+3'UTR |
| 152 | GAAUUCCUCCUCCGGAAUCAG | CUGAUUCCGGAGGAGGAAUUC | | [1646-1666] ORF | [1451-1471] ORF |
| 153 | GCCAUCCCUUCUGAUCGUCUU | AAGACGAUCAGAAGGGAUGGC | | [1596-1616] ORF | [1401-1421] ORF |
| 154 | CUAUCUCGAACGGCUCAGGAU | AUCCUGAGCCGUUCGAGAUAG | | [1135-1155] ORF | [1135-1155] ORF |
| 155 | UGACAGUUCCACAUCCAUAGU | ACUAUGGAUGUGGAACUGUCA | | [2463-2483] 3'UTR | [2172-2192] 3'UTR |
| 156 | GGCUACAGAUACUGACAGCUGI | CAGCUGUCAGUAUCUGUAGCC | | [2413-2433] 3'UTR | [2122-2142] 3'UTR |
| 157 | CCAUGCUUGGCUACAGAUACU | AGUAUCUGUAGCCAAGCAUGG | | [2405-2425] 3'UTR | [2114-2134] 3'UTR |
| 158 | GCCAUCUUUUGUGGGCAGUUA | UAACUGCCCACAAAAGAUGGC | | [2310-2330] 3'UTR | [2019-2039] 3'UTR |
| 159 | UGCCAUCUUUUGUGGGCAGUU | AACUGCCCACAAAAGAUGGCA | | [2309-2329] 3'UTR | [2018-2038] 3'UTR |
| 160 | GGAGAUACUGGAGCUGACCAU | AUGGUCAGCUCCAGUAUCUCC | | [2258-2278] 3'UTR | [1967-1987] 3'UTR |
| 161 | CACCAAAAGGCUAGAGGUAAA | UUUACCUCUAGCCUUUUGGUG | | [2215-2235] 3'UTR | [1924-1944] 3'UTR |
| 162 | CCCACCAAAAGGCUAGAGGUA | UACCUCUAGCCUUUUGGUGGG | | [2213-2233] 3'UTR | [1922-1942] 3'UTR |
| 163 | UGGGCUCUGAAGAAUCACAGA | UCUGUGAUUCUUCAGAGCCCA | | [2113-2133] 3'UTR | [1822-1842] 3'UTR |
| 164 | GGUCACAGAAUGAAUAGAUCA | UGAUCUAUUCAUUCUGUGACC | | [1967-1987] ORF+3'UTR | [1676-1696] ORF+3'UTR |
| 165 | GAAGCUCCAUAACUGCUGCUU | AAGCAGCAGUUAUGGAGCUUC | | [216-236] 5'UTR | [216-236] 5'UTR |
| 166 | CCGUGGUCUAUAUCGAGAUCC | GGAUCUCGAUAUAGACCACGG | | [1081-1101] ORF | [1081-1101] ORF |
| 167 | UCCAUAGUGCAUGGUCUGAUG | CAUCAGACCAUGCACUAUGGA | | [2476-2496] 3'UTR | [2185-2205] 3'UTR |
| 168 | UGAAGGAGGGUGAAAACUUCU | AGAAGUUUUCACCCUCCUUCA | | [2439-2459] 3'UTR | [2148-2168] 3'UTR |
| 169 | GAGCAAAGAUUCCCAUGCUUG | CAAGCAUGGGAAUCUUUGCUC | | [2393-2413] 3'UTR | [2102-2122] 3'UTR |
| 170 | GACCUCCUAUUAAAGAAAAUG | CAUUUUCUUUAAUAGGAGGUC | | [2282-2302] 3'UTR | [1991-2011] 3'UTR |
| 171 | ACCAAAAGGCUAGAGGUAAAG | CUUUACCUCUAGCCUUUUGGU | | [2216-2236] 3'UTR | [1925-1945] 3'UTR |
| 172 | GCACUUCUGAAGGACUUCAGG | CCUGAAGUCCUUCAGAAGUGC | | [489-509] 5'UTR | [489-509] 5'UTR |
| 173 | CUGAUCGUCUUCGAGAGUUUC | GAAACUCUCGAAGACGAUCAG | | [1606-1626] ORF | [1411-1431] ORF |
| 174 | UCUGAUCGUCUUCGAGAGUUU | AAACUCUCGAAGACGAUCAGA | | [1605-1625] ORF | [1410-1430] ORF |
| 175 | AGGUGAUUGGAGUGAACACCA | UGGUGUUCACUCCAAUCACCU | Rat,MO | [1549-1569] ORF | [1354-1374] ORF |
| 176 | GCUCCAUAACUGCUGCUUCAG | CUGAAGCAGCAGUUAUGGAGC | | [219-239] 5'UTR | [219-239] 5'UTR |
| 177 | CCCAUGCUUGGCUACAGAUAC | GUAUCUGUAGCCAAGCAUGGG | | [2404-2424] 3'UTR | [2113-2133] 3'UTR |
| 178 | CCAAAAGGCUAGAGGUAAAGC | GCUUUACCUCUAGCCUUUUGG | | [2217-2237] 3'UTR | [1926-1946] 3'UTR |
| 179 | CCCUGAGGUCACAGAAUGAAU | AUUCAUUCUGUGACCUCAGGG | | [1961-1981] ORF+3'UTR | [1670-1690] ORF+3'UTR |
| 180 | UCCCUUCUGAUCGUCUUCGAG | CUCGAAGACGAUCAGAAGGGA | | [1600-1620] ORF | [1405-1425] ORF |
| 181 | CGUGCACUUCUGAAGGACUUC | GAAGUCCUUCAGAAGUGCACG | | [486-506] 5'UTR | [486-506] 5'UTR |
| 182 | CCACAUCCAUAGUGCAUGGUC | GACCAUGCACUAUGGAUGUGG | | [2471-2491] 3'UTR | [2180-2200] 3'UTR |
| 183 | ACAGAUACUGACAGCUGGCCU | AGGCCAGCUGUCAGUAUCUGU | | [2417-2437] 3'UTR | [2126-2146] 3'UTR |
| 184 | UGGCUACAGAUACUGACAGCU | AGCUGUCAGUAUCUGUAGCCA | | [2412-2432] 3'UTR | [2121-2141] 3'UTR |
| 185 | CUGCCAUCUUUUGUGGGCAGU | ACUGCCCACAAAAGAUGGCAG | | [2308-2328] 3'UTR | [2017-2037] 3'UTR |
| 186 | UCCUGACCUCCUAUUAAAGAA | UUCUUUAAUAGGAGGUCAGGA | | [2278-2298] 3'UTR | [1987-2007] 3'UTR |
| 187 | GGCUAGAGGUAAAGCUGUAUC | GAUACAGCUUUACCUCUAGCC | | [2223-2243] 3'UTR | [1932-1952] 3'UTR |
| 188 | UGCACUUCUGAAGGACUUCAG | CUGAAGUCCUUCAGAAGUGCA | | [488-508] 5'UTR | [488-508] 5'UTR |
| 189 | UGCCAUCCCUUCUGAUCGUCU | AGACGAUCAGAAGGGAUGGCA | | [1595-1615] ORF | [1400-1420] ORF |
| 190 | CCUGGGAGCAAAGAUUCCCAU | AUGGGAAUCUUUGCUCCCAGG | | [2388-2408] 3'UTR | [2097-2117] 3'UTR |
| 191 | UGACCAUCCUGACCUCCUAUU | AAUAGGAGGUCAGGAUGGUCA | | [2272-2292] 3'UTR | [1981-2001] 3'UTR |
| 192 | GAGCAGAUGGUACAAAAUGCU | AGCAUUUUGUACCAUCUGCUC | | [1854-1874] ORF | [1563-1583] ORF |
| 193 | UCACAGCUGGAAUCUCCUUUG | CAAAGGAGAUUCCAGCUGUGA | | [1576-1596] ORF | [1381-1401] ORF |
| 194 | UUCCCAUGCUUGGCUACAGAU | AUCUGUAGCCAAGCAUGGGAA | | [2402-2422] 3'UTR | [2111-2131] 3'UTR |
| 195 | AAAGAUUCCCAUGCUUGGCUA | UAGCCAAGCAUGGGAAUCUUU | | [2397-2417] 3'UTR | [2106-2126] 3'UTR |
| 196 | GGAGCAGAUGGUACAAAAUGC | GCAUUUUGUACCAUCUGCUCC | | [1853-1673] ORF | [1562-1582] ORF |
| 197 | GUGAACACCAUGAAGGUCACA | UGUGACCUUCAUGGUGUUCAC | | [1560-1580] ORF | [1365-1385] ORF |
| 198 | ACAUCCAUAGUGCAUGGUCUG | CAGACCAUGCACUAUGGAUGU | | [2473-2493] 3'UTR | [2162-2202] 3'UTR |
| 199 | GGAAUCUCCUUUGCCAUCCCU | AGGGAUGGCAAAGGAGAUUCC | Rat,MO | [1584-1604] ORF | [1389-1409] ORF |
| 200 | GGUCACAGCUGGAAUCUCCUU | AAGGAGAUUCCAGCUGUGACC | | [1574-1594] ORF | [1379-1399] ORF |
| 201 | GGAGUGAACACCAUGAAGGUC | GACCUUCAUGGUGUUCACUCC | | [1557-1577] ORF | [1362-1382] ORF |
| 202 | UGGAGUGAACACCAUGAAGGU | ACCUUCAUGGUGUUCACUCCA | Rat,MO | [1556-1576] ORF | [1361-1381] ORF |
| 203 | CCUGAGGUCACAGAAUGAAUA | UAUUCAUUCUGUGACCUCAGG | | [1962-1982] ORF+3'UTR | [1671-1691] ORF+3'UTR |
| 204 | CUGAGGUCACAGAAUGAAUAG | CUAUUCAUUCUGUGACCUCAG | | [1963-1983]ORF+3'UTR | [1672-1692] ORF+3'UTR |
| 205 | AGCUGGAAUCUCCUUUGCCAU | AUGGCAAAGGAGAUUCCAGCU | Rat,MO | [1560-1600] ORF | [1385-1405] ORF |
| 206 | AGGUCACAGCUGGAAUCUCCU | AGGAGAUUCCAGCUGUGACCU | | [1573-1593] ORF | [1378-1398] ORF |
| 207 | GCAACGCUGAGGAUUCAGACU | AGUCUGAAUCCUCAGCGUUGC | | [1290-1310] ORF | [1290-1310] ORF |
| 208 | UCCACAUCCAUAGUGCAUGGU | ACCAUGCACUAUGGAUGUGGA | | [2470-2490] 3'UTR | [2179-2199] 3'UTR |
| 209 | AGGCUCCUGCUCUGAUUUCCU | AGGAAAUCAGAGCAGGAGCCU | | [199-2019] 3'UTR | [1708-1728] 3'UTR |

**Table E: 19-mer kelch-like ECH-associated protein 1 (KEAP1)**

| No. | Sense siRNA | AntiSense siRNA | Other Sp | Human 45269144 ORF:186-2060 | Human 45269143 ORF:157-2031 |
|---|---|---|---|---|---|
| 1 | GGAACGAGUGGCGAAUGAU | AUCAUUCGCCACUCGUUCC | CHMP | [1666-1684] ORF | [1637-1655] ORF |
| 2 | GGGCAAAAAUACAGUCCAA | UUGGACUGUAUUUUUGCCC | | [2077-2095] 3'UTR | [2048-2066] 3'UTR |
| 3 | AGAGGAACGAGUGGCGAAU | AUUCGCCACUCGUUCCUCU | CHMP | [1663-1681] ORF | [1634-1652] ORF |
| 4 | GGAGUAUCAUUGUUUUUGU | ACAAAAACAAUGAUACUCC | CHMP | [2099-2117] 3'UTR | [2070-2088] 3'UTR |
| 5 | GCCUCAUUGAAUUCGCCUA | UAGGCGAAUUCAAUGAGGC | | [589-607] ORF | [560-578] ORF |
| 6 | GGCAAAAAUACAGUCCAAU | AUUGGACUGUAUUUUUGCC | | [2076-2096] 3'UTR | [2049-2067] 3'UTR |
| 7 | GGAUGCCUCAGUGUUAAAA | UUUUAACACUGAGGCAUCC | CHMP | [2183-2201] 3'UTR | [2154-2172] 3'UTR |
| 8 | CGGGACUAAAAGAAAAGAC | GUCUUUUCUUUUAGUCCCG | CHMP | [2126-2144] 3'UTR | [2097-2115] 3'UTR |
| 9 | UCAUUGAAUUCGCCUACAC | GUGUAGGCGAAUUCAAUGA | | [592-610] ORF | [563-581] ORF |
| 10 | GCACUGCAAAUAACCCAUC | GAUGGGUUAUUUGCAGUGC | CHMP | [2146-2164] 3'UTR | [2117-2135] 3'UTR |
| 11 | GGGACUAAAAGAAAAGACA | UGUCUUUUCUUUUAGUCCC | CHMP | [2127-2145] 3'UTR | [2098-2116] 3'UTR |
| 12 | CCGGGACUAAAAGAAAAGA | UCUUUUCUUUUAGUCCCGG | CHMP | [2125-2143] 3'UTR | [2096-2114] 3'UTR |
| 13 | AGAAAAGACAGCACUGCAA | UUGCAGUGCUGUCUUUUCU | CHMP | [2136-2154] 3'UTR | [2107-2125] 3'UTR |
| 14 | CUCAUUGAAUUCGCCUACA | UGUAGGCGAAUUCAAUGAG | | [591-609] ORF | [562-580] ORF |
| 15 | UGAUAAGUAACCCUGUAAU | AUUACAGGGUUACUUAUCA | CHMP | [2528-2546] 3'UTR | [2499-2517] 3'UTR |
| 16 | UGGUGGUGUUGCUUAUCUU | AAGAUAAGCAACACCACCA | | [157-175] 5'UTR | [128-146] 5'UTR |
| 17 | GGGAGUAUCAUUGUUUUUG | CAAAAACAAUGAUACUCCC | CHMP | [2098-2116] 3'UTR | [2069-2087] 3'UTR |
| 18 | UGUUGCUUAUCUUCUGGAA | UUCCAGAAGAUAAGCAACA | CHMP | [163-181] 5'UTR | [134-152] 5'UTR |
| 19 | CCUCAUUGAAUUCGCCUAC | GUAGGCGAAUUCAAUGAGG | | [590-608] ORF | [561-579] ORF |
| 20 | CACCAUGUGAUUUAUUCUU | AAGAAUAAAUCACAUGGUG | | [2381-2399] 3'UTR | [2352-2370] 3'UTR |
| 21 | ACUGCAAAUAACCCAUCUU | AAGAUGGGUUAUUUGCAGU | CHMP | [2148-2166] 3'UTR | [2119-2137] 3'UTR |
| 22 | CUAAAAGAAAAGACAGCAC | GUGCUGUCUUUUCUUUUAG | CHMP | [2131-2149] 3'UTR | [2102-2120] 3'UTR |
| 23 | GCAAAAAUACAGUCCAAUG | CAUUGGACUGUAUUUUUGC | | [2079-2097] 3'UTR | [2050-2068] 3'UTR |
| 24 | UCCUGCACAACUGUAUCUA | UAGAUACAGUUGUGCAGGA | CHMP | [1726-1744] ORF | [1697-1715] ORF |
| 25 | CCCGGGAGUACAUCUACAU | AUGUAGAUGUACUCCCGGG | CHMP | [793-611] ORF | [764-782] ORF |
| 26 | CUGUCUUCAAGGCCAUGUU | AACAUGGCCUUGAAGACAG | CHMP | [499-517] ORF | [470-488] ORF |
| 27 | AGAACAGACUAACUAGUGU | ACACUAGUUAGUCUGUUCU | CHMP | [2563-2581] 3'UTR | [2534-2552] 3'UTR |
| 28 | CACUGCAAAUAACCCAUCU | AGAUGGGUUAUUUGCAGUG | CHMP | [2147-2165] 3'UTR | [2118-2136] 3'UTR |
| 29 | GGACUAAAAGAAAAGACAG | CUGUCUUUUCUUUUAGUCC | CHMP | [2128-2146] 3'UTR | [2099-2117] 3'UTR |
| 30 | ACCGGGACUAAAAGAAAAG | CUUUUCUUUUAGUCCCGGU | CHMP | [2124-2142] 3'UTR | [2095-2113] 3'UTR |
| 31 | CCUGCACAACUGUAUCUAU | AUAGAUACAGUUGUGCAGG | CHMP | [1727-1745] ORF | [1698-1716] ORF |
| 32 | CCUCAAUCGUCUCCUUUAU | AUAAAGGAGACGAUUGAGG | | [1586-1604] ORF | [1557-1575] ORF |
| 33 | UCAUGUACCAGAUCGACAG | CUGUCGAUCUGGUACAUGA | CHMP | [664-682] ORF | [635-653] ORF |
| 34 | GUGUUGCUUAUCUUCUGGA | UCCAGAAGAUAAGCAACAC | CHMP | [162-180] 5'UTR | [133-151] 5'UTR |
| 35 | UGGUGUUGCUUAUCUUCUG | CAGAAGAUAAGCAACACCA | CHMP | [160-178] 5'UTR | [131-149] 5'UTR |
| 36 | CCAUGUGAUUUAUUCUUGG | CCAAGAAUAAAUCACAUGG | | [2383-2401] 3'UTR | [2354-2372] 3'UTR |
| 37 | GCAGCUGUCACCAUGUGAU | AUCACAUGGUGACAGCUGC | | [2373-2391] 3'UTR | [2344-2362] 3'UTR |
| 38 | CAUCUCAAAAGAAGUCCAA | UUGGACUUCUUUUGAGAUG | CHMP | [2205-2223] 3'UTR | [2176-2194] 3'UTR |
| 39 | AGCACUGCAAAUAACCCAU | AUGGGUUAUUUGCAGUGCU | CHMP | [2145-2163] 3'UTR | [2116-2134] 3'UTR |
| 40 | AACCGGGACUAAAAGAAAA | UUUUCUUUUAGUCCCGGUU | CHMP | [2123-2141] 3'UTR | [2094-2112] 3'UTR |
| 41 | ACAAAAACCGGGACUAAAA | UUUUAGUCCCGGUUUUUGU | CHMP | [2118-2136] 3'UTR | [2089-2107] 3'UTR |
| 42 | GUACAAAAACCGGGACUAA | UUAGUCCCGGUUUUUGUAC | CHMP | [2116-2134] 3'UTR | [2087-2105] 3'UTR |
| 43 | UGAGGCACUUUUGUUUCUU | AAGAAACAAAAGUGCCUCA | CHMP | [2056-2076] ORF+3'UTR | [2029-2047] ORF+3'UTR |
| 44 | CCUUAAUUCAGCUGAGUGU | ACACUCAGCUGAAUUAAGG | CHMP | [1634-1652] ORF | [1605-1623] ORF |
| 45 | ACAGUGUGGAGAGGUAUGA | UCAUACCUCUCCACACUGU | CHMP | [1498-1516] ORF | [1469-1487] ORF |
| 46 | AGGUGGUGGUGUUGCUUAU | AUAAGCMCACCACCACCU | | [154-172] 5'UTR | [125-143] 5'UTR |
| 47 | CGCCUCAUUGAAUUCGCCU | AGGCGAAUUCAAUGAGGCG | | [588-606] ORF | [559-577] ORF |
| 48 | GUGUUAAAAUGACAUCUCA | UGAGAUGUCAUUUUAACAC | CHMP | [2193-2211] 3'UTR | [2164-2182] 3'UTR |
| 49 | AGGAUGCCUCAGUGUUAAA | UUUAACACUGAGGCAUCCU | CHMP | [2182-2200] 3'UTR | [2153-2171] 3'UTR |
| 50 | ACGUCACACUGCAGGUCAA | UUGACCUGCAGUGUGACGU | CHMP | [418-436] ORF | [389-407] ORF |
| 51 | CGAUGUGGAAACAGAGACG | CGUCUCUGUUUCCACAUCG | CHMP | [1796-1814] ORF | [1767-1785] ORF |
| 52 | CGCUACGAUGUGGAAACAG | CUGUUUCCACAUCGUAGCG | CHMP | [1791-1809] ORF | [1762-1780] ORF |
| 53 | AGCGCUACGAUGUGGAAAC | GUUUCCACAUCGUAGCGCU | CHMP | [1789-1807] ORF | [1760-1778] ORF |
| 54 | GUCCUGCACAACUGUAUCU | AGAUACAGUUGUGCAGGAC | CHMP | [1725-1743] ORF | [1696-1714] ORF |
| 55 | CAACAGUGUGGAGAGGUAU | AUACCUCUCCACACUGUUG | CHMP | [1496-1514] ORF | [1467-1485] ORF |
| 56 | UGCAUCAACUGGGUCAAGU | ACUUGACCCAGUUGAUGCA | CHMP | [930-948] ORF | [901-919] ORF |
| 57 | GAACGGUGCUGUCAUGUAC | GUACAUGACAGCACCGUUC | | [653-671] ORF | [624-642] ORF |
| 58 | UCCAAGGAAAAUAAAGAAC | GUUCUUUAUUUUCCUUGGA | CHMP | [2549-2567] 3'UTR | [2520-2536] 3'UTR |
| 59 | UUGAUAAGUAACCCUGUAA | UUACAGGGUUACUUAUCAA | CHMP | [2527-2545] 3'UTR | [2498-2516] 3'UTR |
| 60 | UGGACAGUUAUUUUGUUGA | UCAACAAAAUAACUGUCCA | CHMP | [2512-2530] 3'UTR | [2463-2501] 3'UTR |
| 61 | GUACAUAGAAGCCACCGGA | UCCGGUGGCUUCUAUGUAC | CHMP | [2475-2493] 3'UTR | [2446-2464] 3'UTR |
| 62 | UCAGUGUUAAAAUGACAUC | GAUGUCAUUUUAACACUGA | CHMP | [2190-2208] 3'UTR | [2161-2179] 3'UTR |
| 63 | CUCAGUGUUAAAAUGACAU | AUGUCAUUUUAACACUGAG | CHMP | [2189-2207] 3'UTR | [2160-2178] 3'UTR |
| 64 | CAAAAAUACAGUCCAAUGG | CCAUUGGACUGUAUUUUUG | | [2080-2098] 3'UTR | [2051-2069] 3'UTR |
| 65 | UCAAGUACGACUGCGAACA | UGUUCGCAGUCGUACUUGA | CHMP | [943-961] ORF | [914-932] ORF |
| 66 | AGAUUGGCUGUGUGGAGUU | AACUCCACACAGCCAAUCU | | [763-781] ORF | [734-752] ORF |
| 67 | UGAACGGUGCUGUCAUGUA | UACAUGACAGCACCGUUCA | | [652-670] ORF | [623-641] ORF |
| 68 | GGAGCGCCUCAUUGAAUUC | GAAUUCAAUGAGGCGCUCC | | [584-602] ORF | [555-573] ORF |
| 69 | GGUGUCCAUUGAGGGUAUC | GAUACCCUCAAUGGACACC | CHMP | [551-569] ORF | [522-540] ORF |
| 70 | AGUGUUAAAAUGACAUCUC | GAGAUGUCAUUUUAACACU | CHMP | [2192-2210] 3'UTR | [2163-2181] 3'UTR |
| 71 | CAGUGUUAAAAUGACAUCU | AGAUGUCAUUUUAACACUG | CHMP | [2191-2209] 3'UTR | [2162-2180] 3'UTR |
| 72 | CCUCAGUGUUAAAAUGACA | UGUCAUUUUAACACUGAGG | CHMP | [2188-2206] 3'UTR | [2159-2177] 3'UTR |
| 73 | UGUACAAAAACCGGGACUA | UAGUCCCGGUUUUUGUACA | CHMP | [2115-2133] 3'UTR | [2086-2104] 3'UTR |
| 74 | GCACUUUUGUUUCUUGGGC | GCCCAAGAAACAAAAGUGC | CHMP | [2062-2080] 3'UTR | [2033-2051] 3'UTR |
| 75 | GAGGCACUUUUGUUUCUUG | CAAGAAACAAAAGUGCCUC | CHMP | [2059-2077] ORF+3'UTR | [2030-2048] ORF+3'UTR |
| 76 | ACAGUGUGGAGUGUUACGA | UCGUAACACUCCACACUGU | CHMP | [1921-1939] ORF | [1892-1910] ORF |
| 77 | CCUUUGGCAUCAUGAACGA | UCGUUCAUGAUGCCAAAGG | CHMP | [373-391] ORF | [344-362] ORF |
| 78 | ACUUUUGUUUCUUGGGCAA | UUGCCCAAGAAACAAAAGU | CHMP | [2064-2082] 3'UTR | [2035-2053] 3'UTR |
| 79 | GCAGAUUGACCAGCAGAAC | GUUCUGCUGGUCAAUCUGC | CHMP | [2030-2048] ORF | [2001-2019] ORF |
| 80 | GGACAGUGUGGAGUGUUAC | GUAACACUCCACACUGUCC | CHMP | [1919-1937] ORF | [1890-1908] ORF |
| 81 | GGAAACAGAGACGUGGACU | AGUCCACGUCUCUGUUUCC | CHMP | [1802-1820] ORF | [1773-1791] ORF |
| 82 | UGGAAACAGAGACGUGGAC | GUCCACGUCUCUGUUUCCA | CHMP | [1801-1819] ORF | [1772-1790] ORF |
| 83 | GAUUUAUUCUUGGAUACCU | AGGUAUCCAAGAAUAAAUC | CHMP | [2389-2407] 3'UTR | [2360-2376] 3'UTR |
| 84 | UUUGUUUCUUGGGCAAAAA | UUUUUGCCCAAGAAACAAA | CHMP | [2067-2085] 3'UTR | [2038-2056] 3'UTR |
| 85 | CACUUUUGUUUCUUGGGCA | UGCCCAAGAAACAAAAGUG | CHMP | [2063-2081] 3'UTR | [2034-2052] 3'UTR |
| 86 | CUGGACAGUGUGGAGUGUU | AACACUCCACACUGUCCAG | CHMP | [1917-1935] ORF | [1888-1906] ORF |
| 87 | CCGGGAGUACAUCUACAUG | CAUGUAGAUGUACUCCCGG | CHMP | [794-812] ORF | [765-783] ORF |
| 88 | CCAAGGAAAAUAAAGAACA | UGUUCUUUAUUUUCCUUGG | CHMP | [2550-2568] 3'UTR | [2521-2539] 3'UTR |
| 89 | GGAAAAUAAAGAACAGACU | AGUCUGUUCUUUAUUUUCC | CHMP | [2554-2572] 3'UTR | [2525-2543] 3'UTR |
| 90 | UGGCGAAUGAUCACAGCAA | UUGCUGUGAUCAUUCGCCA | CHMP | [1674-1692] ORF | [1645-1663] ORF |
| 91 | CCGGAUGGACAGUUAUUUU | AAAAUAACUGUCCAUCCGG | CHMP | [2507-2525] 3'UTR | [2478-2496] 3'UTR |
| 92 | AGCAGGCCUUUGGCAUCAU | AUGAUGCCAAAGGCCUGCU | CHMP | [367-385] ORF | [338-356] ORF |
| 93 | GUGCUGUCAUGUACCAGAU | AUCUGGUACAUGACAGCAC | | [658-676] ORF | [629-647] ORF |
| 94 | CAAGGAAAAUAAAGAACAG | CUGUUCUUUAUUUUCCUUG | CHMP | [2551-2569] 3'UTR | [2522-2540] 3'UTR |
| 95 | GCUGUCACCAUGUGAUUUA | UAAAUCACAUGGUGACAGC | | [2376-2394] 3'UTR | [2347-2365] 3'UTR |
| 96 | CCGCCUUAAUUCAGCUGAG | CUCAGCUGAAUUAAGGCGG | CHMP | [1631-1649] ORF | [1602-1620] ORF |
| 97 | GGAGGAUCAUACCAAGCAG | CUGCUUGGUAUGAUCCUCC | CHMP | [353-371] ORF | [324-342] ORF |
| 98 | UGGUGUCCAUUGAGGGUAU | AUACCCUCAAUGGACACCA | CHMP | [550-566] ORF | [521-539] ORF |
| 99 | CGGAUGGACAGUUAUUUUG | CAAAAUAACUGUCCAUCCG | CHMP | [2508-2526] 3'UTR | [2479-2497] 3'UTR |
| 100 | GCCUCAGUGUUAAAAUGAC | GUCAUUUUAACACUGAGGC | CHMP | [2187-2205] 3'UTR | [2158-2176] 3'UTR |
| 101 | GCGUCCUGCACAACUGUAU | AUACAGUUGUGCAGGACGC | CHMP | [1723-1741] ORF | [1694-1712] ORF |
| 102 | CGAGUGGCGAAUGAUCACA | UGUGAUCAUUCGCCACUCG | CHMP | [1670-1688] ORF | [1641-1659] ORF |
| 103 | CCCUGGAGGAUCAUACCAA | UUGGUAUGAUCCUCCAGGG | CHMP | [349-367] ORF | [320-338] ORF |
| 104 | GGAUGGACAGUUAUUUUGU | ACAAAAUAACUGUCCAUCC | CHMP | [2509-2527] 3'UTR | [2480-2498] 3'UTR |
| 105 | GCAAUGAACACCAUCCGAA | UUCGGAUGGUGUUCAUUGC | CHMP | [1689-1707] ORF | [1660-1678] ORF |
| 106 | AGGAAAAUAAAGAAGAGAC | GUCUGUUCUUUAUUUUCCU | CHMP | [2553-2571] 3'UTR | [2524-2542] 3'UTR |
| 107 | CCUGUAAUUUUCCAAGGAA | UUCCUUGGAAAAUUACAGG | CHMP | [2539-2557] 3'UTR | [2510-2528] 3'UTR |
| 108 | CCACCGGAUGGACAGUUAU | AUAACUGUCCAUCCGGUGG | CHMP | [2504-2522] 3'UTR | [2475-2493] 3'UTR |
| 109 | AGAAGUCCAAAGCGGGAAU | AUUCCCGCUUUGGACUUCU | CHMP | [2214-2232] 3'UTR | [2185-2203] 3'UTR |
| 110 | CGGGACAAACCGCCUUAAU | AUUAAGGCGGUUUGUCCCG | CHMP | [1622-1640] ORF | [1593-1611] ORF |
| 111 | ACACCCUGGAGGAUCAUAC | GUAUGAUCCUCCAGGGUGU | CHMP | [346-364] ORF | [317-335] ORF |
| 112 | CUACACCCUGGAGGAUCAU | AUGAUCCUCCAGGGUGUAG | CHMP | [344-362] ORF | [315-333] ORF |
| 113 | UGAGCAGAUUGGCUGUGUG | CACACAGCCAAUCUGCUCA | | [758-776] ORF | [729-747] ORF |
| 114 | GUACCAGAUCGACAGCGUU | AACGCUGUCGAUCUGGUAC | CHMP | [668-686] ORF | [639-657] ORF |
| 115 | GUGGUGUCCAUUGAGGGUA | UACCCUCAAUGGACACCAC | CHMP | [549-567] ORF | [520-538] ORF |
| 116 | CCCUGUAAUUUUCCAAGGA | UCCUUGGAAAAUUACAGGG | CHMP | [2538-2556] 3'UTR | [2509-2527] 3'UTR |
| 117 | UAACCCUGUAAUUUUCCAA | UUGGAAAAUUACAGGGUUA | CHMP | [2535-2553] 3'UTR | [2506-2524] 3'UTR |
| 118 | GUAACCCUGUAAUUUUCCA | UGGAAAAUUACAGGGUUAC | CHMP | [2534-2552] 3'UTR | [2505-2523] 3'UTR |
| 119 | UCAUACCAAGCAGGCCUUU | AAAGGCCUGCUUGGUAUGA | CHMP | [359-377] ORF | [330-348] ORF |
| 120 | CGCCUUAAUUCAGCUGAGU | ACUCAGCUGAAUUAAGGCG | CHMP | [1632-1650] ORF | [1603-1621] ORF |
| 121 | ACCGCCUUAAUUCAGCUGA | UCAGCUGAAUUAAGGCGGU | CHMP | [1630-1648] ORF | [1601-1619] ORF |
| 122 | UCGUCUCCUUUAUGCCGUG | CACGGCAUAAAGGAGACGA | | [1592-1610] ORF | [1563-1581] ORF |
| 123 | UGGAGGAUCAUACCAAGCA | UGCUUGGUAUGAUCCUCCA | CHMP | [352-370] ORF | [323-341] ORF |
| 124 | UCAUCGAUGGCCACAUCUA | UAGAUGUGGCCAUCGAUGA | CHMP | [1444-1462] ORF | [1415-1433] ORF |
| 125 | GCUACCUGGAGGCUUACAA | UUGUAAGCCUCCAGGUAGC | CHMP | [1204-1222] ORF | [1175-1193] ORF |
| 126 | AGCAAGAGGAGUUCUUCAA | UUGAAGAACUCCUCUUGCU | CHMP | [832-850] ORF | [803-821] ORF |
| 127 | AGUACAUCUACAUGCAUUU | AAAUGCAUGUAGAUGUACU | CHMP | [799-817] ORF | [770-788] ORF |
| 128 | CUGAGCAGAUUGGCUGUGU | ACACAGCCAAUCUGCUCAG | | [757-775] ORF | [726-746] ORF |
| 129 | CAGACUAACUAGUGUCUUU | AAAGACACUAGUUAGUCUG | CHMP | [2567-2585] 3'UTR | [2538-2556] 3'UTR |
| 130 | GGAGUGUUACGACCCAGAU | AUCUGGGUCGUAACACUCC | CHMP | [1928-1946] ORF | [1899-1917] ORF |
| 131 | UGGAGAGGUAUGAGCCAGA | UCUGGCUCAUACCUCUCCA | CHMP | [1504-1522] ORF | [1475-1493] ORF |
| 132 | CCAUGACCAAUCAGUGGUC | GACCACUGAUUGGUCAUGG | CHMP | [1378-1396] ORF | [1349-1367] ORF |
| 133 | AGCUGCAGAAGUGCGAGAU | AUCUCGCACUUCUGCAGCU | CHMP | [1036-1054] ORF | [1007-1025] ORF |
| 134 | AGAUGCAGCUGCAGAAGUG | CACUUCUGCAGCUGCAUCU | CHMP | [1030-1048] ORF | [1001-1019] ORF |
| 135 | CUGUAAUUUUCCAAGGAAA | UUUCCUUGGAAAAUUACAG | CHMP | [2540-2558] 3'UTR | [2511-2529] 3'UTR |
| 136 | CUGUCACCAUGUGAUUUAU | AUAAAUCACAUGGUGACAG | | [2377-2395] 3'UTR | [2348-2366] 3'UTR |
| 137 | CAGCUGUCACCAUGUGAUU | AAUCACAUGGUGACAGCUG | | [2374-2392] 3'UTR | [2345-2363] 3'UTR |
| 138 | GACCAGCAGAACUGUACCU | AGGUACAGUUCUGCUGGUC | CHMP | [2037-2055] ORF | [2008-2026] ORF |
| 139 | GGGAGAAUCUACGUCCUUG | CAAGGACGUAGAUUCUCCC | CHMP | [1875-1893] ORF | [1846-1864] ORF |
| 140 | ACGGGACAAACCGCCUUAA | UUAAGGCGGUUUGUCCCGU | CHMP | [1621-1639] ORF | [1592-1610] ORF |
| 141 | CCCAUGACCAAUCAGUGGU | ACCACUGAUUGGUCAUGGG | CHMP | [1377-1395] ORF | [1348-1366] ORF |
| 142 | AGGAGUUCUUCAACCUGUC | GACAGGUUGAAGAACUCCU | CHMP,Rat,MO | [838-856] ORF | [809-827] ORF |
| 143 | GCAAGAGGAGUUCUUCAAC | GUUGAAGAACUCCUCUUGC | CHMP | [833-851] ORF | [804-622] ORF |
| 144 | CCAAGCAAGAGGAGUUCUU | AAGAACUCCUCUUGCUUGG | CHMP | [829-847] ORF | [800-618] ORF |
| 145 | GAAGUCCAAAGCGGGAAUC | GAUUCCCGCUUUGGACUUC | CHMP | [2215-2233] 3'UTR | [2186-2204] 3'UTR |
| 146 | GGCUAUGAUGGUCACACGU | ACGUGUGACCAUCAUAGCC | CHMP | [1896-1914] ORF | [1867-1885] ORF |
| 147 | GGAGAAUCUACGUCCUUGG | CCAAGGACGUAGAUUCUCC | CHMP | [1876-1894] ORF | [1847-1865] ORF |
| 148 | CGAACUUCCUGCAGAUGCA | UGCAUCUGCAGGAAGUUCG | CHMP | [1016-1036] ORF | [989-1007] ORF |
| 149 | CCUGCAUCAACUGGGUCAA | UUGACCCAGUUGAUGCAGG | CHMP | [928-946] ORF | [899-917] ORF |
| 150 | UGGCCAAGCAAGAGGAGUU | AACUCCUCUUGCUUGGCCA | CHMP | [826-844] ORF | [797-815] ORF |
| 151 | CUGUCAUGUACCAGAUCGA | UCGAUCUGGUACAUGACAG | CHMP | [661-679] ORF | [632-650] ORF |
| 152 | GGUGCUGUCAUGUACCAGA | UCUGGUACAUGACAGCACC | | [657-675] ORF | [628-646] ORF |
| 153 | ACCGGAUGGACAGUUAUUU | AAAUAACUGUCCAUCCGGU | CHMP | [2506-2524] 3'UTR | [2477-2495] 3'UTR |
| 154 | GCCUGUACAUAGAAGCCAC | GUGGCUUCUAUGUACAGGC | CHMP | [2471-2489] 3'UTR | [2442-2460] 3'UTR |
| 155 | UGGGCAAAAAUACAGUCCA | UGGACUGUAUUUUUGCCCA | | [2076-2094] 3'UTR | [2047-2065] 3'UTR |
| 156 | AGGCCUUUGGCAUCAUGAA | UUCAUGAUGCCAAAGGCCU | CHMP | [370-388] ORF | [341-359] ORF |
| 157 | UCCUCAAUCGUCUCCUUUA | UAAAGGAGACGAUUGAGGA | | [1585-1603] ORF | [1556-1574] ORF |
| 158 | CCUGGAGGAUCAUACCAAG | CUUGGUAUGAUCCUCCAGG | CHMP | [350-368] ORF | [321-339] ORF |
| 159 | GUACAUCUACAUGCAUUUU | AAAAUGCAUGUAGAUGUAC | CHMP | [800-818] ORF | [771-789] ORF |
| 160 | ACAGACUAACUAGUGUCUU | AAGACACUAGUUAGUCUGU | CHMP | [2566-2584] 3'UTR | [2537-2555] 3'UTR |
| 161 | GGCCUGUACAUAGAAGCCA | UGGCUUCUAUGUACAGGCC | CHMP | [2470-2488] 3'UTR | [2441-2459] 3'UTR |
| 162 | CUGUACCUGUUGAGGCACU | AGUGCCUCAACAGGUACAG | CHMP | [2048-2066] ORF+3'UTR | [2019-2037] ORF+3'UTR |
| 163 | CCAGAUACAGACACCUGGA | UCCAGGUGUCUGUAUCUGG | CHMP | [1941-1959] ORF | [1912-1930] ORF |
| 164 | CGUCCUUGGAGGCUAUGAU | AUCAUAGCCUCCAAGGACG | CHMP | [1886-1904] ORF | [1857-1875] ORF |
| 165 | CACAACUGUAUCUAUGCUG | CAGCAUAGAUACAGUUGUG | CHMP | [1731-1749] ORF | [1702-1720] ORF |
| 166 | CCAACUUCGCUGAGCAGAU | AUCUGCUCAGCGAAGUUGG | CHMP | [748-766] ORF | [719-737] ORF |
| 167 | ACGGUGCUGUCAUGUACCA | UGGUACAUGACAGCACCGU | | [655-673] ORF | [626-644] ORF |
| 168 | AGUGUGUCCUCCACGUCAU | AUGACGUGGAGGACACACU | CHMP | [634-652] ORF | [605-623] ORF |
| 169 | AGAAGUGUGUCCUCCACGU | ACGUGGAGGACACACUUCU | CHMP | [631-649] ORF | [602-620] ORF |
| 170 | UGGAGGUGGUGUCCAUUGA | UCAAUGGACACCACCUCCA | CHMP,Rat | [544-562] ORF | [515-533] ORF |
| 171 | AGACUAACUAGUGUCUUUC | GAAAGACACUAGUUAGUCU | CHMP | [2568-2586] 3'UTR | [2539-2557] 3'UTR |
| 172 | GAACAGACUAACUAGUGUC | GACACUAGUUAGUCUGUUC | CHMP | [2564-2582] 3'UTR | [2535-2553] 3'UTR |
| 173 | CAGGUCAAGUACCAGGAUG | CAUCCUGGUACUUGACCUG | CHMP | [429-447] ORF | [400-418] ORF |
| 174 | CAGAACUGUACCUGUUGAG | CUCAACAGGUACAGUUCUG | CHMP | [2043-2061] ORF+3'UTR | [2014-2032] ORF+3'UTR |
| 175 | CCAGCAGAACUGUACCUGU | ACAGGUACAGUUCUGCUGG | CHMP | [2039-2057] ORF | [2010-2028] ORF |
| 176 | GCCUUAAUUCAGCUGAGUG | CACUCAGCUGAAUUAAGGC | CHMP | [1633-1651] ORF | [1604-1622] ORF |
| 177 | GUCCUCAAUCGUCUCCUUU | AAAGGAGACGAUUGAGGAC | | [1584-1602] ORF | [1555-1573] ORF |
| 178 | UGUCCUCAAUCGUCUCCUU | AAGGAGACGAUUGAGGACA | | [1583-1601] ORF | [1554-1572] ORF |
| 179 | GAGGAGUUCUUCAACCUGU | ACAGGUUGAAGAACUCCUC | CHMP,Rat,MO | [837-855] ORF | [808-826] ORF |
| 180 | GACUAACUAGUGUCUUUCA | UGAAAGACACUAGUUAGUC | CHMP | [2569-2587] 3'UTR | [2540-2558] 3'UTR |
| 181 | CACCGGAUGGACAGUUAUU | AAUAACUGUCCAUCCGGUG | CHMP | [2505-2523] 3'UTR | [2476-2494] 3'UTR |
| 182 | GUCACCAUGUGAUUUAUUC | GAAUAAAUCACAUGGUGAC | | [2379-2397] 3'UTR | [2350-2368] 3'UTR |
| 183 | ACAUCUCAAAAGAAGUCCA | UGGACUUCUUUUGAGAUGU | CHMP | [2204-2222] 3'UTR | [2175-2193] 3'UTR |
| 184 | UGACAUCUCAAAAGAAGUC | GACUUCUUUUGAGAUGUCA | CHMP | [2202-2220] 3'UTR | [2173-2191] 3'UTR |
| 185 | CUGUUGAGGCACUUUUGUU | AACAAAAGUGCCUCAACAG | CHMP | [2054-2072] ORF+3'UTR | [2025-2043] ORF+3'UTR |
| 186 | GUACCUGUUGAGGCACUUU | AAAGUGCCUCAACAGGUAC | CHMP | [2050-2068] ORF+3'UTR | [2021-2039] ORF+3'UTR |
| 187 | UGUACCUGUUGAGGCACUU | AAGUGCCUCAACAGGUACA | CHMP | [2049-2067] ORF+3'UTR | [2020-2038] ORF+3'UTR |
| 188 | GAAGCAGAUUGACCAGCAG | CUGCUGGUCAAUCUGCUUC | CHMP | [2027-2045] ORF | [1998-2016] ORF |
| 189 | CAGAUACAGACACCUGGAG | CUCCAGGUGUCUGUAUCUG | CHMP | [1942-1960] ORF | [1913-1931] ORF |
| 190 | UGGAGUGUUACGACCCAGA | UCUGGGUCGUAACACUCCA | CHMP | [1927-1945] ORF | [1898-1916] ORF |
| 191 | ACACGUUCCUGGACAGUGU | ACACUGUCCAGGAACGUGU | CHMP | [1909-1927] ORF | [1880-1898] ORF |
| 192 | GGAGGCUAUGAUGGUCACA | UGUGACCAUCAUAGCCUCC | CHMP | [1893-1911] ORF | [1864-1882] ORF |
| 193 | CUGAGUGUUACUACCCAGA | UCUGGGUAGUAACACUCAG | CHMP | [1645-1663] ORF | [1616-1634] ORF |
| 194 | GCUGAGUGUUACUACCCAG | CUGGGUAGUAACACUCAGC | CHMP | [1644-1662] ORF | [1615-1633] ORF |
| 195 | CCUGUCUUCAAGGCCAUGU | ACAUGGCCUUGAAGACAGG | CHMP | [498-516] ORF | [469-487] ORF |
| 196 | ACCCUGUAAUUUUCCAAGG | CCUUGGAAAAUUACAGGGU | CHMP | [2537-2555] 3'UTR | [2508-2526] 3'UTR |
| 197 | UGUCACCAUGUGAUUUAUU | AAUAAAUCACAUGGUGACA | | [2378-2396] 3'UTR | [2349-2367] 3'UTR |
| 198 | UACCUGUUGAGGCACUUUU | AAAAGUGCCUCAACAGGUA | CHMP | [2051-2069] ORF+3'UTR | [2022-2040] ORF+3'UTR |
| 199 | AGAUUGACCAGCAGAACUG | CAGUUCUGCUGGUCAAUCU | CHMP | [2032-2050] ORF | [2003-2021] ORF |
| 200 | AGCAGAUUGACCAGCAGAA | UUCUGCUGGUCAAUCUGCU | CHMP | [2029-2047] ORF | [2000-2018] ORF |
| 201 | GGAAGCAGAUUGACCAGCA | UGCUGGUCAAUCUGCUUCC | CHMP | [2026-2044] ORF | [1997-2015] ORF |
| 202 | GAGGCUAUGAUGGUCACAC | GUGUGACCAUCAUAGCCUC | CHMP | [1894-1912] ORF | [1865-1883] ORF |
| 203 | AGCUGAGUGUUACUACCCA | UGGGUAGUAACACUCAGCU | CHMP | [1643-1661] ORF | [1614-1632] ORF |
| 204 | GGUCAAGUACGACUGCGAA | UUCGCAGUCGUACUUGACC | CHMP | [941-959] ORF | [912-930] ORF |
| 205 | ACAUCUACAUGCAUUUUGG | CCAAAAUGCAUGUAGAUGU | CHMP | [802-820] ORF | [773-791] ORF |
| 206 | GUGUCCUCCACGUCAUGAA | UUCAUGACGUGGAGGACAC | CHMP | [637-655] ORF | [608-626] ORF |
| 207 | GCCUUUGGCAUCAUGAACG | CGUUCAUGAUGCCAAAGGC | CHMP | [372-390] ORF | [343-361] ORF |
| 208 | UGGAGGCUAUGAUGGUCAC | GUGACCAUCAUAGCCUCCA | CHMP | [1892-1910] ORF | [1863-1881] ORF |
| 209 | GGCCUUUGGCAUCAUGAAC | GUUCAUGAUGCCAAAGGCC | CHMP | [371-389] ORF | [342-360] ORF |
| 210 | UGCAAGGACUACCUGGUCA | UGACCAGGUAGUCCUUGCA | CHMP | [1074-1092] ORF | [1045-1063] ORF |
| 211 | CGGGAGUACAUCUACAUGC | GCAUGUAGAUGUACUCCCG | CHMP | [795-813] ORF | [766-784] ORF |
| 212 | UCAUGAACGGUGCUGUCAU | AUGACAGCACCGUUCAUGA | | [649-667] ORF | [620-638] ORF |
| 213 | CCCUCUGGAAAUGUGGUUC | GAACCACAUUUCCAGAGGG | CHMP | [2442-2460] 3'UTR | [2413-2431] 3'UTR |
| 214 | AGCUGUGUGACGUCACACU | AGUGUGACGUCACACAGCU | CHMP | [409-427] ORF | [380-398] ORF |
| 215 | CUAUGAUGGUCACACGUUC | GAACGUGUGACCAUCAUAG | CHMP | [1898-1916] ORF | [1869-1887] ORF |
| 216 | GAAAUGUGGUUCCCAGGGA | UCCCUGGGAACCACAUUUC | CHMP | [2449-2467] 3'UTR | [2420-2438] 3'UTR |
| 217 | CUGGAAAUGUGGUUCCCAG | CUGGGAACCACAUUUCCAG | CHMP | [2446-2464] 3'UTR | [2417-2435] 3'UTR |
| 218 | CUAUGAUGGUCAGGACCAG | CUGGUCCUGACCAUCAUAG | CHMP | [1757-1775] ORF | [1728-1746] ORF |
| 219 | CACUGCAGGUCAAGUACCA | UGGUACUUGACCUGCAGUG | CHMP | [424-442] ORF | [395-413] ORF |
| 220 | UGUUGAGGCACUUUUGUUU | AAACAAAAGUGCCUCAACA | CHMP | [2055-2073] ORF+3'UTR | [2026-2044] ORF+3'UTR |
| 221 | AGAGACGUGGACUUUCGUA | UACGAAAGUCCACGUCUCU | CHMP | [1806-1826] ORF | [1779-1797] ORF |
| 222 | GCCAAGCAAGAGGAGUUCU | AGAACUCCUCUUGCUUGGC | CHMP | [828-846] ORF | [799-817] ORF |
| 223 | AAAUGUGGUUCCCAGGGAU | AUCCCUGGGAACCACAUUU | CHMP | [2450-2468] 3'UTR | [2421-2439] 3'UTR |
| 224 | UGGCUGUCCUCAAUCGUCU | AGACGAUUGAGGACAGCCA | CHMP | [1579-1597] ORF | [1550-1568] ORF |
| 225 | UGCAGAUGCAGCUGCAGAA | UUCUGCAGCUGCAUCUGCA | CHMP | [1027-1045] ORF | [998-1016] ORF |

**Table F: 21-mer kelch-like ECH-associated protein 1 (KEAP1)**

| No. | Sense siRNA | AntiSense siRNA | Other Sp | Human-45269144 ORF:186-2060 | Human-45269143 ORF:157-2031 |
|---|---|---|---|---|---|
| 1 | GAGGAACGAGUGGCGAAUGAU | AUCAUUCGCCACUCGUUCCUC | CHMP | [1664-1684] ORF | [1635-1655] ORF |
| 2 | GCACUGCAAAUAACCCAUCUU | AAGAUGGGUUAUUUGCAGUGC | CHMP | [2146-2166] 3'UTR | [2117-2137] 3'UTR |
| 3 | GGGAGUAUCAUUGUUUUUGUA | UACAAAAACAAUGAUACUCCC | CHMP | [2098-2118] 3'UTR | [2069-2089] 3'UTR |
| 4 | GGAACGAGUGGCGAAUGAUCA | UGAUCAUUCGCCACUCGUUCC | CHMP | [1666-1686] ORF | [1637-1657] ORF |
| 5 | GGAGUAUCAUUGUUUUUGUAC | GUACAAAAACAAUGAUACUCC | CHMP | [2099-2119] 3'UTR | [2070-2090] 3'UTR |
| 6 | AGAGAGGAACGAGUGGCGAAU | AUUCGCCACUCGUUCCUCUCU | CHMP | [1661-1681] ORF | [1632-1652] ORF |
| 7 | GCAGAUUGGCUGUGUGGAGUU | AACUCCACACAGCCAAUCUGC | | [761-781] ORF | [732-752] ORF |
| 8 | GGUGUUGCUUAUCUUCUGGAA | UUCCAGAAGAUAAGCAACACC | CHMP | [161-181] 5'UTR | [132-152] 5'UTR |
| 9 | CCUCAUUGAAUUCGCCUACAC | GUGUAGGCGAAUUCAAUGAGG | | [590-610] ORF | [561-581] ORF |
| 10 | GCCUCAUUGAAUUCGCCUACA | UGUAGGCGAAUUCAAUGAGGC | | [589-609] ORF | [560-580] ORF |
| 11 | CCAUGUGAUUUAUUCUUGGAU | AUCCAAGAAUAAAUCACAUGG | | [2383-2403] 3'UTR | [2354-2374] 3'UTR |
| 12 | GCACUUUUGUUUCUUGGGCAA | UUGCCCAAGAAACAAAAGUGC | CHMP | [2062-2082] 3'UTR | [2033-2053] 3'UTR |
| 13 | GGUGGUGGUGUUGCUUAUCUU | AAGAUAAGCAACACCACCACC | | [155-175] 5'UTR | [126-146] 5'UTR |
| 14 | GGAAACAGAGACGUGGACUUU | AAAGUCCACGUCUCUGUUUCC | CHMP | [1802-1822] ORF | [1773-1793] ORF |
| 15 | CGCCUCAUUGAAUUCGCCUAC | GUAGGCGAAUUCAAUGAGGCG | | [588-608] ORF | [559-579] ORF |
| 16 | GGACUAAAAGAAAAGACAGCA | UGCUGUCUUUUCUUUUAGUCC | CHMP | [2128-2148] 3'UTR | [2099-2119] 3'UTR |
| 17 | CGGGACUAAAAGAAAAGACAG | CUGUCUUUUCUUUUAGUCCCG | CHMP | [2126-2146] 3'UTR | [2097-2117] 3'UTR |
| 18 | CCGGGACUAAAAGAAAAGACA | UGUCUUUUCUUUUAGUCCCGG | CHMP | [2125-2145] 3'UTR | [2096-2116] 3'UTR |
| 19 | GGGCAAAAAUACAGUCCAAUG | CAUUGGACUGUAUUUUUGCCC | | [2077-2097] 3'UTR | [2048-2068] 3'UTR |
| 20 | CUGCAUCAACUGGGUCAAGUA | UACUUGACCCAGUUGAUGCAG | CHMP | [929-949] ORF | [900-920] ORF |
| 21 | GUACAAAAACCGGGACUAAAA | UUUUAGUCCCGGUUUUUGUAC | CHMP | [2116-2136] 3'UTR | [2087-2107] 3'UTR |
| 22 | GGGAUGGGCCUGUACAUAGAA | UUCUAUGUACAGGCCCAUCCC | CHMP | [2464-2484] 3'UTR | [2435-2455] 3'UTR |
| 23 | GAAAAGACAGCACUGCAAAUA | UAUUUGCAGUGCUGUCUUUUC | CHMP | [2137-2157] 3'UTR | [2108-2128] 3'UTR |
| 24 | AGAAAAGACAGCACUGCAAAU | AUUUGCAGUGCUGUCUUUUCU | CHMP | [2136-2156] 3'UTR | [2107-2127] 3'UTR |
| 25 | GCAAAAAUACAGUCCAAUGGG | CCCAUUGGACUGUAUUUUUGC | | [2079-2099] 3'UTR | [2050-2070] 3'UTR |
| 26 | CGAUGUGGAAACAGAGACGUG | CACGUCUCUGUUUCCACAUCG | CHMP | [1796-1816] ORF | [1767-1787] ORF |
| 27 | CGCUACGAUGUGGAAACAGAG | CUCUGUUUCCACAUCGUAGCG | CHMP | [1791-1811] ORF | [1762-1782] ORF |
| 28 | GUCCUGCACAACUGUAUCUAU | AUAGAUACAGUUGUGCAGGAC | CHMP | [1725-1745] ORF | [1696-1716] ORF |
| 29 | AAGAAAAGACAGCACUGCAAA | UUUGCAGUGCUGUCUUUUCUU | CHMP | [2135-2155] 3'UTR | [2106-2126] 3'UTR |
| 30 | CUAAAAGAAAAGACAGCACUG | CAGUGCUGUCUUUUCUUUUAG | CHMP | [2131-2151] 3'UTR | [2102-2122] 3'UTR |
| 31 | CUUUUGUUUCUUGGGCAAAAA | UUUUUGCCCAAGAAACAAAAG | CHMP | [2065-2085] 3'UTR | [2036-2056] 3'UTR |
| 32 | GUGUUGCUUAUCUUCUGGAAC | GUUCCAGAAGAUAAGCAACAC | CHMP | [162-182] 5'UTR | [133-153] 5'UTR |
| 33 | UGAUUUAUUCUUGGAUACCUG | CAGGUAUCCAAGAAUAAAUCA | CHMP | [2388-2408] 3'UTR | [2359-2379] 3'UTR |
| 34 | GGCAGCUGUCACCAUGUGAUU | AAUCACAUGGUGACAGCUGCC | | [2372-2392] 3'UTR | [2343-2363] 3'UTR |
| 35 | GUGGUGGUGUUGCUUAUCUUC | GAAGAUAAGCAACACCACCAC | | [156-176] 5'UTR | [127-147] 5'UTR |
| 36 | ACAGCACUGCAAAUAACCCAU | AUGGGUUAUUUGCAGUGCUGU | CHMP | [2143-2163] 3'UTR | [2114-2134] 3'UTR |
| 37 | GACUAAAAGAAAAGACAGCAC | GUGCUGUCUUUUCUUUUAGUC | CHMP | [2129-2149] 3'UTR | [2100-2120] 3'UTR |
| 38 | UGGUGUUGCUUAUCUUCUGGA | UCCAGAAGAUAAGCAACACCA | CHMP | [160-180] 5'UTR | [131-151] 5'UTR |
| 39 | UGUUGAUAAGUAACCCUGUAA | UUACAGGGUUACUUAUCAACA | CHMP | [2525-2545] 3'UTR | [2496-2516] 3'UTR |
| 40 | CUCAUCCAGCCCUGUCUUCAA | UUGAAGACAGGGCUGGAUGAG | CHMP | [488-508] ORF | [459-479] ORF |
| 41 | CACUGCAAAUAACCCAUCUUC | GAAGAUGGGUUAUUUGCAGUG | CHMP | [2147-2167] 3'UTR | [2118-2138] 3'UTR |
| 42 | AGCACUGCAAAUAACCCAUCU | AGAUGGGUUAUUUGCAGUGCU | CHMP | [2145-2165] 3'UTR | [2116-2136] 3'UTR |
| 43 | ACCGGGACUAAAAGAAAAGAC | GUCUUUUCUUUUAGUCCCGGU | CHMP | [2124-2144] 3'UTR | [2095-2115] 3'UTR |
| 44 | CAAAAACCGGGACUAAAAGAA | UUCUUUUAGUCCCGGUUUUUG | CHMP | [2119-2139] 3'UTR | [2090-2110] 3'UTR |
| 45 | CACUUUUGUUUCUUGGGCAAA | UUUGCCCAAGAAACAAAAGUG | CHMP | [2063-2083] 3'UTR | [2034-2054] 3'UTR |
| 46 | GGCACUUUUGUUUCUUGGGCA | UGCCCAAGAAACAAAAGUGCC | CHMP | [2061-2081] 3'UTR | [2032-2052] 3'UTR |
| 47 | GCGCUACGAUGUGGAAACAGA | UCUGUUUCCACAUCGUAGCGC | CHMP | [1790-1810] ORF | [1761-1781] ORF |
| 48 | CCUUAAUUCAGCUGAGUGUUA | UAACACUCAGCUGAAUUAAGG | CHMP | [1634-1654] ORF | [1605-1625] ORF |
| 49 | GUUGAUAAGUAACCCUGUAAU | AUUACAGGGUUACUUAUCAAC | CHMP | [2526-2546] 3'UTR | [2497-2517] 3'UTR |
| 50 | ACCAUGUGAUUUAUUCUUGGA | UCCAAGAAUAAAUCACAUGGU | | [2382-2402] 3'UTR | [2353-2373] 3'UTR |
| 51 | CAGUGUUAAAAUGACAUCUCA | UGAGAUGUCAUUUUAACACUG | CHMP | [2191-2211] 3'UTR | [2162-2182] 3'UTR |
| 52 | UCAGUGUUAAAAUGACAUCUC | GAGAUGUCAUUUUAACACUGA | CHMP | [2190-2210] 3'UTR | [2161-2181] 3'UTR |
| 53 | AAAAACCGGGACUAAAAGAAA | UUUCUUUUAGUCCCGGUUUUU | CHMP | [2120-2140] 3'UTR | [2091-2111] 3'UTR |
| 54 | AGCGCUACGAUGUGGAAACAG | CUGUUUCCACAUCGUAGCGCU | CHMP | [1789-1809] ORF | [1760-1780] ORF |
| 55 | GAGCGCUACGAUGUGGAAACA | UGUUUCCACAUCGUAGCGCUC | CHMP | [1788-1808] ORF | [1759-1779] ORF |
| 56 | GCAGCUGUCACCAUGUGAUUU | AAAUCACAUGGUGACAGCUGC | | [2373-2393] 3'UTR | [2344-2364] 3'UTR |
| 57 | UUGUACAAAAACCGGGACUAA | UUAGUCCCGGUUUUUGUACAA | CHMP | [2114-2134] 3'UTR | [2085-2105] 3'UTR |
| 58 | GGCAAAAAUACAGUCGAAUGG | CCAUUGGACUGUAUUUUUGCC | | [2076-2098] 3'UTR | [2049-2069] 3'UTR |
| 59 | GGACAGUGUGGAGUGUUACGA | UCGUAACACUCCACACUGUCC | CHMP | [1919-1939] ORF | [1890-1910] ORF |
| 60 | UGCCCGGGAGUACAUCUACAU | AUGUAGAUGUACUCCCGGGCA | CHMP | [791-811] ORF | [762-782] ORF |
| 61 | CCUCAGUGUUAAAAUGACAUC | GAUGUCAUUUUAACACUGAGG | CHMP | [2188-2206] 3'UTR | [2159-2179] 3'UTR |
| 62 | CUGGACAGUGUGGAGUGUUAC | GUAACACUCCACACUGUCCAG | CHMP | [1917-1937] ORF | [1888-1908] ORF |
| 63 | CCUGGACAGUGUGGAGUGUUA | UAACACUCCACACUGUCCAGG | CHMP | [1916-1936] ORF | [1887-1907] ORF |
| 64 | UGGAAACAGAGACGUGGACUU | AAGUCCACGUCUCUGUUUCCA | CHMP | [1801-1821] ORF | [1772-1792] ORF |
| 65 | UUAAUUCAGCUGAGUGUUACU | AGUAACACUCAGCUGAAUUAA | CHMP | [1636-1656] ORF | [1607-1627] ORF |
| 66 | UCCUCAAUCGUCUCCUUUAUG | CAUAAAGGAGACGAUUGAGGA | | [1585-1605] ORF | [1556-1576] ORF |
| 67 | GUCCUCAAUCGUCUCCUUUAU | AUAAAGGAGACGAUUGAGGAC | | [1584-1604] ORF | [1555-1575] ORF |
| 68 | GUGGUGUCCAUUGAGGGUAUC | GAUACCCUCAAUGGACACCAC | CHMP | [549-569] ORF | [520-540] ORF |
| 69 | GGAUGGGCCUGUACAUAGAAG | CUUCUAUGUACAGGCCCAUCC | CHMP | [2465-2485] 3'UTR | [2436-2456] 3'UTR |
| 70 | CUCAGUGUUAAAAUGACAUCU | AGAUGUCAUUUUAACACUGAG | CHMP | [2189-2209] 3'UTR | [2160-2180] 3'UTR |
| 71 | GUUGAGGCACUUUUGUUUCUU | AAGAAACAAAAGUGCCUCAAC | CHMP | [2056-2076]ORF+3'UTR | [2027-2047] ORF+3'UTR |
| 72 | CCUUGGAGGCUAUGAUGGUCA | UGACCAUCAUAGCCUCCAAGG | CHMP | [1889-1909] ORF | [1860-1880] ORF |
| 73 | GUGGAAACAGAGACGUGGACU | AGUCCACGUCUCUGUUUCCAC | CHMP | [1800-1820] ORF | [1771-1791] ORF |
| 74 | UGAUGGUCACACGUUCCUGGA | UCCAGGAACGUGUGACCAUCA | CHMP | [1901-1921] ORF | [1872-1892] ORF |
| 75 | CAAGCAGGCCUUUGGCAUCAU | AUGAUGCCAAAGGCCUGCUUG | CHMP | [365-385] ORF | [336-356] ORF |
| 76 | AGGAUGCCUCAGUGUUAAAAU | AUUUUAACACUGAGGCAUCCU | CHMP | [2182-2202] 3'UTR | [2153-2173] 3'UTR |
| 77 | CCAGGAUGCCUCAGUGUUAAA | UUUAACACUGAGGCAUCCUGG | CHMP | [2180-2200] 3'UTR | [2151-2171] 3'UTR |
| 78 | GGAGUACAUCUACAUGCAUUU | AAAUGCAUGUAGAUGUACUCC | CHMP | [797-817] ORF | [768-788] ORF |
| 79 | CCAAGGAAAAUAAAGAACAGA | UCUGUUCUUUAUUUUCCUUGG | CHMP | [2550-2570] 3'UTR | [2521-2541] 3'UTR |
| 80 | GUGGCGAAUGAUCACAGCAAU | AUUGCUGUGAUCAUUCGCCAC | CHMP | [1673-1693] ORF | [1644-1664] ORF |
| 81 | GGAUCAUACCAAGCAGGCCUU | AAGGCCUGCUUGGUAUGAUCC | CHMP | [356-376] ORF | [327-347] ORF |
| 82 | GGAUGGACAGUUAUUUUGUUG | CAACAAAAUAACUGUCCAUCC | CHMP | [2509-2529] 3'UTR | [2480-2500] 3'UTR |
| 83 | CCGGAUGGACAGUUAUUUUGU | ACAAAAUAACUGUCCAUCCGG | CHMP | [2507-2527] 3'UTR | [2478-2496] 3'UTR |
| 84 | GCCUCAGUGUUAAAAUGACAU | AUGUCAUUUUAACACUGAGGC | CHMP | [2187-2207] 3'UTR | [2158-2178] 3'UTR |
| 85 | AAAAGACAGCACUGCAAAUAA | UUAUUUGCAGUGCUGUCUUUU | CHMP | [2138-2158] 3'UTR | [2109-2129] 3'UTR |
| 86 | GCGAAUGAUCACAGCAAUGAA | UUCAUUGCUGUGAUCAUUCGC | CHMP | [1676-1696] ORF | [1647-1667] ORF |
| 87 | GAAGUGUGUCCUCCACGUCAU | AUGACGUGGAGGACACACUUC | CHMP | [632-652] ORF | [603-623] ORF |
| 88 | CGGAUGGACAGUUAUUUUGUU | AACAAAAUAACUGUCCAUCCG | CHMP | [2508-2526] 3'UTR | [2479-2499] 3'UTR |
| 89 | CUGCAGGUCAAGUACCAGGAU | AUCCUGGUACUUGACCUGCAG | CHMP | [426-446] ORF | [397-417] ORF |
| 90 | GCUGUCACCAUGUGAUUUAUU | AAUAAAUCACAUGGUGACAGC | | [2376-2396] 3'UTR | [2347-2367] 3'UTR |
| 91 | GGAUGCCUCAGUGUUAAAAUG | CAUUUUAACACUGAGGCAUCC | CHMP | [2183-2203] 3'UTR | [2154-2174] 3'UTR |
| 92 | AGCAGGCCUUUGGCAUCAUGA | UCAUGAUGCCAAAGGCCUGCU | CHMP | [367-387] ORF | [338-358] ORF |
| 93 | UGGCGAAUGAUCACAGCAAUG | CAUUGCUGUGAUCAUUCGCCA | CHMP | [1674-1694] ORF | [1645-1665] ORF |
| 94 | CAAGGAAAAUAAAGAACAGAC | GUCUGUUCUUUAUUUUCCUUG | CHMP | [2551-2571] 3'UTR | [2522-2542] 3'UTR |
| 95 | UCCAAGGAAAAUAAAGAACAG | CUGUUCUUUAUUUUCCUUGGA | CHMP | [2549-2569] 3'UTR | [2520-2540] 3'UTR |
| 96 | AGCUGUCACCAUGUGAUUUAU | AUAAAUCACAUGGUGACAGCU | | [2375-2395] 3'UTR | [2346-2366] 3'UTR |
| 97 | CCGCCUUAAUUCAGCUGAGUG | CACUCAGCUGAAUUAAGGCGG | CHMP | [1631-1651] ORF | [1602-1622] ORF |
| 98 | AGCUACACCCUGGAGGAUCAU | AUGAUCCUCCAGGGUGUAGCU | CHMP | [342-362] ORF | [313-333] ORF |
| 99 | GCAAGAGGAGUUCUUCAACCU | AGGUUGAAGAACUCCUCUUGC | CHMP | [833-853] ORF | [804-824] ORF |
| 100 | GGUGCUGUCAUGUACCAGAUC | GAUCUGGUACAUGACAGCACC | | [657-677] ORF | [628-648] ORF |
| 101 | CGGUGCUGUCAUGUACCAGAU | AUCUGGUACAUGACAGCACCG | | [656-676] ORF | [627-647] ORF |
| 102 | AGAGGUGGUGGUGUUGCUUAU | AUAAGCAACACCACCACCUCU | | [152-172] 5'UTR | [123-143] 5'UTR |
| 103 | GGUGGUGUCCAUUGAGGGUAU | AUACCCUCAAUGGACACCACC | CHMP | [548-568] ORF | [519-539] ORF |
| 104 | GAGGUGGUGGUGUUGCUUAUC | GAUAAGCAACACCACCACCUC | | [153-173] 5'UTR | [124-144] 5'UTR |
| 105 | CCCUGUAAUUUUCCAAGGAAA | UUUCCUUGGAAAAUUACAGGG | CHMP | [2538-2558] 3'UTR | [2509-2529] 3'UTR |
| 106 | AGUAACCCUGUAAUUUUCCAA | UUGGAAAAUUACAGGGUUACU | CHMP | [2533-2553] 3'UTR | [2504-2524] 3'UTR |
| 107 | CUGUACCUGUUGAGGCACUUU | AAAGUGCCUCAACAGGUACAG | CHMP | [2048-2068]ORF+3'UTR | [2019-2039] ORF+3'UTR |
| 108 | GAGUACAUCUACAUGCAUUUU | AAAAUGCAUGUAGAUGUACUC | CHMP | [798-818] ORF | [769-769] ORF |
| 109 | UGCUGUCAUGUACCAGAUCGA | UCGAUCUGGUACAUGACAGCA | | [659-679] ORF | [630-650] ORF |
| 110 | CUAACUAGUGUCUUUCACCCU | AGGGUGAAAGACACUAGUUAG | CHMP | [2571-2591] 3'UTR | [2542-2562] 3'UTR |
| 111 | CCACCGGAUGGACAGUUAUUU | AAAUAACUGUCCAUCCGGUGG | CHMP | [2504-2524] 3'UTR | [2475-2495] 3'UTR |
| 112 | GCCAGGAUGCCUCAGUGUUAA | UUAACACUGAGGCAUCCUGGC | CHMP | [2179-2199] 3'UTR | [2150-2170] 3'UTR |
| 113 | ACUGUACCUGUUGAGGCACUU | AAGUGCCUCAACAGGUACAGU | CHMP | [2047-2067]ORF+3'UTR | [2018-2038] ORF+3'UTR |
| 114 | ACCGCCUUAAUUCAGCUGAGU | ACUCAGCUGAAUUAAGGCGGU | CHMP | [1630-1650] ORF | [1601-1621] ORF |
| 115 | GGGAGUACAUCUACAUGCAUU | AAUGCAUGUAGAUGUACUCCC | CHMP | [796-816] ORF | [767-787] ORF |
| 116 | ACAGACUAACUAGUGUCUUUC | GAAAGACACUAGUUAGUCUGU | CHMP | [2566-2586] 3'UTR | [2537-2557] 3'UTR |
| 117 | CCCUGUCUUCAAGGCCAUGUU | AACAUGGCCUUGAAGACAGGG | CHMP | [497-517] ORF | [468-486] ORF |
| 118 | CCUGUAAUUUUCCAAGGAAAA | UUUUCCUUGGAAAAUUACAGG | CHMP | [2539-2559] 3'UTR | [2510-2530] 3'UTR |
| 119 | CAGCUGUCACCAUGUGAUUUA | UAAAUCACAUGGUGACAGCUG | | [2374-2394] 3'UTR | [2345-2365] 3'UTR |
| 120 | GAUCAUACCAAGCAGGCCUUU | AAAGGCCUGCUUGGUAUGAUC | CHMP | [357-377] ORF | [326-348] ORF |
| 121 | GCCUUAAUUCAGCUGAGUGUU | AACACUCAGCUGAAUUAAGGC | CHMP | [1633-1653] ORF | [1604-1624] ORF |
| 122 | CCACAACAGUGUGGAGAGGUA | UACCUCUCCACACUGUUGUGG | CHMP | [1493-1513] ORF | [1464-7484] ORF |
| 123 | GUCAUCGAUGGCCACAUCUAU | AUAGAUGUGGCCAUCGAUGAC | CHMP | [1443-1463] ORF | [1414-1434] ORF |
| 124 | GGUCAUCGAUGGCCACAUCUA | UAGAUGUGGCCAUCGAUGACC | CHMP | [1442-1462] ORF | [1413-1433] ORF |
| 125 | AAAGAAGUCCAAAGCGGGAAU | AUUCCCGCUUUGGACUUCUUU | CHMP | [2212-2232] 3'UTR | [2183-2203] 3'UTR |
| 126 | AGCAGAACUGUACCUGUUGAG | CUCAACAGGUACAGUUCUGCU | CHMP | [2041-2061]ORF+3'UTR | [2012-2032] ORF+3'UTR |
| 127 | GCAGGCCUUUGGCAUCAUGAA | UUCAUGAUGCCAAAGGCCUGC | CHMP | [368-388] ORF | [339-359] ORF |
| 128 | GGGAGAAUCUACGUCCUUGGA | UCCAAGGACGUAGAUUCUCCC | CHMP | [1875-1895] ORF | [1846-1866] ORF |
| 129 | GGCGAAUGAUCACAGCAAUGA | UCAUUGCUGUGAUCAUUCGCC | CHMP | [1675-1695] ORF | [1646-1666] ORF |
| 130 | GGGACAAACCGCCUUAAUUCA | UGAAUUAAGGCGGUUUGUCCC | CHMP | [1623-1643] ORF | [1594-1614] ORF |
| 131 | CGGGAGUACAUCUACAUGCAU | AUGCAUGUAGAUGUACUCCCG | CHMP | [795-815] ORF | [766-786] ORF |
| 132 | ACCCUGUAAUUUUCCAAGGAA | UUCCUUGGAAAAUUACAGGGU | CHMP | [2537-2557] 3'UTR | [2508-2528] 3'UTR |
| 133 | UGGGCAAAAAUACAGUCCAAU | AUUGGACUGUAUUUUUGCCCA | | [2076-2096] 3'UTR | [2047-2067] 3'UTR |
| 134 | CCAGCAGAACUGUACCUGUUG | CAACAGGUACAGUUCUGCUGG | CHMP | [2039-2059] ORF | [2010-2030] ORF |
| 135 | AUCUACGUCCUUGGAGGCUAU | AUAGCCUCCAAGGACGUAGAU | CHMP | [1881-1901] ORF | [1852-1872] ORF |
| 136 | GCGUCCUGCACAACUGUAUCU | AGAUACAGUUGUGCAGGACGC | CHMP | [1723-1743] ORF | [1694-1714] ORF |
| 137 | GACGGGACAAACCGCCUUAAU | AUUAAGGCGGUUUGUCCCGUC | CHMP | [1620-1640] ORF | [1591-1611] ORF |
| 138 | CCCAAUGCUGACACGAAGGAU | AUCCUUCGUGUCAGCAUUGGG | CHMP | [1547-1567] ORF | [1518-1538] ORF |
| 139 | CACAACAGUGUGGAGAGGUAU | AUACCUCUCCACACUGUUGUG | CHMP | [1494-1514] ORF | [1465-1485] ORF |
| 140 | CAAGGACUACCUGGUCAAGAU | AUCUUGACCAGGUAGUCCUUG | CHMP | [1076-1096] ORF | [1047-1067] ORF |
| 141 | UGGGUCAAGUACGACUGCGAA | UUCGCAGUCGUACUUGACCCA | CHMP | [939-959] ORF | [910-930] ORF |
| 142 | CAAGCAAGAGGAGUUCUUCAA | UUGAAGAACUCCUCUUGCUUG | CHMP | [830-850] ORF | [801-821] ORF |
| 143 | GCGCCUCAUUGAAUUCGCCUA | UAGGCGAAUUCAAUGAGGCGC | | [587-607] ORF | [558-578] ORF |
| 144 | CUGUACAUAGAAGCCACCGGA | UCCGGUGGCUUCUAUGUACAG | CHMP | [2473-2493] 3'UTR | [2444-2464] 3'UTR |
| 145 | ACAUCUCAAAAGAAGUCCAAA | UUUGGACUUCUUUUGAGAUGU | CHMP | [2204-2224] 3'UTR | [2175-2195] 3'UTR |
| 146 | ACCAGCAGAACUGUACCUGUU | AACAGGUACAGUUCUGCUGGU | CHMP | [2038-2058] ORF | [2009-2029] ORF |
| 147 | AGAUUGACCAGCAGAACUGUA | UACAGUUCUGCUGGUCAAUCU | CHMP | [2032-2052] ORF | [2003-2023] ORF |
| 148 | GUGGAGUGUUACGACCCAGAU | AUCUGGGUCGUAACACUCCAC | CHMP | [1926-1946] ORF | [1897-1917] ORF |
| 149 | GGCUAUGAUGGUCACACGUUC | GAACGUGUGACCAUCAUAGCC | CHMP | [1896-1916] ORF | [1867-1887] ORF |
| 150 | AGGCUAUGAUGGUCACACGUU | AACGUGUGACCAUCAUAGCCU | CHMP | [1895-1915] ORF | [1866-1886] ORF |
| 151 | GAGGCUAUGAUGGUCACACGU | ACGUGUGACCAUCAUAGCCUC | CHMP | [1894-1914] ORF | [1865-1685] ORF |
| 152 | GGAGAAUCUACGUCCUUGGAG | CUCCAAGGACGUAGAUUCUCC | CHMP | [1876-1896] ORF | [1847-1867] ORF |
| 153 | CGUCCUGCACAACUGUAUCUA | UAGAUACAGUUGUGCAGGACG | CHMP | [1724-1744] ORF | [1695-1715] ORF |
| 154 | GAUCACAGCAAUGAACACCAU | AUGGUGUUCAUUGCUGUGAUC | CHMP | [1682-1702] ORF | [1653-1673] ORF |
| 155 | GAAUGAUCACAGCAAUGAACA | UGUUCAUUGCUGUGAUCAUUC | CHMP | [1678-1698] ORF | [1649-1669] ORF |
| 156 | CGAAUGAUCACAGCAAUGAAC | GUUCAUUGCUGUGAUCAUUCG | CHMP | [1677-1697] ORF | [1648-1668] ORF |
| 157 | CCAACUUCGCUGAGCAGAUUG | CAAUCUGCUCAGCGAAGUUGG | CHMP | [748-766] ORF | [719-739] ORF |
| 158 | GAACAGACUAACUAGUGUCUU | AAGACACUAGUUAGUCUGUUC | CHMP | [2564-2584] 3'UTR | [2535-2555] 3'UTR |
| 159 | CCCACCGGAUGGACAGUUAUU | AAUAACUGUCCAUCCGGUGGG | CHMP | [2503-2523] 3'UTR | [2474-2494] 3'UTR |
| 160 | GGAAAUGUGGUUCCCAGGGAU | AUCCCUGGGAACCACAUUUCC | CHMP | [2448-2468] 3'UTR | [2419-2439] 3'UTR |
| 161 | GAAGUCCAAAGCGGGAAUCAU | AUGAUUCCCGCUUUGGACUUC | CHMP | [2215-2235] 3'UTR | [2186-2206] 3'UTR |
| 162 | GACAUCUCAAAAGAAGUCCAA | UUGGACUUCUUUUGAGAUGUC | CHMP | [2203-2223] 3'UTR | [2174-2194] 3'UTR |
| 163 | GGAAGCAGAUUGACCAGCAGA | UCUGCUGGUCAAUCUGCUUCC | CHMP | [2026-2046] ORF | [1997-2017] ORF |
| 164 | GCACAACUGUAUCUAUGCUGC | GCAGCAUAGAUACAGUUGUGC | CHMP | [1730-1750] ORF | [1701-1721] ORF |
| 165 | UGAGUGUUACUACCCAGAGAG | CUCUCUGGGUAGUAACACUCA | CHMP | [1646-1666] ORF | [1617-1637] ORF |
| 166 | CGGGACAAACCGCCUUAAUUC | GAAUUAAGGCGGUUUGUCCCG | CHMP | [1622-1642] ORF | [1593-1613] ORF |
| 167 | UGACGGGACAAACCGCCUUAA | UUAAGGCGGUUUGUCCCGUCA | CHMP | [1619-1639] ORF | [1590-1610] ORF |
| 168 | ACCCUGGAGGAUCAUACCAAG | CUUGGUAUGAUCCUCCAGGGU | CHMP | [348-368] ORF | [319-339] ORF |
| 169 | CUGUCCUCAAUCGUCUCCUUU | AAAGGAGACGAUUGAGGACAG | | [1582-1602] ORF | [1553-1573] ORF |
| 170 | GCUGUCCUCAAUCGUCUCCUU | AAGGAGACGAUUGAGGACAGC | | [1581-1601] ORF | [1552-1572] ORF |
| 171 | CAGCUACCUGGAGGCUUACAA | UUGUAAGCCUCCAGGUAGCUG | CHMP | [1202-1222] ORF | [1173-1193] ORF |
| 172 | CAACUGGGUCAAGUACGACUG | CAGUCGUACUUGACCCAGUUG | CHMP | [935-955] ORF | [906-926] ORF |
| 173 | CCAAGCAAGAGGAGUUCUUCA | UGAAGAACUCCUCUUGCUUGG | CHMP | [829-849] ORF | [800-820] ORF |
| 174 | CAGACUAACUAGUGUCUUUCA | UGAAAGACACUAGUUAGUCUG | CHMP | [2567-2587] 3'UTR | [2538-2558] 3'UTR |
| 175 | CCUGUUGAGGCACUUUUGUUU | AAACAAAAGUGCCUCAACAGG | CHMP | [2053-2073]ORF+3'UTR | [2024-2044] ORF+3'UTR |
| 176 | GGCCUUUGGCAUCAUGAACGA | UCGUUCAUGAUGCCAAAGGCC | CHMP | [371-391] ORF | [342-362] ORF |
| 177 | ACCCAGAUACAGACACCUGGA | UCCAGGUGUCUGUAUCUGGGU | CHMP | [1939-1959] ORF | [1910-1930] ORF |
| 178 | GAGUGUUACGACCCAGAUACA | UGUAUCUGGGUCGUAACACUC | CHMP | [1929-1949] ORF | [1900-1920] ORF |
| 179 | GGAGUGUUACGACCCAGAUAC | GUAUCUGGGUCGUAACACUCC | CHMP | [1928-1948] ORF | [1899-1919] ORF |
| 180 | ACGUCCUUGGAGGCUAUGAUG | CAUCAUAGCCUCCAAGGACGU | CHMP | [1885-1905] ORF | [1856-1876] ORF |
| 181 | CACAACUGUAUCUAUGCUGCU | AGCAGCAUAGAUACAGUUGUG | CHMP | [1731-1751] ORF | [1702-1722] ORF |
| 182 | CGCCUUAAUUCAGCUGAGUGU | ACACUCAGCUGAAUUAAGGCG | CHMP | [1632-1652] ORF | [1603-1623] ORF |
| 183 | ACGGGACAAACCGCCUUAAUU | AAUUAAGGCGGUUUGUCCCGU | CHMP | [1621-1641] ORF | [1592-1612] ORF |
| 184 | AGCUACCUGGAGGCUUACAAC | GUUGUAAGCCUCCAGGUAGCU | CHMP | [1203-1223] ORF | [1174-1194] ORF |
| 185 | GCAAGGACUACCUGGUCAAGA | UCUUGACCAGGUAGUCCUUGC | CHMP | [1075-1095] ORF | [1046-1066] ORF |
| 186 | CUGCAAGGACUACCUGGUCAA | UUGACCAGGUAGUCCUUGCAG | CHMP | [1073-1093] ORF | [1044-1064] ORF |
| 187 | CCGGGAGUACAUCUACAUGCA | UGCAUGUAGAUGUACUCCCGG | CHMP | [794-814] ORF | [765-785] ORF |
| 188 | GCCAACUUCGCUGAGCAGAUU | AAUCUGCUCAGCGAAGUUGGC | CHMP | [747-767] ORF | [718-738] ORF |
| 189 | GCUGUCAUGUACCAGAUCGAC | GUCGAUCUGGUACAUGAGAGC | CHMP | [660-680] ORF | [631-651] ORF |
| 190 | CCUGUCUUCAAGGCCAUGUUC | GAACAUGGCCUUGAAGACAGG | CHMP | [498-518] ORF | [469-489] ORF |
| 191 | AGAACAGACUAACUAGUGUCU | AGACACUAGUUAGUCUGUUCU | CHMP | [2563-2583] 3'UTR | [2534-2554] 3'UTR |
| 192 | CAGGUCAAGUACCAGGAUGCA | UGCAUCCUGGUACUUGACCUG | CHMP | [429-449] ORF | [400-420] ORF |
| 193 | CAGGAUGCCUCAGUGUUAAAA | UUUUAACACUGAGGCAUCCUG | CHMP | [2181-2201] 3'UTR | [2152-2172] 3'UTR |
| 194 | UUGGGCAAAAAUACAGUCCAA | UUGGACUGUAUUUUUGCCCAA | | [2075-2095] 3'UTR | [2046-2066] 3'UTR |
| 195 | GUACCUGUUGAGGCACUUUUG | CAAAAGUGCCUCAACAGGUAC | CHMP | [2050-2070]ORF+3'UTR | [2021-2041] ORF+3'UTR |
| 196 | GAACUGUACCUGUUGAGGCAC | GUGCCUCAACAGGUACAGUUC | CHMP | [2045-2065]ORF+3'UTR | [2016-2036] ORF+3'UTR |
| 197 | GCAGAACUGUACCUGUUGAGG | CCUCAACAGGUACAGUUCUGC | CHMP | [2042-2062]ORF+3'UTR | [2013-2033] ORF+3'UTR |
| 198 | UGCACAACUGUAUCUAUGCUG | CAGCAUAGAUACAGUUGUGCA | CHMP | [1729-1749] ORF | [1700-1720] ORF |
| 199 | AGCAAUGAACACCAUCCGAAG | CUUCGGAUGGUGUUCAUUGCU | CHMP | [1688-1708] ORF | [1659-1679] ORF |
| 200 | GUGUUACUACCCAGAGAGGAA | UUCCUCUCUGGGUAGUAACAC | CHMP | [1649-1669] ORF | [1620-1640] ORF |
| 201 | GCUGAGUGUUACUACCCAGAG | CUCUGGGUAGUAACACUCAGC | CHMP | [1644-1664] ORF | [1615-1635] ORF |
| 202 | CUGGAGGAUCAUACCAAGCAG | CUGCUUGGUAUGAUCCUCCAG | CHMP | [351-371] ORF | [322-342] ORF |
| 203 | ACAAACCGCCUUAAUUCAGCU | AGCUGAAUUAAGGCGGUUUGU | CHMP | [1626-1646] ORF | [1597-1617] ORF |
| 204 | GUCAAGUACGACUGCGAACAG | CUGUUCGCAGUCGUACUUGAC | CHMP | [942-962] ORF | [913-933] ORF |
| 205 | AGAGGAGUUCUUCAACCUGUC | GACAGGUUGAAGAACUCCUCU | CHMP | [836-856] ORF | [807-827] ORF |
| 206 | GUGUGUCCUCCACGUCAUGAA | UUCAUGACGUGGAGGACACAC | CHMP | [635-655] ORF | [606-626] ORF |
| 207 | GUGUCCAUUGAGGGUAUCCAC | GUGGAUACCCUCAAUGGACAC | CHMP | [552-572] ORF | [523-543] ORF |
| 208 | AGACUAACUAGUGUCUUUCAC | GUGAAAGACACUAGUUAGUCU | CHMP | [2568-2588] 3'UTR | [2539-2559] 3'UTR |
| 209 | UCACCAUGUGAUUUAUUCUUG | CAAGAAUAAAUCACAUGGUGA | | [2380-2400] 3'UTR | [2351-2371] 3'UTR |
| 210 | GCAGAUUGACCAGCAGAACUG | CAGUUCUGCUGGUCAAUCUGC | CHMP | [2030-2050] ORF | [2001-2021] ORF |
| 211 | GAAGCAGAUUGACCAGCAGAA | UUCUGCUGGUCAAUCUGCUUC | CHMP | [2027-2047] ORF | [1998-2018] ORF |
| 212 | AGAUACAGACACCUGGAGCGA | UCGCUCCAGGUGUCUGUAUCU | CHMP | [1943-1963] ORF | [1914-1934] ORF |
| 213 | UGGAGUGUUACGACCCAGAUA | UAUCUGGGUCGUAACACUCCA | CHMP | [1927-1947] ORF | [1898-1918] ORF |
| 214 | UGCGUCCUGCACAACUGUAUC | GAUACAGUUGUGCAGGACGCA | CHMP | [1722-1742] ORF | [1693-1713] ORF |
| 215 | CUGCGUCCUGCACAACUGUAU | AUACAGUUGUGCAGGACGCAG | CHMP | [1721-174] ORF | [1692-1712] ORF |
| 216 | CAGCAAUGAACACCAUCCGAA | UUCGGAUGGUGUUCAUUGCUG | CHMP | [1687-1707] ORF | [1658-1678] ORF |
| 217 | GGACAAACCGCCUUAAUUCAG | CUGAAUUAAGGCGGUUUGUCC | CHMP | [1624-1644] ORF | [1595-1615] ORF |
| 218 | ACCACAACAGUGUGGAGAGGU | ACCUCUCCACACUGUUGUGGU | CHMP | [1492-1512] ORF | [1463-1483] ORF |
| 219 | GGAGUUCUUCAACCUGUCCCA | UGGGACAGGUUGAAGAACUCC | CHMP | [839-859] ORF | [810-830] ORF |
| 220 | GCCAAGCAAGAGGAGUUCUUC | GAAGAACUCCUCUUGCUUGGC | CHMP | [828-848] ORF | [799-819] ORF |
| 221 | GGCCAAGCAAGAGGAGUUCUU | AAGAACUCCUCUUGCUUGGCC | CHMP | [827-847] ORF | [798-818] ORF |
| 222 | GUACAUCUACAUGCAUUUUGG | CCAAAAUGCAUGUAGAUGUAC | CHMP | [800-820] ORF | [771-791] ORF |
| 223 | UGUACAUAGAAGCCACCGGAU | AUCCGGUGGCUUCUAUGUACA | CHMP | [2474-2494] 3'UTR | [2445-2465] 3'UTR |
| 224 | GAAAUGUGGUUCCCAGGGAUG | CAUCCCUGGGAACCACAUUUC | CHMP | [2449-2469] 3'UTR | [2420-2440) 3'UTR |
| 225 | AGAAGUCCAAAGCGGGAAUCA | UGAUUCCCGCUUUGGACUUCU | CHMP | [2214-2234] 3'UTR | [2185-2205] 3'UTR |
| 226 | ACACUGCAGGUCAAGUACCAG | CUGGUACUUGACCUGCAGUGU | CHMP | [423-443] ORF | [394-414] ORF |
| 227 | UGACAUCUCAAAAGAAGUCCA | UGGACUUCUUUUGAGAUGUCA | CHMP | [2202-2222] 3'UTR | [2173-2193] 3'UTR |
| 228 | CUGUUGAGGCACUUUUGUUUC | GAAACAAAAGUGCCUCAACAG | CHMP | [2054-2074]ORF+3'UTR | [2025-2045] ORF+3'UTR |
| 229 | GCCUUUGGCAUCAUGAACGAG | CUCGUUCAUGAUGCCAAAGGC | CHMP | [372-392] ORF | [343-363] ORF |
| 230 | UCCUGGACAGUGUGGAGUGUU | AACACUCCACACUGUCCAGGA | CHMP | [1915-1935] ORF | [1886-1906] ORF |
| 231 | CCUGCACAACUGUAUCUAUGC | GCAUAGAUACAGUUGUGCAGG | CHMP | [1727-1747] ORF | [1698-1718] ORF |
| 232 | UGAUCACAGCAAUGAACACCA | UGGUGUUCAUUGCUGUGAUCA | CHMP | [1681-1701] ORF | [1652-1672] ORF |
| 233 | AGCUGAGUGUUACUACCCAGA | UCUGGGUAGUAACACUCAGCU | CHMP | [1643-1663] ORF | [1614-1634] ORF |
| 234 | CAGCUGAGUGUUACUACCCAG | CUGGGUAGUAACACUCAGCUG | CHMP | [1642-1662] ORF | [1613-1633] ORF |
| 235 | CAAGAGGAGUUCUUCAACCUG | CAGGUUGAAGAACUCCUCUUG | CHMP | [834-854] ORF | [805-825] ORF |
| 236 | UCAUGAACGGUGCUGUCAUGU | ACAUGACAGCACCGUUCAUGA | | [649-669] ORF | [620-640] ORF |
| 237 | CGUCAUGAACGGUGCUGUCAU | AUGACAGCACCGUUCAUGACG | | [647-667] ORF | [618-638] ORF |
| 238. | UGUCACCAUGUGAUUUAUUCU | AGAAUAAAUCACAUGGUGACA | | [2378-2398] 3'UTR | [2349-2369] 3'UTR |
| 239 | CGUCACACUGCAGGUCAAGUA | UACUUGACCUGCAGUGUGACG | CHMP | [419-439] ORF | [390-410] ORF |
| 240 | CAGAUUGACCAGCAGAACUGU | ACAGUUCUGCUGGUCAAUCUG | CHMP | [2031-2051] ORF | [2002-2022] ORF |
| 241 | CCAAUGCUGACACGAAGGAUC | GAUCCUUCGUGUCAGCAUUGG | CHMP | [1548-1568] ORF | [1519-1539] ORF |
| 242 | UCAUGGAGCGCCUCAUUGAAU | AUUCAAUGAGGCGCUCCAUGA | | [580-600] ORF | [551-571] ORF |
| 243 | GUCACCAUGUGAUUUAUUCUU | AAGAAUAAAUCACAUGGUGAC | | [2379-2399] 3'UTR | [2350-2370] 3'UTR |
| 244 | UGACGUCACACUGCAGGUCAA | UUGACCUGCAGUGUGACGUCA | CHMP | [416-436] ORF | [387-407] ORF |
| 245 | ACCUGUUGAGGCACUUUUGUU | AACAAAAGUGCCUCAACAGGU | CHMP | [2052-2072]ORF+3'UTR | [2023-2043] ORF+3'UTR |
| 246 | GUCAUGGAGCGCCUCAUUGAA | UUCAAUGAGGCGCUCCAUGAC | | [579-599] ORF | [550-570] ORF |
| 247 | AAGGUCAUGGAGCGCCUCAUU | AAUGAGGCGCUCCAUGACCUU | | [576-596] ORF | [547-567] ORF |
| 248 | CCUCUGGAAAUGUGGUUCCCA | UGGGAACCACAUUUCCAGAGG | CHMP | [2443-2463]3'UTR | [2414-2434] 3'UTR |
| 249 | AAAUAACCCAUCUUCCGGGAA | UUCCCGGAAGAUGGGUUAUUU | CHMP | [2153-2173] 3'UTR | [2124-2144] 3'UTR |
| 250 | ACAGCAAUGAACACCAUCCGAA | UCGGAUGGUGUUCAUUGCUGU | CHMP | [1686-1706] ORF | [1657-1677] ORF |
| 251 | CUUCAAGGCCAUGUUCACCAA | UUGGUGAACAUGGCCUUGAAG | CHMP | [503-523] ORF | [474-494] ORF |
| 252 | GUCACACUGCAGGUCAAGUAC | GUACUUGACCUGCAGUGUGAC | CHMP | [420-440] ORF | [391-411] ORF |
| 253 | ACAGAGACGUGGACUUUCGUA | UACGAAAGUCCACGUCUCUGU | CHMP | [1806-1826] ORF | [1777-1797] ORF |
| 254 | CACCGGAUGGACAGUUAUUUU | AAAAUAACUGUCCAUCCGGUG | CHMP | [2505-2525] 3'UTR | [2476-2496] 3'UTR |
| 255 | CAAAUAACCCAUCUUCCGGGA | UCCCGGAAGAUGGGUUAUUUG | CHMP | [2152-2172] 3'UTR | [2123-2143] 3'UTR |
| 256 | UGCCUCAGUGUUAAAAUGACA | UGUCAUUUUAACACUGAGGCA | CHMP | [2186-2206] 3'UTR | [2157-2177] 3'UTR |
| 257 | UGAUGGUCAGGACCAGCUGAA | UUCAGCUGGUCCUGACCAUCA | CHMP | [1760-1780] ORF | [1731-1751] ORF |

**Table G: 19-mer SHC1 - SHC transforming protein 1**

| No. | Sense siRNA | AntiSense siRNA | Other Sp | Human-52693920 ORF:1-1752 | Human-34147725 ORF:121-1545 |
|---|---|---|---|---|---|
| 1 | ACCUGAAAUUUGCUGGAAU | AUUCCAGCAAAUUUCAGGU | | [671-689] ORF | [461-479] ORF |
| 2 | CGAGUAUGUCGCCUAUGUU | AACAUAGGCGACAUACUCG | | [813-831] ORF | [603-621] ORF |
| 3 | CAGAGAGCUUUUUGAUGAU | AUCAUCAAAAAGCUCUCUG | | [1254-1272] ORF | [1047-1065] ORF |
| 4 | GCAGAGAGCUUUUUGAUGA | UCAUCAAAAAGCUCUCUGC | | [1253-1271] ORF | [1046-1064] ORF |
| 5 | CCGCUUUGAAAGUGUCAGU | ACUGACACUUUCAAAGCGG | | [1641-1659] ORF | [1434-1452] ORF |
| 6 | AGCUUUUUGAUGAUCCCUC | GAGGGAUCAUCAAAAAGCU | | [1259-1277] ORF | [1052-1070] ORF |
| 7 | UGACCAUCAGUACUAUAAU | AUUAUAGUACUGAUGGUCA | | [1035-1053] ORF | [825-843] ORF |
| 8 | CACAUGCAAUCUAUCUCAU | AUGAGAUAGAUUGCAUGUG | Rat | [766-784] ORF | [556-574] ORF |
| 9 | CCUACUUGGUUCGGUACAU | AUGUACCGAACCAAGUAGG | Rat,MO | [482-500] ORF | [272-290] ORF |
| 10 | CCUAUGUCAACGUCCAGAA | UUCUGGACGUUGACAUAGG | | [1277-1295] ORF | [1070-1088] ORF |
| 11 | AGAGAGCUUUUUGAUGAUC | GAUCAUCAAAAAGCUCUCU | | [1255-1273] ORF | [1048-1066] ORF |
| 12 | AGAGCUUUUUGAUGAUCCC | GGGAUCAUCAAAAAGCUCU | | [1257-1275] ORF | [1050-1068] ORF |
| 13 | UCAAACAAUACCUCAGGAA | UUCCUGAGGUAUUGUUUGA | | [935-953] ORF | [725-743] ORF |
| 14 | ACAUGCAAUCUAUCUCAUU | AAUGAGAUAGAUUGCAUGU | Rat | [767-785] ORF | [557-575] ORF |
| 15 | AACCUGAAAUUUGCUGGAA | UUCCAGCAAAUUUCAGGUU | | [670-688] ORF | [460-478] ORF |
| 16 | CGCUUUGAAAGUGUCAGUC | GACUGACACUUUCAAAGCG | | [1642-1660] ORF | [1435-1453] ORF |
| 17 | GGGACCUGUUUGACAUGAA | UUCAUGUCAAACAGGUCCC | | [1367-1385] ORF | [1160-1178] ORF |
| 18 | CCUCCUAUGUCAACGUCCA | UGGACGUUGACAUAGGAGG | | [1274-1292] ORF | [1067-1085] ORF |
| 19 | UCCCUCCUAUGUCAACGUC | GACGUUGACAUAGGAGGGA | | [1272-1290] ORF | [1065-1083] ORF |
| 20 | GUACUAUAAUGACUUCCCG | CGGGAAGUCAUUAUAGUAC | | [1044-1062] ORF | [834-852] ORF |
| 21 | AGGGUGUGGUUCGGACUAA | UUAGUCCGAACCACACCCU | | [1616-1634] ORF | [1409-1427] ORF |
| 22 | AGACAUGAGGCUUCGGGAA | UUCCCGAAGCCUCAUGUCU | | [1092-1110] ORF | [882-900] ORF |
| 23 | GCGCUUCAAACAAUACCUC | GAGGUAUUGUUUGAAGCGC | | [930-948] ORF | [720-738] ORF |
| 24 | CCAAUCACUCUCACCGUCU | AGACGGUGAGAGUGAUUGG | | [691-709] ORF | [481-499] ORF |
| 25 | GCAUUUGCUACUGGUGGAC | GUCCACCAGUAGCAAAUGC | | [1593-1611] ORF | [1386-1404] ORF |
| 26 | GUGGUAGACAUGAGGCUUC | GAAGCCUCAUGUCUACCAC | | [1087-1105] ORF | [877-895] ORF |
| 27 | CAAUCACUCUCACCGUCUC | GAGACGGUGAGAGUGAUUG | | [692-710] ORF | [482-500] ORF |
| 28 | CGGGAGCUUUGUCAAUAAG | CUUAUUGACAAAGCUCCCG | Rat,MO | [411-429] ORF | [201-219] ORF |
| 29 | GGACAUGAACAAGCUGAGU | ACUCAGCUUGUUCAUGUCC | Rat,MO | [327-345] ORF | [117-135] 5'UTR+ORF |
| 30 | CCCAAUCCUGCUAUCAAUG | CAUUGAUAGCAGGAUUGGG | | [1336-1354] ORF | [1129-1147] ORF |
| 31 | CGAGUUGCGCUUCAAACAA | UUGUUUGAAGCGCAACUCG | | [924-942] ORF | [714-732] ORF |
| 32 | GCUUUGAAAGUGUCAGUCA | UGACUGACACUUUCAAAGC | | [1643-1661] ORF | [1436-1454] ORF |
| 33 | UGAUGGCUCAGCAUGGGAU | AUCCCAUGCUGAGCCAUCA | | [996-1014] ORF | [786-804] ORF |
| 34 | UGCGCUUCAAACAAUACCU | AGGUAUUGUUUGAAGCGCA | | [929-947] ORF | [719-737] ORF |
| 35 | AUGCAAUCUAUCUCAUUUG | CAAAUGAGAUAGAUUGCAU | | [769-787] ORF | [559-577] ORF |
| 36 | ACCACAUGCAAUCUAUCUC | GAGAUAGAUUGCAUGUGGU | | [64-782] ORF | [554-572] ORF |
| 37 | CCACCACAUGCAAUCUAUC | GAUAGAUUGCAUGUGGUGG | | [762-780] ORF | [552-570] ORF |
| 38 | GUACAUGGGUUGUGUGGAG | CUCCACACAACCCAUGUAC | | [495-513] ORF | [285-303] ORF |
| 39 | GGAUCACCGCUUUGAAAGU | ACUUUCAAAGCGGUGAUCC | | [1635-1653] ORF | [1428-1446] ORF |
| 40 | GGUUCGGACUAAGGAUCAC | GUGAUCCUUAGUCCGAACC | | [1623-1641] ORF | [1416-1434] ORF |
| 41 | CGGGACCUGUUUGACAUGA | UCAUGUCAAACAGGUCCCG | | [1366-1384] ORF | [1159-1177] ORF |
| 42 | CGCUUCAAACAAUACCUCA | UGAGGUAUUGUUUGAAGCG | | [931-949] ORF | [721-739] ORF |
| 43 | GACAUGAACAAGCUGAGUG | CACUCAGCUUGUUCAUGUC | Rat,MO | [328-346] ORF | [118-136]5'UTR+ORF |
| 44 | GCUUCAAACAAUACCUCAG | CUGAGGUAUUGUUUGAAGC | | [932-950] ORF | [722-740] ORF |
| 45 | AGUUGCGCUUCAAACAAUA | UAUUGUUUGAAGCGCAACU | | [926-944] ORF | [716-734] ORF |
| 46 | AGCUACCACAUGGACAAUC | GAUUGUCCAUGUGGUAGCU | Rat,MO | [1669-1687] ORF | [1462-1480] ORF |
| 47 | CAGCUACCACAUGGACAAU | AUUGUCCAUGUGGUAGCUG | Rat,MO | [1668-1686] ORF | [1461-1479] ORF |
| 48 | ACCUUAUCAGCUACCACAU | AUGUGGUAGCUGAUAAGGU | | [1661-1679] ORF | [1454-1472] ORF |
| 49 | GGACUAAGGAUCACCGCUU | AAGCGGUGAUCCUUAGUCC | | [1628-1646] ORF | [1421-1439] ORF |
| 50 | UGGGCAGCCUAAGCAUUUG | CAAAUGCUUAGGCUGCCCA | | [1581-1599] ORF | [1374-1392] ORF |
| 51 | CGAACUGUGUCUACAGCAA | UUGCUGUAGACACAGUUCG | | [1713-1731] ORF | [1506-1524] ORF |
| 52 | GUGUCAGUCACCUUAUCAG | CUGAUAAGGUGACUGACAC | | [1652-1670] ORF | [1445-1463] ORF |
| 53 | GGGAGGAGUAACCUGAAAU | AUUUCAGGUUACUCCUCCC | | [661-679] ORF | [451-469] ORF |
| 54 | GCCUAAGCAUUUGCUACUG | CAGUAGCAAAUGCUUAGGC | | [1587-1605] ORF | [1380-1398] ORF |
| 55 | ACAAUCACUUGCCCAUCAU | AUGAUGGGCAAGUGAUUGU | MO | [1682-1700] ORF | [1475-1493] ORF |
| 56 | AGAUCCAGAAGUCCGCAAA | UUUGCGGACUUCUGGAUCU | | [1206-1224] ORF | [996-1014] ORF |
| 57 | CCAUCAGUACUAUAAUGAC | GUCAUUAUAGUACUGAUGG | | [1038-1056] ORF | [828-846] ORF |
| 58 | AGCCACCUGACCAUCAGUA | UACUGAUGGUCAGGUGGCU | | [1028-1046] ORF | [818-836] ORF |
| 59 | AGCCGAGUAUGUCGCCUAU | AUAGGCGACAUACUCGGCU | | [810-828] ORF | [600-618] ORF |
| 60 | AGGAGGAGAAAGCCCUGUA | UACAGGGCUUUCUCCUCCU | | [619-637] ORF | [409-427] ORF |
| 61 | UCAGCUACCACAUGGACAA | UUGUCCAUGUGGUAGCUGA | Rat,MO | [1667-1685] ORF | [1460-1478] ORF |
| 62 | GGGAGCUACAUUGCCUGUA | UACAGGCAAUGUAGCUCCC | | [1170-1188] ORF | [960-978] ORF |
| 63 | CUGACCAUCAGUACUAUAA | UUAUAGUACUGAUGGUCAG | | [1034-1052] ORF | [824-842] ORF |
| 64 | GAAAUUUGCUGGAAUGCCA | UGGCAUUCCAGCAAAUUUC | | [675-693] ORF | [465-483] ORF |
| 65 | ACGUCCAGAACCUAGACAA | UUGUCUAGGUUCUGGACG | | [1286-1304] ORF | [1079-1097] ORF |
| 66 | ACCAUCAGUACUAUAAUGA | UCAUUAUAGUACUGAUGGU | | [1037-1055] ORF | [827-845] ORF |
| 67 | GCAAACAGAUCAUCGCCAA | UUGGCGAUGAUCUGUUUGC | | [743-761] ORF | [533-551] ORF |
| 68 | CAGACUGCAAACAGAUCAU | AUGAUCUGUUUGCAGUCUG | Rat | [737-755] ORF | [527-545] ORF |
| 69 | ACCACAUGGACAAUCACUU | AAGUGAUUGUCCAUGUGGU | Rat,MO | [1673-1691] ORF | [1466-1484] ORF |
| 70 | CUAAGCAUUUGCUACUGGU | ACCAGUAGCAAAUGCUUAG | | [1589-1607] ORF | [1382-1400] ORF |
| 71 | GAGAUCCAGAAGUCCGCAA | UUGCGGACUUCUGGAUCUC | | [1205-1223] ORF | [995-1013] ORF |
| 72 | CGCCUAUGUUGCCAAAGAC | GUCUUUGGCAACAUAGGCG | | [822-840] ORF | [612-630] ORF |
| 73 | UGGACAAUCACUUGCCCAU | AUGGGCAAGUGAUUGUCCA | MO | [1679-1697] ORF | [1472-1490] ORF |
| 74 | AGGCAGAGAGCUUUUUGAU | AUCAAAAAGCUCUCUGCCU | | [1251-1269] ORF | [1044-1062] ORF |
| 75 | CCUGACCAUCAGUACUAUA | UAUAGUACUGAUGGUCAGG | | [1033-1051] ORF | [823-841] ORF |
| 76 | CCGCAGACUGCAAACAGAU | AUCUGUUUGCAGUCUGCGG | | [734-752] ORF | [524-542] ORF |
| 77 | GGGUUUCCUACUUGGUUCG | CGAACCAAGUAGGAAACCC | Rat,MO | [476-494] ORF | [266-284] ORF |
| 78 | GGACAAUCACUUGCCCAUC | GAUGGGCAAGUGAUUGUCC | MO | [1680-1698] ORF | [1473-1491] ORF |
| 79 | GCAGCCUAAGCAUUUGCUA | UAGCAAAUGCUUAGGCUGC | | [1584-1602] ORF | [1377-1395] ORF |
| 80 | CAGGCAGAGAGCUUUUUGA | UCAAAAAGCUCUCUGCCUG | | [1250-1268] ORF | [1043-1061] ORF |
| 81 | GGAGCUACAUUGCCUGUAG | CUACAGGCAAUGUAGCUCC | | [1171-1189] ORF | [961-979] ORF |
| 82 | GCAAUCUAUCUCAUUUGCA | UGCAAAUGAGAUAGAUUGC | | [771-789] ORF | [561-579] ORF |
| 83 | UGCAUCCCAACGACAAAGU | ACUUUGUCGUUGGGAUGCA | Rat,MO | [446-464] ORF | [236-254] ORF |
| 84 | GAAUGCCAAUCACUCUCAC | GUGAGAGUGAUUGGCAUUC | MO | [686-704] ORF | [476-494] ORF |
| 85 | CAACGACAAAGUCAUGGGA | UCCCAUGACUUUGUCGUUG | Rat,MO | [453-471] ORF | [243-261] ORF |
| 86 | AGCCUAAGCAUUUGCUACU | AGUAGCAAAUGCUUAGGCU | | [1586-1604] ORF | [1379-1397] ORF |
| 87 | GGCAGCCUAAGCAUUUGCU | AGCAAAUGCUUAGGCUGCC | | [1583-1601] ORF | [1376-1394] ORF |
| 88 | ACCUGACCAUCAGUACUAU | AUAGUACUGAUGGUCAGGU | | [1032-1050] ORF | [822-840] ORF |
| 89 | CACCUGACCAUCAGUACUA | UAGUACUGAUGGUCAGGUG | | [1031-1049] ORF | [821-839] ORF |
| 90 | GGCUUUGAUGGCUCAGCAU | AUGCUGAGCCAUCAAAGCC | | [991-1009] ORF | [781-799] ORF |
| 91 | ACCACCACAUGCAAUCUAU | AUAGAUUGCAUGUGGUGGU | | [761-779] ORF | [551-569] ORF |
| 92 | GCAGACUGCAAACAGAUCA | UGAUCUGUUUGCAGUCUGC | Rat | [736-754] ORF | [526-544] ORF |
| 93 | UGAAAGUGUCAGUCACCUU | AAGGUGACUGACACUUUCA | | [1647-1665] ORF | [1440-1458] ORF |
| 94 | GGCAGAGAGCUUUUUGAUG | CAUCAAAAAGCUCUCUGCC | | [1252-1270] ORF | [1045-1063] ORF |
| 95 | GCCACUUGGGAGCUACAUU | AAUGUAGCUCCCAAGUGGC | | [1163-1181] ORF | [953-971] ORF |
| 96 | UGUCGCCUAUGUUGCCAAA | UUUGGCAACAUAGGCGACA | | [619-837] ORF | [609-627] ORF |
| 97 | UGGAGGUCCUCCAGUCAAU | AUUGACUGGAGGACCUCCA | | [509-527] ORF | [299-317] ORF |
| 98 | GUCACCUUAUCAGCUACCA | UGGUAGCUGAUAAGGUGAC | | [1658-1676] ORF | [1451-1469] ORF |
| 99 | UCACCGCUUUGAAAGUGUC | GACACUUUCAAAGCGGUGA | | [1638-1656] ORF | [1431-1449] ORF |
| 100 | CCUAAGCAUUUGCUACUGG | CCAGUAGCAAAUGCUUAGG | | [1588-1606] ORF | [1381-1399] ORF |
| 101 | GCCCUUCGAAGAUGCUCUU | AAGAGCAUCUUCGAAGGGC | | [1386-1404] ORF | [1179-1197] ORF |
| 102 | UGCUAUCAAUGGCAGUGCA | UGCACUGCCAUUGAUAGCA | | [1344-1362] ORF | [1137-1155] ORF |
| 103 | UGACAGGAUGGCUGGCUUU | AAAGCCAGCCAUCCUGUCA | Rat,MO | [976-996] ORF | [768-786] ORF |
| 104 | CCUUCGAGUUGCGCUUCAA | UUGAAGCGCAACUCGAAGG | | [920-938] ORF | [710-726] ORF |
| 105 | UUGCCAAAGACCCUGUGAA | UUCACAGGGUCUUUGGCAA | Rat,MO | [830-848] ORF | [620-638] ORF |
| 106 | UGCAAUCUAUCUCAUUUGC | GCAAAUGAGAUAGAUUGCA | | [770-788] ORF | [560-578] ORF |
| 107 | GCGAACUGUGUCUACAGCA | UGCUGUAGACACAGUUCGC | | [1712-1730] ORF | [1505-1523] ORF |
| 108 | AGUAUGUGCUCACUGGCUU | AAGCCAGUGAGCACAUACU | | [1556-1574] ORF | [1349-1367] ORF |
| 109 | UGGCUUUGAUGGCUCAGCA | UGCUGAGCCAUCAAAGCCA | | [990-1008] ORF | [780-798] ORF |
| 110 | AGACUGCAAACAGAUCAUC | GAUGAUCUGUUUGCAGUCU | | [738-756] ORF | [528-546] ORF |
| 111 | GCAUCCCAACGACAAAGUC | GACUUUGUCGUUGGGAUGC | Rat,MO | [447-465] ORF | [237-255] ORF |
| 112 | GCUGCAUCCCAACGACAAA | UUUGUCGUUGGGAUGCAGC | Rat,MO | [444-482] ORF | [234-252] ORF |
| 113 | AGCAUUUGCUACUGGUGGA | UCCACCAGUAGCAAAUGCU | | [1592-1610] ORF | [1385-1403] ORF |
| 114 | UCAACGUCCAGAACCUAGA | UCUAGGUUCUGGACGUUGA | | [1283-1301] ORF | [1076-1094] ORF |
| 115 | UGCCAAAGACCCUGUGAAU | AUUCACAGGGUCUUUGGCA | Rat,MO | [831-849] ORF | [621-639] ORF |
| 116 | CCAACCACCACAUGCAAUC | GAUUGCAUGUGGUGGUUGG | | [758-776] ORF | [548-566] ORF |
| 117 | UGCAAACAGAUCAUCGCCA | UGGCGAUGAUCUGUUUGCA | | [742-760] ORF | [532-550] ORF |
| 118 | CCAACGACAAAGUCAUGGGt | CCCAUGACUUUGUCGUUGG | Rat,MO | [452-470] ORF | [242-260] ORF |
| 119 | GGAGCUUUGUCAAUAAGCC | GGCUUAUUGACAAAGCUCC | Rat,MO | [413-431] ORF | [203-221] ORF |
| 120 | CCUGCUAUCAAUGGCAGUG | CACUGCCAUUGAUAGCAGG | | [1342-1360] ORF | [1135-1153] ORF |
| 121 | AGCCACUUGGGAGCUACAU | AUGUAGCUCCCAAGUGGCU | | [1162-1180] ORF | [952-970] ORF |
| 122 | CCACCUGACCAUCAGUACU | AGUACUGAUGGUCAGGUGG | | [1030-1048] ORF | [820-838] ORF |
| 123 | GCCAAAGACCCUGUGAAUC | GAUUCACAGGGUCUUUGGC | Rat,MO | [832-850] ORF | [622-640] ORF |
| 124 | AACCACCACAUGCAAUCUA | UAGAUUGCAUGUGGUGGUU | | [760-778] ORF | [550-568] ORF |
| 125 | GCUUUGUCAAUAAGCCCAC | GUGGGCUUAUUGACAAAGC | Rat,MO | [416-434] ORF | [206-224] ORF |
| 126 | CAGUCACCUUAUCAGCUAC | GUAGCUGAUAAGGUGACUG | | [1656-1674] ORF | [1449-1467] ORF |
| 127 | UGACAUGAAGCCCUUCGAA | UUCGAAGGGCUUCAUGUCA | Rat | [1377-1395] ORF | [1170-1188] ORF |
| 128 | CUGCCACAUUCUGGAGUGU | ACACUCCAGAAUGUGGCAG | | [858-876] ORF | [648-666] ORF |
| 129 | GCCAACCACCACAUGCAAU | AUUGCAUGUGGUGGUUGGC | | [757-775] ORF | [547-565] ORF |
| 130 | CGCAGACUGCAAACAGAUC | GAUCUGUUUGCAGUCUGCG | | [735-753] ORF | [525-543] ORF |
| 131 | CACCUUAUCAGCUACCACA | UGUGGUAGCUGAUAAGGUG | | [1660-1678] ORF | [1453-1471] ORF |
| 132 | CAGGAUGUCAUCAGCACCA | UGGUGCUGAUGACAUCCUG | MO | [892-910] ORF | [682-700] ORF |
| 133 | UGGCCAGUAUGUGCUCACU | AGUGAGCACAUACUGGCCA | MO | [1551-1569] ORF | [1344-1362] ORF |
| 134 | AGGAUGUCAUCAGCACCAU | AUGGUGCUGAUGACAUCCU | MO | [893-911] ORF | [683-701] ORF |
| 135 | CCCUUCGAAGAUGCUCUUC | GAAGAGCAUCUUCGAAGGG | | [1387-1405] ORF | [1180-1198] ORF |
| 136 | UGUUUGACAUGAAGCCCUU | AAGGGCUUCAUGUCAAACA | | [1373-1391] ORF | [1166-1184] ORF |
| 137 | CUGUUUGACAUGAAGCCCU | AGGGCUUCAUGUCAAACAG | | [1372-1390] ORF | [1165-1183] ORF |
| 138 | GGAUGUCAUCAGCACCAUU | AAUGGUGCUGAUGACAUCC | | [894-912] ORF | [684-702] ORF |
| 139 | UCAUCGCCAACCACCACAU | AUGUGGUGGUUGGCGAUGA | | [752-770] ORF | [542-560] ORF |
| 140 | GGGUUCUUAUAAUGGAAAA | UUUUCCAUUAUAAGAACCC | | | |
| 141 | CCCAAGCCCAAGUACAAUC | GAUUGUACUUGGGCUUGGG | | | |
| 142 | AGGAAGGGCAGCUGAUGAU | AUCAUCAGCUGCCCUUCCU | | | |

**Table H: 21-mer SHC1-SHC transforming protein 1 (SHC1)**

| No. | Sense siRNA | AntiSense siRNA | Other Sp | Human-52693920 ORF:1-1752 | Human-34147725 ORF:121-1545 |
|---|---|---|---|---|---|
| 1 | UGAUGAUCCCUCCUAUGUCAA | UUGACAUAGGAGGGAUCAUCA | | [1268-1286] ORF | [1059-1079] ORF |
| 2 | CUUUUUGAUGAUCCCUCCUAU | AUAGGAGGGAUCAUCAAAAAG | | [1261-1281] ORF | [1054-1074] ORF |
| 3 | CCACAUGCAAUCUAUCUCAUU | AAUGAGAUAGAUUGCAUGUGG | | [765-785] ORF | [555-575] ORE |
| 4 | GCUUUUUGAUGAUCCCUCCUA | UAGGAGGGAUCAUCAAAAAGC | | [1260-1280] ORF | [1053-1073] ORF |
| 5 | AGCUUUUUGAUGAUCCCUCCU | AGGAGGGAUCAUCAAAAAGCU | | [1259-1279] ORF | [1052-1072] ORF |
| 6 | GCAGAGAGCUUUUUGAUGAUC | GAUCAUCAAAAAGCUCUCUGC | | [1253-1273] ORF | [1046-1066] ORF |
| 7 | CGAGUUGCGCUUCAAACAAUA | UAUUGUUUGAAGCGCAACUCG | | [924-944] ORF | [714-734] ORF |
| 8 | UAACCUGAAAUUUGCUGGAAU | AUUCCAGCAAAUUUCAGGUUA | | [669-689] ORF | [459-479] ORF |
| 9 | GUAACCUGAAAUUUGCUGGAA | UUCCAGCAAAUUUCAGGUUAC | | [668-688] ORF | [458-478] ORF |
| 10 | CCGCUUUGAAAGUGUCAGUCA | UGACUGACACUUUCAAAGCGG | | [1641-1661] ORF | [1434-1454] ORF |
| 11 | UGAUCCCUCCUAUGUCAACGU | ACGUUGACAUAGGAGGGAUCA | | [1269-1289] ORF | [1062-1082] ORF |
| 12 | CCACGGGAGCUUUGUCAAUAA | UUAUUGACAAAGCUCCCGUGG | | [408-428] ORF | [198-218] ORF |
| 13 | ACAUGCAAUCUAUCUCAUUUG | CAAAUGAGAUAGAUUGCAUGU | | [767-787] ORF | [557-577] ORF |
| 14 | ACCACAUGCAAUCUAUCUCAU | AUGAGAUAGAUUGCAUGUGGU | | [764-784] ORF | [554-574] ORF |
| 15 | GGACUAAGGAUCACCGCUUUG | CAAAGCGGUGAUCCUUAGUCC | | [1628-1648] ORF | [1421-1441] ORF |
| 16 | GUAGACAUGAGGCUUCGGGAA | UUCCCGAAGCCUCAUGUCUAC | | [1090-1110] ORF | [880-900] ORF |
| 17 | AACCUGAAAUUUGCUGGAAUG | CAUUCCAGCAAAUUUCAGGUU | | [670-690] ORF | [460-480] ORF |
| 18 | CCGGGACCUGUUUGACAUGAA | UUCAUGUCAAACAGGUCCCGG | | [1365-1385] ORF | [1158-1178] ORF |
| 19 | UCCUAUGUCAACGUCCAGAAC | GUUCUGGACGUUGACAUAGGA | | [1276-1296] ORF | [1069-1089] ORF |
| 20 | CACAUGCAAUCUAUCUCAUUU | AAAUGAGAUAGAUUGCAUGUG | | [766-786] ORF | [556-576] ORF |
| 21 | GGACAUGAACAAGCUGAGUGG | CCACUCAGCUUGUUCAUGUCC | Rat,MO | [327-347] ORF | [117-137] 5'UTR+ORF |
| 22 | CUUCAAACAAUACCUCAGGAA | UUCCUGAGGUAUUGUUUGAAG | | [933-953] ORF | [723-743] ORF |
| 23 | CACCACAUGCAAUCUAUCUCA | UGAGAUAGAUUGCAUGUGGUG | | [763-783] ORF | [553-573] ORF |
| 24 | CCACCACAUGCAAUCUAUCUC | GAGAUAGAUUGCAUGUGGUGG | | [762-782] ORF | [552-572] ORF |
| 25 | GGUUUCCUACUUGGUUCGGUA | UACCGAACCAAGUAGGAAACC | Rat,MO | [477-497] ORF | [267-287] ORF |
| 26 | CAGCUACCACAUGGACAAUCA | UGAUUGUCCAUGUGGUAGCUG | Rat,MO | [1668-1688] ORF | [1461-1481] ORF |
| 27 | GGUUCGGUACAUGGGUUGUGU | ACACAACCCAUGUACCGAACC | | [489-509] ORF | [279-299] ORF |
| 28 | ACUAAGGAUCACCGCUUUGAA | UUCAAAGCGGUGAUCCUUAGU | | [1630-1650] ORF | [1423-1443] ORF |
| 29 | CGGGACCUGUUUGACAUGAAG | CUUCAUGUCAAACAGGUCCCG | | [1366-1386] ORF | [1159-1179] ORF |
| 30 | GCUUCAAACAAUACCUCAGGA | UCCUGAGGUAUUGUUUGAAGC | | [932-952] ORF | [722-742] ORF |
| 31 | CGCUUCAAACAAUACCUCAGG | CCUGAGGUAUUGUUUGAAGCG | | [931-951] ORF | [721-741] ORF |
| 32 | GUACAUGGGUUGUGUGGAGGU | ACCUCCACACAACCCAUGUAC | | [495-515] ORF | [285-305] ORF |
| 33 | GGUGUGGUUCGGACUAAGGAU | AUCCUUAGUCCGAACCACACC | | [1618-1638] ORF | [1411-1431] ORF |
| 34 | UGAGGGUGUGGUUCGGACUAA | UUAGUCCGAACCACACCCUCA | | [1614-1634] ORF | [1407-1427] ORF |
| 35 | GCGCUUCAAACAAUACCUCAG | CUGAGGUAUUGUUUGAAGCGC | | [930-950] ORF | [720-740] ORF |
| 36 | UGCGCUUCAAACAAUACCUCA | UGAGGUAUUGUUUGAAGCGCA | | [929-949] ORF | [719-739] ORF |
| 37 | GAGUUGCGCUUCAAACAAUAC | GUAUUGUUUGAAGCGCAACUC | | [925-945] ORF | [715-735] ORF |
| 38 | UCGAGUUGCGCUUCAAACAAU | AUUGUUUGAAGCGCAACUCGA | | [923-943] ORF | [713-733] ORF |
| 39 | CACGGGAGCUUUGUCAAUAAG | CUUAUUGACAAAGCUCCCGUG | Rat,MO | [409429] ORF | [199-219] ORF |
| 40 | GGACAAUCACUUGCCCAUCAU | AUGAUGGGCAAGUGAUUGUCC | MO | [1680-1700] ORF | [1473-1493] ORF |
| 41 | CCAUCAGUACUAUAAUGACUU | AAGUCAUUAUAGUACUGAUGG | | [1038-1058] ORF | [828-848] ORF |
| 42 | GGGACCUGUUUGACAUGAAGC | GCUUCAUGUCAAACAGGUCCC | | [1367-1387] ORF | [1160-1180] ORF |
| 43 | GAGAUCCAGAAGUCCGCAAAC | GUUUGCGGACUUCUGGAUCUC | | [1205-1225] ORF | [995-1015] ORF |
| 44 | GGGUUUCCUACUUGGUUCGGU | ACCGAACCAAGUAGGAAACCC | Rat,MO | [476-496] ORF | [266-286] ORF |
| 45 | GCCGAGUAUGUCGCCUAUGUU | AACAUAGGCGACAUACUCGGC | | [811-831] ORF | [601-621] ORF |
| 46 | GGAGAUCCAGAAGUCCGCAAA | UUUGCGGACUUCUGGAUCUCC | | [1204-1224] ORF | [994-1014] ORF |
| 47 | GGAGUAACCUGAAAUUUGCUG | CAGCAAAUUUCAGGUUACUCC | | [665-685] ORF | [455-475] ORF |
| 48 | GGGAGGAGUAACCUGAAAUUU | AAAUUUCAGGUUACUCCUCCC | | [661-681] ORF | [451-471] ORF |
| 49 | UGAAAUUUGCUGGAAUGCCAA | UUGGCAUUCCAGCAAAUUUCA | | [674-694] ORF | [464-484] ORF |
| 50 | UGAAAGUGUCAGUCACCUUAU | AUAAGGUGACUGACACUUUCA | | [1647-1667] ORF | [1440-1460] ORF |
| 51 | GGCAGAGAGCUUUUUGAUGAU | AUCAUCAAAAAGCUCUCUGCC | | [1252-1272] ORF | [1045-1065] ORF |
| 52 | CCUGACCAUCAGUACUAUAAU | AUUAUAGUACUGAUGGUCAGG | | [1033-1053] ORF | [623-843] ORF |
| 53 | GCCACCUGACCAUCAGUACUA | UAGUACUGAUGGUCAGGUGGC | | [1029-1049] ORF | [819-839] ORF |
| 54 | GGGCAGCCUAAGCAUUUGCUA | UAGCAAAUGCUUAGGCUGCCC | | [1582-1602] ORF | [1375-1395] ORF |
| 55 | GUAUGUGCUCACUGGCUUGCA | UGCAAGCCAGUGAGCACAUAC | | [1557-1577] ORF | [1350-1370] ORF |
| 56 | GACAUGAAGCCCUUCGAAGAU | AUCUUCGAAGGGCUUCAUGUC | Rat | [1378-1398] ORF | [1171-1191] ORF |
| 57 | CGCAGACUGCAAACAGAUCAU | AUGAUCUGUUUGCAGUCUGCG | | [735-755] ORF | [525-545] ORF |
| 58 | GCGAACUGUGUCUACAGCAAC | GUUGCUGUAGACACAGUUCGC | | [1712-1732] ORF | [1505-1525] ORF |
| 59 | CGGACUAAGGAUCACCGCUUU | AAAGCGGUGAUCCUUAGUCCG | | [1627-1647] ORF | [1420-1440] ORF |
| 60 | CCAGAAGUCCGCAAACAGAUG | CAUCUGUUUGCGGACUUCUGG | | [1210-1230] ORF | [1000-1020] ORF |
| 61 | GGAGGAGUAACCUGAAAUUUG | CAAAUUUCAGGUUACUCCUCC | | [662-682] ORF | [452-472] ORF |
| 62 | CUACCACAUGGACAAUCACUU | AAGUGAUUGUCCAUGUGGUAG | Rat,MO | [1671-169] ORF | [1464-1484] ORF |
| 63 | CAACGUCCAGAACCUAGACAA | UUGUCUAGGUUCUGGACGUUG | | [1284-1304] ORF | [1077-1097] ORF |
| 64 | UGACCAUCAGUACUAUAAUGA | UCAUUAUAGUACUGAUGGUCA | | [1035-1055] ORF | [825-845] ORF |
| 65 | ACAGCCGAGUAUGUCGCCUAU | AUAGGCGACAUACUCGGCUGU | | [808-828] ORF | [598-618] ORF |
| 66 | CUCCUAUGUCAACGUCCAGAA | UUCUGGACGUUGACAUAGGAG | | [1275-1295] ORF | [1068-1088] ORF |
| 67 | GGAGCUACAUUGCCUGUAGGA | UCCUACAGGCAAUGUAGCUCC | | [1171-1191] ORF | [961-981] ORF |
| 68 | GACAGGAUGGCUGGCUUUGAU | AUCAAAGCCAGCCAUCCUGUC | Rat,MO | [979-999] ORF | [769-789] ORF |
| 69 | CCAAAGACCCUGUGAAUCAGA | UCUGAUUCACAGGGUCUUUGG | Rat,MO | [833-853] ORF | [623-643] ORF |
| 70 | CUGCAAACAGAUCAUCGCCAA | UUGGCGAUGAUCUGUUUGCAG | | [741-761] ORF | [531-551] ORF |
| 71 | CUAAGGAUCACCGCUUUGAAA | UUUCAAAGCGGUGAUCCUUAG | | [1631-1651] ORF | [1424-1444] ORF |
| 72 | GACCAUCAGUACUAUAAUGAC | GUCAUUAUAGUACUGAUGGUC | | [1036-1056] ORF | [826-846] ORF |
| 73 | CCACCUGACCAUCAGUACUAU | AUAGUACUGAUGGUCAGGUGG | | [1030-1050] ORF | [820-840] ORF |
| 74 | UCAUGACAGGAUGGCUGGCUU | AAGCCAGCCAUCCUGUCAUGA | | [975995] ORF | [765-785] ORF |
| 75 | GCAAUCUAUCUCAUUUGCAUC | GAUGCAAAUGAGAUAGAUUGC | | [771-791] ORF | [561-581] ORF |
| 76 | GCAGACUGCAAACAGAUCAUC | GAUGAUCUGUUUGCAGUCUGC | | [736-756] ORF | [526-546] ORF |
| 77 | CAAGGAGGAGAAAGCCCUGUA | UACAGGGCUUUCUCCUCCUUG | | [617-637] ORF | [407-427] ORF |
| 78 | AGCGAACUGUGUCUACAGCAA | UUGCUGUAGACACAGUUCGCU | | [1711-1731] ORF | [1504-1524] ORF |
| 79 | GCCUAAGCAUUUGCUACUGGU | ACCAGUAGCAAAUGCUUAGGC | | [1587-1607] ORF | [1380-1400] ORF |
| 60 | GAGAGCUUUUUGAUGAUCCCU | AGGGAUCAUCAAAAAGCUCUC | | [1256-1276] ORF | [1049-1069] ORF |
| 81 | GUAUGUCGCCUAUGUUGCCAA | UUGGCAACAUAGGCGACAUAC | | [816-836] ORF | [606-626] ORF |
| 82 | GAACUGUGUCUACAGCAACCU | AGGUUGCUGUAGACACAGUUC | | [1714-1734] ORF | [1507-1527] ORF |
| 83 | GACAAUCACUUGCCCAUCAUC | GAUGAUGGGCAAGUGAUUGUC | MO | [1681-1701] ORF | [1474-1494] ORF |
| 84 | CCACAUGGACAAUCACUUGCC | GGCAAGUGAUUGUCCAUGUGG | MO | [1674-1694] ORF | [1467-1487] ORF |
| 85 | AGAGAGCUUUUUGAUGAUCCC | GGGAUCAUCAAAAAGCUCUCU | | [1255-1275] ORF | [1048-1068] ORF |
| 86 | CAUGACAGGAUGGCUGGCUUU | AAAGCCAGCCAUCCUGUCAUG | Rat,MO | [976-996] ORF | [766-766] ORF |
| 87 | GCCUUCGAGUUGCGCUUCAAA | UUUGAAGCGCAACUCGAAGGC | | [919-939] ORF | [709-729] ORF |
| 88 | CCGAGUAUGUCGCCUAUGUUG | CAACAUAGGCGACAUACUCGG | | [812-832] ORF | [602-622] ORF |
| 89 | CCAACCACCACAUGCAAUCUA | UAGAUUGCAUGUGGUGGUUGG | | [758-778] ORF | [548-568] ORF |
| 90 | UGCCAAUCACUCUCACCGUCU | AGACGGUGAGAGUGAUUGGCA | | [689-709] ORF | [479-499] ORF |
| 91 | GGAAUGCCAAUCACUCUCACC | GGUGAGAGUGAUUGGCAUUCC | | [685-705] ORF | [475-495] ORF |
| 92 | UGGCUGCAUCCCAACGACAAA | UUUGUCGUUGGGAUGCAGCCA | Rat,MO | [442-462] ORF | [232-252] ORF |
| 93 | AGCUACCACAUGGACAAUCAC | GUGAUUGUCCAUGUGGUAGCU | Rat,MO | [1669-1689] ORF | [1462-1482] ORF |
| 94 | GGAUCACCGCUUUGAAAGUGU | ACACUUUCAAAGCGGUGAUCC | | [1635-1655] ORF | [1428-1448] ORF |
| 95 | CACCUGACCAUCAGUACUAUA | UAUAGUACUGAUGGUCAGGUG | | [1031-1051] ORF | [821-841] ORF |
| 96 | GUCGCCUAUGUUGCCAAAGAC | GUCUUUGGCAACAUAGGCGAC | | [820-840] ORF | [610-630] ORF |
| 97 | GAAAUUUGCUGGAAUGCCAAU | AUUGGCAUUCCAGCAAAUUUC | | [675-695] ORF | [465-485] ORF |
| 98 | UCCUACUUGGUUCGGUACAUG | CAUGUACCGAACCAAGUAGGA | Rat,MO | [481-501] ORF | [271-291] ORF |
| 99 | ACAUGGACAAUCACUUGCCCA | UGGGCAAGUGAUUGUCCAUGU | MO | [1676-1696] ORF | [1469-1489] ORF |
| 100 | GUCAGUCACCUUAUCAGCUAC | GUAGCUGAUAAGGUGACUGAC | | [1654-1674] ORF | [1447-1467] ORF |
| 101 | CACCGCUUUGAAAGUGUCAGU | ACUGACACUUUCAAAGCGGUG | | [1639-1659] ORF | [1432-1452] ORF |
| 102 | UCCAGAAGUCCGCAAACAGAU | AUCUGUUUGCGGACUUCUGGA | | [1209-1229] ORF | [999-1019] ORF |
| 103 | ACAGGAUGGCUGGCUUUGAUG | CAUCAAAGCCAGCCAUCCUGU | Rat,MO | [980-1000] ORF | [770-790] ORF |
| 104 | GCCAAAGACCCUGUGAAUCAG | CUGAUUCACAGGGUCUUUGGC | Rat,MO | [832-852] ORF | [622-642] ORF |
| 105 | GUUGCCAAAGACCCUGUGAAU | AUUCACAGGGUCUUUGGCAAC | Rat,MO | [829-849] ORF | [619-639] ORF |
| 106 | CAACCACCACAUGCAAUCUAU | AUAGAUUGCAUGUGGUGGUUG | | [759-779] ORF | [549-569] ORF |
| 107 | CUGGAAUGCCAAUCACUCUCA | UGAGAGUGAUUGGCAUUCCAG | MO | [683-703] ORF | [473-493] ORF |
| 108 | UCACCUUAUCAGCUACCACAU | AUGUGGUAGCUGAUAAGGUGA | | [1659-1679] ORF | [1452-1472] ORF |
| 109 | CGCUUUGAAAGUGUCAGUCAC | GUGACUGACACUUUCAAAGCG | | [1642-1662] ORF | [1435-1455] ORF |
| 110 | CCUAAGCAUUUGCUACUGGUG | CACCAGUAGCAAAUGCUUAGG | | [1588-1608] ORF | [1381-1401] ORF |
| 111 | GCAGCCUAAGCAUUUGCUACU | AGUAGCAAAUGCUUAGGCUGC | | [1584-1604] ORF | [1377-1397] ORF |
| 112 | CUGGCUUUGAUGGCUCAGCAU | AUGCUGAGCCAUCAAAGCCAG | | [989-1009] ORF | [779-799] ORF |
| 113 | CACAUUCUGGAGUGUCCCGAA | UUCGGGACACUCCAGAAUGUG | | [862-882] ORF | [652-672] ORF |
| 114 | UGCAAUCUAUCUCAUUUGCAU | AUGCAAAUGAGAUAGAUUGCA | | [770-790] ORF | [560-580] ORF |
| 115 | UGGAAUGCCAAUCACUCUCAC | GUGAGAGUGAUUGGCAUUCCA | MO | [684-704] ORF | [474-494] ORF |
| 116 | CCUUAUCAGCUACCACAUGGA | UCCAUGUGGUAGCUGAUAAGG | | [1662-1682] ORF | [1455-1475] ORF |
| 117 | CAGCCUAAGCAUUUGCUACUG | CAGUAGCAAAUGCUUAGGCUG | | [1585-1605] ORF | [1378-1398] ORF |
| 118 | CCUGCUAUCAAUGGCAGUGCA | UGCACUGCCAUUGAUAGCAGG | | [1342-1362] ORF | [1135-1155] ORF |
| 119 | ACCUGACCAUCAGUACUAUAA | UUAUAGUACUGAUGGUCAGGU | | [1032-1052] ORF | [822-842] ORF |
| 120 | CAAACAGAUCAUCGCCAACCA | UGGUUGGCGAUGAUCUGUUUG | | [744-764] ORF | [534-554] ORF |
| 121 | CCAACGACAAAGUCAUGGGAC | GUCCCAUGACUUUGUCGUUGG | Rat,MO | [452-472] ORF | [242-262] ORF |
| 122 | CAGCGAACUGUGUCUACAGCA | UGCUGUAGACACAGUUCGCUG | | [1710-1730] ORF | [1503-1523] ORF |
| 123 | GCUUUGAAAGUGUCAGUCACC | GGUGACUGACACUUUCAAAGC | | [1643-1663] ORF | [1436-1456] ORF |
| 124 | UCGGACUAAGGAUCACCGCUU | AAGCGGUGAUCCUUAGUCCGA | | [1626-1646] ORF | [1419-1439] ORF |
| 125 | CCAGUAUGUGCUCACUGGCUU | AAGCCAGUGAGCACAUACUGG | | [1554-1574] ORF | [1347-1367] ORF |
| 126 | CUGCUAUCAAUGGCAGUGCAC | GUGCACUGCCAUUGAUAGCAG | | [1343-1363] ORF | [1136-1156] ORF |
| 127 | GCUGCAUCCCAACGACAAAGU | ACUUUGUCGUUGGGAUGCAGC | Rat,MO | [444-464] ORF | [234-254] ORF |
| 128 | AAAGUGUCAGUCACCUUAUCA | UGAUAAGGUGACUGACACUUU | | [1649-1669] ORF | [1442-1462] ORF |
| 129 | CCAGGAUGUCAUCAGCACCAU | AUGGUGCUGAUGACAUCCUGG | | [891-911] ORF | [681-701] ORF |
| 130 | GCCUAUGUUGCCAAAGACCCU | AGGGUCUUUGGCAACAUAGGC | Rat,MO | [823-843] ORF | [613-633] ORF |
| 131 | GCCAACCACCACAUGCAAUCU | AGAUUGCAUGUGGUGGUUGGC | | [757-777] ORF | [547-567] ORF |
| 132 | UGCAAACAGAUCAUCGCCAAC | GUUGGCGAUGAUCUGUUUGCA | | [742-762] ORF | [532-552] ORF |
| 133 | GCCACGGGAGCUUUGUCAAUA | UAUUGACAAAGCUCCCGUGGC | | [407-427] ORF | [197-217] ORF |
| 134 | GUCAACGUCCAGAACCUAGAC | GUCUAGGUUCUGGACGUUGAC | | [1282-1302] ORF | [1075-1095] ORF |
| 135 | ACAUUGCCUGUAGGACAGCCU | AGGCUGUCCUACAGGCAAUGU | | [1177-1197] ORF | [967-987] ORF |
| 136 | UGGGAGCUACAUUGCCUGUAG | CUACAGGCAAUGUAGCUCCCA | | [1169-1189] ORF | [959-979] ORF |
| 137 | CAGCCACUUGGGAGCUACAUU | AAUGUAGCUCCCAAGUGGCUG | | [1161-1181] ORF | [951-971] ORF |
| 138 | ACCCUGUGAAUCAGAGAGCCU | AGGCUCUCUGAUUCACAGGGU | Rat,MO | [839-859] ORF | [629-649] ORF |
| 139 | CCGCAGACUGCAAACAGAUCA | UGAUCUGUUUGCAGUCUGCGG | | [734-754] ORF | [524-544] ORF |
| 140 | CUGCAUCCCAACGACAAAGUC | GACUUUGUCGUUGGGAUGCAG | Rat,MO | [445-465] ORF | [235-255] ORF |
| 141 | GAGCUUUGUCAAUAAGCCCAC | GUGGGCUUAUUGACAAAGCUC | Rat,MO | [414-434] ORF | [204-224] ORF |
| 142 | CAGUCACCUUAUCAGCUACCA | UGGUAGCUGAUAAGGUGACUG | | [1656-1676] ORF | [1449-1469] ORF |
| 143 | CCUGUUUGACAUGAAGCCCUU | AAGGGCUUCAUGUCAAACAGG | | [1371-1391] ORF | [1164-1184] ORF |
| 144 | CUAUGUUGCCAAAGACCCUGU | ACAGGGUCUUUGGCAACAUAG | Rat,MO | [825-845] ORF | [615-635] ORF |
| 145 | GGAGCUUUGUCAAUAAGCCCA | UGGGCUUAUUGACAAAGCUCC | Rat,MO | [413-433] ORF | [203-223] ORF |
| 146 | GGGAGCUUUGUCAAUAAGCCC | GGGCUUAUUGACAAAGCUCCC | Rat,MO | [412-432] ORF | [202-222] ORF |
| 147 | CUGUGUCUACAGCAACCUGUG | CACAGGUUGCUGUAGACACAG | Rat | [1717-1737] ORF | [1510-1530] ORF |
| 148 | CAGUAUGUGCUCACUGGCUUG | CAAGCCAGUGAGCACAUACUG | | [1555-1575] ORF | [1348-1368] ORF |
| 149 | CCAAUCCUGCUAUCAAUGGCA | UGCCAUUGAUAGCAGGAUUGG | | [1337-1357] ORF | [1130-1150] ORF |
| 150 | AGGAGUAACCUGAAAUUUGCU | AGCAAAUUUCAGGUUACUCCU | | [664-684] ORF | [454-474] ORF |
| 151 | CAGGAUGUCAUCAGCACCAUU | AAUGGUGCUGAUGACAUCCUG | | [892-912] ORF | [682-702] ORF |
| 152 | GCAUCCCAACGACAAAGUCAU | AUGACUUUGUCGUUGGGAUGC | Rat,MO | [447-467] ORF | [237-257] ORF |
| 153 | CUGUUUGACAUGAAGCCCUUC | GAAGGGCUUCAUGUCAAACAG | | [1372-1392] ORF | [1165-1185] ORF |
| 154 | UCAGAGAGCCUGCCACAUUCU | AGAAUGUGGCAGGCUCUCUGA | | [849-869] ORF | [639-659] ORF |
| 155 | UGAAUCAGAGAGCCUGCCACA | UGUGGCAGGCUCUCUGAUUCA | | [845-865] ORF | [635-655] ORF |
| 156 | CCCAACGACAAAGUCAUGGGA | UCCCAUGACUUUGUCGUUGGG | Rat,MO | [451-471] ORF | [241-261] ORF |
| 157 | GGAUGUCAUCAGCACCAUUGG | CCAAUGGUGCUGAUGACAUCC | | [894-914] ORF | [684-704] ORF |
| 158 | GAUCAUCGCCAACCACCACAU | AUGUGGUGGUUGGCGAUGAUC | | [750-770] ORF | [540-560] ORF |

**Table I: 19-mer zinc finger, HIT type 1 (ZNHIT1)**

| Number | Sense siRNA | AntiSense siRNA | Other Sp | Human-37594439 ORF:493-957 |
|---|---|---|---|---|
| 1 | CCGAGGUGAUCAUUUUAAA | UUUAAAAUGAUCACCUCGG | | [705-723] ORF |
| 2 | CCCGAGGUGAUCAUUUUAA | UUAAAAUGAUCACCUCGGG | | [704-722] ORF |
| 3 | CGUGACCACAUCUUUAAAA | UUUUAAAGAUGUGGUCACG | | [240-258] 5'UTR |
| 4 | CCCUAUAAAACAUGGCGAA | UUCGCCAUGUUUUAUAGGG | | [321-339] 5'UTR |
| 5 | GUGACCACAUCUUUAAAAU | AUUUUAAAGAUGUGGUCAC | | [241-259] 5'UTR |
| 6 | GAAAAAACUUUCAGGCCCU | AGGGCCUGAAAGUUUUUUC | | [734-752] ORF |
| 7 | CCCUGGAGAAUGACAACUU | AAGUUGUCAUUCUCCAGGG | | [593-611] ORF |
| 8 | CUAUAAAACAUGGCGAAAA | UUUUCGCCAUGUUUUAUAG | | [323-341] 5'UTR |
| 9 | CCGUGACCACAUCUUUAAA | UUUAAAGAUGUGGUCACGG | | [239-257] 5'UTR |
| 10 | ACCGUGACCACAUCUUUAA | UUAAAGAUGUGGUCACGGU | | [238-256] 5'UTR |
| 11 | CAGAAAGACAGAAUUUCAU | AUGAAAUUCUGUCUUUCUG | | [1006-1024] 3'UTR |
| 12 | CGGACACUGGAAAGAAAAA | UUUUUCUUUCCAGUGUCCG | CHL | [677-695] ORF |
| 13 | CAGUGCUGUUAGAAUAAAA | UUUUAUUCUAACAGCACUG | | [1156-1174] 3'UTR |
| 14 | GCUCAUUCACCCAACAAAA | UUUUGUUGGGUGAAUGAGC | | [1068-1086] 3'TR |
| 15 | UCAUUUUAAACUUCGCUUC | GAAGCGAAGUUUAAAAUGA | | [714-732] ORF |
| 16 | GAGGUGAUCAUUUUAAACU | AGUUUAAAAUGAUCACCUC | | [707-725] ORF |
| 17 | CGAGGUGAUCAUUUUAAAC | GUUUAAAAUGAUCACCUCG | | [706-724] ORF |
| 18 | GCGGACACUGGAAAGAAAA | UUUUCUUUCCAGUGUCCGC | CHL | [676-694] ORF |
| 19 | CGCCACGUAAUGAGUCAAA | UUUGACUCAUUACGUGGCG | | [363-381] 5'UTR |
| 20 | CCUUCAGAAAGACAGAAUU | AAUUCUGUCUUUCUGAAGG | | [1002-1020] 3'UTR |
| 21 | CCUAUAAAACAUGGCGAAA | UUUCGCCAUGUUUUAUAGG | | [322-340] 5'UTR |
| 22 | ACAGCAUCUUCACAAGGAC | GUCCUUGUGAAGAUGCUGU | | [190-208] 5'UTR |
| 23 | CACAGCAUCUUCACAAGGA | UCCUUGUGAAGAUGCUGUG | | [189-207] 5'UTR |
| 24 | CCCAGUGCUGUUAGAAUAA | UUAUUCUAACAGCACUGGG | | [1154-1172] 3'UTR |
| 25 | ACCCGAGGUGAUCAUUUUA | UAAAAUGAUCACCUCGGGU | | [703-721] ORF |
| 26 | AACCCGAGGUGAUCAUUUU | AAAAUGAUCACCUCGGGUU | | [702-720] ORF |
| 27 | GCCCUGGAGAAUGACAACU | AGUUGUCAUUCUCCAGGGC | | [592-610] ORF |
| 28 | ACACCGUGACCACAUCUUU | AAAGAUGUGGUCACGGUGU | | [236-254] 5'UTR |
| 29 | GCAUCUUCACAAGGACCAA | UUGGUCCUUGUGAAGAUGC | | [193-211] 5'UTR |
| 30 | GAUCACAGCAUCUUC6CAA | UUGUGAAGAUGCUGUGAUC | | [186-204] 5'UTR |
| 31 | GGAGCAGAACUUGAGUGUG | CACACUCAAGUUCUGCUCC | | [759-777] ORF |
| 32 | UGAUCAUUUUAAACUUCGC | GCGAAGUUUAAAAUGAUCA | | [711-729] ORF |
| 33 | GUGAUCAUUUUAAACUUCG | CGAAGUUUAAAAUGAUCAC | | [710-728] ORF |
| 34 | CUGGAAAGAAAAAGAAGAA | UUCUUCUUUUUCUUUCCAG | CHL | [683-701] ORF |
| 35 | AGGCCCUGGAGAAUGACAA | UUGUCAUUCUCCAGGGCCU | | [590-608] ORF |
| 36 | GUACAAGCUACCGGAAGUG | CACUUCCGGUAGCUUGUAC | | [402-420] 5'UTR |
| 37 | CACCGUGACCACAUCUUUA | UAAAGAUGUGGUCACGGUG | | [237-255] 5'UTR |
| 38 | GACCAAAGGAAAAUAAGAU | AUCUUAUUUUCCUUUGGUC | | [206-224] 5'UTR |
| 39 | CUGUUAGAAUAAAAAGCCU | AGGCUUUUUAUUCUAACAG | | [1161-1179]3'UTR |
| 40 | UCAGAAAGACAGAAUUUCA | UGAAAUUCUGUCUUUCUGA | | [1005-1023] 3'UTR |
| 41 | AGGAGCAGAACUUGAGUGU | ACACUCAAGUUCUGCUCCU | | [758-776] ORF |
| 42 | CCACUUUCUUUGACAGUCC | GGACUGUCAAAGAAAGUGG | | [120-138] 5'UTR |
| 43 | GGAGGAGCAGAACUUGAGU | ACUCAAGUUCUGCUCCUCC | | [756-774] ORF |
| 44 | ACAAGCUACCGGAAGUGAU | AUCACUUCCGGUAGCUUGU | | [404-422] 5'UTR |
| 45 | AGAACACCGUGACCACAUC | GAUGUGGUCACGGUGUUCU | | [233-251] 5'UTR |
| 46 | CAAGGACCAAAGGAAAAUA | UAUUUUCCUUUGGUCCUUG | | [202-220] 5'UTR |
| 47 | AGCAUCUUCACAAGGACCA | UGGUCCUUGUGAAGAUGCU | | [192-210] 5'UTR |
| 48 | UGUCUUAUCUGCCAGGAAA | UUUCCUGGCAGAUAAGACA | | [1090-1108] 3'UTR |
| 49 | CUUCAGAAAGACAGAAUUU | AAAUUCUGUCUUUCUGAAG | | [1003-1021] 3'UTR |
| 50 | ACACUGGAAAGAAAAAGAA | UUCUUUUUCUUUCCAGUGU | CHL | [680-698] ORF |
| 51 | GCCACGUAAUGAGUCAAAG | CUUUGACUCAUUACGUGGC | | [364-382] 5'UTR |
| 52 | GGAAACGCCACGUAAUGAG | CUCAUUACGUGGCGUUUCC | | [358-376] 5'UTR |
| 53 | CAAGGAAACGCCACGUAAU | AUUACGUGGCGUUUCCUUG | | [355-373] 5'UTR |
| 54 | GGACCAAAGGAAAAUAAGA | UCUUAUUUUCCUUUGGUCC | | [205-223] 5'UTR |
| 55 | ACAAGGACCAAAGGAAAAU | AUUUUCCUUUGGUCCUUGU | | [201-219] 5'UTR |
| 56 | GUGUCUUAUCUGCCAGGAA | UUCCUGGCAGAUAAGACAC | | [1089-1107] 3'UTR |
| 57 | CACUUUCUUUGACAGUCCA | UGGACUGUCAAAGAAAGUG | | [121-139] 5'UTR |
| 58 | ACGAUGCGGACACUGGAAA | UUUCCAGUGUCCGCAUCGU | | [671-689] ORF |
| 59 | AUGGUGGAGAAGAAMCUU | AAGUUUUCUUCUCCACCAU | | [493-511] ORF |
| 60 | GCAGUUUCUUCCGACAGUU | AACUGUCGGAAGAAACUGC | | [463-481] 5'UTR |
| 61 | CAUCUUCACAAGGACCAAA | UUUGGUCCUUGUGAAGAUG | | [194-212] 5'UTR |
| 62 | UGUUGGAGGAGCAGAACUU | AAGUUCUGCUCCUCCAACA | | [752-770] ORF |
| 63 | GGACACUGGAAAGAAAAAG | CUUUUUCUUUCCAGUGUCC | CHL | [678-696] ORF |
| 64 | ACGCCACGUAAUGAGUCAA | UUGACUCAUUACGUGGCGU | | [362-380] 5'UTR |
| 65 | AAGGACCAAAGGAAAAUAA | UUAUUUUCCUUUGGUCCUU | | [203-221] 5'UTR |
| 66 | UCAGUUUGAUGACGAUGCG | CGCAUCGUCAUCAAACUGA | | [660-678] ORF |
| 67 | CGUAAUGAGUCAAAGCUGU | ACAGCUUUGACUCAUUACG | | [366-366] 5'UTR |
| 68 | CACGUAAUGAGUCAAAGCU | AGCUUUGACUCAUUACGUG | | [366-384] 5'UTR |
| 69 | UCACAAGGACCAAAGGAAA | UUUCCUUUGGUCCUUGUGA | | [199-217] 5'UTR |
| 70 | GCCUUUGUGAUCACAGCAU | AUGCUGUGAUCACAAAGGC | | [178-195] 5'UTR |
| 71 | GCAAGAGACUGCCUCAGUU | AACUGAGGCAGUCUCUUGC | | [647-665] ORF |
| 72 | CACAAGGACCAAAGGAAAA | UUUUCCUUUGGUCCUUGUG | | [200-218] 5'UTR |
| 73 | CCACGUAAUGAGUCAAAGC | GCUUUGACUCAUUACGUGG | | [365-383] 5'UTR |
| 74 | ACAGAAUUUCAUCACCCAA | UUGGGUGAUGAAAUUCUGU | | [1013-1031] 3'UTR |
| 75 | GACACUGGAAAGAAAAAGA | UCUUUUUCUUUCCAGUGUC | CHL | [679-697] ORF |
| 76 | GGAGAAGAAAACUUCGGUU | AACCGAAGUUUUCUUCUCC | | [498-516] ORF |
| 77 | CCAACAAAACUGUGUCUUA | UAAGACACAGUUUUGUUGG | | [1078-1096] 3'UTR |
| 78 | UCGCUUCCGAAAAAACUUU | AAAGUUUUUUCGGAAGCGA | | [726-744] ORF |
| 79 | AAACUUCGCUUCCGAAAAA | UUUUUCGGAAGCGAAGUUU | GP | [721-739] ORF |
| 80 | CGCGCAGAAGUACAAGCUA | UAGCUUGUACUUCUGCGCG | | [393-411] 5'UTR |
| 81 | ACAUCUUUAAAAUGACCCA | UGGGUCAUUUUAAAGAUGU | | [247-265] 5'UTR |
| 82 | ACCACAUCUUUAAAAUGAC | GUCAUUUUAAAGAUGUGGU | | [244-262] 5'UTR |
| 83 | CCCAACAAAACUGUGUCUU | AAGACACAGUUUUGUUGGG | | [1077-1095] 3'UTR |
| 84 | CGCUCAUUCACCCAACAAA | UUUGUUGGGUGAAUGAGCG | | [1067-1085] 3'UTR |
| 85 | AAAACUUUCAGGCCCUGUU | AACAGGGCCUGAAAGUUUU | | [737-755] ORF |
| 86 | CGAAAAAACUUUCAGGCCC | GGGCCUGAAAGUUUUUUCG | | [733-751] ORF |
| 87 | GAAGUACAAGCUACCGGAA | UUCCGGUAGCUUGUACUUC | | [399-417] 5'UTR |
| 88 | AACUUCGCUUCCGAAAAAA | UUUUUUCGGAAGCGAAGUU | GP | [722-740] ORF |
| 89 | AGAAGUACAAGCUACCGGA | UCCGGUAGCUUGUACUUCU | | [398-416] 5'UTR |
| 90 | CCACUAGGAACCUCGGAUU | AAUCCGAGGUUCCUAGUGG | | [86-104] 5'UTR |
| 91 | ACAAGAUUUACACCUCCAC | GUGGAGGUGUAAAUCUUGU | | [279-297] 5'UTR |
| 92 | CACAAGAUUUACACCUCCA | UGGAGGUGUAAAUCUUGUG | | [278-296] 5'UTR |
| 93 | GGCUCCCACAAGAUUUACA | UGUAAAUCUUGUGGGAGCC | | [272-290] 5'UTR |
| 94 | CCACAUCUUUAAAAUGACC | GGUCAUUUUAAAGAUGUGG | | [245-263] 5'UTR |
| 95 | CGCUUCCGAAAAAACUUUC | GAAAGUUUUUUCGGAAGCG | | [727-745] ORF |
| 96 | GAGACUGCCUCAGUUUGAU | AUCAAACUGAGGCAGUCUC | | [651-669] ORF |
| 97 | CAAGAGACUGCCUCAGUUU | AAACUGAGGCAGUCUCUUG | | [648-666] ORF |
| 98 | UCCAAGGAAACGCCACGUA | UACGUGGCGUUUCCUUGGA | | [353-371] 5'UTR |
| 99 | GACCACAUCUUUAAAAUGA | UCAUUUUAAAGAUGUGGUC | | [243-261] 5'UTR |
| 100 | CCGCUCAUUCACCCAACAA | UUGUUGGGUGAAUGAGCGG | | [1066-1064] 3'UTR |
| 101 | GACAGAAUUUCAUCACCCA | UGGGUGAUGAAAUUCUGUC | | [1012-1030] 3'UTR |
| 102 | GCCUUCAGAAAGACAGAAU | AUUCUGUCUUUCUGAAGGC | | [1001-1019] 3'UTR |
| 103 | GAAAACCCGAGGUGAUCAU | AUGAUCACCUCGGGUUUUC | | [699-717] ORF |
| 104 | GGAGAAUGACAACUUCCAG | CUGGAAGUUGUCAUUCUCC | CHL,MO | [597-615] ORF |
| 105 | CAAUGGUGGAGAAGAAAAC | GUUUUCUUCUCCACCAUUG | | [491-509] 5'UTR+ORF |
| 106 | GCGCAGAAGUACAAGCUAC | GUAGCUUGUACUUCUGCGC | | [394-412] 5'UTR |
| 107 | CAACAAAACUGUGUCUUAU | AUAAGACACAGUUUUGUUG | | [1079-1097] 3'UTR |
| 108 | GCUUCCGAAAAAACUUUCA | UGAAAGUUUUUUCGGAAGC | | [728-746] ORF |
| 109 | CCCACAAGAUUUACACCUC | GAGGUGUAAAUCUUGUGGG | | [276-294] 5'UTR |
| 110 | GCUCCCACAAGAUUUACAC | GUGUAAAUCUUGUGGGAGC | | [273-291] 5'UTR |
| 111 | GAAAGACAGAAUUUCAUCA | UGAUGAAAUUCUGUCUUUC | | [1008-1026] 3'UTR |
| 112 | GCCUCAGUUUGAUGACGAU | AUCGUCAUCAAACUGAGGC | | [657-675] ORF |
| 113 | GAGAAGAAAACUUCGGUUC | GAACCGAAGUUUUCUUCUC | | [499-517] ORF |
| 114 | CAGUUGUGUUGUGCCAAUG | CAUUGGCACAACACAACUG | | [477-495] 5'UTR+ORF |
| 115 | CGACAGUUGUGUUGUGCCA | UGGCACAACACAACUGUCG | | [474-492] 5'UTR |
| 116 | GCAGCAGUUUCUUCCGACA | UGUCGGAAGAAACUGCUGC | | [460-478] 5'UTR |
| 117 | GCUUUCUUGUCUCCAAGGA | UCCUUGGAGACAAGAAAGC | | [342-360] 5'UTR |
| 118 | CCACAAGAUUUACACCUCC | GGAGGUGUAAAUCUUGUGG | | [277-295] 5'UTR |
| 119 | CAUCUUUAAAAUGACCCAU | AUGGGUCAUUUUAAAGAUG | | [248-266] 5'UTR |
| 120 | UGACCACAUCUUUAAAAUG | CAUUUUAAAGAUGUGGUCA | | [242-260] 5'UTR |
| 121 | CCUUUGUGAUCACAGCAUC | GAUGCUGUGAUCACAAAGG | | [179-197] 5'UTR |
| 122 | AGACAGAAUUUCAUCACCC | GGGUGAUGAAAUUCUGUCU | | [1011-1029] 3'UTR |
| 123 | GGCCUUCAGAAAGACAGAA | UUCUGUCUUUCUGAAGGCC | | [1000-1018] 3'UTR |
| 124 | UGAUGACGAUGCGGACACU | AGUGUCCGCAUCGUCAUCA | | [666-684] ORF |
| 125 | UGGAGAAGAAAACUUCGGU | ACCGAAGUUUUCUUCUCCA | | [497-515] ORF |
| 126 | UUGUGCCAAUGGUGGAGAA | UUCUCCACCAUUGGCACAA | | [485-503] 5'UTR+ORF |
| 127 | CCAAGGAAACGCCACGUAA | UUACGUGGCGUUUCCUUGG | | [354-372] 5'UTR |
| 128 | UGAGGCCGGAAGUGGUUUU | AAAACCACUUCCGGCCUCA | | [300-318] 5'UTR |
| 129 | ACAAAACUGUGUCUUAUCU | AGAUAAGACACAGUUUUGU | | [1081-1099] 3'UTR |
| 130 | CGCAGAAGUACAAGCUACC | GGUAGCUUGUACUUCUGCG | | [395-413] 5'UTR |
| 131 | AGAUUUCUUGUAAGAACAC | GUGUUCUUACAAGAAAUCU | | [221-239] 5'UTR |
| 132 | UCACCCAACAAAACUGUGU | ACACAGUUUUGUUGGGUGA | | [1074-1092] 3'UTR |
| 133 | CGGCCUUCAGAAAGACAGA | UCUGUCUUUCUGAAGGCCG | | [999-1017] 3'UTR |
| 134 | GCCACUUUCUUUGACAGUC | GACUGUCAAAGAAAGUGGC | | [119-137] 5'UTR |
| 135 | AGAAUGACAACUUCCAGGA | UCCUGGAAGUUGUCAUUCU | CHL,MO | [599-617] ORF |
| 136 | AGCAGUUUCUUCCGACAGU | ACUGUCGGAAGAAACUGCU | | [462-480] 5'UTR |
| 137 | ACGCGCAGAAGUACAAGCU | AGCUUGUACUUCUGCGCGU | | [392-410] 5'UTR |
| 138 | GGAAGCCAGCCACUUUCUU | AAGAAAGUGGCUGGCUUCC | | [111-129] 5'UTR |
| 139 | GUGGCUCCCACAAGAUUUA | UAAAUCUUGUGGGAGCCAC | | [270-288] 5'UTR |
| 140 | GCUGUUAGAAUAAAAAGCC | GGCUUUUUAUUCUAACAGC | | [116-178] 3'UTR |
| 141 | ACCCAACAAAACUGUGUCU | AGACACAGUUUUGUUGGGU | | [1076-1094] 3'UTR |
| 142 | GGUGUCUGAAGUGGACUGU | ACAGUCCACUUCAGACACC | | [935-953] ORF |
| 143 | GGCUGCCUUUGUGAUCACA | UGUGAUCACAAAGGCAGCC | | [174-192] 5'UTR |
| 144 | UGGAGAAUGACAACUUCCA | UGGAAGUUGUCAUUCUCCA | CHL,MO | [596-614] ORF |
| 145 | CAGCCACUUUCUUUGACAG | CUGUCAAAGAAAGUGGCUG | | [117-135] 5'UTR |
| 146 | CCAGCCACUUUCUUUGACA | UGUCAAAGAAAGUGGCUGG | | [116-134] 5'UTR |
| 147 | UCCCACAAGAUUUACACCU | AGGUGUAAAUCUUGUGGGA | | [275-293] 5'UTR |
| 148 | GGAGUUCAGCUUUCUGCAG | CUGCAGAAAGCUGAACUCC | | [63-81] 5'UTR |
| 149 | CACCCAACAAAACUGUGUC | GACACAGUUUUGUUGGGUG | | [1075-1093] 3'TR |
| 150 | UGGUGGAGAAGAAAACUUC | GAAGUUUUCUUCUCCACCA | | [494-512] ORF |
| 151 | GCCAAUGGUGGAGAAGAAA | UUUCUUCUCCACCAUUGGC | | [489-507] 5'UTR+ORF |
| 152 | GGAACUGGGAGUUCAGCUU | AAGCUGAACUCCCAGUUCC | | [56-74] 5'UTR |
| 153 | GAAGAAAACUUCGGUUCGC | GCGAACCGAAGUUUUCUUC | | [501-519] ORF |
| 154 | GGGAGUUCAGCUUUCUGCA | UGCAGAAAGCUGAACUCCC | | [62-80] 5'UTR |
| 155 | UGCCUCAGUUUGAUGACGA | UCGUCAUCAAACUGAGGCA | | [656-674] ORF |
| 156 | GCCAGCCACUUUCUUUGAC | GUCAAAGAAAGUGGCUGGC | | [115-133] 5'UTR |
| 157 | GAGACCAGGUGUCUGAAGU | ACUUCAGACACCUGGUCUC | MO | [928-946] ORF |
| 158 | CGUGGCUCCCACAAGAUUU | AAAUCUUGUGGGAGCCACG | | [269-287] 5'UTR |
| 159 | GAGUUCAGCUUUCUGCAGA | UCUGCAGAAAGCUGAACUC | | [64-82] 5'UTR |
| 160 | GAACUGGGAGUUCAGCUUU | AAAGCUGAACUCCCAGUUC | | [57-75] 5'UTR |
| 161 | GAAGCCAGCCACUUUCUUU | AAAGAAAGUGGCUGGCUUC | | [112-130] 5'UTR |
| 162 | CCAAUGGUGGAGAAGAAAA | UUUUCUUCUCCACCAUUGG | | [490-508] 5'UTR+ORF |

**Table J: 21-mer zinc finger, HIT type 1 (ZNHIT1)**

| Number | Sense siRNA | AntiSense siRNA | Other Sp | Human-37594439 ORF:493-957 |
|---|---|---|---|---|
| 1 | CCGUGACCACAUCUUUAAAAU | AUUUUAAAGAUGUGGUCACGG | | [239-259] 5'UTR |
| 2 | CGAGGUGAUCAUUUUAAACUU | AAGUUUAAAAUGAUCACCUCG | | [706-726] ORF |
| 3 | CCAGUGCUGUUAGAAUAAAAA | UUUUUAUUCUAACAGCACUGG | | [1155-1175] 3'UTR |
| 4 | CCCAGUGCUGUUAGAAUAAAA | UUUUAUUCUAACAGCACUGGG | | [1154-1174] 3'UTR |
| 5 | CCCGAGGUGAUCAUUUUAAAC | GUUUAAAAUGAUCACCUCGGG | | [704-724] ORF |
| 6 | ACCCGAGGUGAUCAUUUUAAA | UUUAAAAUGAUCACCUCGGGU | | [703-723] ORF |
| 7 | CCUAUAAAACAUGGCGAAAAG | CUUUUCGCCAUGUUUUAUAGG | | [322-342] 5'UTR |
| 8 | CCCUAUAAAACAUGGCGAAAA | UUUUCGCCAUGUUUUAUAGGG | | [321-341] 5'UTR |
| 9 | CACCGUGACCACAUCUUUAAA | UUUAAAGAUGUGGUCACGGUG | | [237-257] 5'UTR |
| 10 | GGCCCUGGAGAAUGACAACUU | AAGUUGUCAUUCUCCAGGGCC | | [591-611] ORF |
| 11 | GGACCAAAGGAAAAUAAGAUU | AAUCUUAUUUUCCUUUGGUCC | | [205-225] 5'UTR |
| 12 | GAAUGACAACUUCCAGGAUGA | UCAUCCUGGAAGUUGUCAUUC | MO | [600-620] ORF |
| 13 | GAACACCGUGACCACAUCUUU | AAAGAUGUGGUCACGGUGUUC | | [234-254] 5'UTR |
| 14 | UCACAGCAUCUUCACAAGGAC | GUCCUUGUGAAGAUGCUGUGA | | [188-208] 5'UTR |
| 15 | GGAGGAGCAGAACUUGAGUGU | ACACUCAAGUUCUGCUCCUCC | | [756-776] ORF |
| 16 | AAACCCGAGGUGAUCAUUUUA | UAAAAUGAUCACCUCGGGUUU | | [701-721] ORF |
| 17 | ACCGUGACCACAUCUUUAAAA | UUUUAAAGAUGUGGUCACGGU | | [238-258] 5'UTR |
| 18 | ACACCGUGACCACAUCUUUAA | UUAAAGAUGUGGUCACGGUGU | | [236-256] 5'UTR |
| 19 | GAGGUGAUCAUUUUAAACUUC | GAAGUUUAAAAUGAUCACCUC | | [707-727] ORF |
| 20 | CCGAGGUGAUCAUUUUAAACU | AGUUUAAAAUGAUCACCUCGG | | [705-725] ORF |
| 21 | CGAUGCGGACACUGGAAAGAA | UUCUUUCCAGUGUCCGCAUCG | | [672-692] ORF |
| 22 | GCCCUGGAGAAUGACAACUUC | GAAGUUGUCAUUCUCCAGGGC | | [592-612] ORF |
| 23 | GCAUCUUCACAAGGACCAAAG | CUUUGGUCCUUGUGAAGAUGC | | [193-213] 5'UTR |
| 24 | CAGCAUCUUCACAAGGACCAA | UUGGUCCUUGUGAAGAUGCUG | | [191-211] 5'UTR |
| 25 | GUGAUCAUUUUAAACUUCGCU | AGCGAAGUUUAAAAUGAUCAC | | [710-730] ORF |
| 26 | UCAUUCACCCAACAAAACUGU | ACAGUUUUGUUGGGUGAAUGA | | [1070-1090] 3'UTR |
| 27 | GGUGAUCAUUUUAAACUUCGC | GCGAAGUUUAAAAUGAUCACC | | [709-729] ORF |
| 28 | GGACACUGGAAAGAAAAAGAA | UUCUUUUUCUUUCCAGUGUCC | CHL | [678-698] ORF |
| 29 | CCACUUUCUUUGACAGUCCAG | CUGGACUGUCAAAGAAAGUGG | | [120-140] 5'UTR |
| 30 | GUACAAGCUACCGGAAGUGAU | AUCACUUCCGGUAGCUUGUAC | | [402-422] 5'UTR |
| 31 | GCUCAUUCACCCAACAAAACU | AGUUUUGUUGGGUGAAUGAGC | | [1068-1088] 3'UTR |
| 32 | GGAAAGAAAAAGAAGAAAACC | GGUUUUCUUCUUUUUCUUUCC | | [685-705] ORF |
| 33 | UGCGGACACUGGAAAGAAAAA | UUUUUCUUUCCAGUGUCCGCA | CHL | [675-695] ORF |
| 34 | CUAUAAAACAUGGCGAAAAGC | GCUUUUCGCCAUGUUUUAUAG | | [323-343] 5'UTR |
| 35 | CAGUGCUGUUAGAAUAAAAAG | CUUUUUAUUCUAACAGCACUG | | [1156-1176] 3'TR |
| 36 | CUUCAGAAAGACAGAAUUUCA | UGAAAUUCUGUCUUUCUGAAG | | [1003-1023] 3'UTR |
| 37 | CACUGGAAAGAAAAAGAAGAA | UUCUUCUUUUUCUUUCCAGUG | CHL | [681-701] ORF |
| 38 | AUGCGGACACUGGAAAGAAAA | UUUUCUUUCCAGUGUCCGCAU | CHL | [674-694] ORF |
| 39 | AGAACCCGUGACCACAUCUU | AAGAUGUGGUCACGGUGUUCU | | [233-253] 5'UTR |
| 40 | CCACGUAAUGAGUCAAAGCUG | CAGCUUUGACUCAUUACGUGG | | [365-385] 5'UTR |
| 41 | GUAAGAACACCGUGACCACAU | AUGUGGUCACGGUGUUCUUAC | | [230-250] 5'UTR |
| 42 | ACAAGGACCAAAGGAAAAUAA | UUAUUUUCCUUUGGUCCUUGU | | [201-221] 5'UTR |
| 43 | GUGUCUUAUCUGCCAGGAAAG | CUUUCCUGGCAGAUAAGACAC | | [1089-1109] 3'UTR |
| 44 | CACAAGGACCAAAGGAAAAUA | UAUUUUCCUUUGGUCCUUGUG | | [200-220] 5'UTR |
| 45 | CGGACACUGGAAAGAAAAAGA | UCUUUUUCUUUCCAGUGUCCG | CHL | [677-697] ORF |
| 46 | AGGACCAAAGGAAAAUAAGAU | AUCUUAUUUUCCUUUGGUCCU | | [204-224] 5'UTR |
| 47 | CUUCACAAGGACCAAAGGAAA | UUUCCUUUGGUCCUUGUGAAG | | [197-217] 5'UTR |
| 48 | ACGUAAUGAGUCAAAGCUGUG | CACAGCUUUGACUCAUUACGU | | [367-387] 5'TR |
| 49 | CAAGGACCAAAGGAAAAUAAG | CUUAUUUUCCUUUGGUCCUUG | | [202-222] 5'UTR |
| 50 | UCACAAGGACCAAAGGAAAAU | AUUUUCCUUUGGUCCUUGUGA | | [199-219] 5'UTR |
| 51 | ACGCCACGUAAUGAGUCAAAG | CUUUGACUCAUUACGUGGCGU | | [362-382] 5'UTR |
| 52 | AGGAAACGCCACGUAAUGAGU | ACUCAUUACGUGGCGUUUCCU | | [357-377] 5'UTR |
| 53 | GAAUUUCAUCACCCAAUGCAG | CUGCAUUGGGUGAUGAAAUUC | | [1016-1036] 3'UTR |
| 54 | AGACAGAAUUUCAUCACCCAA | UUGGGUGAUGAAAUUCUGUCU | | [1011-1031] 3'UTR |
| 55 | CAGUUUGAUGACGAUGCGGAC | GUCCGCAUCGUCAUCAAACUG | | [661-681] ORF |
| 56 | CAGCAGUUUCUUCCGACAGUU | AACUGUCGGAAGAAACUGCUG | | [461-481] 5'UTR |
| 57 | GACACUGGAAAGAAAAAGAAG | CUUCUUUUUCUUUCCAGUGUC | CHL | [679-699] ORF |
| 58 | CCGAAAAAACUUUCAGGCCCU | AGGGCCUGAAAGUUUUUUCGG | | [732-752] ORF |
| 59 | GCGGACACUGGAAAGAAAAAG | CUUUUUCUUUCCAGUGUCCGC | CHL | [676-696] ORF |
| 60 | CCACAAGAUUUACACCUCCAC | GUGGAGGUGUAAAUCUUGUGG | | [277-297] 5'UTR |
| 61 | CGAAAAAACUUUCAGGCCCUG | CAGGGCCUGAAAGUUUUUUCG | | [733-753] ORF |
| 62 | GCAGUUUCUUCCGACAGUUGU | ACAACUGUCGGAAGAAACUGC | | [463-483] 5'UTR |
| 63 | UGACCACAUCUUUAAAAUGAC | GUCAUUUUAAAGAUGUGGUCA | | [242-262] 5'UTR |
| 64 | CCGCUCAUUCACCCAACAAAA | UUUUGUUGGGUGAAUGAGCGG | | [1066-1086] 3'UTR |
| 65 | ACAGAAUUUCAUCACCCAAUG | CAUUGGGUGAUGAAAUUCUGU | | [1013-1033] 3'UTR |
| 66 | GACAGAAUUUCAUCACCCAAU | AUUGGGUGAUGAAAUUCUGUC | | [1012-1032] 3'UTR |
| 67 | AGAAAGACAGAAUUUCAUCAC | GUGAUGAAAUUCUGUCUUUCU | | [1007-1027] 3'UTR |
| 68 | UAAACUUCGCUUCCGAAAAAA | UUUUUUCGGAAGCGAAGUUUA | GP | [720-740] ORF |
| 69 | AGCAGUUUCUUCCGACAGUUG | CAACUGUCGGAAGAAACUGCU | | [462-482] 5'UTR |
| 70 | CCCACAAGAUUUACACCUCCA | UGGAGGUGUAAAUCUUGUGGG | | [276-296] 5'UTR |
| 71 | CGGCCUUCAGAAAGACAGAAU | AUUCUGUCUUUCUGAAGGCCG | | [999-1019] 3'UTR |
| 72 | GGAGAAUGACAACUUCCAGGA | UCCUGGAAGUUGUCAUUCUCC | CHL,MO | [597-617] ORF |
| 73 | ACCCAACAAAACUGUGUCUUA | UAAGACACAGUUUUGUUGGGU | | [1076-1096] 3'UTR |
| 74 | GGCAAGAGACUGCCUCAGUUU | AAACUGAGGCAGUCUCUUGCC | | [646-666] ORF |
| 75 | CCACAUCUUUAAAAUGACCCA | UGGGUCAUUUUAAAGAUGUGG | | [245-265] 5'UTR |
| 76 | GCCUUCAGAAAGACAGAAUUU | AAAUUCUGUCUUUCUGAAGGC | | [1001-1021] 3'UTR |
| 77 | UCGCUUCCGAAAAAACUUUCA | UGAAAGUUUUUUCGGAAGCGA | | [726-746] ORF |
| 78 | UUAAACUUCGCUUCCGAAAAA | UUUUUCGGAAGCGAAGUUUAA | GP | [719-739] ORF |
| 79 | CAAUGGUGGAGAAGAAAACUU | AAGUUUUCUUCUCCACCAUUG | | [491-511] 5'UTR+ORF |
| 80 | GAAGUACAAGCUACCGGAAGU | ACUUCCGGUAGCUUGUACUUC | | [399-419] 5'UTR |
| 81 | GCAGAAGUACAAGCUACCGGA | UCCGGUAGCUUGUACUUCUGC | | [396-416] 5'UTR |
| 82 | AGCUUUCUUGUCUCCAAGGAA | UUCCUUGGAGACAAGAAAGCU | | [341-361] 5'UTR |
| 83 | CACAAGAUUUACACCUCCACA | UGUGGAGGUGUAAAUCUUGUG | | [278-298] 5'UTR |
| 84 | CAGAAUUUCAUCACCCAAUGC | GCAUUGGGUGAUGAAAUUCUG | | [1014-1034] 3'UTR |
| 85 | CCUUUGUGAUCACAGCAUCUU | AAGAUGCUGUGAUCACAAAGG | | [179-199] 5'UTR |
| 86 | GCCUUUGUGAUCACAGCAUCU | AGAUGCUGUGAUCACAAAGGC | | [178-198] 5'UTR |
| 87 | GAAAAAACUUUCAGGCCCUGU | ACAGGGCCUGAAAGUUUUUUC | | [734-754] ORF |
| 88 | AGAAAACCCGAGGUGAUCAUU | AAUGAUCACCUCGGGUUUUCU | | [698-718] ORF |
| 89 | GGAGAAGAAAACUUCGGUUCG | CGAACCGAAGUUUUCUUCUCC | | [498-518] ORF |
| 90 | CAGAAGUACAAGCUACCGGAA | UUCCGGUAGCUUGUACUUCUG | | [397-417] 5'UTR |
| 91 | CGUGACCACAUCUUUAAAAUG | CAUUUUAAAGAUGUGGUCACG | | [240-260] 5'UTR |
| 92 | CCAACAAAACUGUGUCUUAUC | GAUAAGACACAGUUUUGUUGG | | [1078-1098] 3'UTR |
| 93 | CACCCAACAAAACUGUGUCUU | AAGACACAGUUUUGUUGGGUG | | [1075-1095] 3'UTR |
| 94 | GGCCUUCAGAAAGACAGAAUU | AAUUCUGUCUUUCUGAAGGCC | | [1000-1020] 3'UTR |
| 95 | CGCUUCCGAAAAAACUUUCAG | CUGAAAGUUUUUUCGGAAGCG | | [727-747] ORF |
| 96 | UGACGAUGCGGACACUGGAAA | UUUCCAGUGUCCGCAUCGUCA | | [669-689] ORF |
| 97 | GUGCCAAUGGUGGAGAAA | UUUCUUCUCCACCAUUGGCAC | | [487-507] 5'UTR+ORF |
| 98 | CUCCAAGGAAACGCCACGUAA | UUACGUGGCGUUUCCUUGGAG | | [352-372] 5'UTR |
| 99 | CAAGAUUUACACCUCCACACU | AGUGUGGAGGUGUAAAUCUUG | | [280-300] 5'UTR |
| 100 | CACAUCUUUAAAAUGACCCAU | AUGGGUCAUUUUAAAGAUGUG | | [246266] 5'UTR |
| 101 | GUGAUCACAGCAUCUUCACAA | UUGUGAAGAUGCUGUGAUCAC | | [184-204] 5'UTR |
| 102 | GCCGCUCAUUCACCCAACAAA | UUUGUUGGGUGAAUGAGCGGC | | [1065-1085] 3'UTR |
| 103 | UCAGAAAGACAGAAUUUCAUC | GAUGAAAUUCUGUCUUUCUGA | | [1005-1025] 3'UTR |
| 104 | CUGCCUCAGUUUGAUGACGAU | AUCGUCAUCAAACUGAGGCAG | | [655-675] ORF |
| 105 | GCAAGAGACUGCCUCAGUUUG | CAAACUGAGGCAGUCUCUUGC | | [647-667] ORF |
| 106 | GUGGAGAAGAAAACUUCGGUU | AACCGAAGUUUUCUUCUCCAC | | [496-516] ORF |
| 107 | GACAGUUGUGUUGUGCCAAUG | CAUUGGCACAACACAACUGUC | | [475-495] 5'UTR+ORF |
| 108 | CUUUAAAAUGACCCAUUUCGU | ACGAAAUGGGUCAUUUUAAAG | | [251-271] 5'UTR |
| 109 | AGCCGCUCAUUCACCCAACAA | UUGUUGGGUGAAUGAGCGGCU | | [1064-1084] 3'UTR |
| 110 | CAUUUUAAACUUCGCUUCCGA | UCGGAAGCGAAGUUUAAAAUG | | [715-735] ORF |
| 111 | GAGAAUGACAACUUCCAGGAU | AUCCUGGAAGUUGUCAUUCUC | MO | [598-618] ORF |
| 112 | GGUGGAGAAGAAAACUUCGGU | ACCGAAGUUUUCUUCUCCACC | | [495-515] ORF |
| 113 | CCGACAGUUGUGUUGUGCCAA | UUGGCACAACACAACUGUCGG | | [473-493] 5'UTR |
| 114 | GCUUUCUUGUCUCCAAGGAAA | UUUCCUUGGAGACAAGAAAGC | | [342-362] 5'UTR |
| 115 | GGGAACUGGGAGUUCAGCUUU | AAAGCUGAACUCCCAGUUCCC | | [55-75] 5'UTR |
| 116 | CCCAACAAAACUGUGUCUUAU | AUAAGACACAGUUUUGUUGGG | | [1077-1097] 3'UTR |
| 117 | CGCUCAUUCACCCAACAAAAC | GUUUUGUUGGGUGAAUGAGCG | | [1067-1087] 3'UTR |
| 118 | GAAAACCCGAGGUGAUCAUUU | AAAUGAUCACCUCGGGUUUUC | | [699-719] ORF |
| 119 | UGGAGAAGAAAACUUCGGUUC | GAACCGAAGUUUUCUUCUCCA | | [497-517] ORF |
| 120 | UGUUGUGCCAAUGGUGGAGAA | UUCUCCACCAUUGGCACAACA | | [483-503] 5'UTR+ORF |
| 121 | CGACAGUUGUGUUGUGCCAAU | AUUGGCACAACACAACUGUCG | | [474-494] 5'UTR+ORF |
| 122 | UCCAAGGAAACGCCACGUAAU | AUUACGUGGCGUUUCCUUGGA | | [353-373] 5'UTR |
| 123 | UGGCUCCCACAAGAUUUACAC | GUGUAAAUCUUGUGGGAGCCA | | [271-291] 5'UTR |
| 124 | UGAUCACAGCAUCUUCACAAG | CUUGUGAAGAUGCUGUGAUCA | | [185-205] 5'UTR |
| 125 | UGCCUUUGUGAUCACAGCAUC | GAUGCUGUGAUCACAAAGGCA | | [177-197] 5'UTR |
| 126 | ACAGUUGUGUUGUGCCAAUGG | CCAUUGGCACAACACAACUGU | | [476-496] 5'UTR+ORF |
| 127 | GCCAGCCACUUUCUUUGACAG | CUGUCAAAGAAAGUGGCUGGC | | [115-135] 5'UTR |
| 128 | CACGCGCAGAAGUACAAGCUA | UAGCUUGUACUUCUGCGCGUG | | [391-411] 5'UTR |
| 129 | GCUGUUAGAAUAAAAAGCCUC | GAGGCUUUUUAUUCUAACAGC | | [1160-1180] 3'UTR |
| 130 | AGAGACUGCCUCAGUUUGAUG | CAUCAAACUGAGGCAGUCUCU | | [650-670] ORF |
| 131 | GCCACUUUCUUUGACAGUCCA | UGGACUGUCAAAGAAAGUGGC | | [119-139] 5'UTR |
| 132 | AGAAGAAAACUUCGGUUCGCU | AGCGAACCGAAGUUUUCUUCU | | [500-520] ORF |
| 133 | UGCUGUUAGAAUAAAAAGCCU | AGGCUUUUUAUUCUAACAGCA | | [1159-1179] 3'UTR |
| 134 | CUGUGUCUUAUCUGCCAGGAA | UUCCUGGCAGAUAAGACACAG | | [1087-1107] 3'UTR |
| 135 | UCACCCAACAAAACUGUGUCU | AGACACAGUUUUGUUGGGUGA | | [1074-1094] 3'UTR |
| 136 | GUGUCUGAAGUGGACUGUGUG | CACACAGUCCACUUCAGACAC | | [936-956] ORF |
| 137 | CAGGUGUCUGAAGUGGACUGU | ACAGUCCACUUCAGACACCUG | | [933-953] ORF |
| 138 | CCUGUUGGAGGAGCAGAACUU | AAGUUCUGCUCCUCCAACAGG | | [750-770] ORF |
| 139 | GCUGCCUUUGUGAUCACAGCA | UGCUGUGAUCACAAAGGCAGC | | [175-195] 5'UTR |
| 140 | CCCUGGAGAAUGACAACUUCC | GGAAGUUGUCAUUCUCCAGGG | | [593-613] ORF |
| 141 | CCAGCCACUUUCUUUGACAGU | ACUGUCAAAGAAAGUGGCUGG | | [116-136] 5'UTR |
| 142 | GCAGCAGUUUCUUCCGACAGU | ACUGUCGGAAGAAACUGCUGC | | [460-480] 5'UTR |
| 143 | CCAAGGAAACGCCACGUAAUG | CAUUACGUGGCGUUUCCUUGG | | [354-374] 5'UTR |
| 144 | AGAUUUACACCUCCACACUGA | UCAGUGUGGAGGUGUAAAUCU | | [282-302] 5'UTR |
| 145 | CUGCCUUUGUGAUCACAGCAU | AUGCUGUGAUCACAAAGGCAG | | [176-196] 5'UTR |
| 146 | AGACUGCCUCAGUUUGAUGAC | GUCAUCAAACUGAGGCAGUCU | | [652-672] ORF |
| 147 | CUGGAGAAUGACAACUUCCAG | CUGGAAGUUGUCAUUCUCCAG | | [595-615] ORF |
| 148 | GAAGAAAACUUCGGUUCGCUC | GAGCGAACCGAAGUUUUCUUC | | [501-521] ORF |
| 149 | GUUUCUUCCGACAGUUGUGUU | AACACAACUGUCGGAAGAAAC | | [466-486] 5'UTR |
| 150 | CUCCCACAAGAUUUACACCUC | GAGGUGUAAAUCUUGUGGGAG | | [274-294] 5'UTR |
| 151 | GGAGUUCAGCUUUCUGCAGAG | CUCUGCAGAAAGCUGAACUCC | | [63-83] 5'UTR |
| 152 | CAACAAAACUGUGUCUUAUCU | AGAUAAGACACAGUUUUGUUG | | [1079-1099] 3'UTR |
| 153 | CGUGGCUCCCACAAGAUUUAC | GUAAAUCUUGUGGGAGCCACG | | [269-289] 5'UTR |
| 154 | UCGUGGCUCCCACAAGAUUUA | UAAAUCUUGUGGGAGCCACGA | | [268-288] 5'UTR |
| 155 | CCGGCCUUCAGAAAGACAGAA | UUCUGUCUUUCUGAAGGCCGG | | [998-1018] 3'UTR |
| 156 | UGAAGUGGACUGUGUGAGCCU | AGGCUCACACAGUCCACUUCA | | [941-961] ORF+3'UTR |
| 157 | CGCCACGUAAUGAGUCAAAGC | GCUUUGACUCAUUACGUGGCG | | [363-383] 5'UTR |
| 158 | UCUCCAAGGAAACGCCACGUA | UACGUGGCGUUUCCUUGGAGA | | [351-371] 5'UTR |
| 159 | ACGGAAGCCAGCCACUUUCUU | AAGAAAGUGGCUGGCUUCCGU | | [109-129] 5'UTR |
| 160 | GGAACUGGGAGUUCAGCUUUC | GAAAGCUGAACUCCCAGUUCC | | [56-76] 5'UTR |
| 161 | CUGGGAGUUCAGCUUUCUGCA | UGCAGAAAGCUGAACUCCCAG | | [60-80] 5'UTR |
| 162 | GGAAGCCAGCCACUUUCUUUG | CAAAGAAAGUGGCUGGCUUCC | | [111-131] 5'UTR |
| 163 | CGGAAGCCAGCCACUUUCUUU | AAAGAAAGUGGCUGGCUUCCG | | [110-130] 5'UTR |
| 164 | CCUCAGUUUGAUGACGAUGCG | CGCAUCGUCAUCAAACUGAGG | | [658-678] ORF |
| 165 | AGCCACUUUCUUUGACAGUCC | GGACUGUCAAAGAAAGUGGCU | | [118-138] 5'UTR |
| 166 | AGCCAGCCACUUUCUUUGACA | UGUCAAAGAAAGUGGCUGGCU | | [114-134] 5'UTR |
| 167 | UGCCUCAGUUUGAUGACGAUG | CAUCGUCAUCAAACUGAGGCA | | [656-676] ORF |
| 168 | GAACUGGGAGUUCAGCUUUCU | AGAAAGCUGAACUCCCAGUUC | | [57-77] 5'UTR |
| 169 | GCCACGUAAUGAGUCAAAGCU | AGCUUUGACUCAUUACGUGGC | | [364-384] 5'UTR |
| 170 | GAAGCCAGCCACUUUCUUUGA | UCAAAGAAAGUGGCUGGCUUC | | [112-132] 5'UTR |
| 171 | GGGAGUUCAGCUUUCUGCAGA | UCUGCAGAAAGCUGAACUCCC | | [62-82] 5'UTR |
| 172 | UGCCAAUGGUGGAGAAGAAAA | UUUUCUUCUCCACCAUUGGCA | | [488-508] 5'UTR+ORF |
| 173 | CCAAUGGUGGAGAAGAAAACU | AGUUUUCUUCUCCACCAUUGG | | [490-510] 5'UTR+ORF |
| 174 | GCCAAUGGUGGAGAAGAAAAC | GUUUUCUUCUCCACCAUUGGC | | [489-509] 5'UTR+ORF |
| 175 | AGACCAGGUGUCUGAAGUGGA | UCCACUUCAGACACCUGGUCU | MO | [929-949] ORF |

**Table K: 19-mer lectin, galactoside-binding, soluble, 3 (LGALS3)**

| Number | Sense siRNA | AntiSense siRNA | Other Sp | Human-115430222 ORF:152-904 | Human-115430224 |
|---|---|---|---|---|---|
| 1 | UGCCUUAUAACCUGCCUUU | AAAGGCAGGUUAUAAGGCA | | [498-516] ORF | [1186-1204] 3'UTR |
| 2 | CAAUACAAAGCUGGAUAAU | AUUAUCCAGCUUUGUAUUG | | [670-688] ORF | [1358-1376] 3'UTR |
| 3 | GUGCCUUAUAACCUGCCUU | AAGGCAGGUUAUAAGGCAC | | [497-515] ORF | [1185-1203] 3'UTR |
| 4 | GGGAAUUUCUGGUGACAUA | UAUGUCACCAGAAAUUCCC | | [853-871] ORF | [1541-1559] 3'UTR |
| 5 | ACAUUCAUCAAUAUCCCUC | GAGGGAUAUUGAUGAAUGU | | [990-1008] 3'UTR | [1678-1696] 3'UTR |
| 6 | CAAAAUACAAGUACUGGUU | AACCAGUACUUGUAUUUUG | | [745-763] ORF | [1433-1451] 3'UTR |
| 7 | CCAUUCAAAAUACAAGUAC | GUACUUGUAUUUUGAAUGG | | [740-758] ORF | [1428-1446] 3'UTR |
| 8 | GGAAGAAAGACAGUCGGUU | AACCGACUGUCUUUCUUCC | | [700-718] ORF | [1388-1406] 3'UTR |
| 9 | GGAAAAUGGCAGACAAUUU | AAAUUGUCUGCCAUUUUCC | | [147-165] 5'UTR+ORF | [182-200] 3'UTR |
| 10 | CCAUGAUAUAAUCUGAAAG | CUUUCAGAUUAUAUCAUGG | | [894-912] ORF+3'UTR | [1582-1600] 3'UTR |
| 11 | GCAGUACAAUCAUCGGGUU | AACCCGAUGAUUGUACUGC | | [808-826] ORF | [1496-1514] 3'UTR |
| 12 | GAGAGUCAUUGUUUGCAAU | AUUGCAAACAAUGACUCUC | | [655-673] ORF | [1343-1361] 3'UTR |
| 13 | GAAAAUGGCAGACAAUUUU | AAAAUUGUCUGCCAUUUUC | | [148-166] 5'UTR+ORF | [183-201] 3'UTR |
| 14 | GCGUUAUCUGGGUCUGGAA | UUCCAGACCCAGAUAACGC | | [179-197] ORF | [867-885] 3'UTR |
| 15 | GGGUUAAAAAACUCAAUGA | UCAUUGAGUUUUUUAACCC | | [822-840] ORF | [1510-1528] 3'UTR |
| 16 | CGGGUUAAAAAACUCAAUG | CAUUGAGUUUUUUAACCCG | | [821-839] ORF | [1509-1527] 3'UTR |
| 17 | UCAAAAUACAAGUACUGGU | ACCAGUACUUGUAUUUUGA | | [744-762] ORF | [1432-1450] 3'UTR |
| 18 | CAAGGUUGCAGUGAAUGAU | AUCAUUCACUGCAACCUUG | | [778-796] ORF | [1466-1484] 3'UTR |
| 19 | UCUAAACCUUACAUGUGUA | UACACAUGUAAGGUUUAGA | | [938-956] 3'UTR | [1626-1644] 3'UTR |
| 20 | CCAGUGCUUCAUAUACCAU | AUGGUAUAUGAAGCACUGG | | [879-897] ORF | [1567-1585] 3'UTR |
| 21 | GACCUCACCAGUGCUUCAU | AUGAAGCACUGGUGAGGUC | | [872-890] ORF | [1560-1578] 3'UTR |
| 22 | CUUUAACCCACGCUUCAAU | AUUGAAGCGUGGGUUAAAG | | [625-643] ORF | [1313-1331] 3'UTR |
| 23 | UCCACUUUAACCCACGCUU | AAGCGUGGGUUAAAGUGGA | | [621-639] ORF | [1309-1327] 3'UTR |
| 24 | GCCUUCCACUUUAACCCAC | GUGGGUUAAAGUGGAAGGC | | [617-635] ORF | [1305-1323] 3'UTR |
| 25 | CGUUAUCUGGGUCUGGAAA | UUUCCAGACCCAGAUAACG | | [180-198] ORF | [868-886] 3'UTR |
| 26 | ACCUCACCAGUGCUUCAUA | UAUGAAGCACUGGUGAGGU | | [873-891] ORF | [1561-1579] 3'UTR |
| 27 | AAGAAAGACAGUCGGUUUU | AAAACCGACUGUCUUUCUU | | [702-720] ORF | [1390-1408] 3'UTR |
| 28 | GGGAAGAAAGACAGUCGGU | ACCGACUGUCUUUCUUCCC | | [699-717] ORF | [1387-1405] 3'UTR |
| 29 | CCACUUUAACCCACGCUUC | GAAGCGUGGGUUAAAGUGG | | [622-640] ORF | [1310-1328] 3'UTR |
| 30 | CCUCGCAUGCUGAUAACAA | UUGUUAUCAGCAUGCGAGG | | [533-551] ORF | [1221-1239] 3'UTR |
| 31 | ACAUGUGUAAAGGUUUCAU | AUGAAACCUUUACACAUGU | | [948-966] 3'UTR | [1636-1654] 3'UTR |
| 32 | CUCACCAGUGCUUCAUAUA | UAUAUGAAGCACUGGUGAG | | [875-893] ORF | [1563-1581] 3'UTR |
| 33 | GGAAUUUCUGGUGACAUAG | CUAUGUCACCAGAAAUUCC | | [854-872] ORF | [1542-1560] 3'UTR |
| 34 | UGGGAAUUUCUGGUGACAU | AUGUCACCAGAAAUUCCCA | | [852-870] ORF | [1540-1558] 3'UTR |
| 35 | ACAAUCAUCGGGUUAAAAA | UUUUUAACCCGAUGAUUGU | | [813-831] ORF | [1501-1519] 3'UTR |
| 36 | GCCUUAUAACCUGCCUUUG | CAAAGGCAGGUUAUAAGGC | | [499-517] ORF | [1187-1205] 3'UTR |
| 37 | UGCGUUAUCUGGGUCUGGA | UCCAGACCCAGAUAACGCA | | [178-196] ORF | [866-884] 3'UTR |
| 38 | CCCUCUUGUAAGUCAUCUA | UAGAUGACUUACAAGAGGG | | [1004-1022] 3'UTR | [1692-1710] 3'UTR |
| 39 | ACCAGUGCUUCAUAUACCA | UGGUAUAUGAAGCACUGGU | | [878-896] ORF | [1566-1584] 3'UTR |
| 40 | CUGGGAAUUUCUGGUGACA | UGUCACCAGAAAUUCCCAG | | [851-869] ORF | [1539-1557] 3'UTR |
| 41 | CAAUCAUCGGGUUAAAAAA | UUUUUUAACCCGAUGAUUG | | [814-832] ORF | [1502-1520] 3'UTR |
| 42 | UGAUAACAAUUCUGGGCAC | GUGCCCAGAAUUGUUAUCA | | [543-561] ORF | [1231-1249] 3'UTR |
| 43 | UCGCAUGCUGAUAACAAUU | AAUUGUUAUCAGCAUGCGA | | [535-553] ORF | [1223-1241] 3'UTR |
| 44 | GCCUCGCAUGCUGAUAACA | UGUUAUCAGCAUGCGAGGC | | [532-550] ORF | [1220-1238] 3'UTR |
| 45 | GAAUUUCUGGUGACAUAGA | UCUAUGUCACCAGAAAUUC | | [855-873] ORF | [1543-1561] 3'UTR |
| 46 | UCAUCGGGUUAAAAAACUC | GAGUUUUUUAACCCGAUGA | | [817-835] ORF | [1505-1523] 3'UTR |
| 47 | AGUACAAUCAUCGGGUUAA | UUAACCCGAUGAUUGUACU | | [810-828] ORF | [1498-1516] 3'UTR |
| 48 | AGGAGAGUCAUUGUUUGCA | UGCAAACAAUGACUCUCCU | | [653-671] ORF | [1341-1359] 3'UTR |
| 49 | CAGUGCUUCAUAUACCAUG | CAUGGUAUAUGAAGCACUG | | [880-898] ORF | [1568-1586] 3'UTR |
| 50 | CAGUACAAUCAUCGGGUUA | UAACCCGAUGAUUGUACUG | | [809-827] ORF | [1497-1515] 3'UTR |
| 51 | CAAUUCUGGGCACGGUGAA | UUCACCGUGCCCAGAAUUG | | [549-567] ORF | [1237-1255] 3'UTR |
| 52 | GUAAAGGUUUCAUGUUCAC | GUGAACAUGAAACCUUUAC | | [954-972] 3'UTR | [1642-1660] 3'UTR |
| 53 | GUCAUUGUUUGCAAUACAA | UUGUAUUGCAAACAAUGAC | | [659-677] ORF | [1347-1365] 3'UTR |
| 54 | CUGAUAACAAUUCUGGGCA | UGCCCAGAAUUGUUAUCAG | | [542-560] ORF | [1230-1248] 3'UTR |
| 55 | GGUUUCAUGUUCACUGUGA | UCACAGUGAACAUGAAACC | | [959-977] 3'UTR | [1647-1665] 3'UTR |
| 56 | UGCAGUACAAUCAUCGGGU | ACCCGAUGAUUGUACUGCA | | [807-825] ORF | [1495-1513] 3'UTR |
| 57 | UGCAAUACAAAGCUGGAUA | UAUCCAGCUUUGUAUUGCA | | [668-686] ORF | [1356-1374] 3'UTR |
| 58 | CACGCUUCAAUGAGAACAA | UUGUUCUCAUUGAAGCGUG | | [633-651] ORF | [1321-1339] 3'UTR |
| 59 | UGUAAAGGUUUCAUGUUCA | UGAACAUGAAACCUUUACA | | [953-971] 3'UTR | [1641-1659] 3'UTR |
| 60 | UGUGUAAAGGUUUCAUGUU | AACAUGAAACCUUUACACA | | [951-969] 3'UTR | [1639-1657] 3'UTR |
| 61 | GGGAAGGGAAGAAAGACAG | CUGUCUUUCUUCCCUUCCC | Rat | [694-712] ORF | [1382-1400] 3'UTR |
| 62 | UUGCAAUACAAAGCUGGAU | AUCCAGCUUUGUAUUGCAA | | [667-685] ORF | [1355-1373] 3'UTR |
| 63 | AGAAUUGCUUUAGAUUUCC | GGAAAUCUAAAGCAAUUCU | | [581-599] ORF | [1269-1287] 3'UTR |
| 64 | GCUUCAUAUACCAUGAUAU | AUAUCAUGGUAUAUGAAGC | | [884-902] ORF | [1572-1590] 3'UTR |
| 65 | UGCUUCAUAUACCAUGAUA | UAUCAUGGUAUAUGAAGCA | | [883-901] ORF | [1571-1589] 3'UTR |
| 66 | CUUCAAGGUUGCAGUGAAU | AUUCACUGCAACCUUGAAG | | [775-793] ORF | [1463-1481] 3'UTR |
| 67 | UCAAUGAGAACAACAGGAG | CUCCUGUUGUUCUCAUUGA | MO | [639-657] ORF | [1327-1345] 3'UTR |
| 68 | CCACGCUUCAAUGAGAACA | UGUUCUCAUUGAAGCGUGG | | [632-650] ORF | [1320-1338] 3'UTR |
| 69 | UCCCUCUUGUAAGUCAUCU | AGAUGACUUACAAGAGGGA | | [1003-1021] 3'UTR | [1691-1709] 3'UTR |
| 70 | ACUUCAAGGUUGCAGUGAA | UUCACUGCAACCUUGAAGU | | [774-792] ORF | [1462-1480] 3'UTR |
| 71 | ACAGUCGGUUUUCCCAUUU | AAAUGGGAAAACCGACUGU | | [709-727] ORF | [1397-1415] 3'UTR |
| 72 | CGCUUCAAUGAGAACAACA | UGUUGUUCUCAUUGAAGCG | Rat,MO | [635-653] ORF | [1323-1341] 3'UTR |
| 73 | ACGCUUCAAUGAGAACAAC | GUUGUUCUCAUUGAAGCGU | | [634-652] ORF | [1322-1340] 3'UTR |
| 74 | GGAAUGAUGUUGCCUUCCA | UGGAAGGCAACAUCAUUCC | MO | [606-624] ORF | [1294-1312] 3'UTR |
| 75 | UCUUGUAAGUCAUCUACUU | AAGUAGAUGACUUACAAGA | | [1007-1025] 3'UTR | [1695-1713] 3'UTR |
| 76 | AGUCAUUGUUUGCAAUACA | UGUAUUGCAAACAAUGACU | | [658-676] ORF | [1346-1364] 3'UTR |
| 77 | GCUUCAAUGAGAACAACAG | CUGUUGUUCUCAUUGAAGC | Rat,MO | [636-654] ORF | [1324-1342] 3'UTR |
| 78 | ACCCACGCUUCAAUGAGAA | UUCUCAUUGAAGCGUGGGU | | [630-648] ORF | [1318-1336] 3'UTR |
| 79 | GAGGGAAUGAUGUUGCCUU | AAGGCAACAUCAUUCCCUC | MO | [603-621] ORF | [1291-1309] 3'UTR |
| 80 | UGAAGCCCAAUGCAAACAG | CUGUUUGCAUUGGGCUUCA | | [564-582] ORF | [1252-1270] 3'UTR |
| 81 | CUCAAUGAAAUCAGCAAAC | GUUUGCUGAUUUCAUUGAG | | [833-851] ORF | [1521-1539] 3'UTR |
| 82 | CCCAUUUGAAAGUGGGAAA | UUUCCCACUUUCAAAUGGG | | [721-739] ORF | [1409-1427] 3'UTR |
| 83 | ACGGUGAAGCCCAAUGCAA | UUGCAUUGGGCUUCACCGU | | [560-578] ORF | [1248-1266] 3'UTR |
| 84 | GCCACUGAUUGUGCCUUAU | AUAAGGCACAAUCAGUGGC | | [487-505] ORF | [1175-1193] 3'UTR |
| 85 | CCUCUUGUAAGUCAUCUAC | GUAGAUGACUUACAAGAGG | | [1005-1023] 3'UTR | [1693-1711] 3'UTR |
| 86 | CUAAACCUUACAUGUGUAA | UUACACAUGUAAGGUUUAG | | [939-957] 3'UTR | [1627-1645] 3'UTR |
| 87 | GUGCUUCAUAUACCAUGAU | AUCAUGGUAUAUGAAGCAC | | [882-900] ORF | [1570-1588] 3'UTR |
| 88 | CAGUCGGUUUUCCCAUUUG | CAAAUGGGAAAACCGACUG | | [710-728] ORF | [1398-1416] 3'UTR |
| 89 | GGAAACCCAAACCCUCAAG | CUUGAGGGUUUGGGUUUCC | MO | [194-212] ORF | [882-900] 3'UTR |
| 90 | CUCACUUGUUGCAGUACAA | UUGUACUGCAACAAGUGAG | | [798-816] ORF | [1486-1504] 3'UTR |
| 91 | AGAGUCAUUGUUUGCAAUA | UAUUGCAAACAAUGACUCU | | [656-674] ORF | [1344-1362] 3'UTR |
| 92 | AUGUUGCCUUCCACUUUAA | UUAAAGUGGAAGGCAACAU | MO | [612-630] ORF | [1300-1318] 3'UTR |
| 93 | UCGGGUUAAAAAACUCAAU | AUUGAGUUUUUUAACCCGA | | [820-838] ORF | [1508-1526] 3'UTR |
| 94 | CCACUUCAAGGUUGCAGUG | CACUGCAACCUUGAAGUGG | | [772-790] ORF | [1460-1478] 3'UTR |
| 95 | CCAUUUGAAAGUGGGAAAC | GUUUCCCACUUUCAAAUGG | | [722-740] ORF | [1410-1428] 3'UTR |
| 96 | GGUUUUCCCAUUUGAAAGU | ACUUUCAAAUGGGAAAACC | | [715-733] ORF | [1403-1421] 3'UTR |
| 97 | GUCGGUUUUCCCAUUUGAA | UUCAAAUGGGAAAACCGAC | | [712-730] ORF | [1400-1418] 3'UTR |
| 98 | GAGUCAUUGUUUGCAAUAC | GUAUUGCAAACAAUGACUC | | [657-675] ORF | [1345-1363] 3'UTR |
| 99 | GAAUGAUGUUGCCUUCCAC | GUGGAAGGCAACAUCAUUC | MO | [607-625] ORF | [1295-1313] 3'UTR |
| 100 | GCUUUAGAUUUCCAAAGAG | CUCUUUGGAAAUCUAAAGC | | [587-605] ORF | [1275-1293] 3'UTR |
| 101 | UGCUUUAGAUUUCCAAAGA | UCUUUGGAAAUCUAAAGCA | | [586-604] ORF | [1274-1292] 3'UTR |
| 102 | CUCGCAUGCUGAUAACAAU | AUUGUUAUCAGCAUGCGAG | | [534-552] ORF | [1222-1240] 3'UTR |
| 103 | CCAUCUUCUGGACAGCCAA | UUGGCUGUCCAGAAGAUGG | | [419-437] ORF | [1107-1125] 3'UTR |
| 104 | UGGAAACCCAAACCCUCAA | UUGAGGGUUUGGGUUUCCA | MO | [193-211] ORF | [881-899] 3'UTR |
| 105 | GAAAUCAGCAAACUGGGAA | UUCCCAGUUUGCUGAUUUC | | [839-857] ORF | [1527-1545] 3'UTR |
| 106 | GCUCACUUGUUGCAGUACA | UGUACUGCAACAAGUGAGC | | [797-815] ORF | [1485-1503] 3'UTR |
| 107 | CAGUGAAUGAUGCUCACUU | AAGUGAGCAUCAUUCACUG | | [786-804] ORF | [1474-1492] 3'UTR |
| 108 | CCCACGCUUCAAUGAGAAC | GUUCUCAUUGAAGCGUGGG | | [631-649] ORF | [1319-1337] 3'UTR |
| 109 | AGAUUUCCAAAGAGGGAAU | AUUCCCUCUUUGGAAAUCU | | [592-610] ORF | [1280-1298] 3'UTR |
| 110 | GCAAACAGAAUUGCUUUAG | CUAAAGCAAUUCUGUUUGC | | [575-593] ORF | [1263-1281] 3'UTR |
| 111 | CCCAAUGCAAACAGAAUUG | CAAUUCUGUUUGCAUUGGG | | [569-587] ORF | [1257-1275] 3'UTR |
| 112 | CCUUACAUGUGUAAAGGUU | AACCUUUACACAUGUAAGG | | [944-962] 3'UTR | [1632-1650] 3'UTR |
| 113 | AGUCGGUUUUCCCAUUUGA | UCAAAUGGGAAAACCGACU | | [711-729] ORF | [1399-1417] 3'UTR |
| 114 | CGCAUGCUGAUAACAAUUC | GAAUUGUUAUCAGCAUGCG | | [536-554] ORF | [1224-1242] 3'UTR |
| 115 | UGCCUCGCAUGCUGAUAAC | GUUAUCAGCAUGCGAGGCA | | [531-549] ORF | [1219-1237] 3'UTR |
| 116 | CUACCCAUCUUCUGGACAG | CUGUCCAGAAGAUGGGUAG | | [415-433] ORF | [1103-1121] 3'UTR |
| 117 | CUGGUGACAUAGACCUCAC | GUGAGGUCUAUGUCACCAG | | [861-879] ORF | [1549-1567] 3'UTR |
| 118 | GCAGUGAAUGAUGCUCACU | AGUGAGCAUCAUUCACUGC | | [785-803] ORF | [1473-1491] 3'UTR |
| 119 | ACCACUUCAAGGUUGCAGU | ACUGCAACCUUGAAGUGGU | | [771-789] ORF | [1459-1477] 3'UTR |
| 120 | UGCAAACAGAAUUGCUUUA | UAAAGCAAUUCUGUUUGCA | | [574-592] ORF | [1262-1280] 3'UTR |
| 121 | AAAUCAGCAAACUGGGAAU | AUUCCCAGUUUGCUGAUUU | | [840-858] ORF | [1528-1546] 3'UTR |
| 122 | UGCAGUGAAUGAUGCUCAC | GUGAGCAUCAUUCACUGCA | | [784-802] ORF | [1472-1490] 3'UTR |
| 123 | CCUGACCACUUCAAGGUUG | CAACCUUGAAGUGGUCAGG | | [767-785] ORF | [1455-1473] 3'UTR |
| 124 | UCCCAUUUGAAAGUGGGAA | UUCCCACUUUCAAAUGGGA | | [720-738] ORF | [1408-1426] 3'UTR |
| 125 | AGACAGUCGGUUUUCCCAU | AUGGGAAAACCGACUGUCU | | [707-725] ORF | [1395-1413] 3'UTR |
| 126 | CAACAGGAGAGUCAUUGUU | AACAAUGACUCUCCUGUUG | | [649-667] ORF | [1337-1355] 3'UTR |
| 127 | AGGGAAUGAUGUUGCCUUC | GAAGGCAACAUCAUUCCCU | MO | [604-622] ORF | [1292-1310] 3'UTR |
| 128 | CCAAUGCAAACAGAAUUGC | GCAAUUCUGUUUGCAUUGG | | [570-588] ORF | [1258-1276] 3'UTR |
| 129 | AGCCCAAUGCAAACAGAAU | AUUCUGUUUGCAUUGGGCU | | [567-585] ORF | [1255-1273] 3'UTR |
| 130 | UGUUCACUGUGAGUGAAAA | UUUUCACUCACAGUGAACA | | [966-984] 3'UTR | [1654-1672] 3'UTR |
| 131 | UGAUGCGUUAUCUGGGUCU | AGACCCAGAUAACGCAUCA | | [175-193] ORF | [863-881] 3'UTR |
| 132 | AAACCUUACAUGUGUAAAG | CUUUACACAUGUAAGGUUU | | [941-959] 3'UTR | [1629-1647] 3'UTR |
| 133 | GUGACAUAGACCUCACCAG | CUGGUGAGGUCUAUGUCAC | | [864-882] ORF | [1552-1570] 3'UTR |
| 134 | UCAGCAAACUGGGAAUUUC | GAAAUUCCCAGUUUGCUGA | | [843-861] ORF | [1531-1549] 3'UTR |
| 135 | GGUUGAACCUGACCACUUC | GAAGUGGUCAGGUUCAACC | | [760-778] ORF | [1448-1466] 3'UTR |
| 136 | UGGUUGAACCUGACCACUU | AAGUGGUCAGGUUCAACCA | | [759-777] ORF | [1447-1465] 3'UTR |
| 137 | GUGGGAAACCAUUCAAAAU | AUUUUGAAUGGUUUCCCAC | | [732-750] ORF | [1420-1438] 3'UTR |
| 138 | UCGGUUUUCCCAUUUGAAA | UUUCAAAUGGGAAAACCGA | | [713-731] ORF | [1401-1419] 3'UTR |
| 139 | UGGGUCUGGAAACCCAAAC | GUUUGGGUUUCCAGACCCA | | [187-205] ORF | [875-893] 3'UTR |
| 140 | CAGUGAAUUACCUGUCUCA | UGAGACAGGUAAUUCACUG | | [1037-1055] 3'UTR | [1725-1743] 3'UTR |
| 141 | ACAGUGAAUUACCUGUCUC | GAGACAGGUAAUUCACUGU | | [1036-1054] 3'UTR | [1724-1742] 3'UTR |
| 142 | AUCGGGUUAAAAAACUCAA | UUGAGUUUUUUAACCCGAU | | [819-837] ORF | [1507-1525] 3'UTR |
| 143 | AUAGACCUCACCAGUGCUU | AAGCACUGGUGAGGUCUAU | | [869-887] ORF | [1557-1575] 3'UTR |
| 144 | AGUGAAUGAUGCUCACUUG | CAAGUGAGCAUCAUUCACU | | [787-805] ORF | [1475-1493] 3'UTR |
| 145 | ACCUGACCACUUCAAGGUU | AACCUUGAAGUGGUCAGGU | | [766-784] ORF | [1454-1472] 3'UTR |
| 146 | UUGAACCUGACCACUUCAA | UUGAAGUGGUCAGGUUCAA | | [762-780] ORF | [1450-1468] 3'UTR |
| 147 | CGGUUUUCCCAUUUGAAAG | CUUUCAAAUGGGAAAACCG | | [714-732] ORF | [1402-1420] 3'UTR |
| 148 | AGAACAACAGGAGAGUCAU | AUGACUCUCCUGUUGUUCU | MO | [645-663] ORF | [1333-1351] 3'UTR |
| 149 | UCACUUGUUGCAGUACAAU | AUUGUACUGCAACAAGUGA | | [799-817] ORF | [1487-1505] 3'UTR |
| 150 | AACUCAAUGAAAUCAGCAA | UUGCUGAUUUCAUUGAGUU | | [831-849] ORF | [1519-1537] 3'UTR |
| 151 | AUGCUCACUUGUUGCAGUA | UACUGCAACAAGUGAGCAU | | [795-813] ORF | [1483-1501] 3'UTR |
| 152 | AAAGUGGGAAACCAUUCAA | UUGAAUGGUUUCCCACUUU | | [729-747] ORF | [1417-1435] 3'UTR |
| 153 | CAUGUUCACUGUGAGUGAA | UUCACUCACAGUGAACAUG | | [964-982] 3'UTR | [1652-1670] 3'UTR |
| 154 | AAGUGGGAAACCAUUCAAA | UUUGAAUGGUUUCCCACUU | | [730-746] ORF | [1418-1436] 3'UTR |
| 155 | CACUGUGAGUGAAAAUUUU | AAAAUUUUCACUCACAGUG | | [970-988] 3'UTR | [1658-1676] 3'UTR |
| 156 | UCACUGUGAGUGAAAAUUU | AAAUUUUCACUCACAGUGA | | [969-987] 3'UTR | [1657-1675] 3'UTR |
| 157 | ACUCAAUGAAAUCAGCAAA | UUUGCUGAUUUCAUUGAGU | | [832-850] ORF | [1520-1538] 3'UTR |
| 158 | UUCACUGUGAGUGAAAAUU | AAUUUUCACUCACAGUGAA | | [968-986] 3'UTR | [1656-1674] 3'UTR |
| 159 | GCAGACGGCUUCUCAGUUA | UAAGUGAGAAGCCGUCUGC | | | |
| 160 | AGCGGAAAAUGGCAGACAA | UUGUCUGCCAUUUUCCGCU | | | |

**Table L: 21-mer lectin, galactoside-binding, soluble, 3 (LGALS3)**

| No. | Sense siRNA | AntiSense siRNA | Other Sp | Human-115430222 ORF:152-904 | Human-115430224 |
|---|---|---|---|---|---|
| 1 | ACAUUCAUCAAUAUCCCUCUU | AAGAGGGAUAUUGAUGAAUGU | | [990-1010] 3'UTR | [1678-1698] 3'UTR |
| 2 | GCAAUACAAAGCUGGAUAAUA | UAUUAUCCAGCUUUGUAUUGC | | [669-689] ORF | [1357-1377] 3'UTR |
| 3 | GGGAAACCAUUCAAAAUACAA | UUGUAUUUUGAAUGGUUUCCC | | [734-754] ORF | [1422-1442] 3'UTR |
| 4 | CCAUUCAAAAUACAAGUACUG | CAGUACUUGUAUUUUGAAUGG | | [740-760] ORF | [1428-1448] 3'UTR |
| 5 | GGAAAAUGGCAGACAAUUUUU | AAAAAUUGUCUGCCAUUUUCC | | [147-167] 5'UTR+ORF | [182-202] 3'UTR |
| 6 | GGGAAGAAAGACAGUCGGUUU | AAACCGACUGUCUUUCUUCCC | | [699-719] ORF | [1387-1407] 3'UTR |
| 7 | UGUGCCUUAUAACCUGCCUUU | AAAGGCAGGUUAUAAGGCACA | | [496-516] ORF | [1184-1204] 3'UTR |
| 8 | CGGGUUAAAAAACUCAAUGAA | UUCAUUGAGUUUUUUAACCCG | | [821-841] ORF | [1509-1529] 3'UTR |
| 9 | CCACUUUAACCCACGCUUCAA | UUGAAGCGUGGGUUAAAGUGG | | [622-642] ORF | [1310-1330] 3'UTR |
| 10 | ACCUCACCAGUGCUUCAUAUA | UAUAUGAAGCACUGGUGAGGU | | [873-893] ORF | [1561-1581] 3'UTR |
| 11 | GGAAGAAAGACAGUCGGUUUU | AAAACCGACUGUCUUUCUUCC | | [700-720] ORF | [1388-1408] 3'UTR |
| 12 | CAGUACAAUCAUCGGGUUAAA | UUUAACCCGAUGAUUGUACUG | | [809-829] ORF | [1497-1517] 3'UTR |
| 13 | AGGAGAGUCAUUGUUUGCAAU | AUUGCAAACAAUGACUCUCCU | | [653-673] ORF | [1341-1361] 3'UTR |
| 14 | UCAAAAUACAAGUACUGGUUG | CAACCAGUACUUGUAUUUUGA | | [744-764] ORF | [1432-1452] 3'UTR |
| 15 | GGAAACCAUUCAAAAUACAAG | CUUGUAUUUUGAAUGGUUUCC | | [735-755] ORF | [1423-1443] 3'UTR |
| 16 | CAGGAGAGUCAUUGUUUGCAA | UUGCAAACAAUGACUCUCCUG | | [652-672] ORF | [7340-1360] 3'UTR |
| 17 | GCGUUAUCUGGGUCUGGAAAC | GUUUCCAGACCCAGAUAACGC | | [179-199] ORF | [867-887] 3'UTR |
| 18 | CCAUGAUAUAAUCUGAAAGGG | CCCUUUCAGAUUAUAUCAUGG | | [894-914] ORF+3'UTR | [1582-1602] 3'UTR |
| 19 | GGAAUUUCUGGUGACAUAGAC | GUCUAUGUCACCAGAAAUUCC | | [854-874] ORF | [1542-1562] 3'UTR |
| 20 | GCAGUACAAUCAUCGGGUUAA | UUAACCCGAUGAUUGUACUGC | | [808-828] ORF | [1496-1516] 3'UTR |
| 21 | CACUUUAACCCACGCUUCAAU | AUUGAAGCGUGGGUUAAAGUG | | [623-643] ORF | [1311-1331] 3'UTR |
| 22 | AUGCGUUAUCUGGGUCUGGAA | UUCCAGACCCAGAUAACGCAU | | [177-197] ORF | [865-885] 3'UTR |
| 23 | GAAUCUAAACCUUACAUGUGU | ACACAUGUAAGGUUUAGAUUC | | [935-955] 3'UTR | [1623-1643] 3'UTR |
| 24 | CACCAGUGCUUCAUAUACCAU | AUGGUAUAUGAAGCACUGGUG | | [877-897] ORF | [1565-1585] 3'UTR |
| 25 | GCAAACUGGGAAUUUCUGGUG | CACCAGAAAUUCCCAGUUUGC | | [846-666] ORF | [1534-1554] 3'UTR |
| 26 | CAAAAUACAAGUACUGGUUGA | UCAACCAGUACUUGUAUUUUG | | [745-765] ORF | [1433-1453] 3'UTR |
| 27 | UGCGUUAUCUGGGUCUGGAAA | UUUCCAGACCCAGAUAACGCA | | [178-198] ORF | [866-886] 3'UTR |
| 28 | UCACCAGUGCUUCAUAUACCA | UGGUAUAUGAAGCACUGGUGA | | [876-896] ORF | [1564-1584] 3'UTR |
| 29 | GACCUCACCAGUGCUUCAUAU | AUAUGAAGCACUGGUGAGGUC | | [872-892] ORF | [1560-1580] 3'UTR |
| 30 | GGGAAUUUCUGGUGACAUAGA | UCUAUGUCACCAGAAAUUCCC | | [853-873] ORF | [1541-1561] 3'UTR |
| 31 | UGGGAAUUUCUGGUGACAUAG | CUAUGUCACCAGAAAUUCCCA | | [852-872] ORF | [1540-1560] 3'UTR |
| 32 | CUGGGAAUUUCUGGUGACAUA | UAUGUCACCAGAAAUUCCCAG | | [851-871] ORF | [1539-1559] 3'UTR |
| 33 | GUACAAUCAUCGGGUUAAAAA | UUUUUAACCCGAUGAUUGUAC | | [811-831] ORF | [1499-1519] 3'UTR |
| 34 | AGUACAAUCAUCGGGUUAAAA | UUUUAACCCGAUGAUUGUACU | | [810-830] ORF | [1498-1518] 3'UTR |
| 35 | UGCAGUACAAUCAUCGGGUUA | UAACCCGAUGAUUGUACUGCA | | [807-827] ORF | [1495-1515] 3'UTR |
| 36 | UCAAGGUUGCAGUGAAUGAUG | CAUCAUUCACUGCAACCUUGA | | [777-797] ORF | [1465-1485] 3'UTR |
| 37 | CUUCCACUUUAACCCACGCUU | AAGCGUGGGUUAAAGUGGAAG | | [619-639] ORF | [1307-1327] 3'UTR |
| 38 | CCUUCCACUUUAACCCACGCU | AGCGUGGGUUAAAGUGGAAGG | | [618-638] ORF | [1306-1326] 3'UTR |
| 39 | CCAAAGAGGGAAUGAUGUUGC | GCAACAUCAUUCCCUCUUUGG | | [598-618] ORF | [1286-1306] 3'UTR |
| 40 | GCUGAUAACAAUUCUGGGCAC | GUGCCCAGAAUUGUUAUCAGC | | [541-561] ORF | [1229-1249] 3'UTR |
| 41 | ACUGGGAAUUUCUGGUGACAU | AUGUCACCAGAAAUUCCCAGU | | [850-870] ORF | [1538-1558] 3'UTR |
| 42 | CAAACUGGGAAUUUCUGGUGA | UCACCAGAAAUUCCCAGUUUG | | [847-867] ORF | [1535-1555] 3'UTR |
| 43 | CACGCUUCAAUGAGAACAACA | UGUUGUUCUCAUUGAAGCGUG | | [633-653] ORF | [1321-1341] 3'UTR |
| 44 | GUGCCUUAUAACCUGCCUUUG | CAAAGGCAGGUUAUAAGGCAC | | [497-517] ORF | [1185-1205] 3'UTR |
| 45 | CAGAAUUGCUUUAGAUUUCCA | UGGAAAUCUAAAGCAAUUCUG | | [580-600] ORF | [1268-1288] 3'UTR |
| 46 | GCAUGCUGAUAACAAUUCUGG | CCAGAAUUGUUAUCAGCAUGC | | [537-557] ORF | [1225-1245] 3'UTR |
| 47 | UUCAUCAAUAUCCCUCUUGUA | UACAAGAGGGAUAUUGAUGAA | | [993-1013] 3'UTR | [1681-1701] 3'UTR |
| 48 | ACAGUCGGUUUUCCCAUUUGA | UCAAAUGGGAAAACCGACUGU | | [709-729] ORF | [1397-1417] 3'UTR |
| 49 | UGCUGAUAACAAUUCUGGGCA | UGCCCAGAAUUGUUAUCAGCA | | [540-560] ORF | [1228-1248] 3'UTR |
| 50 | UCAUCAAUAUCCCUCUUGUAA | UUACAAGAGGGAUAUUGAUGA | | [994-1014] 3'UTR | [1682-1702] 3'UTR |
| 51 | UUGCAAUACAAAGCUGGAUAA | UUAUCCAGCUUUGUAUUGCAA | | [667-687] ORF | [1355-1375] 3'UTR |
| 52 | ACAGAAUUGCUUUAGAUUUCC | GGAAAUCUAAAGCAAUUCUGU | | [579-599] ORF | [1267-1287] 3'UTR |
| 53 | UGUGUAAAGGUUUCAUGUUCA | UGAACAUGAAACCUUUACACA | | [951-971] 3'UTR | [1639-1659] 3'UTR |
| 54 | UUGCAGUACAAUCAUCGGGUU | AACCCGAUGAUUGUACUGCAA | | [806-826] ORF | [1494-1514] 3'UTR |
| 55 | UGCAAUACAAAGCUGGAUAAU | AUUAUCCAGCUUUGUAUUGCA | | [668-688] ORF | [1356-1376] 3'UTR |
| 56 | UGAAAUCAGCAAACUGGGAAU | AUUCCCAGUUUGCUGAUUUCA | | [838-858] ORF | [1526-1546] 3'UTR |
| 57 | GGAAGGGAAGAAAGACAGUCG | CGACUGUCUUUCUUCCCUUCC | | [695-715] ORF | [1383-1403] 3'UTR |
| 58 | GAGUCAUUGUUUGCAAUACAA | UUGUAUUGCAAACAAUGACUC | | [657-677] ORF | [1345-1365] 3'UTR |
| 59 | UCAAUAUCCCUCUUGUAAGUC | GACUUACAAGAGGGAUAUUGA | | [997-1017] 3'UTR | [1685-1705] 3'UTR |
| 60 | GAAAUCAGCAAACUGGGAAUU | AAUUCCCAGUUUGCUGAUUUC | | [839-859] ORF | [1527-1547] 3'UTR |
| 61 | AGAGUCAUUGUUUGCAAUACA | UGUAUUGCAAACAAUGACUCU | | [656-676] ORF | [1344-1364] 3'UTR |
| 62 | CAAUGAGAACAACAGGAGAGU | ACUCUCCUGUUGUUCUCAUUG | MO | [640-660] ORF | [1328-1348] 3'UTR |
| 63 | AUCCCUCUUGUAAGUCAUCUA | UAGAUGACUUACAAGAGGGAU | | [1002-1022] 3'UTR | [1690-1710] 3'UTR |
| 64 | CCACGCUUCAAUGAGAACAAC | GUUGUUCUCAUUGAAGCGUGG | | [632-652] ORF | [1320-1340] 3'UTR |
| 65 | CCUCUUGUAAGUCAUCUACUU | AAGUAGAUGACUUACAAGAGG | | [1005-1025] 3'UTR | [1693-1713] 3'UTR |
| 66 | GUGCUUCAUAUACCAUGAUAU | AUAUCAUGGUAUAUGAAGCAC | | [882-902] ORF | [1570-1590] 3'UTR |
| 67 | GCAGUGAAUGAUGCUCACUUG | CAAGUGAGCAUCAUUCACUGC | | [785-805] ORF | [1473-1493] 3'UTR |
| 68 | AGACAGUCGGUUUUCCCAUUU | AAAUGGGAAAACCGACUGUCU | | [707-727] ORF | [1395-1415] 3'UTR |
| 69 | GGAGAGUCAUUGUUUGCAAUA | UAUUGCAAACAAUGACUCUCC | | [654-674] ORF | [1342-1362] 3'UTR |
| 70 | UAACCCACGCUUCAAUGAGAA | UUCUCAUUGAAGCGUGGGUUA | | [628-648] ORF | [1316-1336] 3'UTR |
| 71 | CCCACGCUUCAAUGAGAACAA | UUGUUCUCAUUGAAGCGUGGG | | [631-651] ORF | [1319-1339] 3'UTR |
| 72 | UGAAGCCCAAUGCAAACAGAA | UUCUGUUUGCAUUGGGCUUCA | | [564-584] ORF | [1252-1272] 3'UTR |
| 73 | CGCUUCAAUGAGAACAACAGG | CCUGUUGUUCUCAUUGAAGCG | MO | [635-655] ORF | [1323-1343] 3'UTR |
| 74 | CCUCGCAUGCUGAUAACAAUU | AAUUGUUAUCAGCAUGCGAGG | | [533-553] ORF | (1221-1241] 3'UTR |
| 75 | CCACUGAUUGUGCCUUAUAAC | GUUAUAAGGCACAAUCAGUGG | | [488-508] ORF | [1176-1196] 3'UTR |
| 76 | GGCCACUGAUUGUGCCUUAUA | UAUAAGGCACAAUCAGUGGCC | | [486-506] ORF | [1174-1194] 3'UTR |
| 77 | CUCUUGUAAGUCAUCUACUUA | UAAGUAGAUGACUUACAAGAG | | [1006-1026] 3'UTR | [1694-1714] 3'UTR |
| 78 | CCCUCUUGUAAGUCAUCUACU | AGUAGAUGACUUACAAGAGGG | | [1004-1024] 3'UTR | [1692-1712] 3'UTR |
| 79 | CAGUCGGUUUUCCCAUUUGAA | UUCAAAUGGGAAAACCGACUG | | [710-730] ORF | [1398-1418] 3'UTR |
| 80 | GCAAACAGAAUUGCUUUAGAU | AUCUAAAGCAAUUCUGUUUGC | | [575-595] ORF | [1263-1283] 3'UTR |
| 81 | CAAUGCAAACAGAAUUGCUUU | AAAGCAAUUCUGUUUGCAUUG | | [571-591] ORF | [1259-1279] 3'UTR |
| 82 | CCAAUGCAAACAGAAUUGCUU | AAGCAAUUCUGUUUGCAUUGG | | [570-590] ORF | [1258-1278] 3'UTR |
| 83 | AGUGCUUCAUAUACCAUGAUA | UAUCAUGGUAUAUGAAGCACU | | [881-901] ORF | [1569-1589] 3'UTR |
| 84 | UGAUGUUGCCUUCCACUUUAA | UUAAAGUGGAAGGCAACAUCA | MO | [610-630] ORF | [1298-1318] 3'UTR |
| 85 | GGAAUGAUGUUGCCUUCCACU | AGUGGAAGGCAACAUCAUUCC | MO | [606-626] ORF | [1294-1314] 3'UTR |
| 86 | CACGGUGAAGCCCAAUGCAAA | UUUGCAUUGGGCUUCACCGUG | | [559-579] ORF | [1247-1267] 3'UTR |
| 87 | GGGCCACUGAUUGUGCCUUAU | AUAAGGCACAAUCAGUGGCCC | | [485-505] ORF | [1173-1193] 3'UTR |
| 88 | CUGGAAACCCAAACCCUCAAG | CUUGAGGGUUUGGGUUUCCAG | MO | [192-212] ORF | [880-900] 3'UTR |
| 89 | UGACCACUUCAAGGUUGCAGU | ACUGCAACCUUGAAGUGGUCA | | [769-789] ORF | [1457-1477] 3'UTR |
| 90 | GAAGCCCAAUGCAAACAGAAU | AUUCUGUUUGCAUUGGGCUUC | | [565-585] ORF | [1253-1273] 3'UTR |
| 91 | CCUUAUAACCUGCCUUUGCCU | AGGCAAAGGCAGGUUAUAAGG | | [500-520] ORF | [1188-1208] 3'UTR |
| 92 | GCCACUGAUUGUGCCUUAUAA | UUAUAAGGCACAAUCAGUGGC | | [487-507] ORF | [1175-1195] 3'UTR |
| 93 | UGGGCCACUGAUUGUGCCUUA | UAAGGCACAAUCAGUGGCCCA | | [484-504] ORF | [1172-1192] 3'UTR |
| 94 | UCCCUCUUGUAAGUCAUCUAC | GUAGAUGACUUACAAGAGGGA | | [1003-1023] 3'UTR | [1691-1711] 3'UTR |
| 95 | AGGUUUCAUGUUCACUGUGAG | CUCACAGUGAACAUGAAACCU | | [958-978] 3'UTR | [1646-1666] 3'UTR |
| 96 | UGCUCACUUGUUGCAGUACAA | UUGUACUGCAACAAGUGAGCA | | [796-816] ORF | [1484-1504] 3'UTR |
| 97 | GAAAGUGGGAAACCAUUCAAA | UUUGAAUGGUUUCCCACUUUC | | [728-748] ORF | [1416-1436] 3'UTR |
| 98 | GACAGUCGGUUUUCCCAUUUG | CAAAUGGGAAAACCGACUGUC | | [708-728] ORF | [1396-1416] 3'UTR |
| 99 | AGAUUUCCAAAGAGGGAAUGA | UCAUUCCCUCUUUGGAAAUCU | | [592-612] ORF | [1280-1300] 3'UTR |
| 100 | CCAUCUUCUGGACAGCCAAGU | ACUUGGCUGUCCAGAAGAUGG | | [419-439] ORF | [1107-1127] 3'UTR |
| 101 | GGAAACCCAAACCCUCAAGGA | UCCUUGAGGGUUUGGGUUUCC | MO | [194-214] ORF | [882-902] 3'UTR |
| 102 | GGUUUCAUGUUCACUGUGAGU | ACUCACAGUGAACAUGAAACC | | [959-979] 3'UTR | [1647-1667] 3'UTR |
| 103 | CCAGUGCUUCAUAUACCAUGA | UCAUGGUAUAUGAAGCACUGG | | [879-899] ORF | [1567-1587] 3'UTR |
| 104 | AGACCUCACCAGUGCUUCAUA | UAUGAAGCACUGGUGAGGUCU | | [871-891] ORF | [1559-1579] 3'UTR |
| 105 | CCAUGAUGCGUUAUCUGGGUC | GACCCAGAUAACGCAUCAUGG | | [172-192] ORF | [860-880] 3'UTR |
| 106 | GCUCACUUGUUGCAGUACAAU | AUUGUACUGCAACAAGUGAGC | | [797-817] ORF | [1485-1505] 3'UTR |
| 107 | GUGCCUCGCAUGCUGAUAACA | UGUUAUCAGCAUGCGAGGCAC | | [530-550] ORF | [1218-1238] 3'UTR |
| 108 | ACCCAUCUUCUGGACAGCCAA | UUGGCUGUCCAGAAGAUGGGU | | [417-437] ORF | [1105-1125] 3'UTR |
| 109 | CAGUGCUUCAUAUACCAUGAU | AUCAUGGUAUAUGAAGCACUG | | [880-900] ORF | [1568-1586] 3'UTR |
| 110 | GGUUGCAGUGAAUGAUGCUCA | UGAGCAUCAUUCACUGCAACC | | [781-801] ORF | [1469-1489] 3'UTR |
| 111 | UGGGAAACCAUUCAAAAUACA | UGUAUUUUGAAUGGUUUCCCA | | [733-753] ORF | [1421-1441] 3'UTR |
| 112 | UGAGAACAACAGGAGAGUCAU | AUGACUCUCCUGUUGUUCUCA | MO | [643-663] ORF | [1331-1351] 3'UTR |
| 113 | GGGAAUGAUGUUGCCUUCCAC | GUGGAAGGCAACAUCAUUCCC | MO | [605-625] ORF | [1293-1313] 3'UTR |
| 114 | UGCUUUAGAUUUCCAAAGAGG | CCUCUUUGGAAAUCUAAAGCA | | [586-606] ORF | [1274-1294] 3'UTR |
| 115 | UUGCUUUAGAUUUCCAAAGAG | CUCUUUGGAAAUCUAAAGCAA | | [585-605] ORF | [1273-1293] 3'UTR |
| 116 | CCCAAUGCAAACAGAAUUGCU | AGCAAUUCUGUUUGCAUUGGG | | [569-589] ORF | [1257-1277] 3'UTR |
| 117 | GCCUCGCAUGCUGAUAACAAU | AUUGUUAUCAGCAUGCGAGGC | | [532-552] ORF | [1220-1240] 3'UTR |
| 118 | ACCUUACAUGUGUAAAGGUUU | AAACCUUUACACAUGUAAGGU | | [943-963] 3'UTR | [1631-1651] 3'UTR |
| 119 | CAAGUACUGGUUGAACCUGAC | GUCAGGUUCAACCAGUACUUG | | [752-772] ORF | [1440-1460] 3'UTR |
| 120 | GAAGAAAGACAGUCGGUUUUC | GAAAACCGACUGUCUUUCUUC | | [701-721] ORF | [1389-1409] 3'UTR |
| 121 | GAAUGAUGUUGCCUUCCACUU | AAGUGGAAGGCAACAUCAUUC | MO | [607-627] ORF | [1295-1316] 3'UTR |
| 122 | GGUGAAGCCCAAUGCAAACAG | CUGUUUGCAUUGGGCUUCACC | | [562-582] ORF | [1250-1270] 3'UTR |
| 123 | CGGUGAAGCCCAAUGCAAACA | UGUUUGCAUUGGGCUUCACCG | | [561-581] ORF | [1249-1269] 3'UTR |
| 124 | ACAGUGAAUUACCUGUCUCAA | UUGAGACAGGUAAUUCACUGU | | [1036-1056] 3'UTR | [1724-1744] 3'UTR |
| 125 | AUGUUCACUGUGAGUGAAAAU | AUUUUCACUCACAGUGAACAU | | [965-985] 3'UTR | [1653-1673] 3'UTR |
| 126 | UGAUGCUCACUUGUUGCAGUA | UACUGCAACAAGUGAGCAUCA | | [793-813] ORF | [1481-1501] 3'UTR |
| 127 | GACCACUUCAAGGUUGCAGUG | CACUGCAACCUUGAAGUGGUC | | [770-790] ORF | [1458-1478] 3'UTR |
| 128 | AUACAAGUACUGGUUGAACCU | AGGUUCAACCAGUACUUGUAU | | [749-769] ORF | [1437-1457] 3'UTR |
| 129 | GUGGGAAACCAUUCAAAAUAC | GUAUUUUGAAUGGUUUCCCAC | | [732-752] ORF | [1420-1440] 3'UTR |
| 130 | ACAACAGGAGAGUCAUUGUUU | AAACAAUGACUCUCCUGUUGU | | [648-668] ORF | [1336-1356] 3'UTR |
| 131 | AGAGGGAAUGAUGUUGCCUUC | GAAGGCAACAUCAUUCCCUCU | MO | [602-622] ORF | [1290-1310] 3'UTR |
| 132 | GUGAAGCCCAAUGCAAACAGA | UCUGUUUGCAUUGGGCUUCAC | | [563-583] ORF | [1251-1271] 3'UTR |
| 133 | CGCAUGCUGAUAACAAUUCUG | CAGAAUUGUUAUCAGCAUGCG | | [536-556] ORF | [1224-1244] 3'UTR |
| 134 | GGUGACAUAGACCUCACCAGU | ACUGGUGAGGUCUAUGUCACC | | [863-883] ORF | [1551-1571] 3'UTR |
| 135 | AAAUCAGCAAACUGGGAAUUU | AAAUUCCCAGUUUGCUGAUUU | | [840-860] ORF | [1528-1548] 3'UTR |
| 136 | UCGGGUUAAAAAACUCAAUGA | UCAUUGAGUUUUUUAACCCGA | | [820-840] ORF | [1508-1528] 3'UTR |
| 137 | UCAUCGGGUUAAAAAACUCAA | UUGAGUUUUUUAACCCGAUGA | | [817-837] ORF | [1505-1525] 3'UTR |
| 138 | CCUGACCACUUCAAGGUUGCA | UGCAACCUUGAAGUGGUCAGG | | [767-787] ORF | [1455-1475] 3'UTR |
| 139 | GAACCUGACCACUUCAAGGUU | AACCUUGAAGUGGUCAGGUUC | | [764-784] ORF | [1452-1472] 3'UTR |
| 140 | UGGUUGAACCUGACCACUUCA | UGAAGUGGUCAGGUUCAACCA | | [759-779] ORF | [1447-1467] 3'UTR |
| 141 | GUACUGGUUGAACCUGACCAC | GUGGUCAGGUUCAACCAGUAC | | [755-775] ORF | [1443-1463] 3'UTR |
| 142 | CAUUUGAAAGUGGGAAACCAU | AUGGUUUCCCACUUUCAAAUG | | [723-743] ORF | [1411-1431] 3'UTR |
| 143 | CCAUUUGAAAGUGGGAAACCA | UGGUUUCCCACUUUCAAAUGG | | [722-742] ORF | [1410-1430] 3'UTR |
| 144 | CGGUUUUCCCAUUUGAAAGUG | CACUUUCAAAUGGGAAAACCG | | [714-734] ORF | [1402-1422] 3'UTR |
| 145 | GAACAACAGGAGAGUCAUUGU | ACAAUGACUCUCCUGUUGUUC | MO | [646-666] ORF | [1334-1354] 3'UTR |
| 146 | AGCCCAAUGCAAACAGAAUUG | CAAUUCUGUUUGCAUUGGGCU | | [567-587] ORF | [1255-1275] 3'UTR |
| 147 | UCGCAUGCUGAUAACAAUUCU | AGAAUUGUUAUCAGCAUGCGA | | [535-555] ORF | [1223-1243] 3'UTR |
| 148 | GAAACCCAAACCCUCAAGGAU | AUCCUUGAGGGUUUGGGUUUC | MO | [195-215] ORF | [883-903] 3'UTR |
| 149 | CUGGUGACAUAGACCUCACCA | UGGUGAGGUCUAUGUCACCAG | | [861-881] ORF | [1549-1569] 3'UTR |
| 150 | CACUUGUUGCAGUACAAUCAU | AUGAUUGUACUGCAACAAGUG | | [800-820] ORF | [1488-1508] 3'UTR |
| 151 | CAGUGAAUGAUGCUCACUUGU | ACAAGUGAGCAUCAUUCACUG | | [786-806] ORF | [1474-1494] 3'UTR |
| 152 | CCCAUUUGAAAGUGGGAAACC | GGUUUCCCACUUUCAAAUGGG | | [721-741] ORF | [1409-1429] 3'UTR |
| 153 | GAGAACAACAGGAGAGUCAUU | AAUGACUCUCCUGUUGUUCUC | MO | [644-664] ORF | [1332-1352] 3'UTR |
| 154 | UUAGAUUUCCAAAGAGGGAAU | AUUCCCUCUUUGGAAAUCUAA | | [590-610] ORF | [1278-1298] 3'UTR |
| 155 | UGCAAACAGAAUUGCUUUAGA | UCUAAAGCAAUUCUGUUUGCA | | [574-594] ORF | [1262-1282] 3'UTR |
| 156 | GCCCAAUGCAAACAGAAUUGC | GCAAUUCUGUUUGCAUUGGGC | | [566-588] ORF | [1256-1276] 3'UTR |
| 157 | UGCCUCGCAUGCUGAUAACAA | UUGUUAUCAGCAUGCGAGGCA | | [531-551] ORF | [1219-1239] 3'UTR |
| 158 | UCAUGUUCACUGUGAGUGAAA | UUUCACUCACAGUGAACAUGA | | [963-983] 3'UTR | [1651-1671] 3'UTR |
| 159 | GACAUAGACCUCACCAGUGCU | AGCACUGGUGAGGUCUAUGUC | | [866-886] ORF | [1554-1574] 3'UTR |
| 160 | CAUCGGGUUAAAAAACUCAAU | AUUGAGUUUUUUAACCCGAUG | | [818-838] ORF | [1506-1526] 3'UTR |
| 161 | UGCAGUGAAUGAUGCUCACUU | AAGUGAGCAUCAUUCACUGCA | | [784-804] ORF | [1472-1492] 3'UTR |
| 162 | CACUUCAAGGUUGCAGUGAAU | AUUCACUGCAACCUUGAAGUG | | [773-793] ORF | [1461-1481] 3'UTR |
| 163 | CCACUUCAAGGUUGCAGUGAA | UUCACUGCAACCUUGAAGUGG | | [772-792] ORF | [1460-1480] 3'UTR |
| 164 | GGUUGAACCUGACCACUUCAA | UUGAAGUGGUCAGGUUCAACC | | [760-780] ORF | [1448-1468] 3'UTR |
| 165 | UCCCAUUUGAAAGUGGGAAAC | GUUUCCCACUUUCAAAUGGGA | | [720-740] ORF | [1408-1428] 3'UTR |
| 166 | UUCCCAUUUGAAAGUGGGAAA | UUUCCCACUUUCAAAUGGGAA | | [719-739] ORF | [1407-1427] 3'UTR |
| 167 | GCCUUAUAACCUGCCUUUGCC | GGCAAAGGCAGGUUAUAAGGC | | [499-519] ORF | [1187-1207] 3'UTR |
| 168 | ACAUAGACCUCACCAGUGCUU | AAGCACUGGUGAGGUCUAUGU | | [867-887] ORF | [1555-1575] 3'UTR |
| 169 | GUGUAAAGGUUUCAUGUUCAC | GUGAACAUGAAACCUUUACAC | | [952-972] 3'UTR | [1640-1660] 3'UTR |
| 170 | ACAUGUGUAAAGGUUUCAUGU | ACAUGAAACCUUUACACAUGU | | [948-968] 3'UTR | [1636-1656] 3'UTR |
| 171 | UCACUUGUUGCAGUACAAUCA | UGAUUGUACUGCAACAAGUGA | | [799-819] ORF | [1487-1507] 3'UTR |
| 172 | AAAGUGGGAAACCAUUCAAAA | UUUUGAAUGGUUUCCCACUUU | | [729-749] ORF | [1417-1437] 3'UTR |
| 173 | UGAAAGUGGGAAACCAUUCAA | UUGAAUGGUUUCCCACUUUCA | | [727-747] ORF | [1415-1435] 3'UTR |
| 174 | GUUCACUGUGAGUGAAAAUUU | AAAUUUUCACUCACAGUGAAC | | [967-987] 3'UTR | [1655-1675] 3'UTR |
| 175 | CUCAAUGAAAUCAGCAAACUG | CAGUUUGCUGAUUUCAUUGAG | | [833-853] ORF | [1521-1541] 3'UTR |
| 176 | ACUCAAUGAAAUCAGCAAACU | AGUUUGCUGAUUUCAUUGAGU | | [832-852] ORF | [1520-1540] 3'UTR |

**Table M: 19-mer Sestrin2 (SESN2, Hi95)**

| Number | Sense siRNA | AntiSense siRNA | Other Sp | Human-32454742 ORF:354-1796 |
|---|---|---|---|---|
| 1 | CGGAAUUAAUGUGCCACAA | UUGUGGCACAUUAAUUCCG | | [2103-2121] 3'UTR |
| 2 | GACUUCCUUUGCCCOUUUU | AAAAAGGGCAAAGGAAGUC | MK | [2373-2391] 3'UTR |
| 3 | CCAGUGUGCCACAUUAAAU | AUUUAAUGUGGCACACUGG | MK | [3224-3242] 3'UTR |
| 4 | AGAUUUCAUUACCUCCUAC | GUAGGAGGUAAUGAAAUCU | MK | [2235-2253] 3'UTR |
| 5 | GCCACUACAUUGCCAUCAU | AUGAUGGCAAUGUAGUGGC | MK,MO | [688-706] ORF |
| 6 | CAGUGUGCCACAUUAAAUA | UAUUUAAUGUGGCACACUG | MK | [3225-3243] 3'UTR |
| 7 | UCAGUUUUUUGACUUCCUU | AAGGAAGUCAAAAAACUGA | MK | [2363-2381] 3'UTR |
| 8 | GUCAUUUCCAGAUUUCAUU | AAUGAAAUCUGGAAAUGAC | MK | [2226-2244] 3'UTR |
| 9 | ACAUUAAAUACCCGUGCAG | CUGCACGGGUAUUUAAUGU | MK | [3234-3252] 3'UTR |
| 10 | GCCAGUGUGCCACAUUAAA | UUUAAUGUGGCACACUGGC | MK | [3223-3241] 3'UTR |
| 11 | CCAACAGCAAGCGGAUUUU | AAAAUCCGCUUGCUGUUGG | MK | [2912-2930] 3'UTR |
| 12 | CUACUUGCCAUUCACCCAU | AUGGGUGAAUGGCAAGUAG | MK | [2250-2268] 3'UTR |
| 13 | GGGCCAUCUGGAACUAUAU | AUAUAGUUCCAGAUGGCCC | MK | [1525-1543] ORF |
| 14 | GCAGAUGUCUCCCAAAAAG | CUUUUUGGGAGACAUCUGC | | [2683-2701] 3'UTR |
| 15 | UUGCCAUUCACCCAUCAAU | AUUGAUGGGUGAAUGGCAA | MK | [2254-2272] 3'UTR |
| 16 | CCUCCUACUUGCCAUUCAC | GUGAAUGGCAAGUAGGAGG | MK | [2246-2264] 3'UTR |
| 17 | ACCUCCUACUUGCCAUUCA | UGAAUGGCAAGUAGGAGGU | MK | [2245-2263] 3'UTR |
| 18 | GAAUUAAUGUGCCACAAGU | ACUUGUGGCACAUUAAUUC | | [2105-2123] 3'UTR |
| 19 | GGAAUUAAUGUGCCACAAG | CUUGUGGCACAUUAAUUCC | | [2104-2122] 3'UTR |
| 20 | UGCUACCAGUUCCAUAUGA | UCAUAUGGAACUGGUAGCA | MK | [3432-3450] 3'UTR |
| 21 | CCAGUGUUCAAGUGCAGAA | UUCUGCACUUGAACACUGG | MK | [3402-3420] 3'UTR |
| 22 | GCAUGUGAUGACUGUAAAU | AUUUACAGUCAUCACAUGC | | [3356-3374] 3'UTR |
| 23 | CGGAUUUUCUUGCAAGAUC | GAUCUUGCAAGAAAAUCCG | MK | [2923-2941] 3'UTR |
| 24 | CCUCUUUAUUUUGGUCCUU | AAGGACCAAAAUAAAGAGG | MK | [2535-2553] 3'UTR |
| 25 | GAAUCCUAGUUCAGUUUUU | AAAAACUGAACUAGGAUUC | MK | [2353-2371] 3'UTR |
| 26 | CCAUCAAUGUGAAAGUCAG | CUGACUUUCACAUUGAUGG | MK | [2265-2283] 3'UTR |
| 27 | GGCCAAGCUCGGAAUUAAU | AUUAAUUCCGAGCUUGGCC | | [2094-2112] 3'UTR |
| 28 | CAGUUCCAUAUGAUGAGAA | UUCUCAUCAUAUGGAACUG | MK | [3438-3456] 3'UTR |
| 29 | UCAUCACCAAGGAACACAU | AUGUGUUCCUUGGUGAUGA | MK | [868-886] ORF |
| 30 | UGACUGUGAAUUACGACUU | AAGUCGUAAUUCACAGUCA | | [3097-3115] 3'UTR |
| 31 | GGAUUUUCUUGCAAGAUCA | UGAUCUUGCAAGAAAAUCC | MK | [2924-2942] 3'UTR |
| 32 | GCGGAUUUUCUUGCAAGAU | AUCUUGCAAGAAAAUCCGC | MK | [2922-2940] 3'UTR |
| 33 | CGCAGAUGUCUCCCAAAAA | UUUUUGGGAGACAUCUGCG | | [2682-2700] 3'UTR |
| 34 | CUCUUUAUUUUGGUCCUUU | AAAGGACCAAAAUAAAGAG | MK | [2536-2554] 3'UTR |
| 35 | AGAGGAGGGAGUAUUAGAU | AUCUAAUACUCCCUCCUCU | MK | [2509-2527] 3'UTR |
| 36 | CCUUUGCCCUUUUUCCCUU | AAGGGAAAAAGGGCAAAGG | | [2378-2396] 3'UTR |
| 37 | CGAAUCCUAGUUCAGUUUU | AAAACUGAACUAGGAUUCG | MK | [2352-2370] 3'UTR |
| 38 | CUGGAAUGGACAGUUCAUU | AAUGAACUGUCCAUUCCAG | MK | [1954-1972] 3'UTR |
| 39 | AGUUCCAUAUGAUGAGAAA | UUUCUCAUCAUAUGGAACU | MK | [3439-3457] 3'UTR |
| 40 | CCAGGGUUCUUGUUUGGAC | GUCCAAACAAGAACCCUGG | MK | [2760-2778] 3'UTR |
| 41 | UUACCUCCUACUUGCCAUU | AAUGGCAAGUAGGAGGUAA | MK | [2243-2261] 3'UTR |
| 42 | UCGGAAUUAAUGUGCCACA | UGUGGCACAUUAAUUCCGA | | [2102-2120] 3'UTR |
| 43 | GGCCAUCUGGAACUAUAUC | GAUAUAGUUCCAGAUGGCC | MK | [1526-1544] ORF |
| 44 | GCCUCACCUACAAUACCAU | AUGGUAUUGUAGGUGAGGC | MK | [1468-1486] ORF |
| 45 | AGAAAUCUUUGGCUUUGCU | AGCAAAGCCAAAGAUUUCU | MK | [3417-3435] 3'UTR |
| 46 | CUGUAGCAAAUGACUGUGA | UCACAGUCAUUUGCUACAG | MK | [3087-3105] 3'UTR |
| 47 | GCUCGGAAUUAAUGUGCCA | UGGCACAUUAAUUCCGAGC | | [2100-2118] 3'UTR |
| 48 | CCAAGCUCGGAAUUAAUGU | ACAUUAAUUCCGAGCUUGG | | [2096-2114] 3'UTR |
| 49 | GAAGAGUCCAGCACCAAAG | CUUUGGUGCUGGACUCUUC | | [172-190] 5'UTR |
| 50 | GGAUUAUACCUGGGAAGAC | GUCUUCCCAGGUAUAAUCC | MK,Rat,MO | [1373-1391] ORF |
| 51 | CUCUCCUCCUUCGUGUUUG | CAAACACGAAGGAGGAGAG | MK | [972-990] ORF |
| 52 | GCUUUGCUACCAGUUCCAU | AUGGAACUGGUAGCAAAGC | MK | [3428-3446] 3'UTR |
| 53 | GACUUCUCUUGCCCUUCUU | AAGAAGGGCAAGAGAAGUC | | [3111-3129] 3'UTR |
| 54 | GUAGCAAAUGACUGUGAAU | AUUCACAGUCAUUUGCUAC | MK | [3089-3107] 3'UTR |
| 55 | CCAUCCCAGUAUCUCAUCU | AGAUGAGAUACUGGGAUGG | MK | [2998-3016] 3'UTR |
| 56 | AGCGGAUUUUCUUGCAAGA | UCUUGCAAGAAAAUCCGCU | MK | [2921-2939] 3'UTR |
| 57 | GGCACCAGGGUUCUUGUUU | AAACAAGAACCCUGGUGCC | MK | [2756-2774] 3'UTR |
| 58 | CUGAAGAGGAGGGAGUAUU | AAUACUCCCUCCUCUUCAG | MK | [2505-2523] 3'UTR |
| 59 | CUUUUUCCCUUUUcUCCAU | AUGGAGAAAAGGGAAAAAG | | [2386-2404] 3'UTR |
| 60 | CUUUGCCCUUUUUCCCUUU | AAAGGGAAAAAGGGCAAAG | | [2379-2397] 3'UTR |
| 61 | CAGUUUUUUGACUUCCUUU | AAAGGAAGUCAAAAAACUG | MK | [2364-2382] 3'UTR |
| 62 | CAAGCUCGGAAUUAAUGUG | CACAUUAAUUCCGAGCUUG | | [2097-2115] 3'UTR |
| 63 | ACUAUAUCCACUGCGUCUU | AAGACGCAGUGGAUAUAGU | MK | [1537-1555] ORF |
| 64 | UGCACCCUGACUACUUUAC | GUAAAGUAGUCAGGGUGCA | MK | [607-625] ORF |
| 65 | CAGACAUGCUGUGCUUUGU | ACAAAGCACAGCAUGUCUG | MK | [1282-1300] ORF |
| 66 | CUGCACCCUGACUACUUUA | UAAAGUAGUCAGGGUGCAG | MK | [606-624] ORF |
| 67 | ACCAGUUCCAUAUGAUGAG | CUCAUCAUAUGGAACUGGU | MK | [3436-3454] 3'UTR |
| 68 | CUAGAGGGCAUGUGAUGAC | GUCAUCACAUGCCCUCUAG | | [3349-3367] 3'UTR |
| 69 | CCCUUCUUCUAGCAGUCUG | CAGACUGCUAGAAGAAGGG | | [3122-3140] 3'UTR |
| 70 | UCCUCUUUAUUUUGGUCCU | AGGACCAAAAUAAAGAGGA | MK | [2534-2552] 3'UTR |
| 71 | GGAAUGGACAGUUCAUUGC | GCAAUGAACUGUCCAUUCC | MK | [1956-1974] 3'UTR |
| 72 | CACCCUGACUACUUUACCA | UGGUAAAGUAGUCAGGGUG | MK | [609-627] ORF |
| 73 | UGCUGUGCUUUGUGGAAGA | UCUUCCACAAAGCACAGCA | MK | [1288-1306] ORF |
| 74 | UGAUGAGAAAUAAACGUUC | GAACGUUUAUUUCUCAUCA | MK | [3448-3466] 3'UTR |
| 75 | UGAUGACUGUAAAUGUUCA | UGAACAUUUACAGUCAUCA | | [3361-3379] 3'UTR |
| 76 | CCGAUUCCAGGCACUUUCU | AGAAAGUGCCUGGAAUCGG | MK | [3070-3088] 3'UTR |
| 77 | ACAGCAAGCGGAUUUUCUU | AAGAAAAUCCGCUUGCUGU | MK | [2915-2933] 3'UTR |
| 78 | CCCACAAUGUCUGAAGCUG | CAGCUUCAGACAUUGUGGG | MK | [2868-2886] 3'UTR |
| 79 | CUAGAUGGCUUAGGUGGCA | UGCCACCUAAGCCAUCUAG | | [2712-2730] 3'UTR |
| 80 | UUGCCCUUUUUCCCUUUUC | GAAAAGGGAAAAAGGGCAA | | [2381-2399] 3'UTR |
| 81 | UCCUAGUUCAGUUUUUUGA | UCAAAAAACUGAACUAGGA | MK | [2356-2374] 3'UTR |
| 82 | CGAGUUUCUGCAGACUGGU | ACCAGUCUGCAGAAACUCG | MK | [755-773] ORF |
| 83 | CCAGAUUUCAUUACCUCCU | AGGAGGUAAUGAAAUCUGG | MK | [2233-2251] 3'UTR |
| 84 | CGCCAUCAGUGUUCUUACC | GGUAAGAACACUGAUGGCG | MK | [717-735] ORF |
| 85 | UGAGAAAUAAACGUUCGCU | AGCGAACGUUUAUUUCUCA | MK | [3451-3469] 3'UTR |
| 86 | UUCAAGUGCAGAAAUCUUU | AAAGAUUUCUGCACUUGAA | MK | [3408-3426] 3'UTR |
| 87 | GUGGUAUCCAGUGUUCAAG | CUUGAACACUGGAUACCAC | MK | [3395-3413] 3'UTR |
| 88 | UCUGGGUUCUAGAGGGCAU | AUGCCCUCUAGAACCCAGA | | [3341-3359] 3'UTR |
| 89 | GAGUAGACAACCUGGCAGU | ACUGCCAGGUUGUCUACUC | MK | [577-595] ORF |
| 90 | CUCAGCGAGAUCAACAAGU | ACUUGUUGAUCUCGCUGAG | MK | [828-846] ORF |
| 91 | CACAUUGCCUUCUGAAGAG | CUCUUCAGAAGGCAAUGUG | MK | [2494-2512] 3'UTR |
| 92 | CCUUUUCUCCAUGCUUAAU | AUUAAGCAUGGAGAAAAGG | MK | [2393-2411] 3'UTR |
| 93 | GCUUGUGUGUGAUGUGCAG | CUGCACAUCACACACAAGC | MK | [1906-1924] 3'UTR |
| 94 | GGCUUGUGUGUGAUGUGCA | UGCACAUCACACACAAGCC | MK | [1905-1923] 3'UTR |
| 95 | CCGCCAUCAGUGUUCUUAC | GUAAGAACACUGAUGGCGG | MK | [716-734] ORF |
| 96 | CAGUGGGCUUGUGUGUGAU | AUCACACACAAGCCCACUG | | [1900-1918] 3'UTR |
| 97 | CUUUCGGAUAUGAGGACUU | AAGUCCUCAUAUCCGAAAG | MK | [1312-1330] ORF |
| 98 | GAGAAAUAAACGUUCGCUG | CAGCGAACGUUUAUUUCUC | MK | [3452-3470] 3'UTR |
| 99 | CAGAAAUCUUUGGCUUUGC | GCAAAGCCAAAGAUUUCUG | MK | [3416-3434] 3'UTR |
| 100 | CCUUCUUCUAGCAGUCUGU | ACAGACUGCUAGAAGAAGG | | [3123-3141] 3'UTR |
| 101 | CCUUUAUGCUUGAGGUUCC | GGAACCUCAAGCAUAAAGG | MK | [2550-2568] 3'UTR |
| 102 | GGUCACAUUGCCUUCUGAA | UUCAGAAGGCAAUGUGACC | MK | [2491-2509] 3'UTR |
| 103 | UCUAGAGAGUUUGAGCCUU | AAGGCUCAAACUCUCUAGA | MK | [2468-2486] 3'UTR |
| 104 | GCAAACUCAGCGAGAUCAA | UUGAUCUCGCUGAGUUUGC | MK | [823-841] ORF |
| 105 | UCAAUGUGAAAGUCAGGGU | ACCCUGACUUUCACAUUGA | MK | [2268-2286] 3'UTR |
| 106 | GCCAUCAGUGUUCUUACCU | AGGUAAGAACACUGAUGGC | MK | [718-736] ORF |
| 107 | AGCUGCUGGAUGAGAAGUU | AACUUCUCAUCCAGCAGCU | MK,Rat,MO | [1435-1453] ORF |
| 108 | CGUUCGCUGAGGUUUUGUU | AACAAAACCUCAGCGAACG | MK | [3462-3480] 3'UTR |
| 109 | CCCAUCCCAGUAUCUCAUC | GAUGAGAUACUGGGAUGGG | MK | [2997-3015] 3'UTR |
| 110 | AGGCACCAGGGUUCUUGUU | AACAAGAACCCUGGUGCCU | MK | [2755-2773] 3'UTR |
| 111 | AGAGGGCACAGGAAAGAAG | CUUCUUUCCUGUGCCCUCU | MK | [2072-2090] 3'UTR |
| 112 | GUGGGCUUGUGUGUGAUGU | ACAUCACACACAAGCCCAC | MK | [1902-1920] 3'UTR |
| 113 | AGCCUCACCUACAAUACCA | UGGUAUUGUAGGUGAGGCU | MK | [1467-1485] ORF |
| 114 | AGAGGGAGAAUUCUGUUCU | AGAACAGAAUUCUCCCUCU | | [2601-2619] 3'UTR |
| 115 | CAAUGUGAAAGUCAGGGUC | GACCCUGACUUUCACAUUG | MK | [2269-2287] 3'UTR |
| 116 | GGCUUAAGUGGGUUGCUUC | GAAGCAACCCACUUAAGCC | MK | [3031-3049] 3'UTR |
| 117 | GGAGGAGAGGGAGAAUUCU | AGAAUUCUCCCUCUCCUCC | | [2596-2614] 3'UTR |
| 118 | GCUGCUGGAUGAGAAGUUC | GAACUUCUCAUCCAGCAGC | MK,MO | [1436-1454] ORF |
| 119 | CGCUGAGGUUUUGUUUCAU | AUGAAACAAAACCUCAGCG | MK | [3466-3484] 3'UTR |
| 120 | GGGCACUGAAGAAAGGCAA | UUGCCUUUCUUCAGUGCCC | MK | [3165-3183] 3'UTR |
| 121 | CCCAAAAAGUUGAGCCUUU | AAAGGCUCAACUUUUUGGG | | [2693-2711] 3'UTR |
| 122 | GCUUAAUGGUGUGAGGCGU | ACGCCUCACACCAUUAAGC | MK | [2405-2423] 3'UTR |
| 123 | CCCUUUUCUCCAUGCUUAA | UUAAGCAUGGAGAAAAGGG | | [2392-2410] 3'UTR |
| 124 | UCCCUUUUCUCCAUGCUUA | UAAGCAUGGAGAAAAGGGA | | [2391-2409] 3'UTR |
| 125 | UGCCCUUUUUCCCUUUUCU | AGAAAAGGGAAAAAGGGCA | | [2382-2400] 3'UTR |
| 126 | UGACUUCCUUUGCCCUUUU | AAAAGGGCAAAGGAAGUCA | MK | [2372-2390] 3'UTR |
| 127 | ACCCUCCCUUUUCCUCACU | AGUGAGGAAAAGGGAGGGU | MK | [1937-1955] 3'UTR |
| 128 | GGAACCUCAAGGUCUAUAU | AUAUAGACCUUGAGGUUCC | MK | [1609-1627] ORF |
| 129 | GCACUGAAGAAAGGCAAGG | CCUUGCCUUUCUUCAGUGC | MK | [3167-3165] 3'UTR |
| 130 | GCCCUUUUUCCCUUUUCUC | GAGAAAAGGGAAAAAGGGC | | [2383-2401] 3'UTR |
| 131 | CCCUGUCAUUUCCAGAUUU | AAAUCUGGAAAUGACAGGG | MK | [2222-2240] 3'UTR |
| 132 | GACAGUUCAUUGCACUGAC | GUCAGUGCAAUGAACUGUC | MK | [1962-1980] 3'UTR |
| 133 | CAGUGUUCAAGUGCAGAAA | UUUCUGCACUUGAACACUG | MK | [3403-3421] 3'UTR |
| 134 | UGCGCAAACUCAGCGAGAU | AUCUCGCUGAGUUUGCGCA | MK | [820-838] ORF |
| 135 | AGAAGGUCCACGUGAACUU | AAGUUCACGUGGACCUUCU | | [1705-1723] ORF |
| 136 | GUGUUCAAGUGCAGAAAUC | GAUUUCUGCACUUGAACAC | MK | [3405-3423] 3'UTR |
| 137 | GGGAGUGGUAUCCAGUGUU | AACACUGGAUACCACUCCC | MK | [3391-3409] 3'UTR |
| 138 | CCAAAAAGUUGAGCCUUUC | GAAAGGCUCAACUUUUUGG | MK | [2694-2712] 3'UTR |
| 139 | ACCCAUCAAUGUGAAAGUC. | GACUUUCACAUUGAUGGGU | MK | [2263-2281] 3'UTR |
| 140 | ACAGUUCAUUGCACUGACU | AGUCAGUGCAAUGAACUGU | MK | [1963-1981] 3'UTR |
| 141 | GGACAGUUCAUUGCACUGA | UCAGUGCAAUGAACUGUCC | MK | [1961-1979] 3'UTR |
| 142 | CCCUUUUCCUCACUGGAAU | AUUCCAGUGAGGAAAAGGG | MK | [1942-1960] 3'UTR |
| 143 | CCCGAAGAAUGUACAACCU | AGGUUGUACAUUCUUCGGG | MK | [1660-1678] ORF |
| 144 | AGGUCUAUAUCAAGACAGU | ACUGUCUUGAUAUAGACCU | MK | [1618-1636] ORF |
| 145 | CUGACUACUUUACCAGCUU | AAGCUGGUAAAGUAGUCAG | MK | [613-631] ORF |
| 146 | CCAGGAUUAUACCUGGGAA | UUCCCAGGUAUAAUCCUGG | MK,MO | [1370-1388] ORF |
| 147 | CCCAGACAUGCUGUGCUUU | AAAGCACAGCAUGUCUGGG | MK | [1280-1298] ORF |
| 148 | GGCCAGUGUGCCACAUUAA | UUAAUGUGGCACACUGGCC | MK | [3222-3240] 3'UTR |
| 149 | CAGGCACUUUCUGUAGCAA | UUGCUACAGAAAGUGCCUG | MK | [3077-3095] 3'UTR |
| 150 | AGGUGACCAUGGCUACAUU | AAUGUAGCCAUGGUCACCU | MK | [2799-2817] 3'UTR |
| 151 | UUGACUUCCUUUGCCCUUU | AAAGGGCAAAGGAAGUCAA | MK | [2371-2389] 3'UTR |
| 152 | UGACACUCCAGGCAGCUUU | AAAGCUGCCUGGAGUGUCA | MK | [2193-2211] 3'UTR |
| 153 | CGAAGAAUGUACAACCUCU | AGAGGUUGUACAUUCUUCG | MK | [1662-1680] ORF |
| 154 | CGGAACCUCAAGGUCUAUA | UAUAGACCUUGAGGUUCCG | MK | [1608-1626] ORF |
| 155 | GCGUCUUUGGCAUCAGAUA | UAUCUGAUGCCAAAGACGC | MK,MO | [1549-1567] ORF |
| 156 | CCUACAAUACCAUCGCCAU | AUGGCGAUGGUAUUGUAGG | MK | [1474-1492] ORF |
| 157 | GUGCAGAAAUCUUUGGCUU | AAGCCAAAGAUUUCUGCAC | MK | [3413-3431] 3'UTR |
| 158 | AGGGAGUGGUAUCCAGUGU | ACACUGGAUACCACUCCCU | MK | [3390-3408] 3'UTR |
| 159 | CACAUUAAAUACCCGUGCA | UGCACGGGUAUUUAAUGUG | MK | [3233-3251] 3'UTR |
| 160 | CCACAUUAAAUACCCGUGC | GCACGGGUAUUUAAUGUGG | MK | [3232-3250] 3'UTR |
| 161 | AGGCACUUUCUGUAGCAAA | UUUGCUACAGAAAGUGCCU | MK | [3078-3096] 3'UTR |
| 162 | CCGAAUCCUAGUUCAGUUU | AAACUGAACUAGGAUUCGG | MK | [2351-2369] 3'UTR |
| 163 | CCCGAAUCCUAGUUCAGUU | AACUGAACUAGGAUUCGGG | MK | [2350-2368] 3'UTR |
| 164 | CCUAAAAGCCUGUUCUGUU | AACAGAACAGGCUUUUAGG | MK | [2312-2330] 3'UTR |
| 165 | CCCUAAAAGCCUGUUCUGU | ACAGAACAGGCUUUUAGGG | MK | [2311-2329] 3'UTR |
| 166 | ACGUGAACUUGCUGCUCCU | AGGAGCAGCAAGUUCACGU | | [1714-1732] ORF |
| 167 | GAAGAAUGUACAACCUCUU | AAGAGGUUGUACAUUCUUC | MK | [1663-1681] ORF |
| 168 | GGAACUAUAUCCACUGCGU | ACGCAGUGGAUAUAGUUCC | MK | [1534-1552] ORF |
| 169 | CCAUCUGGAACUAUAUCCA | UGGAUAUAGUUCCAGAUGG | MK | [1528-1546] ORF |
| 170 | ACCUACAAUACCAUCGCCA | UGGCGAUGGUAUUGUAGGU | MK | [1473-1491] ORF |
| 171 | UUGUGGAAGACCCUACUUU | AAAGUAGGGUCUUCCACAA | MK | [1297-1315] ORF |
| 172 | UCGCUGAGGUUUUGUUUCA | UGAAACAAAACCUCAGCGA | MK | [3465-3483] 3'UTR |
| 173 | GGCUUUGCUACCAGUUCCA | UGGAACUGGUAGCAAAGCC | MK | [3427-3445] 3'UTR |
| 174 | AGUGUUCAAGUGCAGAAAU | AUUUCUGCACUUGAACACU | MK | [3404-3422] 3'UTR |
| 175 | GCCACAUUAAAUACCCGUG | CACGGGUAUUUAAUGUGGC | MK | [3231-3249] 3'UTR |
| 176 | UGGCCAGUGUGCCACAUUA | UAAUGUGGCACACUGGCCA | MK | [3221-3239] 3'UTR |
| 177 | GCACUUUCUGUAGCAAAUG | CAUUUGCUACAGAAAGUGC | MK | [3080-3098] 3'UTR |
| 178 | CAGGUGACCAUGGCUACAU | AUGUAGCCAUGGUCACCUG | MK | [2798-2816] 3'UTR |
| 179 | GCGAGAUCAACAAGUUGCU | AGCAACUUGUUGAUCUCGC | MK | [832-850] ORF |
| 180 | GCCUUUGCUGGUCACAUUG | CAAUGUGACCAGCAAAGGC | MK | [2482-2500] 3'UTR |
| 181 | CACCCAUCAAUGUGAAAGU | ACUUUCACAUUGAUGGGUG | MK,MO | [2262-2280] 3'UTR |
| 182 | GCCAUUCACCCAUCAAUGU | ACAUUGAUGGGUGAAUGGC | MK | [2256-2274] 3'UTR |
| 183 | CCUGUCAUUUCCAGAUUUC | GAAAUCUGGAAAUGACAGG | MK | [2223-2241] 3'UTR |
| 184 | CCACAAGUGUUGUGGCCUU | AAGGCCACAACACUUGUGG | MK | [2116-2134] 3'UTR |
| 185 | CCACCCGAAGAAUGUACAA | UUGUACAUUCUUCGGGUGG | MK | [1657-1675] ORF |
| 186 | GCAUCAGAUAUGAUGACUA | UAGUCAUCAUAUCUGAUGC | MK | [1558-1576] ORF |
| 187 | GGAAGACCCUACUUUCGGA | UCCGAAAGUAGGGUCUUCC | MK | [1301-1319] ORF |
| 188 | GGAAGAGUCCAGCACCAAA | UUUGGUGCUGGACUCUUCC | | [171-189] 5'UTR |
| 189 | UGACUGUAAAUGUUCACUG | CAGUGAACAUUUACAGUCA | | [3364-3382] 3'UTR |
| 190 | CAAGCGGAUUUUCUUGCAA | UUGCAAGAAAAUCCGCUUG | MK | [2919-2937] 3'UTR |
| 191 | GCAAGCGGAUUUUCUUGCA | UGCAAGAAAAUCCGCUUGC | MK | [2918-2936] 3'UTR |
| 192 | UGGCUACAUUGCCAAACCU | AGGUUUGGCAAUGUAGCCA | MK | [2808-2826] 3'UTR |
| 193 | AGCCUUUCUAGAUGGCUUA | UAAGCCAUCUAGAAAGGCU | | [2705-2723] 3'UTR |
| 194 | CUCCCAAAAAGUUGAGCCU | AGGCUCAACUUUUUGGGAG | | [2691-2709] 3'UTR |
| 195 | AGAUGUCUCCCAAAAAGUU | AACUUUUUGGGAGACAUCU | | [2685-2703] 3'UTR |
| 196 | GUCCUUUAUGCUUGAGGUU | AACCUCAAGCAUAAAGGAC | MK | [2548-2566] 3'UTR |
| 197 | GAUUAUCUCUAGAGAGUUU | AAACUCUCUAGAGAUAAUC | MK | [2461-2479] 3'UTR |
| 198 | CCAUUCACCCAUCAAUGUG | CACAUUGAUGGGUGAAUGG | MK | [2257-2275] 3'UTR |
| 199 | CACUGACUCUGGGAUCUCA | UGAGAUCCCAGAGUCAGUG | MK | [1974-1992] 3'UTR |
| 200 | ACAAGGACUUCUCUGUCUG | CAGACAGAGAAGUCCUUGU | MK | [1832-1850] 3'UTR |
| 201 | AGGUCCACGUGAACUUGCU | AGCAAGUUCACGUGGACCU | | [1708-1726] ORF |
| 202 | ACCUCAAGGUCUAUAUCAA | UUGAUAUAGACCUUGAGGU | MK | [1612-1630] ORF |
| 203 | GACUACUUUACCAGCUUCU | AGAAGCUGGUAAAGUAGUC | MK | [615-633] ORF |
| 204 | GAAGACCCUACUUUCGGAU | AUCCGAAAGUAGGGUCUUC | MK | [1302-1320] ORF |
| 205 | ACCCAGACAUGCUGUGCUU | AAGCACAGCAUGUCUGGGU | MK | [1279-1297] ORF |
| 206 | CACCAAGGAACACAUCCAG | CUGGAUGUGUUCCUUGGUG | MK | [872-890] ORF |
| 207 | GAGCCUUUCUAGAUGGCUU | AAGCCAUCUAGAAAGGCUC | | [2704-2722] 3'UTR |
| 208 | AAAAAGUUGAGCCUUUCUA | UAGAAAGGCUCAACUUUUU | MK | [2696-2714] 3'UTR |
| 209 | GAGGAGGAGAGGGAGAAUU | AAUUCUCCCUCUCCUCCUC | | [2594-2612] 3'UTR |
| 210 | CUGGUCACAUUGCCUUCUG | CAGAAGGCAAUGUGACCAG | MK | [2489-2507] 3'UTR |
| 211 | AGAUUAUCUCUAGAGAGUU | AACUCUCUAGAGAUAAUCU | MK | [2460-2478] 3'UTR |
| 212 | AGUUCAGUUUUUUGACUUC | GAAGUCAAAAAACUGAACU | MK | [2360-2378] 3'UTR |
| 213 | GUGUCCAGUUCCCUAAAAG | CUUUUAGGGAACUGGACAC | MK | [2301-2319] 3'UTR |
| 214 | CCCAUCAAUGUGAAAGUCA | UGACUUUCACAUUGAUGGG | MK | [2264-2282] 3'UTR |
| 215 | UCACCCAUCAAUGUGAAAG | CUUUCACAUUGAUGGGUGA | MK | [2261-2279] 3'UTR |
| 216 | UCAGUGUUCUUACCUGGUA | UACCAGGUAAGAACACUGA | MK | [722-740] ORF |
| 217 | GAGCACUUGGAGAUCCUAA | UUAGGAUCUCCAAGUGCUC | | [2015-2033] 3'UTR |
| 218 | AGAGCACUUGGAGAUCCUA | UAGGAUCUCCAAGUGCUCU | | [2014-2032] 3'UTR |
| 219 | CCCGCCAUCAGUGUUCUUA | UAAGAACACUGAUGGCGGG | MK | [715-733] ORF |
| 220 | GGACUUCUCUGUCUGGAGA | UCUCCAGACAGAGAAGUCC | MK | [1836-1854] 3'UTR |
| 221 | CCCACAAGGACUUCUCUGU | ACAGAGAAGUCCUUGUGGG | MK | [1829-1847] 3'UTR |
| 222 | AGAAUGUACAACCUCUUCU | AGAAGAGGUUGUACAUUCU | MK | [1665-1683] ORF |
| 223 | CCGAAGAAUGUACAACCUC | GAGGUUGUACAUUCUUCGG | MK | [1661-1679] ORF |
| 224 | CAAGGUCUAUAUCAAGACA | UGUCUUGAUAUAGACCUUG | MK | [1616-1634] ORF |
| 225 | GCGGAACCUCAAGGUCUAU | AUAGACCUUGAGGUUCCGC | MK | [1607-1625] ORF |
| 226 | AGCGGAACCUCAAGGUCUA | UAGACCUUGAGGUUCCGCU | MK | [1606-1624] ORF |
| 227 | CGUCUUUGGCAUCAGAUAU | AUAUCUGAUGCCAAAGACG | MK | [1550-1568] ORF |
| 228 | GACCCUACUUUCGGAUAUG | CAUAUCCGAAAGUAGGGUC | MK | [1305-1323] ORF |
| 229 | CUGUAAAUGUUCACUGGGU | ACCCAGUGAACAUUUACAG | | [3367-3385] 3'UTR |
| 230 | CUGUGAAUUACGACUUCUC | GAGAAGUCGUAAUUCACAG | | [3100-3118] 3'UTR |
| 231 | CCCAGUAUCUCAUCUGUCC | GGACAGAUGAGAUACUGGG | MK | [3002-3020] 3'UTR |
| 232 | GGCUACAUUGCCAAACCUC | GAGGUUUGGCAAUGUAGCC | MK | [2809-2827] 3'UTR |
| 233 | UCAAGGUCUAUAUCAAGAC | GUCUUGAUAUAGACCUUGA | MK | [1615-1633] ORF |
| 234 | CGCCACUACAUUGCCAUCA | UGAUGGCAAUGUAGUGGCG | MK,MO | [687-705] ORF |
| 235 | CCCAGGAUUAUACCUGGGA | UCCCAGGUAUAAUCCUGGG | MK,MO | [1369-1387] ORF |
| 236 | ACCCUACUUUCGGAUAUGA | UCAUAUCCGAAAGUAGGGU | MK | [1306-1324] ORF |
| 237 | GAGCUGGAGAAGUCAGAGA | UCUCUGACUUCUCCAGCUC | | [1215-1233] ORF |
| 238 | AGGAACACAUCCAGGCCUU | AAGGCCUGGAUGUGUUCCU | MK | [877-895] ORF |
| 239 | UGCCACAUUAAAUACCCGU | ACGGGUAUUUAAUGUGGCA | MK | [3230-248] 3'UTR |
| 240 | GUGAAUUACGACUUCUCUU | AAGAGAAGUCGUAAUUCAC | | [3102-3120] 3'UTR |
| 241 | CCAUUUCUGCAGCCAGUGU | ACACUGGCUGCAGAAAUGG | | [2949-2967] 3'UTR |
| 242 | GGGUUCUUGUUUGGACCCU | AGGGUCCAAACAAGAACCC | MK | [2763-2781] 3'UTR |
| 243 | CAAAAAGUUGAGCCUUUCU | AGAAAGGCUCAACUUUUUG | MK | [2695-2713] 3'UTR |
| 244 | GGAGAAUUCUGUUCUCCCA | UGGGAGAACAGAAUUCUCC | | [2605-2623] 3'UTR |
| 245 | GCUGGUCACAUUGCCUUCU | AGAAGGCAAUGUGACCAGC | MK | [2486-2506] 3'UTR |
| 246 | UCAAGGACUACCUGCGGUU | AACCGCAGGUAGUCCUUGA | | [388-406] ORF |
| 247 | CUAGUUCAGUUUUUUGACU | AGUCAAAAAACUGAACUAG | MK | [2358-2376] 3'UTR |
| 248 | UGGUCUGUGUGUCCAGUUC | GAACUGGACACACAGACCA | MK | [2293-2311] 3'UTR |
| 249 | CCAUCAGUGUUCUUACCUG | CAGGUAAGAACACUGAUGG | MK | [719-737] ORF |
| 250 | AGACCCUUUUGUGUCCCAU | AUGGGACACAAAAGGGUCU | | [1862-1880] 3'UTR |
| 251 | AGAAGACCACCCGAAGAAU | AUUCUUCGGGUGGUCUUCU | MK | [1651-1669] ORF |
| 252 | GCCAUCUGGAACUAUAUCC | GGAUAUAGUUCCAGAUGGC | MK | [1527-1545] ORF |
| 253 | AGGGACCCGUUGAACAACU | AGUUGUUCAACGGGUCCCU | MK | [1080-1098] ORF |
| 254 | UACGACUUCUCUUGCCCUU | AAGGGCAAGAGAAGUCGUA | | [3108-3126] 3'UTR |
| 255 | GGCUCAUCACCAAGGAACA | UGUUCCUUGGUGAUGAGCC | MK | [865-883] ORF |
| 256 | ACAUUGCCAAACCUCUGAC | GUCAGAGGUUUGGCAAUGU | MK | [2813-2831] 3'UTR |
| 257 | ACCAUGGCUACAUUGCCAA | UUGGCAAUGUAGCCAUGGU | MK | [2804-2822] 3'UTR |
| 258 | GCCUUUCUAGAUGGCUUAG | CUAAGCCAUCUAGAAAGGC | | [2706-2724] 3'UTR |
| 259 | UCCCAAAAAGUUGAGCCUU | AAGGCUCAACUUUUUGGGA | | [2692-2710] 3'UTR |
| 260 | GGUCCUUUAUGCUUGAGGU | ACCUCAAGCAUAAAGGACC | MK | [2547-2565] 3'UTR |
| 261 | GAGAGUUUGAGCCUUUGCU | AGCAAAGGCUCAAACUCUC | MK | [2472-2490] 3'UTR |
| 262 | UUCAGUUUUUUGACUUCCU | AGGAAGUCAAAAAACUGAA | MK | [2362-2380] 3'UTR |
| 263 | CCAGUUCCCUAAAAGCCUG | CAGGCUUUUAGGGAACUGG | MK | [2305-2323] 3'UTR |
| 264 | UGUGUCCAGUUCCCUAAAA | UUUUAGGGAACUGGACACA | MK | [2300-2318] 3'UTR |
| 265 | AGCUGGAAGAGCACUUGGA | UCCAAGUGCUCUUCCAGCU | | [2007-2025] 3'UTR |
| 266 | UCCCUUUUCCUCACUGGAA | UUCCAGUGAGGAAAAGGGA | MK | [1941-1959] 3'UTR |
| 267 | CCACAAGGACUUCUCUGUC | GACAGAGAAGUCCUUGUGG | MK | [1830-1848] 3'UTR |
| 268 | ACAACCUCUUCUGGAGGCA | UGCCUCCAGAAGAGGUUGU | MK | [1672-1690] ORF |
| 269 | CAUCAGAUAUGAUGACUAU | AUAGUCAUCAUAUCUGAUG | MK | [1559-1577] ORF |
| 270 | GGCAUCAGAUAUGAUGACU | AGUCAUCAUAUCUGAUGCC | MK | [1557-1575] ORF |
| 271 | CACUGCGUCUUUGGCAUCA | UGAUGCCAAAGACGCAGUG | MK,MO | [1545-1563] ORF |
| 272 | CAGGGCCAUCUGGAACUAU | AUAGUUCCAGAUGGCCCUG | MK | [1523-1541] ORF |
| 273 | UGACUACUUUACCAGCUUC | GAAGCUGGUAAAGUAGUCA | MK | [614-632] ORF |
| 274 | CCUGACUACUUUACCAGCU | AGCUGGUAAAGUAGUCAGG | MK | [612-630] ORF |
| 275 | ACCAUGGCUACUCGCUGAU | AUCAGCGAGUAGCCAUGGU | MK,Rat,MO | [1390-1408] ORF |
| 276 | CAGGAUUAUACCUGGGAAG | CUUCCCAGGUAUAAUCCUG | MK,Rat,MO | [1371-1389] ORF |
| 277 | CCCUACUUUCGGAUAUGAG | CUCAUAUCCGAAAGUAGGG | MK | [1307-1325] ORF |
| 278 | UCACCAAGGAACACAUCCA | UGGAUGUGUUCCUUGGUGA | MK | [871-889] ORF |
| 279 | AGAAUUCUGUUCUCCCAGA | UCUGGGAGAACAGAAUUCU | | [2607-2625] 3'UTR |
| 280 | CUCCAUGCUUAAUGGUGUG | CACACCAUUAAGCAUGGAG | MK | [2399-2417] 3'UTR |
| 281 | UGCACUGACUCUGGGAUCU | AGAUCCCAGAGUCAGUGCA | MK | [1972-1990] 3'UTR |
| 282 | UGAACUUGCUGCUCCUGGA | UCCAGGAGCAGCAAGUUCA | MK | [1717-1735] ORF |
| 283 | CGGAUAUGAGGACUUCACU | AGUGAAGUCCUCAUAUCCG | MK | [1316-1334] ORF |
| 284 | UCGGAUAUGAGGACUUCAC | GUGAAGUCCUCAUAUCCGA | MK | [1315-1333] ORF |
| 285 | GCUUUGUGGAAGACCCUAC | GUAGGGUCUUCCACAAAGC | MK | [1294-1312] ORF |
| 286 | GGACCCGUUGAACAACUCU | AGAGUUGUUCAACGGGUCC | MK | [1082-1100] ORF |
| 287 | UCCUUCGUGUUUGGCUGUG | CACAGCCAAACACGAAGGA | MK | [978-996] ORF |
| 288 | CAGUUUGGAGGGCACUGAA | UUCAGUGCCCUCCAAACUG | MK | [3156-3174] 3'UTR |
| 289 | UGGCUCAUCACCAAGGAAC | GUUCCUUGGUGAUGAGCCA | MK | [864-882] ORF |
| 290 | ACCGAUUCCAGGCACUUUC | GAAAGUGCCUGGAAUCGGU | MK | [30693087] 3'UTR |
| 291 | CAACAAGUUGCUGGCGCAU | AUGCGCCAGCAACUUGUUG | MK | [839-857] ORF |
| 292 | UCAACAAGUUGCUGGCGCA | UGCGCCAGCAACUUGUUGA | MK | [838-856] ORF |
| 293 | CCAUGGCUACAUUGCCAAA | UUUGGCAAUGUAGCCAUGG | MK | [2805-2823] 3'UTR |
| 294 | GAAUUCUGUUCUCCCAGAG | CUCUGGGAGAACAGAAUUC | | [2608-2626] 3'UTR |
| 295 | UGCUUGAGGUUCCAACCUG | CAGGUUGGAACCUCAAGCA | MK | [2556-2574] 3'UTR |
| 296 | GAGCCUUUGCUGGUCACAU | AUGUGACCAGCAAAGGCUC | MK | [2480-2498] 3'UTR |
| 297 | CAGAUUAUCUCUAGAGAGU | ACUCUCUAGAGAUAAUCUG | MK | [2459-2477] 3'UTR |
| 298 | GCAGAUUAUCUCUAGAGAG | CUCUCUAGAGAUAAUCUGC | MK | [2458-2476] 3'UTR |
| 299 | CCAUGCUUAAUGGUGUGAG | CUCACACCAUUAAGCAUGG | MK | [2401-2419] 3'UTR |
| 300 | GCCCGAAUCCUAGUUCAGU | ACUGAACUAGGAUUCGGGC | MK | [2349-2367] 3'UTR |
| 301 | CAAGUGUUGUGGCCUUCCU | AGGAAGGCCACAACACUUG | MK | [2119-2137] 3'UTR |
| 302 | UCACUGGAAUGGACAGUUC | GAACUGUCCAUUCCAGUGA | MK | [1951-1969] 3'UTR |
| 303 | GUCUUUGGCAUCAGAUAUG | CAUAUCUGAUGCCAAAGAC | MK | [1551-1569] ORF |
| 304 | UGCGUCUUUGGCAUCAGAU | AUCUGAUGCCAAAGACGCA | MK,MO | [1548-1566] ORF |
| 305 | CUGCGUCUUUGGCAUCAGA | UCUGAUGCCAAAGACGCAG | MK,MO | [1547-1565] ORF |
| 306 | GCCUAUAGCCUCACCUACA | UGUAGGUGAGGCUAUAGGC | MK | [1461-1479] ORF |
| 307 | ACCCUGACUACUUUACCAG | CUGGUAAAGUAGUCAGGGU | MK | [610-628] ORF |
| 308 | GGAUAUGAGGACUUCACUC | GAGUGAAGUCCUCAUAUCC | MK | [1317-1335] ORF |
| 309 | CGCUUUGAGCUGGAGAAGU | ACUUCUCCAGCUCAAAGCG | MK | [1209-1227] ORF |
| 310 | UGCAGAAAUCUUUGGCUUU | AAAGCCAAAGAUUUCUGCA | MK | [3414-3432] 3'UTR |
| 311 | GCCCUUCUUCUAGCAGUCU | AGACUGCUAGAAGAAGGGC | MK | [3121-3139] 3'UTR |
| 312 | CGAUUCCAGGCACUUUCUG | CAGAAAGUGCCUGGAAUCG | MK | [3071-3089] 3'UTR |
| 313 | GACCAUGGCUACAUUGCCA | UGGCAAUGUAGCCAUGGUC | MK | [2803-2821] 3'UTR |
| 314 | GGUUCUUGUUUGGACCCUG | CAGGGUCCAAACAAGAACC | MK | [2764-2782] 3'UTR |
| 315 | UGGUCACAUUGCCUUCUGA | UCAGAAGGCAAUGUGACCA | MK | [2490-2508] 3'UTR |
| 316 | GUGUGUCCAGUUCCCUAAA | UUUAGGGAACUGGACACAC | MK | [2299-2317] 3'UTR |
| 317 | UGUGUGUCCAGUUCCCUAA | UUAGGGAACUGGACACACA | MK | [2298-2316] 3'UTR |
| 318 | CAGUGUUCUUACCUGGUAG | CUACCAGGUAAGAACACUG | MK | [723-741] ORF |
| 319 | CAGAGCUCAAGGACUACCU | AGGUAGUCCUUGAGCUCUG | | [382-400] ORF |
| 320 | CAGACCCUUUUGUGUCCCA | UGGGACACAAAAGGGUCUG | | [1861-1879] 3'UTR |
| 321 | UGGCAUCAGAUAUGAUGAC | GUCAUCAUAUCUGAUGCCA | MK | [1556-1574] ORF |
| 322 | UCCACUGCGUCUUUGGCAU | AUGCCAAAGACGCAGUGGA | MK,MO | [1543-1561] ORF |
| 323 | CUAUAGCCUCACCUACAAU | AUUGUAGGUGAGGCUAUAG | MK | [1463-1481] ORF |
| 324 | AGAAGUUCCAGGCAGCCUA | UAGGCUGCCUGGAACUUCU | MK | [1447-1465] ORF |
| 325 | GACCGAUUCCAGGCACUUU | AAAGUGCCUGGAAUCGGUC | MK | [3068-3086] 3'UTR |
| 326 | CGAGAUCAACAAGUUGCUG | CAGCAACUUGUUGAUCUCG | MK | [633-851] ORF |
| 327 | GAGGGAGAAUUCUGUUCUC | GAGAACAGAAUUCUCCCUC | | [2602-2620] 3'UTR |
| 328 | CCUUCCUGAACUGGGAAGU | ACUUCCCAGUUCAGGAAGG | MK | [2131-2149] 3'UTR |
| 329 | CAGCCUAUAGCCUCACCUA | UAGGUGAGGCUAUAGGCUG | MK | [1459-1477] ORF |
| 330 | GAAGUUCCAGGCAGCCUAU | AUAGGCUGCCUGGAACUUC | MK | [1446-1466] ORF |
| 331 | GGAUGAGAAGUUCCAGGCA | UGCCUGGAACUUCUCAUCC | MK | [1442-1460] ORF |
| 332 | AGCUCAUUCAGGCUCUGGU | ACCAGAGCCUGAAUGAGCU | MK | [931-949] ORF |
| 333 | UGCCCUUCUUCUAGCAGUC | GACUGCUAGAAGAAGGGCA | MK | [3120-3138] 3'UTR |
| 334 | CGCAAACUCAGCGAGAUCA | UGAUCUCGCUGAGUUUGCG | MK | [822-840] ORF |
| 335 | CUGGUCUGUGUGUCCAGUU | AACUGGACACACAGACCAG | MK | [2292-2310] 3'UTR |
| 336 | AGGCAGCUUUGCCUUCUCU | AGAGAAGGCAAAGCUGCCU | MK | [2202-2220] 3'UTR |
| 337 | CACAAGUGUUGUGGCCUUC | GAAGGCCACAACACUUGUG | MK | [2117-2135] 3'UTR |
| 338 | UCCAUGCUUAAUGGUGUGA | UCACACCAUUAAGCAUGGA | MK | [2400-2418] 3'UTR |
| 339 | UGCUGGAUGAGAAGUUCCA | UGGAACUUCUCAUCCAGCA | MK,MO | [1438-1456] ORF |
| 340 | AGCACUUGGAGAUCCUAAG | CUUAGGAUCUCCAAGUGCU | | [2016-2034] 3'UTR |
| 341 | CUCACUGGAAUGGACAGUU | AACUGUCCAUUCCAGUGAG | MK | [1950-1968] 3'UTR |
| 342 | GCUGUGCUUUGUGGAAGAC | GUCUUCCACAAAGCACAGC | MK | [1289-1307] ORF |
| 343 | GGCACUGAAGAAAGGCAAG | CUUGCCUUUCUUCAGUGCC | MK | [3166-3184] 3'UTR |
| 344 | CCUCACUGGAAUGGACAGU | ACUGUCCAUUCCAGUGAGG | MK | [1949-1967] 3'UTR |

**Table N: 21-mer Sestrin2 (SESN2, Hi95)**

| Number | Sense siRNA | AntiSense siRNA | Other Sp | Human-32454742 ORF:354-1796 |
|---|---|---|---|---|
| 1 | GGAGGGAGUAUUAGAUUAUAA | UUAUAAUCUAAUACUCCCUCC | MK | [2512-2532] 3'UTR |
| 2 | AGAUUUCAUUACCUCCUACUU | AAGUAGGAGGUAAUGAAAUCU | MK | [2235-2255] 3'UTR |
| 3 | GGAAUUAAUGUGCCACAAGUG | CACUUGUGGCACAUUAAUUCC | | [2104-2124] 3'UTR |
| 4 | CCAGUUCCAUAUGAUGAGAAA | UUUCUCAUCAUAUGGAACUGG | MK | [3437-3457] 3'UTR |
| 5 | CCAGUGUGCCACAUUAAAUAC | GUAUUUAAUGUGGCACACUGG | MK | [3224-3244] 3'UTR |
| 6 | GGGCAUGUGAUGACUGUAAAU | AUUUACAGUCAUCACAUGCCC | | [3354-3374] 3'UTR |
| 7 | GCCAGUGUGCCACAUUAAAUA | UAUUUAAUGUGGCACACUGGC | MK | [3223-3243] 3'UTR |
| 8 | GCCGCAGAUGUCUCCCAAAAA | UUUUUGGGAGACAUCUGCGGC | | [2680-2700] 3'UTR |
| 9 | CCUCUUUAUUUUGGUCCUUUA | UAAAGGACCAAAAUAAAGAGG | MK | [2535-2555] 3'UTR |
| 10 | CGAAUCCUAGUUCAGUUUUUU | AAAAAACUGAACUAGGAUUCG | MK | [2352-2372] 3'UTR |
| 11 | CUACAUGACCUGACUCCUGAG | CUCAGGAGUCAGGUCAUGUAG | MK | [1784-1804] ORF+3'UTR |
| 12 | GAGGAGGGAGUAUUAGAUUAU | AUAAUCUAAUACUCCCUCCUC | MK | [2510-2530] 3'UTR |
| 13 | CCUUUGCCCUUUUUCCCUUUU | AAAAGGGAAAAAGGGCAAAGG | | [2378-2398] 3'UTR |
| 14 | CUACUUGCCAUUCACCCAUCA | UGAUGGGUGAAUGGCAAGUAG | MK | [2250-2270] 3'UTR |
| 15 | GCAGAAAUCUUUGGCUUUGCU | AGCAAAGCCAAAGAUUUCUGC | MK | [3415-3435] 3'UTR |
| 16 | GUAGCAAAUGACUGUGAAUUA | UAAUUCACAGUCAUUUGCUAC | MK | [3089-3109] 3'UTR |
| 17 | UCCUUUGCCCUUUUUCCCUUU | AAAGGGAAAAAGGGCAAAGGA | | [2377-2397] 3'UTR |
| 18 | CGGAAUUAAUGUGCCACAAGU | ACUUGUGGCACAUUAAUUCCG | | [2103-2123] 3'UTR |
| 19 | CCAUAUGAUGAGAAAUAAACG | CGUUUAUUUCUCAUCAUAUGG | MK | [3443-3463] 3'UTR |
| 20 | CAGUUCCAUAUGAUGAGAAAU | AUUUCUCAUCAUAUGGAACUG | MK | [3438-3458] 3'UTR |
| 21 | GCAUGUGAUGACUGUAAAUGU | ACAUUUACAGUCAUCACAUGC | | [3356-3376] 3'UTR |
| 22 | CUGUAGCAAAUGACUGUGAAU | AUUCACAGUCAUUUGCUACAG | MK | [3087-3107] 3'UTR |
| 23 | GCGGAUUUUCUUGCAAGAUCA | UGAUCUUGCAAGAAAAUCCGC | MK | [2922-2942] 3'UTR |
| 24 | GCUCAUCACCAAGGAACACAU | AUGUGUUCCUUGGUGAUGAGC | MK | [866-886] ORF |
| 25 | AGGAGGGAGUAUUAGAUUAUA | UAUAAUCUAAUACUCCCUCCU | MK | [2511-2531] 3'UTR |
| 26 | UACUUGCCAUUCACCCAUCAA | UUGAUGGGUGAAUGGCAAGUA | MK | [2251-2271] 3'UTR |
| 27 | CCUACUUGCCAUUCACCCAUC | GAUGGGUGAAUGGCAAGUAGG | MK | [2249-2269] 3'UTR |
| 28 | CAUUACCUCCUACUUGCCAUU | AAUGGCAAGUAGGAGGUAAUG | MK | [2241-2261] 3'UTR |
| 29 | UCAUUACCUCCUACUUGCCAU | AUGGCAAGUAGGAGGUAAUGA | MK | [2240-2260] 3'UTR |
| 30 | UCAUUUCCAGAUUUCAUUACC | GGUAAUGAAAUCUGGAAAUGA | MK | [2227-2247] 3'UTR |
| 31 | UCGGAAUUAAUGUGCCACAAG | CUUGUGGCACAUUAAUUCCGA | | [2102-2122] 3'UTR |
| 32 | CUCGGAAUUAAUGUGCCACAA | UUGUGGCACAUUAAUUCCGAG | | [2101-2121] 3'UTR |
| 33 | UUGCUACCAGUUCCAUAUGAU | AUCAUAUGGAACUGGUAGCAA | MK | [3431-3451] 3'UTR |
| 34 | GCUUUGCUACCAGUUCCAUAU | AUAUGGAACUGGUAGCAAAGC | MK | [3428-3448] 3'UTR |
| 35 | CAGAAAUCUUUGGCUUUGCUA | UAGCAAAGCCAAAGAUUUCUG | MK | [3416-3436] 3'UTR |
| 36 | CGCAGAUGUCUCCCAAAAAGU | ACUUUUUGGGAGACAUCUGCG | | [2682-2702] 3'UTR |
| 37 | UCCUCUUUAUUUUGGUCCUUU | AAAGGACCAAAAUAAAGAGGA | MK | [2534-2554] 3'UTR |
| 38 | ACAUGACCUGACUCCUGAGCA | UGCUCAGGAGUCAGGUCAUGU | MK | [1786-1806] ORF+3'UTR |
| 39 | ACCAGUUCCAUAUGAUGAGAA | UUCUCAUCAUAUGGAACUGGU | MK | [3436-3456] 3'UTR |
| 40 | AGGGCAUGUGAUGACUGUAAA | UUUACAGUCAUCACAUGCCCU | | [3353-3373] 3'UTR |
| 41 | GGGUUCUAGAGGGCAUGUGAU | AUCACAUGCCCUCUAGAACCC | | [3344-3364] 3'UTR |
| 42 | GCAAAUGACUGUGAAUUACGA | UCGUAAUUCACAGUCAUUUGC | MK | [3092-3112] 3'UTR |
| 43 | CGGAUUUUCUUGCAAGAUCAG | CUGAUCUUGCAAGAAAAUCCG | MK | [2923-2943] 3'UTR |
| 44 | GCAGAUGUCUCCCAAAAAGUU | AACUUUUUGGGAGACAUCUGC | | [2683-2703] 3'UTR |
| 45 | CCUUUAUGCUUGAGGUUCCAA | UUGGAACCUCAAGCAUAAAGG | MK | [2550-2570] 3'UTR |
| 46 | CAAAUGACUGUGAAUUACGAC | GUCGUAAUUCACAGUCAUUUG | MK | [3093-3113] 3'UTR |
| 47 | UCUGUAGCAAAUGACUGUGAA | UUCACAGUCAUUUGCUACAGA | MK | [3086-3106] 3'UTR |
| 48 | CCUUUCUAGAUGGCUUAGGUG | CACCUAAGCCAUCUAGAAAGG | | [2707-2727] 3'UTR |
| 49 | AGAGGAGGGAGUAUUAGAUUA | UAAUCUAAUACUCCCUCCUCU | MK | [2509-2529] 3'UTR |
| 50 | GAAGAGGAGGGAGUAUUAGAU | AUCUAAUACUCCCUCCUCUUC | MK | [2507-2527] 3'UTR |
| 51 | GGCCAAGCUCGGAAUUAAUGU | ACAUUAAUUCCGAGCUUGGCC | | [2094-2114] 3'UTR |
| 52 | AACUAUAUCCACUGCGUCUUU | AAAGACGCAGUGGAUAUAGUU | MK | [1536-1556] ORF |
| 53 | GAACUAUAUCCACUGCGUCUU | AAGACGCAGUGGAUAUAGUUC | MK | [1535-1555] ORF |
| 54 | AGACAUGCUGUGCUUUGUGGA | UCCACAAAGCACAGCAUGUCU | MK | [1283-1303] ORF |
| 55 | UGGGUUCUAGAGGGCAUGUGA | UCACAUGCCCUCUAGAACCCA | | [3343-3363] 3'UTR |
| 56 | CAAGAAGGUGUGCAGGAGAGA | UCUCUCCUGCACACCUUCUUG | | [3301-3321] 3'UTR |
| 57 | UCGCUCUCCUCCUUCGUGUUU | AAACACGAAGGAGGAGAGCGA | MK | [969-989] ORF |
| 58 | CCUUUUUCCCUUUUCUCCAUG | CAUGGAGAAAAGGGAAAAAGG | | [2385-2405] 3'UTR |
| 59 | CCGCCAUCAGUGUUCUUACCU | AGGUAAGAACACUGAUGGCGG | MK | [716-736] ORF |
| 60 | GAAAUAAACGUUCGCUGAGGU | ACCUCAGCGAACGUUUAUUUC | MK | [3454-3474] 3'UTR |
| 61 | AGAGGGCAUGUGAUGACUGUA | UACAGUCAUCACAUGCCCUCU | | [3351-3371] 3'UTR |
| 62 | GAAGGUGUGCAGGAGAGAAGA | UCUUCUCUCCUGCACACCUUC | | [3304-3324] 3'UTR |
| 63 | CUCAUCACCAAGGAACACAUC | GAUGUGUUCCUUGGUGAUGAG | MK | [867-887] ORF |
| 64 | GCACCAGGGUUCUUGUUUGGA | UCCAAACAAGAACCCUGGUGC | MK | [2757-2777] 3'UTR |
| 65 | CCGCAGAUGUCUCCCAAAAAG | CUUUUUGGGAGACAUCUGCGG | | [2681-2701] 3'UTR |
| 66 | AUCCUCUUUAUUUUGGUCCUU | AAGGACCAAAAUAAAGAGGAU | MK | [2533-2553] 3'UTR |
| 67 | GCUCGGAAUUAAUGUGCCACA | UGUGGCACAUUAAUUCCGAGC | | [2100-2120] 3'UTR |
| 68 | CCAAGCUCGGAAUUAAUGUGC | GCACAUUAAUUCCGAGCUUGG | | [2096-2116] 3'UTR |
| 69 | GCCAAGCUCGGAAUUAAUGUG | CACAUUAAUUCCGAGCUUGGC | | [2095-2115] 3'UTR |
| 70 | GGGCCAAGCUCGGAAUUAAUG | CAUUAAUUCCGAGCUUGGCCC | | [2093-2113] 3'UTR |
| 71 | GGCUUGUGUGUGAUGUGCAGU | ACUGCACAUCACACACAAGCC | MK | [1905-1925] 3'UTR |
| 72 | UGAGAAAUAAACGUUCGCUGA | UCAGCGAACGUUUAUUUCUCA | MK | [3451-3471] 3'UTR |
| 73 | AAGCGGAUUUUCUUGCAAGAU | AUCUUGCAAGAAAAUCCGCUU | MK | [2920-2940] 3'UTR |
| 74 | CAACAGCAAGCGGAUUUUCUU | AAGAAAAUCCGCUUGCUGUUG | MK | [2913-2933] 3'UTR |
| 75 | CCAACAGCAAGCGGAUUUUCU | AGAAAAUCCGCUUGCUGUUGG | MK | [2912-2932] 3'UTR |
| 76 | UCAGUUUUUUGACUUCCUUUG | CAAAGGAAGUCAAAAAACUGA | MK | [2363-2383] 3'UTR |
| 77 | GAAUCCUAGUUCAGUUUUUUG | CAAAAAACUGAACUAGGAUUC | MK | [2353-2373] 3'UTR |
| 78 | GCAGCUGCUGGAUGAGAAGUU | AACUUCUCAUCCAGCAGCUGC | MK,Rat,MO | [1433-1453] ORF |
| 79 | ACGUUCGCUGAGGUUUUGUUU | AAACAAAACCUCAGCGAACGU | MK | [3461-3481] 3'UTR |
| 80 | AAAUAAACGUUCGCUGAGGUU | AACCUCAGCGAACGUUUAUUU | MK | [3455-3475] 3'UTR |
| 81 | GGCAUGUGAUGACUGUAAAUG | CAUUUACAGUCAUCACAUGCC | | [3355-3375] 3'UTR |
| 82 | CUAGAGGGCAUGUGAUGACUG | CAGUCAUCACAUGCCCUCUAG | | [3349-3369] 3'UTR |
| 83 | UGCCGCAGAUGUCUCCCAAAA | UUUUGGGAGACAUCUGCGGCA | | [2679-2699] 3'UTR |
| 84 | CAGUUUUUUGACUUCCUUUGC | GCAAAGGAAGUCAAAAAACUG | MK | [2364-2384] 3'UTR |
| 85 | UGGGCUUGUGUGUGAUGUGCA | UGCACAUCACACACAAGCCCA | MK | [1903-1923] 3'UTR |
| 86 | GUAAAUGUUCACUGGGUGGGU | ACCCACCCAGUGAACAUUUAC | | [3369-3389] 3'UTR |
| 87 | CCCUUCUUCUAGCAGUCUGUG | CACAGACUGCUAGAAGAAGGG | | [3122-3142] 3'UTR |
| 88 | GGAGAGGGAGAAUUCUGUUCU | AGAACAGAAUUCUCCCUCUCC | | [2599-2619] 3'UTR |
| 89 | CUCAGCGAGAUCAACAAGUUG | CAACUUGUUGAUCUCGCUGAG | MK | [828-848] ORF |
| 90 | CCAGAUUUCAUUACCUCCUAC | GUAGGAGGUAAUGAAAUCUGG | MK | [2233-2253] 3'UTR |
| 91 | CUCAGAGAAGGUCCACGUGAA | UUCACGUGGACCUUCUCUGAG | | [1700-1720] ORF |
| 92 | GUAUCCAGUGUUCAAGUGCAG | CUGCACUUGAACACUGGAUAC | MK | [3398-3418] 3'UTR |
| 93 | GAGGGCAUGUGAUGACUGUAA | UUACAGUCAUCACAUGCCCUC | | [3352-3372] 3'UTR |
| 94 | UCAAUGUGAAAGUCAGGGUCA | UGACCCUGACUUUCACAUUGA | MK | [2268-2288] 3'UTR |
| 95 | GAGUUUCUGCAGACUGGUGGU | ACCACCAGUCUGCAGAAACUC | MK | [756-776] ORF |
| 96 | CCAGUGUUCAAGUGCAGAAAU | AUUUCUGCACUUGAACACUGG | MK | [3402-3422] 3'UTR |
| 97 | GAGGAGAGGGAGAAUUCUGUU | AACAGAAUUCUCCCUCUCCUC | | [2597-2617] 3'UTR |
| 98 | UCCAGAUUUCAUUACCUCCUA | UAGGAGGUAAUGAAAUCUGGA | MK | [2232-2252] 3'UTR |
| 99 | CCCUUUUUCCCUUUUCUCCAU | AUGGAGAAAAGGGAAAAAGGG | | [2384-2404] 3'UTR |
| 100 | CCGAAGAAUGUACAACCUCUU | AAGAGGUUGUACAUUCUUCGG | MK | [1661-1681] ORF |
| 101 | CCAUUUCUGCAGCCAGUGUCU | AGACACUGGCUGCAGAAAUGG | | [2949-2969] 3'UTR |
| 102 | GGAGGAGAGGGAGAAUUCUGU | ACAGAAUUCUCCCUCUCCUCC | | [2596-2616] 3'UTR |
| 103 | GCAAACUCAGCGAGAUCAACA | UGUUGAUCUCGCUGAGUUUGC | MK | [823-843] ORF |
| 104 | ACACCCUCCCUUUUCCUCACU | AGUGAGGAAAAGGGAGGGUGU | MK | [1935-1955] 3'UTR |
| 105 | CAGCUGCUGGAUGAGAAGUUC | GAACUUCUCAUCCAGCAGCUG | MK,MO | [1434-1454] ORF |
| 106 | GCCACAUUAAAUACCCGUGCA | UGCACGGGUAUUUAAUGUGGC | MK | [3231-3251] 3'UTR |
| 107 | UGCUUAAUGGUGUGAGGCGUC | GACGCCUCACACCAUUAAGCA | MK | [2404-2424] 3'UTR |
| 108 | CAAACUCAGCGAGAUCAACAA | UUGUUGAUCUCGCUGAGUUUG | MK | [824-844] ORF |
| 109 | CCAUUCACCCAUCAAUGUGAA | UUCACAUUGAUGGGUGAAUGG | MK | [2257-2277] 3'UTR |
| 110 | UCUGAAGAGGAGGGAGUAUUA | UAAUACUCCCUCCUCUUCAGA | MK | [2504-2524] 3'UTR |
| 111 | GCGCCACUACAUUGCCAUCAU | AUGAUGGCAAUGUAGUGGCGC | MK,MO | [686-706] ORF |
| 112 | CCACAUUAAAUACCCGUGCAG | CUGCACGGGUAUUUAAUGUGG | MK | [3232-3252] 3'UTR |
| 113 | GCAGAUUAUCUCUAGAGAGUU | AACUCUCUAGAGAUAAUCUGC | MK | [2458-2478] 3'UTR |
| 114 | GCCCUUUUUCCCUUUUCUCCA | UGGAGAAAAGGGAAAAAGGGC | | [2383-2403] 3'UTR |
| 115 | GCCCGAAUCCUAGUUCAGUUU | AAACUGAACUAGGAUUCGGGC | MK | [2349-2369] 3'UTR |
| 116 | GCUGGAAGAGCACUUGGAGAU | AUCUCCAAGUGCUCUUCCAGC | | [2008-2028] 3'UTR |
| 117 | GGACAGUUCAUUGCACUGACU | AGUCAGUGCAAUGAACUGUCC | MK | [1961-1981] 3'UTR |
| 118 | CCCUGACUACUUUACCAGCUU | AAGCUGGUAAAGUAGUCAGGG | MK | [611-631] ORF |
| 119 | UCCAGUGUUCAAGUGCAGAAA | UUUCUGCACUUGAACACUGGA | MK | [3401-3421] 3'UTR |
| 120 | UUCCCUUUUCUCCAUGCUUAA | UUAAGCAUGGAGAAAAGGGAA | | [2390-2410] 3'UTR |
| 121 | UUGACUUCCUUUGCCCUUUUU | AAAAAGGGCAAAGGAAGUCAA | MK | [2371-2391] 3'UTR |
| 122 | CUGGAAGAGCACUUGGAGAUC | GAUCUCCAAGUGCUCUUCCAG | | [2009-2029] 3'UTR |
| 123 | CCAGACCCUUUUGUGUCCCAU | AUGGGACACAAAAGGGUCUGG | | [1860-1880] 3'UTR |
| 124 | GCGGAACCUCAAGGUCUAUAU | AUAUAGACCUUGAGGUUCCGC | MK | [1607-1627] ORF |
| 125 | CACCUACAAUACCAUCGCCAU | AUGGCGAUGGUAUUGUAGGUG | MK | [1472-1492] ORF |
| 126 | GCUUUGUGGAAGACCCUACUU | AAGUAGGGUCUUCCACAAAGC | MK | [1294-1314] ORF |
| 127 | GUGUUCAAGUGCAGAAAUCUU | AAGAUUUCUGCACUUGAACAC | MK | [3405-3425] 3'UTR |
| 128 | GCACUUUCUGUAGCAAAUGAC | GUCAUUUGCUACAGAAAGUGC | MK | [3080-3100] 3'UTR |
| 129 | UGAGCCUUUCUAGAUGGCUUA | UAAGCCAUCUAGAAAGGCUCA | | [2703-2723] 3'UTR |
| 130 | CCAAAAAGUUGAGCCUUUCUA | UAGAAAGGCUCAACUUUUUGG | MK | [2694-2714] 3'UTR |
| 131 | CCCAAAAAGUUGAGCCUUUCU | AGAAAGGCUCAACUUUUUGGG | | [2693-2713] 3'UTR |
| 132 | CCCUUUUCUCCAUGCUUAAUG | CAUUAAGCAUGGAGAAAAGGG | | [2392-2412] 3'UTR |
| 133 | GUUCAGUUUUUUGACUUCCUU | AAGGAAGUCAAAAAACUGAAC | MK | [2361-2381] 3'UTR |
| 134 | CCCUAAAAGCCUGUUCUGUUG | CAACAGAACAGGCUUUUAGGG | MK | [2311-2331] 3'UTR |
| 135 | UCCCUAAAAGCCUGUUCUGUU | AACAGAACAGGCUUUUAGGGA | MK | [2310-2330] 3'UTR |
| 136 | CAGAUUUCAUUACCUCCUACU | AGUAGGAGGUAAUGAAAUCUG | MK | [2234-2254] 3'UTR |
| 137 | CGGGCCAAGCUCGGAAUUAAU | AUUAAUUCCGAGCUUGGCCCG | | [2092-2112] 3'UTR |
| 138 | GCCUGCACCCUGACUACUUUA | UAAAGUAGUCAGGGUGCAGGC | MK | [604-624] ORF |
| 139 | CCACAAGGACUUCUCUGUCUG | CAGACAGAGAAGUCCUUGUGG | MK | [1830-1850] 3'UTR |
| 140 | GGCCAGUGUGCCACAUUAAAU | AUUUAAUGUGGCACACUGGCC | MK | [3222-3242] 3'UTR |
| 141 | ACGACUUCUCUUGCCCUUCUU | AAGAAGGGCAAGAGAAGUCGU | | [3109-3129] 3'UTR |
| 142 | UCCCUUUUCUCCAUGCUUAAU | AUUAAGCAUGGAGAAAAGGGA | | [2391-2411] 3'UTR |
| 143 | CACCCAUCAAUGUGAAAGUCA | UGACUUUCACAUUGAUGGGUG | MK | [2262-2282] 3'UTR |
| 144 | CAACCUCUUCUGGAGGCACUU | AAGUGCCUCCAGAAGAGGUUG | MK | [1673-1693] ORF |
| 145 | GACCACCCGAAGAAUGUACAA | UUGUACAUUCUUCGGGUGGUC | MK | [1655-1675] ORF |
| 146 | AGACCCUACUUUCGGAUAUGA | UCAUAUCCGAAAGUAGGGUCU | MK | [1304-1324] ORF |
| 147 | AGUGUUCAAGUGCAGAAAUCU | AGAUUUCUGCACUUGAACACU | MK | [3404-3424] 3'UTR |
| 148 | CCAGGCACUUUCUGUAGCAAA | UUUGCUACAGAAAGUGCCUGG | MK | [3076-3096] 3'UTR |
| 149 | UUUGACUUCCUUUGCCCUUUU | AAAAGGGCAAAGGAAGUCAAA | MK | [2370-2390] 3'UTR |
| 150 | CUAGUUCAGUUUUUUGACUUC | GAAGUCAAAAAACUGAACUAG | MK | [2358-2378] 3'UTR |
| 151 | CACCCGAAGAAUGUACAACCU | AGGUUGUACAUUCUUCGGGUG | MK | [1658-1678] ORF |
| 152 | CAAGGUCUAUAUCAAGACAGU | ACUGUCUUGAUAUAGACCUUG | MK | [1616-1636] ORF |
| 153 | CCAUCUGGAACUAUAUCCACU | AGUGGAUAUAGUUCCAGAUGG | MK | [1528-1548] ORF |
| 154 | GAAGACCCUACUUUCGGAUAU | AUAUCCGAAAGUAGGGUCUUC | MK | [1302-1322] ORF |
| 155 | GGAAGACCCUACUUUCGGAUA | UAUCCGAAAGUAGGGUCUUCC | MK | [1301-1321] ORF |
| 156 | CCAGACAUGCUGUGCUUUGUG | CACAAAGCACAGCAUGUCUGG | MK | [1281-1301] ORF |
| 157 | CCCAGACAUGCUGUGCUUUGU | ACAAAGCACAGCAUGUCUGGG | MK | [1280-1300] ORF |
| 158 | AGGGAGUGGUAUCCAGUGUUC | GAACACUGGAUACCACUCCCU | MK | [3390-3410] 3'UTR |
| 159 | UAGGGAGUGGUAUCCAGUGUU | AACACUGGAUACCACUCCCUA | MK | [3389-3409] 3'UTR |
| 160 | CUGGCCAGUGUGCCACAUUAA | UUAAUGUGGCACACUGGCCAG | MK | [3220-3240] 3'UTR |
| 161 | CAAGGAACACAUCCAGGCCUU | AAGGCCUGGAUGUGUUCCUUG | MK | [875-895] ORF |
| 162 | CAGGCACUUUCUGUAGCAAAU | AUUUGCUACAGAAAGUGCCUG | MK | [3077-3097] 3'UTR |
| 163 | AGAAGCAGCCAAGGACCGAUU | AAUCGGUCCUUGGCUGCUUCU | MK | [3055-3075] 3'UTR |
| 164 | CCAGGUGACCAUGGCUACAUU | AAUGUAGCCAUGGUCACCUGG | MK | [2797-2817] 3'UTR |
| 165 | GCCUUUCUAGAUGGCUUAGGU | ACCUAAGCCAUCUAGAAAGGC | | [2706-2726] 3'UTR |
| 166 | CUCCCAAAAAGUUGAGCCUUU | AAAGGCUCAACUUUUUGGGAG | | [2691-2711] 3'UTR |
| 167 | UGGUCCUUUAUGCUUGAGGUU | AACCUCAAGCAUAAAGGACCA | MK | [2546-2566] 3'UTR |
| 168 | CUGGUCACAUUGCCUUCUGAA | UUCAGAAGGCAAUGUGACCAG | MK | [2489-2509] 3'UTR |
| 169 | CCCUGUCAUUUCCAGAUUUCA | UGAAAUCUGGAAAUGACAGGG | MK | [2222-2242] 3'UTR |
| 170 | UCCCUUUUCCUCACUGGAAUG | CAUUCCAGUGAGGAAAAGGGA | MK | [1941-1961] 3'UTR |
| 171 | AGAGAAGGUCCACGUGAACUU | AAGUUCACGUGGACCUUCUCU | | [1703-1723] ORF |
| 172 | CGAAGAAUGUACAACCUCUUC | GAAGAGGUUGUACAUUCUUCG | MK | [1662-1682] ORF |
| 173 | CGGAACCUCAAGGUCUAUAUC | GAUAUAGACCUUGAGGUUCCG | MK | [1608-1628] ORF |
| 174 | GGCCAUCUGGAACUAUAUCCA | UGGAUAUAGUUCCAGAUGGCC | MK | [1526-1546] ORF |
| 175 | CAGGGCCAUCUGGAACUAUAU | AUAUAGUUCCAGAUGGCCCUG | MK | [1523-1543] ORF |
| 176 | UCACCUACAAUACCAUCGCCA | UGGCGAUGGUAUUGUAGGUGA | MK | [1471-1491] ORF |
| 177 | GGAUUAUACCUGGGAAGACCA | UGGUCUUCCCAGGUAUAAUCC | MK,Rat,MO | [1373-1393] ORF |
| 178 | UUCGCUGAGGUUUUGUUUCAU | AUGAAACAAAACCUCAGCGAA | MK | [3464-3484] 3'UTR |
| 179 | GGCUUUGCUACCAGUUCCAUA | UAUGGAACUGGUAGCAAAGCC | MK | [3427-3447] 3'UTR |
| 180 | GGAGUGGUAUCCAGUGUUCAA | UUGAACACUGGAUACCACUCC | MK | [3392-3412] 3'UTR |
| 181 | GAGGGCACUGAAGAAAGGCAA | UUGCCUUUCUUCAGUGCCCUC | MK | [3163-3183] 3'UTR |
| 182 | UCCAGGCACUUUCUGUAGCAA | UUGCUACAGAAAGUGCCUGGA | MK | [3075-3095] 3'UTR |
| 183 | CGAUUCCAGGCACUUUCUGUA | UACAGAAAGUGCCUGGAAUCG | MK | [3071-3091] 3'UTR |
| 184 | ACAUUGCCAAACCUCUGACUG | CAGUCAGAGGUUUGGCAAUGU | MK | [2813-2833] 3'UTR |
| 185 | CAAAAAGUUGAGCCUUUCUAG | CUAGAAAGGCUCAACUUUUUG | MK | [2695-2715] 3'UTR |
| 186 | CCAUGGCUCAUCACCAAGGAA | UUCCUUGGUGAUGAGCCAUGG | MK | [861-881] ORF |
| 187 | CCUAGUUCAGUUUUUUGACUU | AAGUCAAAAAACUGAACUAGG | MK | [2357-2377] 3'UTR |
| 188 | CCCGAAUCCUAGUUCAGUUUU | AAAACUGAACUAGGAUUCGGG | MK | [2350-2370] 3'UTR |
| 189 | CAUUCACCCAUCAAUGUGAAA | UUUCACAUUGAUGGGUGAAUG | MK | [2258-2278] 3'UTR |
| 190 | GACAGUUCAUUGCACUGACUC | GAGUCAGUGCAAUGAACUGUC | MK | [1962-1982] 3'UTR |
| 191 | CAAGGACUUCUCUGUCUGGAG | CUCCAGACAGAGAAGUCCUUG | MK | [1833-1853] 3'UTR |
| 192 | AGAGAAGACCACCCGAAGAAU | AUUCUUCGGGUGGUCUUCUCU | MK | [1649-1669] ORF |
| 193 | UCAAGGUCUAUAUCAAGACAG | CUGUCUUGAUAUAGACCUUGA | MK | [1615-1635] ORF |
| 194 | GGCAUCAGAUAUGAUGACUAU | AUAGUCAUCAUAUCUGAUGCC | MK | [1557-1577] ORF |
| 195 | GCAGGGCCAUCUGGAACUAUA | UAUAGUUCCAGAUGGCCCUGC | MK | [1522-1542] ORF |
| 196 | GCCUAUAGCCUCACCUACAAU | AUUGUAGGUGAGGCUAUAGGC | MK | [1461-1481] ORF |
| 197 | GCCCAGGAUUAUACCUGGGAA | UUCCCAGGUAUAAUCCUGGGC | MK,MO | [1368-1388] ORF |
| 198 | UCUUUGGCUUUGCUACCAGUU | AACUGGUAGCAAAGCCAAAGA | MK | [3422-3442] 3'UTR |
| 199 | GGCACUUUCUGUAGCAAAUGA | UCAUUUGCUACAGAAAGUGCC | MK | [3079-3099] 3'UTR |
| 200 | GCACCCUGACUACUUUACCAG | CUGGUAAAGUAGUCAGGGUGC | MK | [608-628] ORF |
| 201 | CUGCCAACAGCAAGCGGAUUU | AAAUCCGCUUGCUGUUGGCAG | MK | [2909-2929] 3'UTR |
| 202 | UCCCAAAAAGUUGAGCCUUUC | GAAAGGCUCAACUUUUUGGGA | | [2692-2712] 3'UTR |
| 203 | GCUGGUCACAUUGCCUUCUGA | UCAGAAGGCAAUGUGACCAGC | MK | [2488-2508] 3'UTR |
| 204 | CUCUAGAGAGUUUGAGCCUUU | AAAGGCUCAAACUCUCUAGAG | MK | [2467-2487] 3'UTR |
| 205 | AGUUCAGUUUUUUGACUUCCU | AGGAAGUCAAAAAACUGAACU | MK | [2360-2380] 3'UTR |
| 206 | GCUGUUGCCCGAAUCCUAGUU | AACUAGGAUUCGGGCAACAGC | MK | [2343-2363] 3'UTR |
| 207 | CCAGUUCCCUAAAAGCCUGUU | AACAGGCUUUUAGGGAACUGG | MK | [2305-2325] 3'UTR |
| 208 | AGCUGGUCUGUGUGUCCAGUU | AACUGGACACACAGACCAGCU | MK | [2290-2310] 3'UTR |
| 209 | GCCAUUCACCCAUCAAUGUGA | UCACAUUGAUGGGUGAAUGGC | MK | [2256-2276] 3'UTR |
| 210 | CUGUCAUUUCCAGAUUUCAUU | AAUGAAAUCUGGAAAUGACAG | MK | [2224-2244] 3'UTR |
| 211 | CCUGUCAUUUCCAGAUUUCAU | AUGAAAUCUGGAAAUGACAGG | MK | [2223-2243] 3'UTR |
| 212 | UGGACAGUUCAUUGCACUGAC | GUCAGUGCAAUGAACUGUCCA | MK | [1960-1980] 3'UTR |
| 213 | CCUUUUCCUCACUGGAAUGGA | UCCAUUCCAGUGAGGAAAAGG | MK | [1943-1963] 3'UTR |
| 214 | CCAUCAGUGUUCUUACCUGGU | ACCAGGUAAGAACACUGAUGG | MK | [719-739] ORF |
| 215 | AGACCACCCGAAGAAUGUACA | UGUACAUUCUUCGGGUGGUCU | MK | [1654-1674] ORF |
| 216 | GGAACCUCAAGGUCUAUAUCA | UGAUAUAGACCUUGAGGUUCC | MK | [1609-1629] ORF |
| 217 | UGCGUCUUUGGCAUCAGAUAU | AUAUCUGAUGCCAAAGACGCA | MK | [1548-1568] ORF |
| 218 | CGCCACUACAUUGCCAUCAUG | CAUGAUGGCAAUGUAGUGGCG | MK,MO | [687-707] ORF |
| 219 | CUACAAUACCAUCGCCAUGCA | UGCAUGGCGAUGGUAUUGUAG | MK | [1475-1495] ORF |
| 220 | CCAGGAUUAUACCUGGGAAGA | UCUUCCCAGGUAUAAUCCUGG | MK,MO | [1370-1390] ORF |
| 221 | ACACCCAGACAUGCUGUGCUU | AAGCACAGCAUGUCUGGGUGU | MK | [1277-1297] ORF |
| 222 | UGGCUUUGCUACCAGUUCCAU | AUGGAACUGGUAGCAAAGCCA | MK | [3426-3446] 3'UTR |
| 223 | AGUGCAGAAAUCUUUGGCUUU | AAAGCCAAAGAUUUCUGCACU | MK | [3412-3432] 3'UTR |
| 224 | GGGAGUGGUAUCCAGUGUUCA | UGAACACUGGAUACCACUCCC | MK | [3391-3411] 3'UTR |
| 225 | ACCAGUUUGGAGGGCACUGAA | UUCAGUGCCCUCCAAACUGGU | MK | [3154-3174] 3'UTR |
| 226 | GACUGUGAAUUACGACUUCUC | GAGAAGUCGUAAUUCACAGUC | | [3098-3118] 3'UTR |
| 227 | AGGCACUUUCUGUAGCAAAUG | CAUUUGCUACAGAAAGUGCCU | MK | [307-3098] 3'UTR |
| 228 | AGAUUAUCUCUAGAGAGUUUG | CAAACUCUCUAGAGAUAAUCU | MK | [2460-2480] 3'UTR |
| 229 | CAGAUUAUCUCUAGAGAGUUU | AAACUCUCUAGAGAUAAUCUG | MK | [2459-2479] 3'UTR |
| 230 | CCGAAUCCUAGUUCAGUUUUU | AAAAACUGAACUAGGAUUCGG | MK | [2351-2371] 3'UTR |
| 231 | GUGUGUCCAGUUCCCUAAAAG | CUUUUAGGGAACUGGACACAC | MK | [2299-2319] 3'UTR |
| 232 | UCACCCAUCAAUGUGAAAGUC | GACUUUCACAUUGAUGGGUGA | MK | [2261-2281] 3'UTR |
| 233 | GUACAACCUCUUCUGGAGGCA | UGCCUCCAGAAGAGGUUGUAC | MK | [1670-1690] ORF |
| 234 | CCUCAAGGUCUAUAUCAAGAC | GUCUUGAUAUAGACCUUGAGG | MK | [1613-1633] ORF |
| 235 | GAGCGGAACCUCAAGGUCUAU | AUAGACCUUGAGGUUCCGCUC | MK | [1605-1625] ORF |
| 236 | CGUCUUUGGCAUCAGAUAUGA | UCAUAUCUGAUGCCAAAGACG | MK | [1550-1570] ORF |
| 237 | GCGUCUUUGGCAUCAGAUAUG | CAUAUCUGAUGCCAAAGACGC | MK | [1549-1569] ORF |
| 238 | ACUGCGUCUUUGGCAUCAGAU | AUCUGAUGCCAAAGACGCAGU | MK,MO | [1546-1566] ORF |
| 239 | GCCAUCUGGAACUAUAUCCAC | GUGGAUAUAGUUCCAGAUGGC | MK | [1527-1547] ORF |
| 240 | UGGAAGACCCUACUUUCGGAU | AUCCGAAAGUAGGGUCUUCCA | MK | [1300-1320] ORF |
| 241 | CACCCAGACAUGCUGUGCUUU | AAAGCACAGCAUGUCUGGGUG | MK | [1278-1298] ORF |
| 242 | UGAGCUGGAGAAGUCAGAGAG | CUCUCUGACUUCUCCAGCUCA | | [1214-1234] ORF |
| 243 | UGCUACCAGUUCCAUAUGAUG | CAUCAUAUGGAACUGGUAGGA | MK | [3432-3452] 3'UTR |
| 244 | UGGCCAGUGUGCCACAUUAAA | UUUAAUGUGGCACACUGGCCA | MK | [3221-4241] 3'UTR |
| 245 | CGACUUCUCUUGCCCUUCUUC | GAAGAAGGGCAAGAGAAGUCG | | [3110-3130] 3'UTR |
| 246 | CACUUUCUGUAGCAAAUGACU | AGUCAUUUGCUACAGAAAGUG | MK | [3081-3101] 3'UTR |
| 247 | GCAAGCGGAUUUUCUUGCAAG | CUUGCAAGAAAAUCCGCUUGC | MK | [2918-2938] 3'UTR |
| 248 | UGCCAACAGCAAGCGGAUUUU | AAAAUCCGCUUGCUGUUGGCA | MK | [2910-2930] 3'UTR |
| 249 | CAGGUGACCAUGGCUACAUUG | CAAUGUAGCCAUGGUCACCUG | MK | [2798-2818] 3'UTR |
| 250 | UGCUGGUCACAUUGCCUUCUG | CAGAAGGCAAUGUGACCAGCA | MK | [2487-2507] 3'UTR |
| 251 | UGCCCGAAUCCUAGUUCAGUU | AACUGAACUAGGAUUCGGGCA | MK | [2348-2368] 3'UTR |
| 252 | CCCAUCAAUGUGAAAGUCAGG | CCUGACUUUCACAUUGAUGGG | MK | [2264-2284] 3'UTR |
| 253 | ACAAGUGUUGUGGCCUUCCUG | CAGGAAGGCCACAACACUUGU | MK | [2118-2138] 3'UTR |
| 254 | GAAUGGACAGUUCAUUGCACU | AGUGCAAUGAACUGUCCAUUC | MK | [1957-1977] 3'UTR |
| 255 | CCCGAAGAAUGUACAACCUCU | AGAGGUUGUACAUUCUUCGGG | MK | [1660-1680] ORF |
| 256 | ACCCGAAGAAUGUACAACCUC | GAGGUUGUACAUUCUUCGGGU | MK | [1659-1679] ORF |
| 257 | CCACCCGAAGAAUGUACAACC | GGUUGUACAUUCUUCGGGUGG | MK | [1657-1677] ORF |
| 258 | GAAGACCACCCGMGAAUGUA | UACAUUCUUCGGGUGGUCUUC | MK | [1652-1672] ORF |
| 259 | GCAUCAGAUAUGAUGACUAUG | CAUAGUCAUCAUAUCUGAUGC | MK | [1558-1578] ORF |
| 260 | CACUGCGUCUUUGGCAUCAGA | UCUGAUGCCAAAGACGCAGUG | MK,MO | [1545-1565] ORF |
| 261 | CUGGAACUAUAUCCACUGCGU | ACGCAGUGGAUAUAGUUCCAG | MK | [1532-1552] ORF |
| 262 | AGACCAUGGCUACUCGCUGAU | AUCAGCGAGUAGCCAUGGUCU | MK,Rat,MO | [1388-1408] ORF |
| 263 | CCCAGGAUUAUACCUGGGAAG | CUUCCCAGGUAUAAUCCUGGG | MK,MO | [1369-1389] ORF |
| 264 | GGAUAUGAGGACUUCACUCGG | CCGAGUGAAGUCCUCAUAUCC | MK | [1317-1337] ORF |
| 265 | CUACUUUCGGAUAUGAGGACU | AGUCCUCAUAUCCGAAAGUAG | MK | [1309-1329] ORF |
| 266 | CGCUUUGAGCUGGAGAAGUCA | UGACUUCUCCAGCUCAAAGCG | | [1209-1229] ORF |
| 267 | UCCUCCUUCGUGUUUGGCUGU | ACAGCCAAACACGAAGGAGGA | MK | [975-995] ORF |
| 268 | GCCCUUCUUCUAGCAGUCUGU | ACAGACUGCUAGAAGAAGGGC | | [3121-3141] 3'UTR |
| 269 | CAAGCGGAUUUUCUUGCAAGA | UCUUGCAAGAAAAUCCGCUUG | MK | [2919-2939] 3'UTR |
| 270 | AGCAAGCGGAUUUUCUUGCAA | UUGCAAGAAAAUCCGCUUGCU | MK | [2917-2937] 3'UTR |
| 271 | GCCAACAGCAAGCGGAUUUUC | GAAAAUCCGCUUGCUGUUGGC | MK | [2911-2931] 3'UTR |
| 272 | CUACAUUGCCAAACCUCUGAC | GUCAGAGGUUUGGCAAUGUAG | MK | [2811-2831] 3'UTR |
| 273 | GGCUACAUUGCCAAACCUCUG | CAGAGGUUUGGCAAUGUAGCC | MK | [2809-2829] 3'UTR |
| 274 | UGACCAUGGCUACAUUGCCAA | UUGGCAAUGUAGCCAUGGUCA | MK | [2802-2822] 3'UTR |
| 275 | CGAGAUCAACAAGUUGCUGGC | GCCAGCAACUUGUUGAUCUCG | MK | [833-853] ORF |
| 276 | GCGCAAACUCAGCGAGAUCAA | UUGAUCUCGCUGAGUUUGCGC | MK | [821-841] ORF |
| 277 | CUCCCUUUUCCUCACUGGAAU | AUUCCAGUGAGGAAAAGGGAG | MK | [1940-1960] 3'UTR |
| 278 | GAACCUCAAGGUCUAUAUCAA | UUGAUAUAGACCUUGAGGUUC | MK | [1610-1630] ORF |
| 279 | AGACAACCUGGCAGUGGUGAU | AUCACCACUGCCAGGUUGUCU | MK | [581-601] ORF |
| 280 | GGAACUAUAUCCACUGCGUCU | AGACGCAGUGGAUAUAGUUCC | MK | [1534-1554] ORF |
| 281 | UACUUUCGGAUAUGAGGACUU | AAGUCCUCAUAUCCGAAAGUA | MK | [1310-1330] ORF |
| 282 | GACCCUACUUUCGGAUAUGAG | CUCAUAUCCGAAAGUAGGGUC | MK | [1305-1325] ORF |
| 283 | UGCCUCCUCUCUGACCAGUUU | AAACUGGUCAGAGAGGAGGCA | MK | [3141-3161] 3'UTR |
| 284 | CUGUGAAUUACGACUUCUCUU | AAGAGAAGUCGUAAUUCACAG | | [3100-3120] 3'UTR |
| 285 | UGACUGUGAAUUACGACUUCU | AGAAGUCGUAAUUCACAGUCA | | [3097-3117] 3'UTR |
| 286 | AGCAAAUGACUGUGAAUUACG | CGUAAUUCACAGUCAUUUGCU | MK | [3091-3111] 3'UTR |
| 287 | CCAUCCCAGUAUCUCAUCUGU | ACAGAUGAGAUACUGGGAUGG | MK | [2998-3018] 3'UTR |
| 288 | CCCAUCCCAGUAUCUCAUCUG | CAGAUGAGAUACUGGGAUGGG | MK | [2997-3017] 3'UTR |
| 289 | GGCUCAUCACCAAGGAACACA | UGUGUUCCUUGGUGAUGAGCC | MK | [865-885] ORF |
| 290 | UCCUUUAUGCUUGAGGUUCCA | UGGAACCUCAAGCAUAAAGGA | MK | [2549-2569] 3'UTR |
| 291 | GAGCCUUUGCUGGUCACAUUG | CAAUGUGACCAGCAAAGGCUC | MK | [2480-2500] 3'UTR |
| 292 | UGAGCCUUUGCUGGUCACAUU | AAUGUGACCAGCAAAGGCUCA | MK | [2479-2499] 3'UTR |
| 293 | AGAGAGUUUGAGCCUUUGCUG | CAGCAAAGGCUCAAACUCUCU | MK | [2471-2491] 3'UTR |
| 294 | ACUCAGCGAGAUCAACAAGUU | AACUUGUUGAUCUCGCUGAGU | MK | [827-847] ORF |
| 295 | CUGUGUGUCCAGUUCCCUAAA | UUUAGGGAACUGGACACACAG | MK | [2297-2317] 3'UTR |
| 296 | GGAAUGGACAGUUCAUUGCAC | GUGCAAUGAACUGUCCAUUCC | MK | [1956-1976] 3'UTR |
| 297 | UGGAAUGGACAGUUCAUUGCA | UGCAAUGAACUGUCCAUUCCA | MK | [1955-1975] 3'UTR |
| 298 | UCAGAGAAGGUCCACGUGAAC | GUUCACGUGGACCUUCUCUGA | | [1701-1721] ORF |
| 299 | CUAUAUCAAGACAGUGGCCUG | CAGGCCACUGUCUUGAUAUAG | MK | [1622-1642] ORF |
| 300 | CUCAAGGUCUAUAUCAAGACA | UGUCUUGAUAUAGACCUUGAG | MK | [1614-1634] ORF |
| 301 | GUCUUUGGCAUCAGAUAUGAU | AUCAUAUCUGAUGCCAAAGAC | MK | [1551-1571] ORF |
| 302 | UGGAACUAUAUCCACUGCGUC | GACGCAGUGGAUAUAGUUCCA | MK | [1533-1553] ORF |
| 303 | UGCUUUGUGGAAGACCCUACU | AGUAGGGUCUUCCACAAAGCA | MK | [1293-1313] ORF |
| 304 | AGGGACCCGUUGAACAACUCU | AGAGUUGUUCAACGGGUCCCU | MK | [1080-1100] ORF |
| 305 | ACUGUAAAUGUUCACUGGGUG | CACCCAGUGAACAUUUACAGU | | [3366-3386] 3'UTR |
| 306 | GUGAUGACUGUAAAUGUUCAC | GUGAACAUUUACAGUCAUCAC | | [3360-3380] 3'UTR |
| 307 | UGCAAGAAGGUGUGCAGGAGA | UCUCCUGCACACCUUCUUGCA | | [3299-3319] 3'UTR |
| 308 | CUCCUCUCUGACCAGUUUGGA | UCCAAACUGGUCAGAGAGGAG | MK | [3144-3164] 3'UTR |
| 309 | GACCGAUUCCAGGCACUUUCU | AGAAAGUGCCUGGAAUCGGUC | MK | [3068-3088] 3'UTR |
| 310 | GGGAGAAUUCUGUUCUCCCAG | CUGGGAGAACAGAAUUCUCCC | | [2604-2624] 3'UTR |
| 311 | CCCACAAGGACUUCUCUGUCU | AGACAGAGAAGUCCUUGUGGG | MK | [1829-1849] 3'UTR |
| 312 | UGGCAUCAGAUAUGAUGACUA | UAGUCAUCAUAUCUGAUGCCA | MK | [1556-1576] ORF |
| 313 | CUAUAUCCACUGCGUCUUUGG | CCAAAGACGCAGUGGAUAUAG | MK | [1538-1558] ORF |
| 314 | GCCACUACAUUGCCAUCAUGG | CCAUGAUGGCAAUGUAGUGGC | MK,MO | [688-708] ORF |
| 315 | AGCCUAUAGCCUCACCUACAA | UUGUAGGUGAGGCUAUAGGCU | MK | [1460-1480] ORF |
| 316 | AGAAGUUCCAGGCAGCCUAUA | UAUAGGCUGCCUGGAACUUCU | MK | [1447-1467] ORF |
| 317 | GAGAAGUUCCAGGCAGCCUAU | AUAGGCUGCCUGGAACUUCUC | MK | [1446-1466] ORF |
| 318 | CCCUACUUUCGGAUAUGAGGA | UCCUCAUAUCCGAAAGUAGGG | MK | [1307-1327] ORF |
| 319 | CCUGACUACUUUACCAGCUUC | GAAGCUGGUAAAGUAGUCAGG | MK | [612-632] ORF |
| 320 | CAGCAAGCGGAUUUUCUUGCA | UGCAAGAAAAUCCGCUUGCUG | MK | [2916-2936] 3'UTR |
| 321 | CCAUGGCUACAUUGCCAAACC | GGUUUGGCAAUGUAGCCAUGG | MK | [2805-2825] 3'UTR |
| 322 | GAGCCUUUCUAGAUGGCUUAG | CUAAGCCAUCUAGAAAGGCUC | | [2704-2724] 3'UTR |
| 323 | GAGAAUUCUGUUCUCCCAGAG | CUCUGGGAGAACAGAAUUCUC | | [2606-2626] 3'UTR |
| 324 | GGAGAAUUCUGUUCUCCCAGA | UCUGGGAGAACAGAAUUCUCC | | [2605-2625] 3'UTR |
| 325 | CCUUUGCUGGUCACAUUGCCU | AGGCAAUGUGACCAGCAAAGG | MK | [2483-2503] 3'UTR |
| 326 | CUAGAGAGUUUGAGCCUUUGC | GCAAAGGCUCAAACUCUCUAG | MK | [2469-2489] 3'UTR |
| 327 | UCACUGGAAUGGACAGUUCAU | AUGAACUGUCCAUUCCAGUGA | MK | [1951-1971] 3'UTR |
| 328 | UGCAGUGGGCUUGUGUGUGAU | AUCACACACAAGCCCACUGCA | | [1898-1918] 3'UTR |
| 329 | GGACUUCUCUGUCUGGAGACA | UGUCUCCAGACAGAGAAGUCC | MK | [1836-1856] 3'UTR |
| 330 | AGGACUUCUCUGUCUGGAGAC | GUCUCCAGACAGAGAAGUCCU | MK | [1835-1855] 3'UTR |
| 331 | CUGCGUCUUUGGCAUCAGAUA | UAUCUGAUGCCAAAGACGCAG | MK,MO | [1547-1567] ORF |
| 332 | UGACUACUUUACCAGCUUCUG | CAGAAGCUGGUAAAGUAGUCA | MK | [614-634] ORF |
| 333 | CUGACUACUUUACCAGCUUCU | AGAAGCUGGUAAAGUAGUCAG | MK | [613-633] ORF |
| 334 | ACACAUCCAGGCCUUGCUGAA | UUCAGCAAGGCCUGGAUGUGU | MK | [881-901] ORF |
| 335 | CCGAUUCCAGGCACUUUCUGU | ACAGAAAGUGCCUGGAAUCGG | MK | [3070-3090] 3'UTR |
| 336 | CAGCGAGAUCAACAAGUUGCU | AGCAACUUGUUGAUCUCGCUG | MK | [830-850] ORF |
| 337 | CAGUUCCCUAAAAGCCUGUUC | GAACAGGCUUUUAGGGAACUG | MK | [2306-2326] 3'UTR |
| 338 | GAAGAGCACUUGGAGAUCCUA | UAGGAUCUCCAAGUGCUCUUC | | [2012-2032] 3'UTR |
| 339 | CUAUAGCCUCACCUACAAUAC | GUAUUGUAGGUGAGGCUAUAG | MK | [1463-1483] ORF |
| 340 | UGCAGAAAUCUUUGGCUUUGC | GCAAAGCCAAAGAUUUCUGCA | MK | [3414-3434] 3'UTR |
| 341 | CAAGUGCAGAAAUCUUUGGCU | AGCCAAAGAUUUCUGCACUUG | MK | [3410-3430] 3'UTR |
| 342 | UGAUGACUGUAAAUGUUCACU | AGUGAACAUUUACAGUCAUCA | | [3361-3381] 3'UTR |
| 343 | GACCAUGGCUACAUUGCCAAA | UUUGGCAAUGUAGCCAUGGUC | MK | [2803-2823] 3'UTR |
| 344 | AGGGUUCUUGUUUGGACCCUG | CAGGGUCCAAACAAGAACCCU | MK | [2762-2782] 3'UTR |
| 345 | GAGGCACCAGGGUUCUUGUUU | AAACAAGAACCCUGGUGCCUC | MK | [2754-2774] 3'UTR |
| 346 | UGCUUGAGGUUCCAACCUGGA | UCCAGGUUGGAACCUCAAGCA | MK | [2556-2576] 3'UTR |
| 347 | UGACUUCCUUUGCCCUUUUUC | GAAAAAGGGCAAAGGAAGUCA | | [2372-2392] 3'UTR |
| 348 | UCCAGUUCCCUAAAAGCCUGU | ACAGGCUUUUAGGGAACUGGA | MK | [2304-2324] 3'UTR |
| 349 | GCACUUGGAGAUCCUAAGGGA | UCCCUUAGGAUCUCCAAGUGC | | [2017-2037] 3'UTR |
| 350 | CUCACUGGAAUGGACAGUUCA | UGAACUGUCCAUUCCAGUGAG | MK | [1950-1970] 3'UTR |
| 351 | CCUCACUGGAAUGGACAGUUC | GAACUGUCCAUUCCAGUGAGG | MK | [1949-1969] 3'UTR |
| 352 | CAGACCCUUUUGUGUCCCAUG | CAUGGGACACAAAAGGGUCUG | | [1861-1881] 3'UTR |
| 353 | GACUUCUCUGUCUGGAGACAG | CUGUCUCCAGACAGAGAAGUC | MK | [1837-1857] 3'UTR |
| 354 | GCCAUCAGUGUUCUUACCUGG | CCAGGUAAGAACACUGAUGGC | MK | [718-738] ORF |
| 355 | CGCCAUCAGUGUUCUUACCUG | CAGGUAAGAACACUGAUGGCG | MK | [717-737] ORF |
| 356 | UAGCCUCACCUACAAUACCAU | AUGGUAUUGUAGGUGAGGCUA | MK | [1466-1486] ORF |
| 357 | CCUAUAGCCUCACCUACAAUA | UAUUGUAGGUGAGGCUAUAGG | MK | [1462-1482] ORF |
| 358 | GGCUUAAGUGGGUUGCUUCCA | UGGAAGCAACCCACUUAAGCC | MK | [3031-3051] 3'UTR |
| 359 | CACAUUGCCUUCUGAAGAGGA | UCCUCUUCAGAAGGCAAUGUG | MK | [2494-2514] 3'UTR |
| 360 | CCUUUUCUCCAUGCUUAAUGG | CCAUUAAGCAUGGAGAAAAGG | MK | [2393-2413] 3'UTR |
| 361 | UGCCACAAGUGUUGUGGCCUU | AAGGCCACAACACUUGUGGCA | MK | [2114-2134] 3'UTR |
| 362 | GGAAGAGCACUUGGAGAUCCU | AGGAUCUCCAAGUGCUCUUCC | | [2011-2031] 3'UTR |
| 363 | CAGAGAAGACCACCCGAAGAA | UUCUUCGGGUGGUCUUCUCUG | MK | [1648-1668] ORF |
| 364 | UGAGAAGUUCCAGGCAGCCUA | UAGGCUGCCUGGAACUUCUCA | MK | [1445-1465] ORF |
| 365 | CUGUGCUUUGUGGAAGACCCU | AGGGUCUUCCACAAAGCACAG | MK | [1290-1310] ORF |
| 366 | CCAUGCUUAAUGGUGUGAGGC | GCCUCACACCAUUAAGCAUGG | MK | [2401-2421] 3'UTR |
| 367 | CUGGAUGAGAAGUUCCAGGCA | UGCCUGGAACUUCUCAUCCAG | MK | [1440-1460] ORF |
| 368 | GAGAGGGAGAAUUCUGUUCUC | GAGAACAGAAUUCUCCCUCUC | | [2600-2620] 3'UTR |
| 369 | CGCAAACUCAGCGAGAUCAAC | GUUGAUCUCGCUGAGUUUGCG | MK | [822-842] ORF |
| 370 | CACUGGAAUGGACAGUUCAUU | AAUGAACUGUCCAUUCCAGUG | MK | [1952-1972] 3'UTR |
| 371 | UCCUCACUGGAAUGGACAGUU | AACUGUCCAUUCCAGUGAGGA | MK | [1948-1968] 3'UTR |
| 372 | CCUCCCUUUUCCUCACUGGAA | UUCCAGUGAGGAAAAGGGAGG | MK | [1939-1959] 3'UTR |
| 373 | CCUUCUGAAGAGGAGGGAGUA | UACUCCCUCCUCUUCAGAAGG | MK | [2501-2521] 3'UTR |

**Table P: Various preferred siRNA**

| **HTRA2 Sense oligomer 5'-3'** | **AntiSense oligomer 5'-3'** |
|---|---|
| GAAUCACAGAAACACUUUU | AAAAGUGUUUCUGUGAUUC |
| CCGUGGUCUAUAUCGAGAU | AUCUCGAUAUAGACCACGG |
| GACAUGGGUUUCUUGGUAA | UUACCAAGAAACCCAUGUC |
| CCGAGACAGAGGGUUAAAU | AUUUAACCCUCUGUCUCGG |
| GAUGGUACAAAAUGCUGAA | UUCAGCAUUUUGUACCAUC |
| CGUGGUCUAUAUCGAGAUC | GAUCUCGAUAUAGACCACG |
| GCCGUGGUCUAUAUCGAGA | UCUCGAUAUAGACCACGGC |
| GUGCUGCUCUUUGUGGUGU | ACACCACAAAGAGCAGCAC |
| AGGAUUCAGACUAAGUUUG | CAAACUUAGUCUGAAUCCU |
| GGUGAAAACUUCUGCUUGA | UCAAGCAGAAGUUUUCACC |
| CCUGCUCUGAUUUCCUCCU | AGGAGGAAAUCAGAGCAGG |
| UGAGUGCGGUUGCUGACAU | AUGUCAGCAACCGCACUCA |
| AGGGUGAAAACUUCUGCUU | AAGCAGAAGUUUUCACCCU |
| GUCAGGUGCUGCUCUUUGU | ACAAAGAGCAGCACCUGAC |
| GUGAUGUGAUUUUGGCCAU | AUGGCCAAAAUCACAUCAC |
| GCCUGGUGAUGUGAUUUUG | CAAAAUCACAUCACCAGGC |
| CUGCCGUGGUCUAUAUCGA | UCGAUAUAGACCACGGCAG |
| CAUCUUUUGUGGGCAGUUA | UAACUGCCCACAAAAGAUG |
| GGUGAUGUGAUUUUGGCCA | UGGCCAAAAUCACAUCACC |
| GCUGCUGCCAUCUUUUGUG | CACAAAAGAUGGCAGCAGC |
| ACCAUCCUGACCUCCUAUU | AAUAGGAGGUCAGGAUGGU |
| UGGUACAAAAUGCUGAAGA | UCUUCAGCAUUUUGUACCA |
| GAGGGUGAAAACUUCUGCU | AGCAGAAGUUUUCACCCUC |
| GCCUCAGAGAACUCUGGAA | UUCCAGAGUUCUCUGAGGC |
| AGGUCACAGAAUGAAUAGA | UCUAUUCAUUCUGUGACCU |
| UUGCUGACAUGGGUUUCUU | AAGAAACCCAUGUCAGCAA |
| CCAUAGUGCAUGGUCUGAU | AUCAGACCAUGCACUAUGG |
| CUGCUGACGUCAGGAACUU | AAGUUCCUGACGUCAGCAG |
| CUCUGAAGAAUCACAGAAA | UUUCUGUGAUUCUUCAGAG |
| CGCUGAGGAUUCAGACUAA | UUAGUCUGAAUCCUCAGCG |
| CCCGGAGUCAGUACAACUU | AAGUUGUACUGACUCCGGG |
| ACGCUGAGGAUUCAGACUA | UAGUCUGAAUCCUCAGCGU |
| GCUCUGAAGAAUCACAGAA | UUCUGUGAUUCUUCAGAGC |
| AGGGUUAAAUGAACCAGUG | CACUGGUUCAUUUAACCCU |
| GUGCACUUCUGAAGGACUU | AAGUCCUUCAGAAGUGCAC |
| CCUCAGAGAACUCUGGAAC | GUUCCAGAGUUCUCUGAGG |
| GAGGGUUAAAUGAACCAGU | ACUGGUUCAUUUAACCCUC |
| CCAAGAGUAUGAGGCUCCU | AGGAGCCUCAUACUCUUGG |
| CAGAAUGAAUAGAUCACCA | UGGUGAUCUAUUCAUUCUG |
| UCCCAUGCUUGGCUACAGA | UCUGUAGCCAAGCAUGGGA |
| GGGCUCUGAAGAAUCACAG | CUGUGAUUCUUCAGAGCCC |
| CUCCUGGGCUCUGAAGAAU | AUUCUUCAGAGCCCAGGAG |
| GACUAAGUUUGGAAACUCU | AGAGUUUCCAAACUUAGUC |
| | |

| **KEAP1 Sense oligomer** | **AntiSense oligomer** |
|---|---|
| GCACUGCAAAUAACCCAUC | GAUGGGUUAUUUGCAGUGC |
| ACUGCAAAUAACCCAUCUU | AAGAUGGGUUAUUUGCAGU |
| GGAUGCCUCAGUGUUAAAA | UUUUAACACUGAGGCAUCC |
| CACUGCAAAUAACCCAUCU | AGAUGGGUUAUUUGCAGUG |
| GGGCAAAAAUACAGUCCAA | UUGGACUGUAUUUUUGCCC |
| UGAUAAGUAACCCUGUAAU | AUUACAGGGUUACUUAUCA |
| GGAGUAUCAUUGUUUUUGU | ACAAAAACAAUGAUACUCC |
| GCCUCAUUGAAUUCGCCUA | UAGGCGAAUUCAAUGAGGC |
| GCAGCUGUCACCAUGUGAU | AUCACAUGGUGACAGCUGC |
| GGCAAAAAUACAGUCCAAU | AUUGGACUGUAUUUUUGCC |
| CACCAUGUGAUUUAUUCUU | AAGAAUAAAUCACAUGGUG |
| CGGGACUAAAAGAAAAGAC | GUCUUUUCUUUUAGUCCCG |
| GGGACUAAAAGAAAAGACA | UGUCUUUUCUUUUAGUCCC |
| CCGGGACUAAAAGAAAAGA | UCUUUUCUUUUAGUCCCGG |
| GGGAGUAUCAUUGUUUUUG | CAAAAACAAUGAUACUCCC |
| CUCAUUGAAUUCGCCUACA | UGUAGGCGAAUUCAAUGAG |
| CAUCUCAAAAGAAGUCCAA | UUGGACUUCUUUUGAGAUG |
| GUGUUAAAAUGACAUCUCA | UGAGAUGUCAUUUUAACAC |
| CUCAGUGUUAAAAUGACAU | AUGUCAUUUUAACACUGAG |
| CCUCAGUGUUAAAAUGACA | UGUCAUUUUAACACUGAGG |
| CCUUAAUUCAGCUGAGUGU | ACACUCAGCUGAAUUAAGG |
| CCCGGGAGUACAUCUACAU | AUGUAGAUGUACUCCCGGG |
| CCUCAUUGAAUUCGCCUAC | GUAGGCGAAUUCAAUGAGG |
| ACCGGGACUAAAAGAAAAG | CUUUUCUUUUAGUCCCGGU |
| CCUCAAUCGUCUCCUUUAU | AUAAAGGAGACGAUUGAGG |
| CUGUCUUCAAGGCCAUGUU | AACAUGGCCUUGAAGACAG |
| ACGUCACACUGCAGGUCAA | UUGACCUGCAGUGUGACGU |
| AACCGGGACUAAAAGAAAA | UUUUCUUUUAGUCCCGGUU |
| UGAGGCACUUUUGUUUCUU | AAGAAACAAAAGUGCCUCA |
| AGAGGAACGAGUGGCGAAU | AUUCGCCACUCGUUCCUCU |
| CAGUGUUAAAAUGACAUCU | AGAUGUCAUUUUAACACUG |
| AGCGCUACGAUGUGGAAAC | GUUUCCACAUCGUAGCGCU |
| ACAGUGUGGAGAGGUAUGA | UCAUACCUCUCCACACUGU |
| GGUGUCCAUUGAGGGUAUC | GAUACCCUCAAUGGACACC |
| GAGGCACUUUUGUUUCUUG | CAAGAAACAAAAGUGCCUC |
| CCUUUGGCAUCAUGAACGA | UCGUUCAUGAUGCCAAAGG |
| UCCUGCACAACUGUAUCUA | UAGAUACAGUUGUGCAGGA |
| AGGUGGUGGUGUUGCUUAU | AUAAGCAACACCACCACCU |
| GCUGUCACCAUGUGAUUUA | UAAAUCACAUGGUGACAGC |
| GCCUCAGUGUUAAAAUGAC | GUCAUUUUAACACUGAGGC |
| UGCAUCAACUGGGUCAAGU | ACUUGACCCAGUUGAUGCA |
| UGGUGGUGUUGCUUAUCUU | AAGAUAAGCAACACCACCA |
| UGGACAGUUAUUUUGUUGA | UCAACAAAAUAACUGUCCA |
| UUUGUUUCUUGGGCAAAAA | UUUUUGCCCAAGAAACAAA |
| GCAAUGAACACCAUCCGAA | UUCGGAUGGUGUUCAUUGC |
| CCAAGGAAAAUAAAGAACA | UGUUCUUUAUUUUCCUUGG |
| GUCCUGCACAACUGUAUCU | AGAUACAGUUGUGCAGGAC |
| | |

| **LGALS3 Sense siRNA** | **AntiSense siRNA** |
|---|---|
| UGCCUUAUAACCUGCCUUU | AAAGGCAGGUUAUAAGGCA |
| GUGCCUUAUAACCUGCCUU | AAGGCAGGUUAUAAGGCAC |
| GGGAAUUUCUGGUGACAUA | UAUGUCACCAGAAAUUCCC |
| CCAUGAUAUAAUCUGAAAG | CUUUCAGAUUAUAUCAUGG |
| GAGAGUCAUUGUUUGCAAU | AUUGCAAACAAUGACUCUC |
| GAAAAUGGCAGACAAUUUU | AAAAUUGUCUGCCAUUUUC |
| AGCGGAAAAUGGCAGACAA | UUGUCUGCCAUUUUCCGCU |
| GGGUUAAAAAACUCAAUGA | UCAUUGAGUUUUUUAACCC |
| CGGGUUAAAAAACUCAAUG | CAUUGAGUUUUUUAACCCG |
| CAAGGUUGCAGUGAAUGAU | AUCAUUCACUGCAACCUUG |
| GACCUCACCAGUGCUUCAU | AUGAAGCACUGGUGAGGUC |
| CUUUAACCCACGCUUCAAU | AUUGAAGCGUGGGUUAAAG |
| AAGAAAGACAGUCGGUUUU | AAAACCGACUGUCUUUCUU |
| GAGCGGAAAAUGGCAGACA | UGUCUGCCAUUUUCCGCUC |
| UGCGUUAUCUGGGUCUGGA | UCCAGACCCAGAUAACGCA |
| CCCUCUUGUAAGUCAUCUA | UAGAUGACUUACAAGAGGG |
| CUGGGAAUUUCUGGUGACA | UGUCACCAGAAAUUCCCAG |
| AGUACAAUCAUCGGGUUAA | UUAACCCGAUGAUUGUACU |
| GUCAUUGUUUGCAAUACAA | UUGUAUUGCAAACAAUGAC |
| GGUUUCAUGUUCACUGUGA | UCACAGUGAACAUGAAACC |
| UGCAAUACAAAGCUGGAUA | UAUCCAGCUUUGUAUUGCA |
| CACGCUUCAAUGAGAACAA | UUGUUCUCAUUGAAGCGUG |
| GGGAAGGGAAGAAAGACAG | CUGUCUUUCUUCCCUUCCC |
| GCUUCAUAUACCAUGAUAU | AUAUCAUGGUAUAUGAAGC |
| CUUCAAGGUUGCAGUGAAU | AUUCACUGCAACCUUGAAG |
| CCACGCUUCAAUGAGAACA | UGUUCUCAUUGAAGCGUGG |
| UCCCUCUUGUAAGUCAUCU | AGAUGACUUACAAGAGGGA |
| ACAGUCGGUUUUCCCAUUU | AAAUGGGAAAACCGACUGU |
| ACGCUUCAAUGAGAACAAC | GUUGUUCUCAUUGAAGCGU |
| AGUCAUUGUUUGCAAUACA | UGUAUUGCAAACAAUGACU |
| ACCCACGCUUCAAUGAGAA | UUCUCAUUGAAGCGUGGGU |
| GAGGGAAUGAUGUUGCCUU | AAGGCAACAUCAUUCCCUC |
| CUCAAUGAAAUCAGCAAAC | GUUUGCUGAUUUCAUUGAG |
| CCCAUUUGAAAGUGGGAAA | UUUCCCACUUUCAAAUGGG |
| GCCACUGAUUGUGCCUUAU | AUAAGGCACAAUCAGUGGC |
| CCUCUUGUAAGUCAUCUAC | GUAGAUGACUUACAAGAGG |
| CUAAACCUUACAUGUGUAA | UUACACAUGUAAGGUUUAG |
| GUGCUUCAUAUACCAUGAU | AUCAUGGUAUAUGAAGCAC |
| CAGUCGGUUUUCCCAUUUG | CAAAUGGGAAAACCGACUG |
| GGAAACCCAAACCCUCAAG | CUUGAGGGUUUGGGUUUCC |
| CUCACUUGUUGCAGUACAA | UUGUACUGCAACAAGUGAG |
| AUGUUGCCUUCCACUUUAA | UUAAAGUGGAAGGCAACAU |
| CAGCCAACGAGCGGAAAAU | AUUUUCCGCUCGUUGGCUG |
| CGCUCCAUGAUGCGUUAUC | GAUAACGCAUCAUGGAGCG |
| UCGGGUUAAAAAACUCAAU | AUUGAGUUUUUUAACCCGA |
| CCAUUUGAAAGUGGGAAAC | GUUUCCCACUUUCAAAUGG |
| GGUUUUCCCAUUUGAAAGU | ACUUUCAAAUGGGAAAACC |
| GAGUCAUUGUUUGCAAUAC | GUAUUGCAAACAAUGACUC |
| | |

| **TP53 Sense oligomer** | **Antisense oligomer** |
|---|---|
| GAGAAUAUUUCACCCUUCA | UGAAGGGUGAAAUAUUCUC |
| CCGAGUGGAAGGAAAUUUG | CAAAUUUCCUUCCACUCGG |
| GACAGAAACACUUUUCGAC | GUCGAAAAGUGUUUCUGUC |
| | |

| **SHC1-SHC Sense oligomer** | **Antisense oligomer** |
|---|---|
| ACCUGAAAUUUGCUGGAAU | AUUCCAGCAAAUUUCAGGU |
| CAGAGAGCUUUUUGAUGAU | AUCAUCAAAAAGCUCUCUG |
| CACAUGCAAUCUAUCUCAU | AUGAGAUAGAUUGCAUGUG |
| | |

| **ZNHIT1 Sense oligomer** | **Antisense oligomer** |
|---|---|
| CCGAGGUGAUCAUUUUAAA | UUUAAAAUGAUCACCUCGG |
| GUGACCACAUCUUUAAAAU | AUUUUAAAGAUGUGGUCAC |

## Claims

1. A double-stranded siRNA compound which reduces or inhibits expression of the TP53 gene, for use in preventing or reducing Delayed Graft Function in a recipient of a kidney transplant, said siRNA compound having the structure:
5' (N)ₓ-Z 3' (antisense strand)
3' Z'-(N')_{y} 5' (sense strand)
wherein each of N and N' is a ribonucleotide which may be modified or unmodified in its sugar residue;
wherein each of (N)ₓ and (N')_{y} is an oligonucleotide in which each consecutive N or N' is joined to the next N or N' by a covalent bond;
wherein each of x and y is an integer between 18 and 40;
wherein in each of (N)ₓ and (N')_{y} the ribonucleotides alternate between modified ribonucleotides and unmodified ribonucleotides, each modified ribonucleotide being a 2'-O-methyl sugar modified ribonucleotide;
wherein each of Z and Z' may be present or absent, but if present is 1-5 deoxyribonucleotides covalently attached at the 3' terminus of the oligonucleotide in which it is present;
wherein the sequence of (N)ₓ comprises the sequence 5' UGAAGGGUGAAAUAUUCUC 3' (SEQ ID NO:2) and the sequence of (N')_{y} comprises the sequence 5' GAGAAUAUUUCACCCUUCA 3' (SEQ ID NO:1); and wherein a therapeutically effective dose of said siRNA compound is to be administered intravenously to the recipient of the kidney transplant between 15 minutes and 4 hours following revascularization of the transplanted kidney, thereby preventing or reducing Delayed Graft Function in the recipient.

2. The double-stranded siRNA compound for use as in claim 1, wherein the Delayed Graft Function is cold ischemia-associated Delayed Graft Function.

3. The double-stranded siRNA compound for use as in claim 1, wherein said kidney transplant is from a deceased donor.

4. The double-stranded siRNA compound for use as in claim 3, wherein the donor died from brain or cardiac death.

5. The double-stranded siRNA compound for use as in any one of claims 1-4, wherein x=y=19.

6. The double-stranded siRNA compound for use as in any one of claims 1-5, said siRNA compound having the structure:
5' UGAAGGGUGAAAUAUUCUC 3' (antisense strand)
3' ACUUCCCACUUUAUAAGAG 5' (sense strand)
wherein each of A, C, U and G is an unmodified ribonucleotide or a 2'-O-methyl) sugar modified ribonucleotide and each consecutive ribonucleotide is joined to the next ribonucleotide by a covalent bond;
wherein in the antisense strand the first, third, fifth, seventh, ninth, eleventh, thirteenth, fifteenth, seventeenth and nineteenth ribonucleotide is a 2'-O-methyl sugar modified ribonucleotide; and
wherein in the sense strand the second, fourth, sixth, eighth, tenth, twelfth, fourteenth, sixteenth and eighteenth ribonucleotide is a 2'-O-methyl sugar modified ribonucleotide.

7. The double-stranded siRNA compound for use as in any one of claim 1-6, wherein the antisense strand and the sense strand are non-phosphorylated at both the 3' and the 5' termini.

8. The double-stranded siRNA compound for use as in any one of claims 1-6, wherein either or both the antisense strand and the sense strand are phosphorylated at the 3' termini.

9. The double-stranded siRNA compound for use as in any one of claims 1-8, wherein both Z and Z' are absent.

10. The double-stranded siRNA compound for use as in any one of claims 1-9, wherein the dose of said siRNA compound is between 0.1-50 mg/kg body weight of the recipient.

11. The double-stranded siRNA compound for use as in any one of claims 1-9, wherein the dose of said siRNA compound is between 0.5-10 mg/kg body weight of the recipient.

12. The double-stranded siRNA compound for use as in any one of claims 1-9, wherein the dose of said siRNA compound is 10 mg/kg body weight of the recipient.

13. The double-stranded siRNA compound for use as in any one of claims 1-12, wherein the intravenous administration comprises a single, slow intravenous push.

14. The double stranded siRNA compound for use as in any one of claims 1-13, wherein said siRNA compound is to be administered as a pharmaceutically acceptable salt thereof.

## Patentansprüche

1. Doppelsträngige siRNA-Verbindung, die die Expression des TP53-Gens reduziert oder hemmt, zur Verwendung bei der Prävention oder Reduzierung der verzögerten Transplantatfunktion bei einem Empfänger eines Nierentransplantats, wobei die siRNA-Verbindung die folgende Struktur aufweist:
5' (N)ₓ-Z 3' (Antisense-Strang)
3' Z'-(N')_{y} 5' (Sense-Strang),
wobei jedes von N und N' ein Ribokuleotid ist, das an seinem Zuckerrest modifiziert oder nicht modifiziert sein kann;
wobei jedes von (N)ₓ und (N')_{y} ein Oligonukleotid ist, in dem jedes aufeinanderfolgende N oder N' mit dem nächsten N oder N' durch eine kovalente Bindung verbunden ist;
wobei jedes von x und y eine ganze Zahl zwischen 18 und 40 ist; wobei sich in jedem von (N)ₓ und (N')_{y} die Ribonukleotide zwischen modifizierten Ribonukleotiden und nicht modifizierten Ribonukleotiden abwechseln, wobei jedes modifizierte Ribonukleotid ein am Zucker 2'-O-Methyl-modifiziertes Ribonukleotid ist;
wobei jedes von Z und Z' vorhanden oder nicht vorhanden sein kann, aber, falls vorhanden, 1-5 Desoxyribonukleotide ist, die am 3'-Terminus des Oligomers, in dem es vorhanden ist, kovalent gebunden sind;
wobei die Sequenz von (N)ₓ die Sequenz 5'-UGAAGGGUGAAAUAUUCUC-3' (SEQ ID NO:2) umfasst, und die Sequenz von (N')_{y} die Sequenz 5'-GAGAAUAUUUCACCCUUCA-3' (SEQ ID NO:1) umfasst; und wobei eine therapeutisch wirksame Dosis der siRNA-Verbindung dem Empfänger des Nierentransplantats zwischen 15 Minuten und 4 Stunden nach der Revaskularisierung der transplantierten Niere intravenös verabreicht werden soll und dadurch die verzögerte Transplantatfunktion verhindert oder reduziert wird.

2. Doppelsträngige siRNA-Verbindung zur Verwendung nach Anspruch 1, wobei die verzögerte Transplantatfunktion eine mit kalter Ischämie verbundene verzögerte Transplantatfunktion ist.

3. Doppelsträngige siRNA-Verbindung zur Verwendung nach Anspruch 1, wobei das Nierentransplantat von einem verstorbenen Spender stammt.

4. Doppelsträngige siRNA-Verbindung zur Verwendung nach Anspruch 3, wobei der Spender einem Hirn- oder Herztod erlag.

5. Doppelsträngige siRNA-Verbindung zur Verwendung nach einem der Ansprüche 1-4, wobei x=y=19.

6. Doppelsträngige siRNA-Verbindung zur Verwendung nach einem der Ansprüche 1-5, wobei die siRNA-Verbindung die folgende Struktur aufweist:
5' UGAAGGGUGAAAUAUUCUC 3' (Antisense-Strang)
3' ACUUCCCACUUUAUAAGAG 5' (Sense-Strang),
wobei jedes von A, C, U und G ein nicht modifiziertes Ribonukleotid oder ein am Zucker 2'-O-Methyl-modifiziertes Ribonukleotid ist und jedes aufeinanderfolgende Ribonukleotid mit dem nächsten Ribonukleotid durch eine kovalente Bindung verbunden ist;
wobei im Antisense-Strang das erste, dritte, fünfte, siebte, neunte, elfte, dreizehnte, fünfzehnte, siebzehnte und neunzehnte Ribonukleotid ein am Zucker 2'-O-Methyl-modifiziertes Ribonukleotid ist; und
wobei im Sense-Strang das zweite, vierte, sechste, achte, zehnte, zwölfte, vierzehnte und achtzehnte Ribonukleotid ein am Zucker 2'-O-Methyl-modifiziertes Ribonukleotid ist.

7. Doppelsträngige siRNA-Verbindung zur Verwendung nach einem der Ansprüche 1-6, wobei der Antisense-Strang und der Sense-Strang sowohl an den 3'- als auch an den 5'-Termini nicht phosphoryliert sind.

8. Doppelsträngige siRNA-Verbindung zur Verwendung nach einem der Ansprüche 1-6, wobei einer oder beide vom dem Antisense-Strang und dem Sense-Strang an den 3'-Termini phosphoryliert sind.

9. Doppelsträngige siRNA-Verbindung zur Verwendung nach einem der Ansprüche 1-8, wobei sowohl Z als auch Z' abwesend sind.

10. Doppelsträngige siRNA-Verbindung zur Verwendung nach einem der Ansprüche 1-9, wobei die Dosis der siRNA-Verbindung zwischen 0,1-50 mg/kg Körpergewicht des Empfängers liegt.

11. Doppelsträngige siRNA-Verbindung zur Verwendung nach einem der Ansprüche 1-9, wobei die Dosis der siRNA-Verbindung zwischen 0,5-10 mg/kg Körpergewicht des Empfängers liegt.

12. Doppelsträngige siRNA-Verbindung zur Verwendung nach einem der Ansprüche 1-9, wobei die Dosis der siRNA-Verbindung 10 mg/kg Körpergewicht des Empfängers beträgt.

13. Doppelsträngige siRNA-Verbindung zur Verwendung nach einem der Ansprüche 1-12, wobei die die intravenöse Verabreichung einen einzigen langsamen intravenösen Schub umfasst.

14. Doppelsträngige siRNA-Verbindung zur Verwendung nach einem der Ansprüche 1-13, wobei die siRNA-Verbindung als pharmazeutisch annehmbares Salz davon verabreicht werden soll.

## Revendications

1. Composé d'ARNsi à bicaténaire réduisant ou inhibant l'expression du gène TP53, utilisé pour prévenir ou pour réduire le retard du fonctionnement du greffon chez un receveur d'une transplantation de rein, ledit composé ARNsi présentant la structure suivante :
5' (N)ₓ-2 3' (brin antisens)
3' Z' - (N')_{y} 5' (brin sens)
dans laquelle chacun de N et de N'est un ribonucléotide qu'on peut modifier ou non dans son résidu de sucre ;
dans laquelle chacun des (N)ₓ et (N')_{y} représente un oligonucléotide dans lequel chaque N ou N' consécutif est lié au N ou au N' suivant par une liaison covalente ;
dans laquelle chacun des x et y représente un nombre entier compris entre 18 et 40 ;
dans laquelle dans chacun des (N)ₓ et (N')_{y}, les ribonucléotides alternent entre des ribonucléotides modifiés et des ribonucléotides non modifiés, chaque ribonucléotide modifié étant un ribonucléotide modifié par un sucre de 2'-O-méthyle ;
dans laquelle chacun des Z et des Z' peut être présent ou absent, mais si présent, représente 1 à 5 désoxyribonucléotides liés par covalence à la terminaison 3' de l'oligonucléotide dans lequel il est présent ;
dans laquelle la séquence de (N)ₓ comprend la séquence 5' UGAAGGGUGAAAAUAUUCUC 3' (SEQ ID N° 2) et la séquence de (N')_{y} comprend la séquence 5' GAGAAUAUUUCACCCUUCA 3' (SEQ ID N°1) ; et dans lequel une dose thérapeutiquement efficace dudit composé d'ARNsi doit être administrée par voie intraveineuse au receveur de la transplantation de rein entre 15 minutes et 4 heures après revascularisation du rein transplanté, ce qui empêche ou qui réduit le retard du fonctionnement du greffon chez le receveur.

2. Composé d'ARNsi à bicaténaire à employer selon la revendication 1, dans lequel le retard du fonctionnement du greffon est un retard du fonctionnement du greffon associé à l'ischémie froide.

3. Composé d'ARNsi à bicaténaire à employer selon la revendication 1, dans lequel ladite transplantation de rein a lieu à partir d'un donneur décédé.

4. Composé d'ARNsi à bicaténaire à employer selon la revendication 3, dans lequel le donneur est mort d'une mort cérébrale ou cardiaque.

5. Composé d'ARNsi à bicaténaire à employer selon l'une quelconque des revendications 1 à 4, dans lequel x = y = 19.

6. Composé d'ARNsi à bicaténaire à employer selon l'une quelconque des revendications 1 à 5, dans lequel le composé d'ARNsi présentant la structure suivants :
5' UGAAGGGUGAAAUAUUCUC 3' (brin antisens)
3' ACUUCCCACUUUAUAAGAG 5' (brin sens)
dans lequel chacun des A, C, U et G représente un ribonucléotide non modifié ou un ribonucléotide modifié par un sucre de 2'-O-méthyle, et où chaque ribonucléotide consécutif est lié au ribonucléotide suivant par une liaison covalente ;
dans lequel dans le brin anti-sens, le premier, troisième, cinquième, septième, neuvième, onzième, treizième, quinzième, dix-septième et dix-neuvième ribonucléotide est un ribonucléotide modifié par un sucre de 2'-O-méthyle ; et
dans lequel dans le brin sens, le deuxième, quatrième, sixième, huitième, dixième, douzième, quatorzième, seizième et dix-huitième ribonucléotide est un ribonucléotide modifié par un sucre de 2'-O-méthyle.

7. Composé d'ARNsi à bicaténaire à employer selon l'une quelconque des revendications 1 à 6, dans lequel le brin anti-sens et le brin sens sont non phosphorylés aux deux terminaisons 3' et 5'.

8. Composé d'ARNsi à bicaténaire à employer selon l'une quelconque des revendications 1 à 6, dans lequel soit l'un ou l'autre des brins anti-sens et sens soit les deux sont phosphorylés aux terminaisons 3'.

9. Composé d'ARNsi à bicaténaire à employer selon l'une quelconque des revendications 1 à 8, dans lequel Z et Z' sont tous deux absents.

10. Composé d'ARNsi à bicaténaire à employer selon l'une quelconque des revendications 1 à 9, dans lequel la dose dudit composé d'ARNsi est comprise entre 0,1 et 50 mg/kg de poids corporel du receveur.

11. Composé d'ARNsi à bicaténaire à employer selon l'une quelconque des revendications 1 à 9, dans lequel la dose dudit composé d'ARNsi est comprise entre 0,5 et 10 mg/kg de poids corporel du receveur.

12. Composé d'ARNsi à bicaténaire à employer selon l'une quelconque des revendications 1 à 9, dans lequel la dose dudit composé d'ARNsi vaut 10 mg/kg de poids corporel du receveur.

13. Composé d'ARNsi à bicaténaire à employer selon l'une quelconque des revendications 1 à 12, dans lequel l'administration intraveineuse comprend une poussée unique et lente par voie intraveineuse.

14. Composé d'ARNsi à bicaténaire à employer selon l'une quelconque des revendications 1 à 13, dans lequel ledit composé d'ARNsi doit être administré sous forme de sel pharmaceutiquement acceptable de celui-ci.
